# EUROPEAN PATENT APPLICATION

(11) **EP 2 946 764 A1**
(43) Date of publication of application: **25.11.2015**
(21) Application number: 14182110.8
(22) Date of filing: 25.08.2014
(51) Int. Cl.: A61K 8/44, C12N 9/00, C12N 9/10, C12P 13/04

(54) **Biosynthetic production of acyl amino acids**

(30) Priority: 23.05.2014 EP 14169650
(71) Applicant: Evonik Degussa GmbH, 45128 Essen (DE)
(72) Inventor: Reinecke, Liv, 45134 Essen (DE); Schaffer, Steffen, Dr., 45699 Herten (DE); Grammann, Katrin, Dr., 45739 Oer-Erkenschwick (DE); Olfert, Maik, 45665 Recklinghausen (DE); Decker, Nicole, 45657 Recklinghausen (DE); Arto, Nils, 45772 Marl (DE); Hennemann, Hans-Georg, Dr., 50181 Bedburg (DE)

(57) **Abstract**

The present invention relates to a cell for producing acyl amino acids wherein the cell is genetically modified to comprise
- at least a first genetic mutation that increases the expression relative to the wild type cell of an amino acid-N-acyl-transferase,
- at least a second genetic mutation that increases the expression relative to the wild type cell of an acyl-CoA synthetase, and
- at least a third genetic mutation that decreases the expression relative to the wild type cell of at least one enzyme selected from the group consisting of an enzyme of the glycine cleavage system, glycine hydroxymethyltransferase (GlyA) and threonine aldolase (ItaE).

SEQUENCE LISTING

<110>  Evonik Industries AG

<120>  ACYL AMINO ACIDS

<130>  201400254EP

<150>  14169650.0
<151>  2014-05-23

<160>  62

<170>  PatentIn version 3.5

<210>  1
<211>  906
<212>  DNA
<213>  Umbellularia californica


<220>
<221>  misc_feature
<222>  (1)..(906)
<223>  synUcTE (an acyl-CoA thioesterase) gene (codon-optimized)

<400>  1
atgactctag agtggaaacc gaaaccaaaa ctgcctcaac tgctggatga tcacttcggt      60

ctgcacggtc tggtgtttcg tcgtactttc gcaattcgtt cttatgaagt gggtccagat     120

cgttctacct ccatcctggc cgtcatgaac cacatgcagg aagccaccct gaatcacgcg     180

aaatctgttg gtatcctggg tgatggtttc ggcactactc tggaaatgtc taaacgtgac     240

ctgatgtggg tagtgcgtcg cacccacgta gcagtagagc gctaccctac ttggggtgac     300

actgtggaag tcgagtgttg gattggcgcg tccggtaaca atggtatgcg tcgcgatttt     360

ctggtccgtg actgtaaaac gggcgaaatc ctgacgcgtt gcacctccct gagcgttctg     420

atgaacaccc gcactcgtcg cctgtctacc atcccggacg aagtgcgcgg tgagatcggt     480

cctgctttca tcgataacgt ggcagttaaa gacgacgaaa tcaagaaact gcaaaaactg     540

aacgactcca ccgcggacta catccagggc ggtctgactc cgcgctggaa cgacctggat     600

gttaatcagc atgtgaacaa cctgaaatac gttgcttggg tcttcgagac tgtgccggac     660

agcattttcg aaagccatca catttcctct tttactctgg agtaccgtcg cgaatgtact     720

cgcgactccg ttctgcgcag cctgaccacc gtaagcggcg gttctagcga ggcaggtctg     780

gtctgcgacc atctgctgca actggaaggc ggctccgaag tcctgcgtgc gcgtacggag     840

tggcgtccaa agctgacgga ttctttccgc ggcatctccg taattccggc ggaacctcgt     900

gtttaa                                                                906


<210>  2
<211>  159
<212>  DNA

<213>  Artificial Sequence

<220>
<223>  tac promotor for synUcTE expression

<400>  2
ggatccaatt gtgagcggat aacaattacg agcttcatgc acagtgatcg acgctgttga      60

caattaatca tcggctcgta taatgtgtgg atgtggaatt gtgagcgctc acaattccac     120

aacggtttcc ctctagaaat aattttgttt aacaggagg                            159


<210>  3
<211>  4977
<212>  DNA
<213>  Artificial Sequence

<220>
<223>  Vector pJ294[Ptac-synUcTE]

<400>  3
accaatgctt aatcagtgag gcacctatct cagcgatctg tctatttcgt tcatccatag      60

ttgcctgact ccccgtcgtg tagataacta cgatacggga gggcttacca tctggcccca     120

gcgctgcgat gataccgcga gaaccacgct caccggctcc ggatttatca gcaataaacc     180

agccagccgg aagggccgag cgcagaagtg gtcctgcaac tttatccgcc tccatccagt     240

ctattaattg ttgccgggaa gctagagtaa gtagttcgcc agttaatagt ttgcgcaacg     300

ttgttgccat cgctacaggc atcgtggtgt cacgctcgtc gtttggtatg gcttcattca     360

gctccggttc ccaacgatca aggcgagtta catgatcccc catgttgtgc aaaaaagcgg     420

ttagctcctt cggtcctccg atcgttgtca gaagtaagtt ggccgcagtg ttatcactca     480

tggttatggc agcactgcat aattctctta ctgtcatgcc atccgtaaga tgcttttctg     540

tgactggtga gtactcaacc aagtcattct gagaatagtg tatgcggcga ccgagttgct     600

cttgcccggc gtcaatacgg gataataccg cgccacatag cagaacttta aaagtgctca     660

tcattggaaa acgttcttcg gggcgaaaac tctcaaggat cttaccgctg ttgagatcca     720

gttcgatgta acccactcgt gcacccaact gatcttcagc atcttttact ttcaccagcg     780

tttctgggtg agcaaaaaca ggaaggcaaa atgccgcaaa aaagggaata agggcgacac     840

ggaaatgttg aatactcata ttcttccttt ttcaatatta ttgaagcatt tatcagggtt     900

attgtctcat gagcggatac atatttgaat gtatttagaa aaataaacaa ataggggtca     960

gtgttacaac caattaacca attctgaaca ttatcgcgag cccatttata cctgaatatg    1020

gctcataaca ccccttgttt gcctggcggc agtagcgcgg tggtcccacc tgaccccatg    1080

ccgaactcag aagtgaaacg ccgtagcgcc gatggtagtg tggggactcc ccatgcgaga    1140

gtagggaact gccaggcatc aaataaaacg aaaggctcag tcgaaagact gggcctttcg    1200

cccgggctaa ttatggggtg tcgcccttat tcgactctat agtgaagttc ctattctcta    1260

gaaagtatag gaacttctga agtgggggcg gccgcaaatt cagtaagcag aaagtcaaaa 1320

gcctccgacc ggaggctttt gactaaagct taaacacgag gttccgccgg aattacggag 1380

atgccgcgga aagaatccgt cagctttgga cgccactccg tacgcgcacg caggacttcg 1440

gagccgcctt ccagttgcag cagatggtcg cagaccagac ctgcctcgct agaaccgccg 1500

cttacggtgg tcaggctgcg cagaacggag tcgcgagtac attcgcgacg gtactccaga 1560

gtaaaagagg aaatgtgatg gctttcgaaa atgctgtccg gcacagtctc gaagacccaa 1620

gcaacgtatt tcaggttgtt cacatgctga ttaacatcca ggtcgttcca gcgcggagtc 1680

agaccgccct ggatgtagtc cgcggtggag tcgttcagtt tttgcagttt cttgatttcg 1740

tcgtctttaa ctgccacgtt atcgatgaaa gcaggaccga tctcaccgcg cacttcgtcc 1800

gggatggtag acaggcgacg agtgcgggtg ttcatcagaa cgctcaggga ggtgcaacgc 1860

gtcaggattt cgcccgtttt acagtcacgg accagaaaat cgcgacgcat accattgtta 1920

ccggacgcgc caatccaaca ctcgacttcc acagtgtcac cccaagtagg gtagcgctct 1980

actgctacgt gggtgcgacg cactacccac atcaggtcac gtttagacat ttccagagta 2040

gtgccgaaac catcacccag gataccaaca gatttcgcgt gattcagggt ggcttcctgc 2100

atgtggttca tgacggccag gatggaggta gaacgatctg gacccacttc ataagaacga 2160

attgcgaaag tacgacgaaa caccagaccg tgcagaccga agtgatcatc cagcagttga 2220

ggcagttttg gtttcggttt ccactctaga gtcattttat aatcctcctt gtaaacaaaa 2280

ttatttctag agggaaaccg ttgtggaatt gtgagcgctc acaattccac atccacacat 2340

tatacgagcc gatgattaat tgtcaacagc gtcgatcact gtgcatgaag ctcgtaattg 2400

ttatccgctc acaattggat ccaaaatgaa gggaagttcc tatactttct agagaatagg 2460

aacttctata gggagtcgaa taagggcgac acaaaaggta ttctaaatgc ataataaata 2520

ctgataacat cttatagttt gtattatatt ttgtattatc gttgacatgt ataattttga 2580

tatcaaaaac tgattttccc tttattattt tcgagattta ttttcttaat tctctttaac 2640

aaactagaaa tattgtatat acaaaaaatc ataaataata gatgaatagt ttaattatag 2700

gtgttcatca atcgaaaaag caacgtatct tatttaaagt gcgttgcttt tttctcattt 2760

ataaggttaa ataattctca tatatcaagc aaagtgacag gcgcccttaa atattctgac 2820

aaatgctctt tccctaaact ccccccataa aaaaacccgc cgaagcgggt ttttacgtta 2880

tttgcggatt aacgattact cgttatcaga accgcccagg atgcctggca gttccctact 2940

ctcgccgctg cgctcggtcg ttcggctgcg ggacctcagc gctagcggag tgtatactgg 3000

cttactatgt tggcactgat gagggtgtca gtgaagtgct tcatgtggca ggagaaaaaa 3060

ggctgcaccg gtgcgtcagc agaatatgtg atacaggata tattccgctt cctcgctcac 3120

tgactcgcta cgctcggtcg ttcgactgcg gcgagcggaa atggcttacg aacggggcgg 3180

agatttcctg gaagatgcca ggaagatact taacagggaa gtgagagggc cgcggcaaag 3240

ccgtttttcc ataggctccg cccccctgac aagcatcacg aaatctgacg ctcaaatcag 3300

tggtggcgaa acccgacagg actataaaga taccaggcgt ttccccctgg cggctccctc 3360

gtgcgctctc ctgttcctgc ctttcggttt accggtgtca ttccgctgtt atggccgcgt 3420

ttgtctcatt ccacgcctga cactcagttc cgggtaggca gttcgctcca agctggactg 3480

tatgcacgaa ccccccgttc agtccgaccg ctgcgcctta tccggtaact atcgtcttga 3540

gtccaacccg gaaagacatg caaaagcacc actggcagca gccactggta attgatttag 3600

aggagttagt cttgaagtca tgcgccggtt aaggctaaac tgaaaggaca agttttggtg 3660

actgcgctcc tccaagccag ttacctcggt tcaaagagtt ggtagctcag agaaccttcg 3720

aaaaaccgcc ctgcaaggcg gttttttcgt tttcagagca agagattacg cgcagaccaa 3780

aacgatctca agaagatcat cttattaatc actgcccgct ttccagtcgg gaaacctgtc 3840

gtgccagctg cattaatgaa tcggccaacg cgcggggaga ggcggtttgc gtattgggcg 3900

ccagggtggt ttttcttttc accagtgaga ctggcaacag ctgattgccc ttcaccgcct 3960

ggccctgaga gagttgcagc aagcggtcca cgctggtttg ccccagcagg cgaaaatcct 4020

gtttgatggt ggttaacggc gggatataac atgagctatc ttcggtatcg tcgtatccca 4080

ctaccgagat atccgcacca acgcgcagcc cggactcggt aatggcgcgc attgcgccca 4140

gcgccatctg atcgttggca accagcatcg cagtgggaac gatgccctca ttcagcattt 4200

gcatggtttg ttgaaaaccg gacatggcac tccagtcgcc ttcccgttcc gctatcggct 4260

gaatttgatt gcgagtgaga tatttatgcc agccagccag acgcagacgc gccgagacag 4320

aacttaatgg gcccgctaac agcgcgattt gctggtgacc caatgcgacc agatgctcca 4380

cgcccagtcg cgtaccgtcc tcatgggaga aaataatact gttgatgggt gtctggtcag 4440

agacatcaag aaataacgcc ggaacattag tgcaggcagc ttccacagca atggcatcct 4500

ggtcatccag cggatagtta atgatcagcc cactgacgcg ttgcgcgaga agattgtgca 4560

ccgccgcttt acaggcttcg acgccgcttc gttctaccat cgacaccacc acgctggcac 4620

ccagttgatc ggcgcgagat ttaatcgccg cgacaatttg cgacggcgcg tgcagggcca 4680

gactggaggt ggcaacgcca atcagcaacg actgtttgcc cgccagttgt tgtgccacgc 4740

ggttgggaat gtaattcagc tccgccatcg ccgcttccac tttttcccgc gttttcgcag 4800

aaacgtggct ggcctggttc accacgcggg aaacggtctg ataagagaca ccggcatact 4860

ctgcgacatc gtataacgtt actggtttca tattcaccac cctgaattga ctctcttccg 4920

ggcgctatca tgccataccg cgaaaggttt tgcgccattc gatggcgcgc cgctttt 4977

<210>  4
<211>  882
<212>  DNA
<213>  Homo sapiens

<220>
<221>  misc_feature
<222>  (1)..(882)
<223>  hGLYAT2 (an amino acid N-acyl transferase) codon optimised for
       E.coli

<400>  4
atgctggttc tgcacaacag ccaaaaactg caaattctgt acaaatctct ggaaaaatct      60

attcctgaaa gcattaaagt ttatggtgcg atcttcaaca tcaaggataa aaaccctttc     120

aatatggagg tgctggtcga cgcgtggcct gattaccaaa tcgtcatcac ccgtccgcag     180

aagcaagaaa tgaaagatga ccaggaccac tatactaaca cctaccacat cttcactaaa     240

gcgccggaca aactggaaga agttctgagc tactccaatg ttatctcctg ggaacaaacg     300

ctgcagattc aaggttgcca ggaaggtctg gatgaggcga ttcgtaaggt cgctacctct     360

aagtccgttc aagtcgatta catgaaaacc atcctgttta tcccggagct gccgaagaaa     420

cacaaaacct cctctaacga caagatggag ctgttcgaag tagacgatga caacaaagag     480

ggtaactttt ccaatatgtt cctggacgcc tcccacgccg gtctggttaa tgagcactgg     540

gcgttcggta aaaacgaacg ctccctgaag tacattgagc gttgtctgca ggattttctg     600

ggttttggtg tcctgggtcc agagggtcaa ctggtctctt ggatcgttat ggaacagtct     660

tgtgaactgc gtatgggtta tactgtgcca aaataccgtc accaaggtaa tatgctgcaa     720

atcggttatc atctggagaa gtacctgagc cagaaggaaa tcccgttcta tttccatgtt     780

gccgataata acgaaaagag cctgcaagcc ctgaacaatc tgggcttcaa gatctgccct     840

tgcggttggc accagtggaa gtgcacgcct aaaaagtact gt                        882

<210>  5
<211>  864
<212>  DNA
<213>  Homo sapiens

<220>
<221>  misc_feature
<222>  (1)..(864)
<223>  hGLYAT3 (another amino acid N-acyl transferase) (codon optimised
       for E.coli)

<400>  5
atgctggttc tgaattgctc tactaaactg ctgatcctgg agaaaatgct gaaatcctgt      60

ttcccggaat ctctgaaagt ttatggtgcg gtaatgaata tcaaccgtgg taatccgttt     120

caaaaagaag ttgttctgga ctcttggcca gatttcaagg cggttatcac ccgtcgtcag     180

cgtgaagcgg aaaccgacaa cctggaccac tataccaacg cgtatgcggt tttctataaa 240

gatgttcgcg cgtaccgtca gctgctggaa gaatgcgatg ttttcaactg ggaccaggtt 300

tttcagatcc aaggtctgca atccgagctg tacgacgttt ctaaggccgt ggcgaactct 360

aaacaactga acatcaagct gacctctttc aaagcggttc atttctctcc ggtatccagc 420

ctgccggaca cctccttcct gaaaggtccg tctccgcgtc tgacctacct gtctgttgcg 480

aatgcggatc tgctgaaccg tacttggtcc cgtggtggca acgaacagtg cctgcgttac 540

attgctaacc tgatctcttg cttcccgagc gtctgcgttc gtgatgaaaa aggtaaccca 600

gtatcttggt ctatcaccga ccagttcgcg accatgtgtc atggctacac cctgcctgaa 660

caccgtcgta agggttactc tcgtctggtt gcgctgaccc tggcgcgtaa gctgcagtct 720

cgtggttttc cgtcccaggg taacgtcctg gacgacaaca ctgcgtccat ctccctgctg 780

aagtccctgc atgccgaatt cctgccgtgc cgtttccacc gtctgattct gaccccagcg 840

accttctctg gtctgccgca cctg 864

<210> 6
<211> 1686
<212> DNA
<213> Escherichia coli

<220>
<221> misc_feature
<222> (1)..(1686)
<223> fadD (an acyl-CoA synthetase)

<400> 6
ttgaagaagg tttggcttaa ccgttatccc gcggacgttc cgacggagat caaccctgac 60

cgttatcaat ctctggtaga tatgtttgag cagtcggtcg cgcgctacgc cgatcaacct 120

gcgtttgtga atatggggga ggtaatgacc ttccgcaagc tggaagaacg cagtcgcgcg 180

tttgccgctt atttgcaaca agggttgggg ctgaagaaag gcgatcgcgt tgcgttgatg 240

atgcctaatt tattgcaata tccggtggcg ctgtttggca ttttgcgtgc cgggatgatc 300

gtcgtaaacg ttaacccgtt gtataccccg cgtgagcttg agcatcagct taacgatagc 360

ggcgcatcgg cgattgttat cgtgtctaac tttgctcaca cactggaaaa agtggttgat 420

aaaaccgccg ttcagcacgt aattctgacc cgtatgggcg atcagctatc tacggcaaaa 480

ggcacggtag tcaatttcgt tgttaaatac atcaagcgtt tggtgccgaa ataccatctg 540

ccagatgcca tttcatttcg tagcgcactg cataacggct accggatgca gtacgtcaaa 600

cccgaactgg tgccggaaga tttagctttt ctgcaataca ccggcggcac cactggtgtg 660

gcgaaaggcg cgatgctgac tcaccgcaat atgctggcga acctggaaca ggttaacgcg 720

acctatggtc cgctgttgca tccgggcaaa gagctggtgg tgacggcgct gccgctgtat 780

cacatttttg ccctgaccat taactgcctg ctgtttatcg aactgggtgg gcagaacctg 840

cttatcacta acccgcgcga tattccaggg ttggtaaaag agttagcgaa atatccgttt 900

accgctatca cgggcgttaa caccttgttc aatgcgttgc tgaacaataa agagttccag 960

cagctggatt tctccagtct gcatctttcc gcaggcggtg ggatgccagt gcagcaagtg 1020

gtggcagagc gttgggtgaa actgaccgga cagtatctgc tggaaggcta tggccttacc 1080

gagtgtgcgc cgctggtcag cgttaaccca tatgatattg attatcatag tggtagcatc 1140

ggtttgccgg tgccgtcgac ggaagccaaa ctggtggatg atgatgataa tgaagtacca 1200

ccaggtcaac cgggtgagct ttgtgtcaaa ggaccgcagg tgatgctggg ttactggcag 1260

cgtcccgatg ctaccgatga aatcatcaaa aatggctggt tacacaccgg cgacatcgcg 1320

gtaatggatg aagaaggatt cctgcgcatt gtcgatcgta aaaaagacat gattctggtt 1380

tccggtttta acgtctatcc caacgagatt gaagatgtcg tcatgcagca tcctggcgta 1440

caggaagtcg cggctgttgg cgtaccttcc ggctccagtg gtgaagcggt gaaaatcttc 1500

gtagtgaaaa aagatccatc gcttaccgaa gagtcactgg tgactttttg ccgccgtcag 1560

ctcacgggat acaaagtacc gaagctggtg gagtttcgtg atgagttacc gaaatctaac 1620

gtcggaaaaa ttttgcgacg agaattacgt gacgaagcgc gcggcaaagt ggacaataaa 1680

gcctga 1686

<210> 7
<211> 7138
<212> DNA
<213> artificial sequence

<220>
<223> Vector pCDF[atfA1_Ab(co_Ec)-fadD_Ec]

<220>
<221> misc_feature
<222> (1)..(7138)
<223> Vector pCDF[atfA1_Ab(co_Ec)-fadD_Ec]

<400> 7
cgggatctcg acgctctccc ttatgcgact cctgcgttta gggaaagagc atttgtcaga 60

atatttaagg gcgcctgtca ctttgcttga tatatgagaa ttatttaacc ttataaatga 120

gaaaaaagca acgcacttta aataagatac gttgcttttt cgattgatga acacctataa 180

ttaaactatt catctattat ttatgatttt ttgtatatac aatatttcta gtttgttaaa 240

gagaattaag aaaataaatc tcgaaaataa taaagggaaa atcagttttt gatatcaaaa 300

ttatacatgt caacgataat acaaaatata atacaaacta taagatgtta tcagtattta 360

ttatgcattt agaatacctt ttgtgtcgcc cttattcgac tccctataga agttcctatt 420

ctctagaaag tataggaact tcccttcatt ttggatccaa ttgtgagcgg ataacaatta 480

cgagcttcat gcacagtgat cgacgctgtt gacaattaat catcggctcg tataatgtgt 540

ggatgtggaa ttgtgagcgc tcacaattcc acaacggttt ccctctagaa ataattttgt 600

ttaacaggag gtaaaacata tgaaagcgct gtccccggtg gatcagctgt tcctgtggct 660

ggagaaacgt cagcaaccga tgcatgttgg tggtctgcag ctgtttagct tcccagaggg 720

tgcgggtcct aagtacgtta gcgagttggc tcagcaaatg cgtgactact gtcacccggt 780

ggccccgttc aatcagcgcc tgacccgtcg cctgggtcaa tactattgga cgcgcgacaa 840

acaatttgac attgaccacc attttcgcca tgaagcgctg ccgaagccgg gtcgcattcg 900

cgaactgctg tccttggtta gcgcggagca cagcaatctg ttggaccgcg agcgccctat 960

gtgggaagcg cacctgattg agggcatccg tggccgccaa ttcgctttgt actataagat 1020

ccaccatagc gtgatggatg gcatcagcgc tatgcgcatt gcgtctaaaa cgctgagcac 1080

cgacccgtct gagcgcgaaa tggcaccagc atgggccttc aataccaaga aacgtagccg 1140

tagcctgcct tccaatccag ttgatatggc gagcagcatg gcccgtctga cggcttctat 1200

cagcaaacag gcggcgaccg ttccgggtct ggctcgtgaa gtgtacaagg tcacccagaa 1260

agctaagaag gatgagaact acgtgagcat ttttcaagcg ccggatacca tcttgaacaa 1320

caccattacc ggttcccgtc gttttgcagc acagagcttt ccgctgccgc gtctgaaagt 1380

tatcgcgaag gcgtataact gcaccatcaa caccgtcgtc ctgtctatgt gtggccacgc 1440

gctgcgtgaa tatctgatta gccaacacgc actgccggat gagccgctga tcgcgatggt 1500

gccgatgagc ctgcgtcagg acgacagcac gggtggtaat cagattggca tgatcctggc 1560

caacctgggc acccatatct gcgatccggc gaatcgtttg cgtgttattc acgactccgt 1620

cgaggaagcg aaatcgcgtt tcagccagat gagcccggag gagatcctga actttaccgc 1680

actgactatg gccccgacgg gcttgaatct gctgaccggc ctggcaccga aatggcgtgc 1740

gttcaacgtc gtcattagca acattccggg tccgaaggaa ccgctgtatt ggaatggcgc 1800

ccagctgcaa ggtgtgtacc cggtaagcat tgcgttggac cgtattgcac tgaacatcac 1860

gctgactagc tacgtggatc agatggagtt cggtctgatc gcatgccgtc gcacgctgcc 1920

gagcatgcaa cgtctgctgg attatctgga acaaagcatt cgtgagttgg aaatcggtgc 1980

cggcatcaag taagcgttaa gtctgagagg tgaagaattg aagaaggttt ggcttaaccg 2040

ttatcccgcg gacgttccga cggagatcaa ccctgaccgt tatcaatctc tggtagatat 2100

gtttgagcag tcggtcgcgc gctacgccga tcaacctgcg tttgtgaata tgggggaggt 2160

aatgaccttc cgcaagctgg aagaacgcag tcgcgcgttt gccgcttatt tgcaacaagg 2220

gttggggctg aagaaaggcg atcgcgttgc gttgatgatg cctaatttat tgcaatatcc 2280

ggtggcgctg tttggcattt tgcgtgccgg gatgatcgtc gtaaacgtta acccgttgta 2340

taccccgcgt gagcttgagc atcagcttaa cgatagcggc gcatcggcga ttgttatcgt 2400
gtctaacttt gctcacacac tggaaaaagt ggttgataaa accgccgttc agcacgtaat 2460
tctgacccgt atgggcgatc agctatctac ggcaaaaggc acggtagtca atttcgttgt 2520
taaatacatc aagcgtttgg tgccgaaata ccatctgcca gatgccattt catttcgtag 2580
cgcactgcat aacggctacc ggatgcagta cgtcaaaccc gaactggtgc cggaagattt 2640
agcttttctg caatacaccg gcggcaccac tggtgtggcg aaaggcgcga tgctgactca 2700
ccgcaatatg ctggcgaacc tggaacaggt taacgcgacc tatggtccgc tgttgcatcc 2760
gggcaaagag ctggtggtga cggcgctgcc gctgtatcac atttttgccc tgaccattaa 2820
ctgcctgctg tttatcgaac tgggtgggca gaacctgctt atcactaacc cgcgcgatat 2880
tccagggttg gtaaaagagt tagcgaaata tccgtttacc gctatcacgg gcgttaacac 2940
cttgttcaat gcgttgctga acaataaaga gttccagcag ctggatttct ccagtctgca 3000
tctttccgca ggcggtggga tgccagtgca gcaagtggtg gcagagcgtt gggtgaaact 3060
gaccggacag tatctgctgg aaggctatgg ccttaccgag tgtgcgccgc tggtcagcgt 3120
taacccatat gatattgatt atcatagtgg tagcatcggt ttgccggtgc cgtcgacgga 3180
agccaaactg gtggatgatg atgataatga agtaccacca ggtcaaccgg gtgagctttg 3240
tgtcaaagga ccgcaggtga tgctgggtta ctggcagcgt cccgatgcta ccgatgaaat 3300
catcaaaaat ggctggttac acaccggcga catcgcggta atggatgaag aaggattcct 3360
gcgcattgtc gatcgtaaaa aagacatgat tctggtttcc ggttttaacg tctatcccaa 3420
cgagattgaa gatgtcgtca tgcagcatcc tggcgtacag gaagtcgcgg ctgttggcgt 3480
accttccggc tccagtggtg aagcggtgaa aatcttcgta gtgaaaaaag atccatcgct 3540
taccgaagag tcactggtga ctttttgccg ccgtcagctc acgggataca aagtaccgaa 3600
gctggtggag tttcgtgatg agttaccgaa atctaacgtc ggaaaaattt tgcgacgaga 3660
attacgtgac gaagcgcgcg gcaaagtgga caataaagcc tgagcgttaa gtcagtcgtc 3720
agacgccggt taatccggcg ttttttttga cgcccactaa agagaaaaca atctcgagtc 3780
tggtaaagaa accgctgctg cgaaatttga acgccagcac atggactcgt ctactagcgc 3840
agcttaatta acctaggctg ctgccaccgc tgagcaataa ctagcataac cccttggggc 3900
ctctaaacgg gtcttgaggg gttttttgct gaaacctcag gcatttgaga agcacacggt 3960
cacactgctt ccggtagtca ataaaccggt aaaccagcaa tagacataag cggctattta 4020
acgaccctgc cctgaaccga cgaccgggtc atcgtggccg gatcttgcgg cccctcggct 4080
tgaacgaatt gttagacatt atttgccgac taccttggtg atctcgcctt tcacgtagtg 4140
gacaaattct tccaactgat ctgcgcgcga ggccaagcga tcttcttctt gtccaagata 4200
agcctgtcta gcttcaagta tgacgggctg atactgggcc ggcaggcgct ccattgccca 4260

gtcggcagcg acatccttcg gcgcgatttt gccggttact gcgctgtacc aaatgcggga 4320

caacgtaagc actacatttc gctcatcgcc agcccagtcg ggcggcgagt tccatagcgt 4380

taaggtttca tttagcgcct caaatagatc ctgttcagga accggatcaa agagttcctc 4440

cgccgctgga cctaccaagg caacgctatg ttctcttgct tttgtcagca agatagccag 4500

atcaatgtcg atcgtggctg gctcgaagat acctgcaaga atgtcattgc gctgccattc 4560

tccaaattgc agttcgcgct tagctggata acgccacgga atgatgtcgt cgtgcacaac 4620

aatggtgact tctacagcgc ggagaatctc gctctctcca ggggaagccg aagtttccaa 4680

aaggtcgttg atcaaagctc gccgcgttgt ttcatcaagc cttacggtca ccgtaaccag 4740

caaatcaata tcactgtgtg gcttcaggcc gccatccact gcggagccgt acaaatgtac 4800

ggccagcaac gtcggttcga gatggcgctc gatgacgcca actacctctg atagttgagt 4860

cgatacttcg gcgatcaccg cttccctcat actcttcctt tttcaatatt attgaagcat 4920

ttatcagggt tattgtctca tgagcggata catatttgaa tgtatttaga aaaataaaca 4980

aatagctagc tcactcggtc gctacgctcc gggcgtgaga ctgcggcggg cgctgcggac 5040

acatacaaag ttacccacag attccgtgga taagcagggg actaacatgt gaggcaaaac 5100

agcagggccg cgccggtggc gtttttccat aggctccgcc ctcctgccag agttcacata 5160

aacagacgct tttccggtgc atctgtggga gccgtgaggc tcaaccatga atctgacagt 5220

acgggcgaaa cccgacagga cttaaagatc cccaccgttt ccggcgggtc gctccctctt 5280

gcgctctcct gttccgaccc tgccgtttac cggatacctg ttccgccttt ctcccttacg 5340

ggaagtgtgg cgctttctca tagctcacac actggtatct cggctcggtg taggtcgttc 5400

gctccaagct gggctgtaag caagaactcc ccgttcagcc cgactgctgc gccttatccg 5460

gtaactgttc acttgagtcc aacccggaaa agcacggtaa aacgccactg gcagcagcca 5520

ttggtaactg ggagttcgca gaggatttgt ttagctaaac acgcggttgc tcttgaagtg 5580

tgcgccaaag tccggctaca ctggaaggac agatttggtt gctgtgctct gcgaaagcca 5640

gttaccacgg ttaagcagtt ccccaactga cttaaccttc gatcaaacca cctccccagg 5700

tggttttttc gtttacaggg caaaagatta cgcgcagaaa aaaaggatct caagaagatc 5760

ctttgatctt ttctactgaa ccgctctaga tttcagtgca atttatctct tcaaatgtag 5820

cacctgaagt cagccccata cgatataagt tgtaattctc atgttagtca tgccccgcgc 5880

ccaccggaag gagctgactg ggttgaaggc tctcaagggc atcggtcgag atcccggtgc 5940

ctaatgagtg agctaactta cattaattgc gttgcgctca ctgcccgctt tccagtcggg 6000

aaacctgtcg tgccagctgc attaatgaat cggccaacgc gcggggagag gcggtttgcg 6060

tattgggcgc cagggtggtt tttcttttca ccagtgagac gggcaacagc tgattgccct 6120

tcaccgcctg gccctgagag agttgcagca agcggtccac gctggtttgc cccagcaggc 6180

gaaaatcctg tttgatggtg gttaacggcg ggatataaca tgagctgtct tcggtatcgt 6240

cgtatcccac taccgagatg tccgcaccaa cgcgcagccc ggactcggta atggcgcgca 6300

ttgcgcccag cgccatctga tcgttggcaa ccagcatcgc agtgggaacg atgccctcat 6360

tcagcatttg catggtttgt tgaaaaccgg acatggcact ccagtcgcct tcccgttccg 6420

ctatcggctg aatttgattg cgagtgagat atttatgcca gccagccaga cgcagacgcg 6480

ccgagacaga acttaatggg cccgctaaca gcgcgatttg ctggtgaccc aatgcgacca 6540

gatgctccac gcccagtcgc gtaccgtctt catgggagaa aataatactg ttgatgggtg 6600

tctggtcaga gacatcaaga aataacgccg gaacattagt gcaggcagct tccacagcaa 6660

tggcatcctg gtcatccagc ggatagttaa tgatcagccc actgacgcgt tgcgcgagaa 6720

gattgtgcac cgccgcttta caggcttcga cgccgcttcg ttctaccatc gacaccacca 6780

cgctggcacc cagttgatcg gcgcgagatt taatcgccgc gacaatttgc gacggcgcgt 6840

gcagggccag actggaggtg gcaacgccaa tcagcaacga ctgtttgccc gccagttgtt 6900

gtgccacgcg gttgggaatg taattcagct ccgccatcgc cgcttccact ttttcccgcg 6960

ttttcgcaga aacgtggctg gcctggttca ccacgcggga aacggtctga taagagacac 7020

cggcatactc tgcgacatcg tataacgtta ctggtttcac attcaccacc ctgaattgac 7080

tctcttccgg gcgctatcat gccataccgc gaaaggtttt gcgccattcg atggtgtc 7138

<210> 8
<211> 6649
<212> DNA
<213> artificial sequence

<220>
<223> Vector pCDF{Ptac}[hGLYAT2(co_Ec)-fadD_Ec]


<220>
<221> misc_feature
<222> (1)..(6649)
<223> Vector pCDF{Ptac}[hGLYAT2(co_Ec)-fadD_Ec]

<400> 8
cgggatctcg acgctctccc ttatgcgact cctgcgttta gggaaagagc atttgtcaga 60

atatttaagg gcgcctgtca ctttgcttga tatatgagaa ttatttaacc ttataaatga 120

gaaaaaagca acgcacttta aataagatac gttgcttttt cgattgatga acacctataa 180

ttaaactatt catctattat ttatgatttt ttgtatatac aatatttcta gtttgttaaa 240

gagaattaag aaaataaatc tcgaaaataa taaagggaaa atcagttttt gatatcaaaa 300

ttatacatgt caacgataat acaaaatata atacaaacta taagatgtta tcagtattta 360

ttatgcattt agaatacctt ttgtgtcgcc cttattcgac tccctataga agttcctatt 420

ctctagaaag tataggaact tcccttcatt ttggatccaa ttgtgagcgg ataacaatta 480

cgagcttcat gcacagtgat cgacgctgtt gacaattaat catcggctcg tataatgtgt 540

ggatgtggaa ttgtgagcgc tcacaattcc acaacggttt ccctctagaa ataattttgt 600

ttaacaggag gtaaaacata tgctggttct gcacaacagc caaaaactgc aaattctgta 660

caaatctctg gaaaaatcta ttcctgaaag cattaaagtt tatggtgcga tcttcaacat 720

caaggataaa aaccctttca atatggaggt gctggtcgac gcgtggcctg attaccaaat 780

cgtcatcacc cgtccgcaga agcaagaaat gaaagatgac caggaccact atactaacac 840

ctaccacatc ttcactaaag cgccggacaa actggaagaa gttctgagct actccaatgt 900

tatctcctgg gaacaaacgc tgcagattca aggttgccag gaaggtctgg atgaggcgat 960

tcgtaaggtc gctacctcta agtccgttca agtcgattac atgaaaacca tcctgtttat 1020

cccggagctg ccgaagaaac acaaaacctc ctctaacgac aagatggagc tgttcgaagt 1080

agacgatgac aacaaagagg gtaacttttc caatatgttc ctggacgcct cccacgccgg 1140

tctggttaat gagcactggg cgttcggtaa aaacgaacgc tccctgaagt acattgagcg 1200

ttgtctgcag gattttctgg gttttggtgt cctgggtcca gagggtcaac tggtctcttg 1260

gatcgttatg gaacagtctt gtgaactgcg tatgggttat actgtgccaa aataccgtca 1320

ccaaggtaat atgctgcaaa tcggttatca tctggagaag tacctgagcc agaaggaaat 1380

cccgttctat ttccatgttg ccgataataa cgaaaagagc ctgcaagccc tgaacaatct 1440

gggcttcaag atctgccctt gcggttggca ccagtggaag tgcacgccta aaaagtactg 1500

ttaagcgtta agtctgagag gtgaagaatt gaagaaggtt tggcttaacc gttatcccgc 1560

ggacgttccg acggagatca accctgaccg ttatcaatct ctggtagata tgtttgagca 1620

gtcggtcgcg cgctacgccg atcaacctgc gtttgtgaat atgggggagg taatgacctt 1680

ccgcaagctg gaagaacgca gtcgcgcgtt tgccgcttat ttgcaacaag ggttggggct 1740

gaagaaaggc gatcgcgttg cgttgatgat gcctaattta ttgcaatatc cggtggcgct 1800

gtttggcatt ttgcgtgccg ggatgatcgt cgtaaacgtt aacccgttgt ataccccgcg 1860

tgagcttgag catcagctta acgatagcgg cgcatcggcg attgttatcg tgtctaactt 1920

tgctcacaca ctggaaaaag tggttgataa aaccgccgtt cagcacgtaa ttctgacccg 1980

tatgggcgat cagctatcta cggcaaaagg cacggtagtc aatttcgttg ttaaatacat 2040

caagcgtttg gtgccgaaat accatctgcc agatgccatt tcatttcgta gcgcactgca 2100

taacggctac cggatgcagt acgtcaaacc cgaactggtg ccggaagatt tagcttttct 2160

gcaatacacc ggcggcacca ctggtgtggc gaaaggcgcg atgctgactc accgcaatat 2220

gctggcgaac ctggaacagg ttaacgcgac ctatggtccg ctgttgcatc cgggcaaaga 2280

gctggtggtg acggcgctgc cgctgtatca catttttgcc ctgaccatta actgcctgct 2340

gtttatcgaa ctgggtgggc agaacctgct tatcactaac ccgcgcgata ttccagggtt 2400

ggtaaaagag ttagcgaaat atccgtttac cgctatcacg ggcgttaaca ccttgttcaa 2460

tgcgttgctg aacaataaag agttccagca gctggatttc tccagtctgc atctttccgc 2520

aggcggtggg atgccagtgc agcaagtggt ggcagagcgt tgggtgaaac tgaccggaca 2580

gtatctgctg gaaggctatg gccttaccga gtgtgcgccg ctggtcagcg ttaacccata 2640

tgatattgat tatcatagtg gtagcatcgg tttgccggtg ccgtcgacgg aagccaaact 2700

ggtggatgat gatgataatg aagtaccacc aggtcaaccg ggtgagcttt gtgtcaaagg 2760

accgcaggtg atgctgggtt actggcagcg tcccgatgct accgatgaaa tcatcaaaaa 2820

tggctggtta cacaccggcg acatcgcggt aatggatgaa gaaggattcc tgcgcattgt 2880

cgatcgtaaa aaagacatga ttctggtttc cggttttaac gtctatccca acgagattga 2940

agatgtcgtc atgcagcatc ctggcgtaca ggaagtcgcg gctgttggcg taccttccgg 3000

ctccagtggt gaagcggtga aaatcttcgt agtgaaaaaa gatccatcgc ttaccgaaga 3060

gtcactggtg actttttgcc gccgtcagct cacgggatac aaagtaccga agctggtgga 3120

gtttcgtgat gagttaccga aatctaacgt cggaaaaatt ttgcgacgag aattacgtga 3180

cgaagcgcgc ggcaaagtgg acaataaagc ctgagcgtta agtcagtcgt cagacgccgg 3240

ttaatccggc gttttttttg acgcccacta aagagaaaac aatctcgagt ctggtaaaga 3300

aaccgctgct gcgaaatttg aacgccagca catggactcg tctactagcg cagcttaatt 3360

aacctaggct gctgccaccg ctgagcaata actagcataa ccccttgggg cctctaaacg 3420

ggtcttgagg ggttttttgc tgaaacctca ggcatttgag aagcacacgg tcacactgct 3480

tccggtagtc aataaaccgg taaaccagca atagacataa gcggctattt aacgaccctg 3540

ccctgaaccg acgaccgggt catcgtggcc ggatcttgcg gcccctcggc ttgaacgaat 3600

tgttagacat tatttgccga ctaccttggt gatctcgcct ttcacgtagt ggacaaattc 3660

ttccaactga tctgcgcgcg aggccaagcg atcttcttct tgtccaagat aagcctgtct 3720

agcttcaagt atgacgggct gatactgggc cggcaggcgc tccattgccc agtcggcagc 3780

gacatccttc ggcgcgattt tgccggttac tgcgctgtac caaatgcggg acaacgtaag 3840

cactacattt cgctcatcgc cagcccagtc gggcggcgag ttccatagcg ttaaggtttc 3900

atttagcgcc tcaaatagat cctgttcagg aaccggatca aagagttcct ccgccgctgg 3960

acctaccaag gcaacgctat gttctcttgc ttttgtcagc aagatagcca gatcaatgtc 4020

gatcgtggct ggctcgaaga tacctgcaag aatgtcattg cgctgccatt ctccaaattg 4080

cagttcgcgc ttagctggat aacgccacgg aatgatgtcg tcgtgcacaa caatggtgac 4140

ttctacagcg cggagaatct cgctctctcc aggggaagcc gaagtttcca aaaggtcgtt 4200

gatcaaagct cgccgcgttg tttcatcaag ccttacggtc accgtaacca gcaaatcaat 4260

atcactgtgt ggcttcaggc cgccatccac tgcggagccg tacaaatgta cggccagcaa 4320

cgtcggttcg agatggcgct cgatgacgcc aactacctct gatagttgag tcgatacttc 4380

ggcgatcacc gcttccctca tactcttcct ttttcaatat tattgaagca tttatcaggg 4440

ttattgtctc atgagcggat acatatttga atgtatttag aaaaataaac aaatagctag 4500

ctcactcggt cgctacgctc cgggcgtgag actgcggcgg gcgctgcgga cacatacaaa 4560

gttacccaca gattccgtgg ataagcaggg gactaacatg tgaggcaaaa cagcagggcc 4620

gcgccggtgg cgtttttcca taggctccgc cctcctgcca gagttcacat aaacagacgc 4680

ttttccggtg catctgtggg agccgtgagg ctcaaccatg aatctgacag tacgggcgaa 4740

acccgacagg acttaaagat ccccaccgtt tccggcgggt cgctccctct tgcgctctcc 4800

tgttccgacc ctgccgttta ccggatacct gttccgcctt tctcccttac gggaagtgtg 4860

gcgctttctc atagctcaca cactggtatc tcggctcggt gtaggtcgtt cgctccaagc 4920

tgggctgtaa gcaagaactc cccgttcagc ccgactgctg cgccttatcc ggtaactgtt 4980

cacttgagtc caacccggaa aagcacggta aaacgccact ggcagcagcc attggtaact 5040

gggagttcgc agaggatttg tttagctaaa cacgcggttg ctcttgaagt gtgcgccaaa 5100

gtccggctac actggaagga cagatttggt tgctgtgctc tgcgaaagcc agttaccacg 5160

gttaagcagt tccccaactg acttaacctt cgatcaaacc acctccccag gtggtttttt 5220

cgtttacagg gcaaaagatt acgcgcagaa aaaaaggatc tcaagaagat cctttgatct 5280

tttctactga accgctctag atttcagtgc aatttatctc ttcaaatgta gcacctgaag 5340

tcagccccat acgatataag ttgtaattct catgttagtc atgccccgcg cccaccggaa 5400

ggagctgact gggttgaagg ctctcaaggg catcggtcga gatcccggtg cctaatgagt 5460

gagctaactt acattaattg cgttgcgctc actgcccgct ttccagtcgg gaaacctgtc 5520

gtgccagctg cattaatgaa tcggccaacg cgcggggaga ggcggtttgc gtattgggcg 5580

ccagggtggt ttttcttttc accagtgaga cgggcaacag ctgattgccc ttcaccgcct 5640

ggccctgaga gagttgcagc aagcggtcca cgctggtttg ccccagcagg cgaaaatcct 5700

gtttgatggt ggttaacggc gggatataac atgagctgtc ttcggtatcg tcgtatccca 5760

ctaccgagat gtccgcacca acgcgcagcc cggactcggt aatggcgcgc attgcgccca 5820

gcgccatctg atcgttggca accagcatcg cagtgggaac gatgccctca ttcagcattt 5880

gcatggtttg ttgaaaaccg gacatggcac tccagtcgcc ttcccgttcc gctatcggct 5940

gaatttgatt gcgagtgaga tatttatgcc agccagccag acgcagacgc gccgagacag 6000

aacttaatgg gcccgctaac agcgcgattt gctggtgacc caatgcgacc agatgctcca 6060

cgcccagtcg cgtaccgtct tcatgggaga aaataatact gttgatgggt gtctggtcag 6120

agacatcaag aaataacgcc ggaacattag tgcaggcagc ttccacagca atggcatcct 6180

ggtcatccag cggatagtta atgatcagcc cactgacgcg ttgcgcgaga agattgtgca 6240

ccgccgcttt acaggcttcg acgccgcttc gttctaccat cgacaccacc acgctggcac 6300

ccagttgatc ggcgcgagat ttaatcgccg cgacaatttg cgacggcgcg tgcagggcca 6360

gactggaggt ggcaacgcca atcagcaacg actgtttgcc cgccagttgt tgtgccacgc 6420

ggttgggaat gtaattcagc tccgccatcg ccgcttccac tttttcccgc gttttcgcag 6480

aaacgtggct ggcctggttc accacgcggg aaacggtctg ataagagaca ccggcatact 6540

ctgcgacatc gtataacgtt actggtttca cattcaccac cctgaattga ctctcttccg 6600

ggcgctatca tgccataccg cgaaaggttt tgcgccattc gatggtgtc 6649

<210> 9
<211> 6631
<212> DNA
<213> Artificial Sequence

<220>
<223> Vector pCDF{Ptac}[hGLYAT3(co_Ec)-fadD_Ec]


<220>
<221> misc_feature
<222> (1)..(6631)

<400> 9
cgggatctcg acgctctccc ttatgcgact cctgcgttta gggaaagagc atttgtcaga 60

atatttaagg gcgcctgtca ctttgcttga tatatgagaa ttatttaacc ttataaatga 120

gaaaaaagca acgcacttta aataagatac gttgcttttt cgattgatga acacctataa 180

ttaaactatt catctattat ttatgatttt ttgtatatac aatatttcta gtttgttaaa 240

gagaattaag aaaataaatc tcgaaaataa taaagggaaa atcagttttt gatatcaaaa 300

ttatacatgt caacgataat acaaaatata atacaaacta taagatgtta tcagtattta 360

ttatgcattt agaatacctt ttgtgtcgcc cttattcgac tccctataga agttcctatt 420

ctctagaaag tataggaact tcccttcatt ttggatccaa ttgtgagcgg ataacaatta 480

cgagcttcat gcacagtgat cgacgctgtt gacaattaat catcggctcg tataatgtgt 540

ggatgtggaa ttgtgagcgc tcacaattcc acaacggttt ccctctagaa ataattttgt 600

ttaacaggag gtaaaacata tgctggttct gaattgctct actaaactgc tgatcctgga 660

gaaaatgctg aaatcctgtt tcccggaatc tctgaaagtt tatggtgcgg taatgaatat 720

caaccgtggt aatccgtttc aaaaagaagt tgttctggac tcttggccag atttcaaggc 780

ggttatcacc cgtcgtcagc gtgaagcgga aaccgacaac ctggaccact ataccaacgc 840

gtatgcggtt ttctataaag atgttcgcgc gtaccgtcag ctgctggaag aatgcgatgt 900

tttcaactgg gaccaggttt ttcagatcca aggtctgcaa tccgagctgt acgacgtttc 960

taaggccgtg gcgaactcta aacaactgaa catcaagctg acctctttca aagcggttca 1020

tttctctccg gtatccagcc tgccggacac ctccttcctg aaaggtccgt ctccgcgtct 1080

gacctacctg tctgttgcga atgcggatct gctgaaccgt acttggtccc gtggtggcaa 1140

cgaacagtgc ctgcgttaca ttgctaacct gatctcttgc ttcccgagcg tctgcgttcg 1200

tgatgaaaaa ggtaacccag tatcttggtc tatcaccgac cagttcgcga ccatgtgtca 1260

tggctacacc ctgcctgaac accgtcgtaa gggttactct cgtctggttg cgctgaccct 1320

ggcgcgtaag ctgcagtctc gtggttttcc gtcccagggt aacgtcctgg acgacaacac 1380

tgcgtccatc tccctgctga agtccctgca tgccgaattc ctgccgtgcc gtttccaccg 1440

tctgattctg accccagcga ccttctctgg tctgccgcac ctgtaagcgt taagtctgag 1500

aggtgaagaa ttgaagaagg tttggcttaa ccgttatccc gcggacgttc cgacggagat 1560

caaccctgac cgttatcaat ctctggtaga tatgtttgag cagtcggtcg cgcgctacgc 1620

cgatcaacct gcgtttgtga atatggggga ggtaatgacc ttccgcaagc tggaagaacg 1680

cagtcgcgcg tttgccgctt atttgcaaca agggttgggg ctgaagaaag gcgatcgcgt 1740

tgcgttgatg atgcctaatt tattgcaata tccggtggcg ctgtttggca ttttgcgtgc 1800

cgggatgatc gtcgtaaacg ttaacccgtt gtataccccg cgtgagcttg agcatcagct 1860

taacgatagc ggcgcatcgg cgattgttat cgtgtctaac tttgctcaca cactggaaaa 1920

agtggttgat aaaaccgccg ttcagcacgt aattctgacc cgtatgggcg atcagctatc 1980

tacggcaaaa ggcacggtag tcaatttcgt tgttaaatac atcaagcgtt tggtgccgaa 2040

ataccatctg ccagatgcca tttcatttcg tagcgcactg cataacggct accggatgca 2100

gtacgtcaaa cccgaactgg tgccggaaga tttagctttt ctgcaataca ccggcggcac 2160

cactggtgtg gcgaaaggcg cgatgctgac tcaccgcaat atgctggcga acctggaaca 2220

ggttaacgcg acctatggtc cgctgttgca tccgggcaaa gagctggtgg tgacggcgct 2280

gccgctgtat cacatttttg ccctgaccat taactgcctg ctgtttatcg aactgggtgg 2340

gcagaacctg cttatcacta acccgcgcga tattccaggg ttggtaaaag agttagcgaa 2400

atatccgttt accgctatca cgggcgttaa caccttgttc aatgcgttgc tgaacaataa 2460

agagttccag cagctggatt tctccagtct gcatctttcc gcaggcggtg ggatgccagt 2520

gcagcaagtg gtggcagagc gttgggtgaa actgaccgga cagtatctgc tggaaggcta 2580

tggccttacc gagtgtgcgc cgctggtcag cgttaaccca tatgatattg attatcatag 2640

tggtagcatc ggtttgccgg tgccgtcgac ggaagccaaa ctggtggatg atgatgataa 2700

tgaagtacca ccaggtcaac cgggtgagct ttgtgtcaaa ggaccgcagg tgatgctggg 2760

ttactggcag cgtcccgatg ctaccgatga aatcatcaaa aatggctggt tacacaccgg 2820

cgacatcgcg gtaatggatg aagaaggatt cctgcgcatt gtcgatcgta aaaaagacat 2880

gattctggtt tccggtttta acgtctatcc caacgagatt gaagatgtcg tcatgcagca 2940

tcctggcgta caggaagtcg cggctgttgg cgtaccttcc ggctccagtg gtgaagcggt 3000

gaaaatcttc gtagtgaaaa aagatccatc gcttaccgaa gagtcactgg tgactttttg 3060

ccgccgtcag ctcacgggat acaaagtacc gaagctggtg gagtttcgtg atgagttacc 3120

gaaatctaac gtcggaaaaa ttttgcgacg agaattacgt gacgaagcgc gcggcaaagt 3180

ggacaataaa gcctgagcgt taagtcagtc gtcagacgcc ggttaatccg gcgttttttt 3240

tgacgcccac taaagagaaa acaatctcga gtctggtaaa gaaaccgctg ctgcgaaatt 3300

tgaacgccag cacatggact cgtctactag cgcagcttaa ttaacctagg ctgctgccac 3360

cgctgagcaa taactagcat aaccccttgg ggcctctaaa cgggtcttga ggggtttttt 3420

gctgaaacct caggcatttg agaagcacac ggtcacactg cttccggtag tcaataaacc 3480

ggtaaaccag caatagacat aagcggctat ttaacgaccc tgccctgaac cgacgaccgg 3540

gtcatcgtgg ccggatcttg cggcccctcg gcttgaacga attgttagac attatttgcc 3600

gactaccttg gtgatctcgc ctttcacgta gtggacaaat tcttccaact gatctgcgcg 3660

cgaggccaag cgatcttctt cttgtccaag ataagcctgt ctagcttcaa gtatgacggg 3720

ctgatactgg gccggcaggc gctccattgc ccagtcggca gcgacatcct tcggcgcgat 3780

tttgccggtt actgcgctgt accaaatgcg ggacaacgta agcactacat ttcgctcatc 3840

gccagcccag tcgggcggcg agttccatag cgttaaggtt tcatttagcg cctcaaatag 3900

atcctgttca ggaaccggat caaagagttc ctccgccgct ggacctacca aggcaacgct 3960

atgttctctt gcttttgtca gcaagatagc cagatcaatg tcgatcgtgg ctggctcgaa 4020

gatacctgca agaatgtcat tgcgctgcca ttctccaaat tgcagttcgc gcttagctgg 4080

ataacgccac ggaatgatgt cgtcgtgcac aacaatggtg acttctacag cgcggagaat 4140

ctcgctctct ccaggggaag ccgaagtttc caaaaggtcg ttgatcaaag ctcgccgcgt 4200

tgtttcatca agccttacgg tcaccgtaac cagcaaatca atatcactgt gtggcttcag 4260

gccgccatcc actgcggagc cgtacaaatg tacggccagc aacgtcggtt cgagatggcg 4320

ctcgatgacg ccaactacct ctgatagttg agtcgatact tcggcgatca ccgcttccct 4380

catactcttc ctttttcaat attattgaag catttatcag ggttattgtc tcatgagcgg 4440

atacatattt gaatgtattt agaaaaataa acaaatagct agctcactcg gtcgctacgc 4500

tccgggcgtg agactgcggc gggcgctgcg gacacataca aagttaccca cagattccgt 4560

ggataagcag gggactaaca tgtgaggcaa aacagcaggg ccgcgccggt ggcgtttttc 4620

cataggctcc gccctcctgc cagagttcac ataaacagac gcttttccgg tgcatctgtg 4680

ggagccgtga ggctcaacca tgaatctgac agtacgggcg aaacccgaca ggacttaaag 4740

atccccaccg tttccggcgg gtcgctccct cttgcgctct cctgttccga ccctgccgtt 4800

taccggatac ctgttccgcc tttctccctt acgggaagtg tggcgctttc tcatagctca 4860

cacactggta tctcggctcg gtgtaggtcg ttcgctccaa gctgggctgt aagcaagaac 4920

tccccgttca gcccgactgc tgcgccttat ccggtaactg ttcacttgag tccaacccgg 4980

aaaagcacgg taaaacgcca ctggcagcag ccattggtaa ctgggagttc gcagaggatt 5040

tgtttagcta aacacgcggt tgctcttgaa gtgtgcgcca aagtccggct acactggaag 5100

gacagatttg gttgctgtgc tctgcgaaag ccagttacca cggttaagca gttccccaac 5160

tgacttaacc ttcgatcaaa ccacctcccc aggtggtttt ttcgtttaca gggcaaaaga 5220

ttacgcgcag aaaaaaagga tctcaagaag atcctttgat cttttctact gaaccgctct 5280

agatttcagt gcaatttatc tcttcaaatg tagcacctga agtcagcccc atacgatata 5340

agttgtaatt ctcatgttag tcatgccccg cgcccaccgg aaggagctga ctgggttgaa 5400

ggctctcaag ggcatcggtc gagatcccgg tgcctaatga gtgagctaac ttacattaat 5460

tgcgttgcgc tcactgcccg ctttccagtc gggaaacctg tcgtgccagc tgcattaatg 5520

aatcggccaa cgcgcgggga gaggcggttt gcgtattggg cgccagggtg gtttttcttt 5580

tcaccagtga gacgggcaac agctgattgc ccttcaccgc ctggccctga gagagttgca 5640

gcaagcggtc cacgctggtt tgccccagca ggcgaaaatc ctgtttgatg gtggttaacg 5700

gcgggatata acatgagctg tcttcggtat cgtcgtatcc cactaccgag atgtccgcac 5760

caacgcgcag cccggactcg gtaatggcgc gcattgcgcc cagcgccatc tgatcgttgg 5820

caaccagcat cgcagtggga acgatgccct cattcagcat ttgcatggtt tgttgaaaac 5880

cggacatggc actccagtcg ccttcccgtt ccgctatcgg ctgaatttga ttgcgagtga 5940

gatatttatg ccagccagcc agacgcagac gcgccgagac agaacttaat gggcccgcta 6000

acagcgcgat ttgctggtga cccaatgcga ccagatgctc cacgcccagt cgcgtaccgt 6060

cttcatggga gaaaataata ctgttgatgg gtgtctggtc agagacatca agaaataacg 6120

ccggaacatt agtgcaggca gcttccacag caatggcatc ctggtcatcc agcggatagt 6180

taatgatcag cccactgacg cgttgcgcga gaagattgtg caccgccgct ttacaggctt 6240

cgacgccgct tcgttctacc atcgacacca ccacgctggc acccagttga tcggcgcgag 6300

atttaatcgc cgcgacaatt tgcgacggcg cgtgcagggc cagactggag gtggcaacgc 6360

caatcagcaa cgactgtttg cccgccagtt gttgtgccac gcggttggga atgtaattca 6420

gctccgccat cgccgcttcc actttttccc gcgttttcgc agaaacgtgg ctggcctggt 6480

tcaccacgcg ggaaacggtc tgataagaga caccggcata ctctgcgaca tcgtataacg 6540

ttactggttt cacattcacc accctgaatt gactctcttc cgggcgctat catgccatac 6600

cgcgaaaggt tttgcgccat tcgatggtgt c 6631

<210> 10
<211> 693
<212> DNA
<213> Pseudomonas putida

<220>
<221> misc_feature
<222> (1)..(693)
<223> alkL (an importer facilitating transport of hydrophobic acyl
across cell membranes) gene

<400> 10
gtgagttttt ctaattataa agtaatcgcg atgccggtgt tggttgctaa ttttgttttg 60

ggggcggcca ctgcatgggc gaatgaaaat tatccggcga aatctgctgg ctataatcag 120

ggtgactggg tcgctagctt caatttttct aaggtctatg tgggtgagga gcttggcgat 180

ctaaatgttg gagggggggc tttgccaaat gctgatgtaa gtattggtaa tgatacaaca 240

cttacgtttg atatcgccta ttttgttagc tcaaatatag cggtggattt ttttgttggg 300

gtgccagcta gggctaaatt tcaaggtgag aaatcaatct cctcgctggg aagagtcagt 360

gaagttgatt acggccctgc aattctttcg cttcaatatc attacgatag ctttgagcga 420

ctttatccat atgttggggt tggtgttggt cgggtgctat tttttgataa aaccgacggt 480

gctttgagtt cgtttgatat taaggataaa tgggcgcctg cttttcaggt tggccttaga 540

tatgaccttg gtaactcatg gatgctaaat tcagatgtgc gttatattcc tttcaaaacg 600

gacgtcacag gtactcttgg cccggttcct gtttctacta aaattgaggt tgatcctttc 660

attctcagtc ttggtgcgtc atatgttttc taa 693

<210> 11
<211> 153
<212> DNA
<213> Artificial Sequence

<220>
<223> lacuv5 promoter

<220>
<221> misc_feature
<222> (1)..(153)

<400> 11
ggcagtgagc gcaacgcaat taatgtaagt tagctcactc attaggcacc ccaggcttga 60

cactttatgc ttccggctcg tataatgtgt ggaattgtga gcggataaca ataacaattt 120

cacacaggat ctaggaacca aggagagtgg cat 153

<210>  12
<211>  4876
<212>  DNA
<213>  Artificial Sequence

<220>
<223>  Vector pCDF[alkLmod1]

<220>
<221>  misc_feature
<222>  (1)..(4876)
<223>  Vector pCDF[alkLmod1]

<400>  12
ggggaattgt gagcggataa caattcccct gtagaaataa ttttgtttaa ctttaataag      60

gagatatacc atgggcagca gccatcacca tcatcaccac agccaggatc cttcaatatt     120

attgaagcat ttatcagggt tattgtctca tgagcggata catatttgaa tgtatttaga     180

aaaataaaca aataggggtc agtgttacaa ccaattaacc aattctgaac attatcgcga     240

gcccatttat acctgaatat ggctcataac accccttgtt tgcctggcgg cagtagcgcg     300

gtggtcccac ctgaccccat gccgaactca gaagtgaaac gccgtagcgc cgatggtagt     360

gtggggactc cccatgcgag agtagggaac tgccaggcat caaataaaac gaaaggctca     420

gtcgaaagac tgggcctttc gcccgggcta attatggggt gtcgccctta ttcgactcta     480

tagtgaagtt cctattctct agaaagtata ggaacttctg aagtggggat ccggccggcc     540

tgcagccccg cagggcctgt ctcggtcgat cattcagccc ggctcataga tatgcgggca     600

gtgagcgcaa cgcaattaat gtaagttagc tcactcatta ggcaccccag gcttgacact     660

ttatgcttcc ggctcgtata atgtgtggaa ttgtgagcgg ataacaataa caatttcaca     720

caggatctag gaaccaagga gagtggcatg tgagtttttc taattataaa gtaatcgcga     780

tgccggtgtt ggttgctaat tttgttttgg gggcggccac tgcatggcgg aatgaaaatt     840

atccggcgaa atctgctggc tataatcagg gtgactgggt cgctagcttc aatttttcta     900

aggtctatgt gggtgaggag cttggcgatc taaatgttgg agggggggct ttgccaaatg     960

ctgatgtaag tattggtaat gatacaacac ttacgtttga tatcgcctat tttgttagct    1020

caaatatagc ggtggatttt tttgttgggg tgccagctag ggctaaattt caaggtgaga    1080

aatcaatctc ctcgctggga agagtcagtg aagttgatta cggccctgca attctttcgc    1140

ttcaatatca ttacgatagc tttgagcgac tttatccata tgttggggtt ggtgttggtc    1200

gggtgctatt ttttgataaa accgacggtg ctttgagttc gtttgatatt aaggataaat    1260

gggcgcctgc ttttcaggtt ggccttagat atgaccttgg taactcatgg atgctaaatt    1320

cagatgtgcg ttatattcct ttcaaaacgg acgtcacagg tactcttggc ccggttcctg    1380

tttctactaa aattgaggtt gatcctttca ttctcagtct tggtgcgtca tatgttttct 1440

aaggtaccct cgagtctggt aaagaaaccg ctgctgcgaa atttgaacgc cagcacatgg 1500

actcgtctac tagcgcagct taattaacct aggctgctgc caccgctgag caataactag 1560

cataacccct tggggcctct aaacgggtct tgaggggttt tttgctgaaa cctcaggcat 1620

ttgagaagca cacggtcaca ctgcttccgg tagtcaataa accggtaaac cagcaataga 1680

cataagcggc tatttaacga ccctgccctg aaccgacgac cgggtcatcg tggccggatc 1740

ttgcggcccc tcggcttgaa cgaattgtta gacattattt gccgactacc ttggtgatct 1800

cgcctttcac gtagtggaca aattcttcca actgatctgc gcgcgaggcc aagcgatctt 1860

cttcttgtcc aagataagcc tgtctagctt caagtatgac gggctgatac tgggccggca 1920

ggcgctccat tgcccagtcg gcagcgacat ccttcggcgc gattttgccg gttactgcgc 1980

tgtaccaaat gcgggacaac gtaagcacta catttcgctc atcgccagcc cagtcgggcg 2040

gcgagttcca tagcgttaag gtttcattta gcgcctcaaa tagatcctgt tcaggaaccg 2100

gatcaaagag ttcctccgcc gctggaccta ccaaggcaac gctatgttct cttgcttttg 2160

tcagcaagat agccagatca atgtcgatcg tggctggctc gaagatacct gcaagaatgt 2220

cattgcgctg ccattctcca aattgcagtt cgcgcttagc tggataacgc cacggaatga 2280

tgtcgtcgtg cacaacaatg gtgacttcta cagcgcggag aatctcgctc tctccagggg 2340

aagccgaagt ttccaaaagg tcgttgatca aagctcgccg cgttgtttca tcaagcctta 2400

cggtcaccgt aaccagcaaa tcaatatcac tgtgtggctt caggccgcca tccactgcgg 2460

agccgtacaa atgtacggcc agcaacgtcg gttcgagatg gcgctcgatg acgccaacta 2520

cctctgatag ttgagtcgat acttcggcga tcaccgcttc cctcatactc ttcctttttc 2580

aatattattg aagcatttat cagggttatt gtctcatgag cggatacata tttgaatgta 2640

tttagaaaaa taaacaaata gctagctcac tcggtcgcta cgctccgggc gtgagactgc 2700

ggcgggcgct gcggacacat acaaagttac ccacagattc cgtggataag caggggacta 2760

acatgtgagg caaaacagca gggccgcgcc ggtggcgttt ttccataggc tccgccctcc 2820

tgccagagtt cacataaaca gacgcttttc cggtgcatct gtgggagccg tgaggctcaa 2880

ccatgaatct gacagtacgg gcgaaacccg acaggactta aagatcccca ccgtttccgg 2940

cgggtcgctc cctcttgcgc tctcctgttc cgaccctgcc gtttaccgga tacctgttcc 3000

gcctttctcc cttacgggaa gtgtggcgct ttctcatagc tcacacactg gtatctcggc 3060

tcggtgtagg tcgttcgctc caagctgggc tgtaagcaag aactccccgt tcagcccgac 3120

tgctgcgcct tatccggtaa ctgttcactt gagtccaacc cggaaaagca cggtaaaacg 3180

ccactggcag cagccattgg taactgggag ttcgcagagg atttgtttag ctaaacacgc 3240

ggttgctctt gaagtgtgcg ccaaagtccg gctacactgg aaggacagat ttggttgctg 3300

tgctctgcga aagccagtta ccacggttaa gcagttcccc aactgactta accttcgatc 3360

aaaccacctc cccaggtggt tttttcgttt acagggcaaa agattacgcg cagaaaaaaa 3420

ggatctcaag aagatccttt gatcttttct actgaaccgc tctagatttc agtgcaattt 3480

atctcttcaa atgtagcacc tgaagtcagc cccatacgat ataagttgta attctcatgt 3540

tagtcatgcc ccgcgcccac cggaaggagc tgactgggtt gaaggctctc aagggcatcg 3600

gtcgagatcc cggtgcctaa tgagtgagct aacttacatt aattgcgttg cgctcactgc 3660

ccgctttcca gtcgggaaac ctgtcgtgcc agctgcatta atgaatcggc caacgcgcgg 3720

ggagaggcgg tttgcgtatt gggcgccagg gtggtttttc ttttcaccag tgagacgggc 3780

aacagctgat tgcccttcac cgcctggccc tgagagagtt gcagcaagcg gtccacgctg 3840

gtttgcccca gcaggcgaaa atcctgtttg atggtggtta acggcgggat ataacatgag 3900

ctgtcttcgg tatcgtcgta tcccactacc gagatgtccg caccaacgcg cagcccggac 3960

tcggtaatgg cgcgcattgc gcccagcgcc atctgatcgt tggcaaccag catcgcagtg 4020

ggaacgatgc cctcattcag catttgcatg gtttgttgaa aaccggacat ggcactccag 4080

tcgccttccc gttccgctat cggctgaatt tgattgcgag tgagatattt atgccagcca 4140

gccagacgca gacgcgccga gacagaactt aatgggcccg ctaacagcgc gatttgctgg 4200

tgacccaatg cgaccagatg ctccacgccc agtcgcgtac cgtcttcatg ggagaaaata 4260

atactgttga tgggtgtctg gtcagagaca tcaagaaata acgccggaac attagtgcag 4320

gcagcttcca cagcaatggc atcctggtca tccagcggat agttaatgat cagcccactg 4380

acgcgttgcg cgagaagatt gtgcaccgcc gctttacagg cttcgacgcc gcttcgttct 4440

accatcgaca ccaccacgct ggcacccagt tgatcggcgc gagatttaat cgccgcgaca 4500

atttgcgacg gcgcgtgcag ggccagactg gaggtggcaa cgccaatcag caacgactgt 4560

ttgcccgcca gttgttgtgc cacgcggttg ggaatgtaat tcagctccgc catcgccgct 4620

tccacttttt cccgcgtttt cgcagaaacg tggctggcct ggttcaccac gcgggaaacg 4680

gtctgataag agacaccggc atactctgcg acatcgtata acgttactgg tttcacattc 4740

accaccctga attgactctc ttccgggcgc tatcatgcca taccgcgaaa ggttttgcgc 4800

cattcgatgg tgtccgggat ctcgacgctc tcccttatgc gactcctgca ttaggaaatt 4860

aatacgactc actata 4876

<210> 13
<211> 7468
<212> DNA
<213> Artificial Sequence

<220>
<223> Vector pCDF{Ptac}[hGLYAT2(co_Ec)-fadD_Ec]{Placuv5}[alkLmod1]

<220>
<221>  misc_feature
<222>  (1)..(7468)
<223>  Vector pCDF{Ptac}[hGLYAT2(co_Ec)-fadD_Ec]{Placuv5}[alkLmod1]

<400>  13
cgggatctcg acgctctccc ttatgcgact cctgcgttta gggaaagagc atttgtcaga      60

atatttaagg gcgcctgtca ctttgcttga tatatgagaa ttatttaacc ttataaatga     120

gaaaaaagca acgcacttta aataagatac gttgcttttt cgattgatga acacctataa     180

ttaaactatt catctattat ttatgatttt ttgtatatac aatatttcta gtttgttaaa     240

gagaattaag aaaataaatc tcgaaaataa taaagggaaa atcagttttt gatatcaaaa     300

ttatacatgt caacgataat acaaaatata atacaaacta taagatgtta tcagtattta     360

ttatgcattg tctcggtcga tcattcagcc cggctcatag atatgcgggc agtgagcgca     420

acgcaattaa tgtaagttag ctcactcatt aggcacccca ggcttgacac tttatgcttc     480

cggctcgtat aatgtgtgga attgtgagcg gataacaata acaatttcac acaggatcta     540

ggaaccaagg agagtggcat gtgagttttt ctaattataa agtaatcgcg atgccggtgt     600

tggttgctaa ttttgttttg ggggcggcca ctgcatgggc gaatgaaaat tatccggcga     660

aatctgctgg ctataatcag ggtgactggg tcgctagctt caatttttct aaggtctatg     720

tgggtgagga gcttggcgat ctaaatgttg gagggggggc tttgccaaat gctgatgtaa     780

gtattggtaa tgatacaaca cttacgtttg atatcgccta ttttgttagc tcaaatatag     840

cggtggattt ttttgttggg gtgccagcta gggctaaatt tcaaggtgag aaatcaatct     900

cctcgctggg aagagtcagt gaagttgatt acggccctgc aattctttcg cttcaatatc     960

attacgatag ctttgagcga ctttatccat atgttggggt tggtgttggt cgggtgctat    1020

tttttgataa aaccgacggt gctttgagtt cgtttgatat taaggataaa tgggcgcctg    1080

cttttcaggt tggccttaga tatgaccttg gtaactcatg gatgctaaat tcagatgtgc    1140

gttatattcc tttcaaaacg gacgtcacag gtactcttgg cccggttcct gtttctacta    1200

aaattgaggt tgatcctttc attctcagtc ttggtgcgtc atatgttttc taaggtaccc    1260

tcgagtctgg tggatccaat tgtgagcgga taacaattac gagcttcatg cacagtgatc    1320

gacgctgttg acaattaatc atcggctcgt ataatgtgtg gatgtggaat tgtgagcgct    1380

cacaattcca caacggtttc cctctagaaa taattttgtt taacaggagg taaaacatat    1440

gctggttctg cacaacagcc aaaaactgca aattctgtac aaatctctgg aaaaatctat    1500

tcctgaaagc attaaagttt atggtgcgat cttcaacatc aaggataaaa accctttcaa    1560

tatggaggtg ctggtcgacg cgtggcctga ttaccaaatc gtcatcaccc gtccgcagaa    1620

gcaagaaatg aaagatgacc aggaccacta tactaacacc taccacatct tcactaaagc    1680

gccggacaaa ctggaagaag ttctgagcta ctccaatgtt atctcctggg aacaaacgct 1740

gcagattcaa ggttgccagg aaggtctgga tgaggcgatt cgtaaggtcg ctacctctaa 1800

gtccgttcaa gtcgattaca tgaaaaccat cctgtttatc ccggagctgc cgaagaaaca 1860

caaaacctcc tctaacgaca agatggagct gttcgaagta gacgatgaca acaaagaggg 1920

taacttttcc aatatgttcc tggacgcctc ccacgccggt ctggttaatg agcactgggc 1980

gttcggtaaa aacgaacgct ccctgaagta cattgagcgt tgtctgcagg atttctgggg 2040

ttttggtgtc ctgggtccag agggtcaact ggtctcttgg atcgttatgg aacagtcttg 2100

tgaactgcgt atgggttata ctgtgccaaa ataccgtcac caaggtaata tgctgcaaat 2160

cggttatcat ctggagaagt acctgagcca gaaggaaatc ccgttctatt tccatgttgc 2220

cgataataac gaaaagagcc tgcaagccct gaacaatctg ggcttcaaga tctgcccttg 2280

cggttggcac cagtggaagt gcacgcctaa aaagtactgt taagcgttaa gtctgagagg 2340

tgaagaattg aagaaggttt ggcttaaccg ttatcccgcg gacgttccga cggagatcaa 2400

ccctgaccgt tatcaatctc tggtagatat gtttgagcag tcggtcgcgc gctacgccga 2460

tcaacctgcg tttgtgaata tgggggaggt aatgaccttc cgcaagctgg aagaacgcag 2520

tcgcgcgttt gccgcttatt tgcaacaagg gttggggctg aagaaaggcg atcgcgttgc 2580

gttgatgatg cctaatttat tgcaatatcc ggtggcgctg tttggcattt tgcgtgccgg 2640

gatgatcgtc gtaaacgtta acccgttgta taccccgcgt gagcttgagc atcagcttaa 2700

cgatagcggc gcatcggcga ttgttatcgt gtctaacttt gctcacacac tggaaaaagt 2760

ggttgataaa accgccgttc agcacgtaat tctgacccgt atgggcgatc agctatctac 2820

ggcaaaaggc acggtagtca atttcgttgt taaatacatc aagcgtttgg tgccgaaata 2880

ccatctgcca gatgccattt catttcgtag cgcactgcat aacggctacc ggatgcagta 2940

cgtcaaaccc gaactggtgc cggaagattt agcttttctg caatacaccg gcggcaccac 3000

tggtgtggcg aaaggcgcga tgctgactca ccgcaatatg ctggcgaacc tggaacaggt 3060

taacgcgacc tatggtccgc tgttgcatcc gggcaaagag ctggtggtga cggcgctgcc 3120

gctgtatcac atttttgccc tgaccattaa ctgcctgctg tttatcgaac tgggtgggca 3180

gaacctgctt atcactaacc cgcgcgatat tccagggttg gtaaaagagt tagcgaaata 3240

tccgtttacc gctatcacgg gcgttaacac cttgttcaat gcgttgctga acaataaaga 3300

gttccagcag ctggatttct ccagtctgca tctttccgca ggcggtggga tgccagtgca 3360

gcaagtggtg gcagagcgtt gggtgaaact gaccggacag tatctgctgg aaggctatgg 3420

ccttaccgag tgtgcgccgc tggtcagcgt taacccatat gatattgatt atcatagtgg 3480

tagcatcggt ttgccggtgc cgtcgacgga agccaaactg gtggatgatg atgataatga 3540

agtaccacca ggtcaaccgg gtgagctttg tgtcaaagga ccgcaggtga tgctgggtta 3600

ctggcagcgt cccgatgcta ccgatgaaat catcaaaaat ggctggttac acaccggcga 3660

catcgcggta atggatgaag aaggattcct gcgcattgtc gatcgtaaaa aagacatgat 3720

tctggtttcc ggttttaacg tctatcccaa cgagattgaa gatgtcgtca tgcagcatcc 3780

tggcgtacag gaagtcgcgg ctgttggcgt accttccggc tccagtggtg aagcggtgaa 3840

aatcttcgta gtgaaaaaag atccatcgct taccgaagag tcactggtga ctttttgccg 3900

ccgtcagctc acgggataca aagtaccgaa gctggtggag tttcgtgatg agttaccgaa 3960

atctaacgtc ggaaaaattt tgcgacgaga attacgtgac gaagcgcgcg gcaaagtgga 4020

caataaagcc tgagcgttaa gtcagtcgtc agacgccggt taatccggcg ttttttttga 4080

cgcccactaa agagaaaaca atctcgagtc tggtaaagaa accgctgctg cgaaatttga 4140

acgccagcac atggactcgt ctactagcgc agcttaatta acctaggctg ctgccaccgc 4200

tgagcaataa ctagcataac cccttggggc ctctaaacgg gtcttgaggg gttttttgct 4260

gaaacctcag gcatttgaga agcacacggt cacactgctt ccggtagtca ataaaccggt 4320

aaaccagcaa tagacataag cggctattta acgaccctgc cctgaaccga cgaccgggtc 4380

atcgtggccg gatcttgcgg cccctcggct tgaacgaatt gttagacatt atttgccgac 4440

taccttggtg atctcgcctt tcacgtagtg gacaaattct tccaactgat ctgcgcgcga 4500

ggccaagcga tcttcttctt gtccaagata agcctgtcta gcttcaagta tgacgggctg 4560

atactgggcc ggcaggcgct ccattgccca gtcggcagcg acatccttcg gcgcgatttt 4620

gccggttact gcgctgtacc aaatgcggga caacgtaagc actacatttc gctcatcgcc 4680

agcccagtcg ggcggcgagt tccatagcgt taaggtttca tttagcgcct caaatagatc 4740

ctgttcagga accggatcaa agagttcctc cgccgctgga cctaccaagg caacgctatg 4800

ttctcttgct tttgtcagca agatagccag atcaatgtcg atcgtggctg gctcgaagat 4860

acctgcaaga atgtcattgc gctgccattc tccaaattgc agttcgcgct tagctggata 4920

acgccacgga atgatgtcgt cgtgcacaac aatggtgact tctacagcgc ggagaatctc 4980

gctctctcca ggggaagccg aagtttccaa aaggtcgttg atcaaagctc gccgcgttgt 5040

ttcatcaagc cttacggtca ccgtaaccag caaatcaata tcactgtgtg gcttcaggcc 5100

gccatccact gcggagccgt acaaatgtac ggccagcaac gtcggttcga gatggcgctc 5160

gatgacgcca actacctctg atagttgagt cgatacttcg gcgatcaccg cttccctcat 5220

actcttcctt tttcaatatt attgaagcat ttatcagggt tattgtctca tgagcggata 5280

catatttgaa tgtatttaga aaaataaaca aatagctagc tcactcggtc gctacgctcc 5340

gggcgtgaga ctgcggcggg cgctgcggac acatacaaag ttacccacag attccgtgga 5400

taagcagggg actaacatgt gaggcaaaac agcagggccg cgccggtggc gtttttccat 5460

aggctccgcc ctcctgccag agttcacata aacagacgct tttccggtgc atctgtggga 5520

gccgtgaggc tcaaccatga atctgacagt acgggcgaaa cccgacagga cttaaagatc 5580

cccaccgttt ccggcgggtc gctccctctt gcgctctcct gttccgaccc tgccgtttac 5640

cggatacctg ttccgccttt ctcccttacg ggaagtgtgg cgctttctca tagctcacac 5700

actggtatct cggctcggtg taggtcgttc gctccaagct gggctgtaag caagaactcc 5760

ccgttcagcc cgactgctgc gccttatccg gtaactgttc acttgagtcc aacccggaaa 5820

agcacggtaa aacgccactg gcagcagcca ttggtaactg ggagttcgca gaggatttgt 5880

ttagctaaac acgcggttgc tcttgaagtg tgcgccaaag tccggctaca ctggaaggac 5940

agatttggtt gctgtgctct gcgaaagcca gttaccacgg ttaagcagtt ccccaactga 6000

cttaaccttc gatcaaacca cctccccagg tggttttttc gtttacaggg caaaagatta 6060

cgcgcagaaa aaaaggatct caagaagatc ctttgatctt ttctactgaa ccgctctaga 6120

tttcagtgca atttatctct tcaaatgtag cacctgaagt cagccccata cgatataagt 6180

tgtaattctc atgttagtca tgccccgcgc ccaccggaag gagctgactg ggttgaaggc 6240

tctcaagggc atcggtcgag atcccggtgc ctaatgagtg agctaactta cattaattgc 6300

gttgcgctca ctgcccgctt tccagtcggg aaacctgtcg tgccagctgc attaatgaat 6360

cggccaacgc gcggggagag gcggtttgcg tattgggcgc cagggtggtt tttcttttca 6420

ccagtgagac gggcaacagc tgattgccct tcaccgcctg gccctgagag agttgcagca 6480

agcggtccac gctggtttgc cccagcaggc gaaaatcctg tttgatggtg gttaacggcg 6540

ggatataaca tgagctgtct tcggtatcgt cgtatcccac taccgagatg tccgcaccaa 6600

cgcgcagccc ggactcggta atggcgcgca ttgcgcccag cgccatctga tcgttggcaa 6660

ccagcatcgc agtgggaacg atgccctcat tcagcatttg catggtttgt tgaaaaccgg 6720

acatggcact ccagtcgcct tcccgttccg ctatcggctg aatttgattg cgagtgagat 6780

atttatgcca gccagccaga cgcagacgcg ccgagacaga acttaatggg cccgctaaca 6840

gcgcgatttg ctggtgaccc aatgcgacca gatgctccac gcccagtcgc gtaccgtctt 6900

catgggagaa aataatactg ttgatgggtg tctggtcaga gacatcaaga aataacgccg 6960

gaacattagt gcaggcagct tccacagcaa tggcatcctg gtcatccagc ggatagttaa 7020

tgatcagccc actgacgcgt tgcgcgagaa gattgtgcac cgccgcttta caggcttcga 7080

cgccgcttcg ttctaccatc gacaccacca cgctggcacc cagttgatcg gcgcgagatt 7140

taatcgccgc gacaatttgc gacggcgcgt gcagggccag actggaggtg gcaacgccaa 7200

tcagcaacga ctgtttgccc gccagttgtt gtgccacgcg gttgggaatg taattcagct 7260

ccgccatcgc cgcttccact ttttcccgcg ttttcgcaga aacgtggctg gcctggttca 7320

ccacgcggga aacggtctga taagagacac cggcatactc tgcgacatcg tataacgtta 7380

ctggtttcac attcaccacc ctgaattgac tctcttccgg gcgctatcat gccataccgc 7440

gaaaggtttt gcgccattcg atggtgtc 7468

<210> 14
<211> 5367
<212> DNA
<213> Artificial Sequence

<220>
<223> Vector pET-28b

<220>
<221> misc_feature
<222> (1)..(5367)
<223> Vector pET-28b

<400> 14
ggcgaatggg acgcgccctg tagcggcgca ttaagcgcgg cgggtgtggt ggttacgcgc 60

agcgtgaccg ctacacttgc cagcgcccta gcgcccgctc ctttcgcttt cttcccttcc 120

tttctcgcca cgttcgccgg ctttccccgt caagctctaa atcgggggct ccctttaggg 180

ttccgattta gtgctttacg gcacctcgac cccaaaaaac ttgattaggg tgatggttca 240

cgtagtgggc catcgccctg atagacggtt tttcgccctt tgacgttgga gtccacgttc 300

tttaatagtg gactcttgtt ccaaactgga acaacactca accctatctc ggtctattct 360

tttgatttat aagggatttt gccgatttcg gcctattggt taaaaaatga gctgatttaa 420

caaaaattta acgcgaattt taacaaaata ttaacgttta caatttcagg tggcactttt 480

cggggaaatg tgcgcggaac ccctatttgt ttatttttct aaatacattc aaatatgtat 540

ccgctcatga attaattctt agaaaaactc atcgagcatc aaatgaaact gcaatttatt 600

catatcagga ttatcaatac catatttttg aaaaagccgt ttctgtaatg aaggagaaaa 660

ctcaccgagg cagttccata ggatggcaag atcctggtat cggtctgcga ttccgactcg 720

tccaacatca atacaaccta ttaatttccc ctcgtcaaaa ataaggttat caagtgagaa 780

atcaccatga gtgacgactg aatccggtga gaatggcaaa agtttatgca tttctttcca 840

gacttgttca acaggccagc cattacgctc gtcatcaaaa tcactcgcat caaccaaacc 900

gttattcatt cgtgattgcg cctgagcgag acgaaatacg cgatcgctgt taaaaggaca 960

attacaaaca ggaatcgaat gcaaccggcg caggaacact gccagcgcat caacaatatt 1020

ttcacctgaa tcaggatatt cttctaatac ctggaatgct gttttcccgg ggatcgcagt 1080

ggtgagtaac catgcatcat caggagtacg gataaaatgc ttgatggtcg gaagaggcat 1140

aaattccgtc agccagttta gtctgaccat ctcatctgta acatcattgg caacgctacc 1200

tttgccatgt ttcagaaaca actctggcgc atcgggcttc ccatacaatc gatagattgt 1260

cgcacctgat tgcccgacat tatcgcgagc ccatttatac ccatataaat cagcatccat 1320

gttggaattt aatcgcggcc tagagcaaga cgtttcccgt tgaatatggc tcataacacc 1380

ccttgtatta ctgtttatgt aagcagacag ttttattgtt catgaccaaa atcccttaac 1440

gtgagttttc gttccactga gcgtcagacc ccgtagaaaa gatcaaagga tcttcttgag 1500

atcctttttt tctgcgcgta atctgctgct tgcaaacaaa aaaaccaccg ctaccagcgg 1560

tggtttgttt gccggatcaa gagctaccaa ctctttttcc gaaggtaact ggcttcagca 1620

gagcgcagat accaaatact gtccttctag tgtagccgta gttaggccac cacttcaaga 1680

actctgtagc accgcctaca tacctcgctc tgctaatcct gttaccagtg gctgctgcca 1740

gtggcgataa gtcgtgtctt accgggttgg actcaagacg atagttaccg gataaggcgc 1800

agcggtcggg ctgaacgggg ggttcgtgca cacagcccag cttggagcga acgacctaca 1860

ccgaactgag atacctacag cgtgagctat gagaaagcgc cacgcttccc gaagggagaa 1920

aggcggacag gtatccggta agcggcaggg tcggaacagg agagcgcacg agggagcttc 1980

cagggggaaa cgcctggtat ctttatagtc ctgtcgggtt tcgccacctc tgacttgagc 2040

gtcgattttt gtgatgctcg tcaggggggc ggagcctatg gaaaaacgcc agcaacgcgg 2100

cctttttacg gttcctggcc ttttgctggc cttttgctca catgttcttt cctgcgttat 2160

cccctgattc tgtggataac cgtattaccg cctttgagtg agctgatacc gctcgccgca 2220

gccgaacgac cgagcgcagc gagtcagtga gcgaggaagc ggaagagcgc ctgatgcggt 2280

attttctcct tacgcatctg tgcggtattt cacaccgcat atatggtgca ctctcagtac 2340

aatctgctct gatgccgcat agttaagcca gtatacactc cgctatcgct acgtgactgg 2400

gtcatggctg cgccccgaca cccgccaaca cccgctgacg cgccctgacg ggcttgtctg 2460

ctcccggcat ccgcttacag acaagctgtg accgtctccg ggagctgcat gtgtcagagg 2520

ttttcaccgt catcaccgaa acgcgcgagg cagctgcggt aaagctcatc agcgtggtcg 2580

tgaagcgatt cacagatgtc tgcctgttca tccgcgtcca gctcgttgag tttctccaga 2640

agcgttaatg tctggcttct gataaagcgg gccatgttaa gggcggtttt ttcctgtttg 2700

gtcactgatg cctccgtgta agggggattt ctgttcatgg gggtaatgat accgatgaaa 2760

cgagagagga tgctcacgat acgggttact gatgatgaac atgcccggtt actggaacgt 2820

tgtgagggta aacaactggc ggtatggatg cggcgggacc agagaaaaat cactcagggt 2880

caatgccagc gcttcgttaa tacagatgta ggtgttccac agggtagcca gcagcatcct 2940

gcgatgcaga tccggaacat aatggtgcag ggcgctgact tccgcgtttc cagactttac 3000

gaaacacgga aaccgaagac cattcatgtt gttgctcagg tcgcagacgt tttgcagcag 3060

cagtcgcttc acgttcgctc gcgtatcggt gattcattct gctaaccagt aaggcaaccc 3120

cgccagccta gccgggtcct caacgacagg agcacgatca tgcgcacccg tggggccgcc 3180

atgccggcga taatggcctg cttctcgccg aaacgtttgg tggcgggacc agtgacgaag 3240

gcttgagcga gggcgtgcaa gattccgaat accgcaagcg acaggccgat catcgtcgcg 3300

ctccagcgaa agcggtcctc gccgaaaatg acccagagcg ctgccggcac ctgtcctacg 3360

agttgcatga taaagaagac agtcataagt gcggcgacga tagtcatgcc ccgcgcccac 3420

cggaaggagc tgactgggtt gaaggctctc aagggcatcg gtcgagatcc cggtgcctaa 3480

tgagtgagct aacttacatt aattgcgttg cgctcactgc ccgctttcca gtcgggaaac 3540

ctgtcgtgcc agctgcatta atgaatcggc caacgcgcgg ggagaggcgg tttgcgtatt 3600

gggcgccagg gtggtttttc ttttcaccag tgagacgggc aacagctgat tgcccttcac 3660

cgcctggccc tgagagagtt gcagcaagcg gtccacgctg gtttgcccca gcaggcgaaa 3720

atcctgtttg atggtggtta acggcgggat ataacatgag ctgtcttcgg tatcgtcgta 3780

tcccactacc gagatatccg caccaacgcg cagcccggac tcggtaatgg cgcgcattgc 3840

gcccagcgcc atctgatcgt tggcaaccag catcgcagtg ggaacgatgc cctcattcag 3900

catttgcatg gtttgttgaa aaccggacat ggcactccag tcgccttccc gttccgctat 3960

cggctgaatt tgattgcgag tgagatattt atgccagcca gccagacgca gacgcgccga 4020

gacagaactt aatgggcccg ctaacagcgc gatttgctgg tgacccaatg cgaccagatg 4080

ctccacgccc agtcgcgtac cgtcttcatg ggagaaaata atactgttga tgggtgtctg 4140

gtcagagaca tcaagaaata acgccggaac attagtgcag gcagcttcca cagcaatggc 4200

atcctggtca tccagcggat agttaatgat cagcccactg acgcgttgcg cgagaagatt 4260

gtgcaccgcc gctttacagg cttcgacgcc gcttcgttct accatcgaca ccaccacgct 4320

ggcacccagt tgatcggcgc gagatttaat cgccgcgaca atttgcgacg gcgcgtgcag 4380

ggccagactg gaggtggcaa cgccaatcag caacgactgt ttgcccgcca gttgttgtgc 4440

cacgcggttg ggaatgtaat tcagctccgc catcgccgct tccacttttt cccgcgtttt 4500

cgcagaaacg tggctggcct ggttcaccac gcgggaaacg gtctgataag agacaccggc 4560

atactctgcg acatcgtata acgttactgg tttcacattc accaccctga attgactctc 4620

ttccgggcgc tatcatgcca taccgcgaaa ggttttgcgc cattcgatgg tgtccgggat 4680

ctcgacgctc tcccttatgc gactcctgca ttaggaagca gcccagtagt aggttgaggc 4740

cgttgagcac cgccgccgca aggaatggtg catgcaagga gatggcgccc aacagtcccc 4800

cggccacggg gcctgccacc atacccacgc cgaaacaagc gctcatgagc ccgaagtggc 4860

gagcccgatc ttccccatcg gtgatgtcgg cgatataggc gccagcaacc gcacctgtgg 4920

cgccggtgat gccggccacg atgcgtccgg cgtagaggat cgagatctcg atcccgcgaa 4980

attaatacga ctcactatag gggaattgtg agcggataac aattcccctc tagaaataat 5040

tttgtttaac tttaagaagg agatatacca tgggcagcag ccatcatcat catcatcaca 5100

gcagcggcct ggtgccgcgc ggcagccata tggctagcat gactggtgga cagcaaatgg 5160

gtcgggatcc gaattcgagc tccgtcgaca agcttgcggc cgcactcgag caccaccacc 5220

accaccactg agatccggct gctaacaaag cccgaaagga agctgagttg gctgctgcca 5280

ccgctgagca ataactagca taaccccttg gggcctctaa acgggtcttg aggggttttt 5340

tgctgaaagg aggaactata tccggat 5367

<210> 15
<211> 6432
<212> DNA
<213> Artificial Sequence

<220>
<223> Vector pET-28b{Ptac}[hGLYAT2(co_Ec)]

<220>
<221> misc_feature
<222> (1)..(6432)
<223> Vector pET-28b{Ptac}[hGLYAT2(co_Ec)]

<400> 15
ggccgctctg gtggatccaa ttgtgagcgg ataacaatta cgagcttcat gcacagtgat 60

cgacgctgtt gacaattaat catcggctcg tataatgtgt ggatgtggaa ttgtgagcgc 120

tcacaattcc acaacggttt ccctctagaa ataattttgt ttaacaggag gtaaaacata 180

tgctggttct gcacaacagc caaaaactgc aaattctgta caaatctctg gaaaaatcta 240

ttcctgaaag cattaaagtt tatggtgcga tcttcaacat caaggataaa aaccctttca 300

atatggaggt gctggtcgac gcgtggcctg attaccaaat cgtcatcacc cgtccgcaga 360

agcaagaaat gaaagatgac caggaccact atactaacac ctaccacatc ttcactaaag 420

cgccggacaa actggaagaa gttctgagct actccaatgt tatctcctgg gaacaaacgc 480

tgcagattca aggttgccag gaaggtctgg atgaggcgat tcgtaaggtc gctacctcta 540

agtccgttca agtcgattac atgaaaacca tcctgtttat cccggagctg ccgaagaaac 600

acaaaacctc ctctaacgac aagatggagc tgttcgaagt agacgatgac aacaaagagg 660

gtaacttttc caatatgttc ctggacgcct cccacgccgg tctggttaat gagcactggg 720

cgttcggtaa aaacgaacgc tccctgaagt acattgagcg ttgtctgcag gattttctgg 780

gttttggtgt cctgggtcca gagggtcaac tggtctcttg gatcgttatg gaacagtctt 840

gtgaactgcg tatgggttat actgtgccaa aataccgtca ccaaggtaat atgctgcaaa 900

tcggttatca tctggagaag tacctgagcc agaaggaaat cccgttctat ttccatgttg 960

ccgataataa cgaaaagagc ctgcaagccc tgaacaatct gggcttcaag atctgccctt 1020

gcggttggca ccagtggaag tgcacgccta aaaagtactg ttaagcgtta agtctgagag 1080

gtgagagctc gaattcggat cccgacccat ttgctgtcca ccagtcatgc tagccatatg 1140

gctgccgcgc ggcaccaggc cgctgctgtg atgatgatga tgatggctgc tgcccatggt 1200

atatctcctt cttaaagtta aacaaaatta tttctagagg ggaattgtta tccgctcaca 1260

attcccctat agtgagtcgt attaatttcg cgggatcgag atctcgatcc tctacgccgg 1320

acgcatcgtg gccggcatca ccggcgccac aggtgcggtt gctggcgcct atatcgccga 1380

catcaccgat ggggaagatc gggctcgcca cttcgggctc atgagcgctt gtttcggcgt 1440

gggtatggtg gcaggccccg tggccggggg actgttgggc gccatctcct tgcatgcacc 1500

attccttgcg gcggcggtgc tcaacggcct caacctacta ctgggctgct tcctaatgca 1560

ggagtcgcat aagggagagc gtcgagatcc cggacaccat cgaatggcgc aaaacctttc 1620

gcggtatggc atgatagcgc ccggaagaga gtcaattcag ggtggtgaat gtgaaaccag 1680

taacgttata cgatgtcgca gagtatgccg gtgtctctta tcagaccgtt tcccgcgtgg 1740

tgaaccaggc cagccacgtt tctgcgaaaa cgcgggaaaa agtggaagcg gcgatggcgg 1800

agctgaatta cattcccaac cgcgtggcac aacaactggc gggcaaacag tcgttgctga 1860

ttggcgttgc cacctccagt ctggccctgc acgcgccgtc gcaaattgtc gcggcgatta 1920

aatctcgcgc cgatcaactg ggtgccagcg tggtggtgtc gatggtagaa cgaagcggcg 1980

tcgaagcctg taaagcggcg gtgcacaatc ttctcgcgca acgcgtcagt gggctgatca 2040

ttaactatcc gctggatgac caggatgcca ttgctgtgga agctgcctgc actaatgttc 2100

cggcgttatt tcttgatgtc tctgaccaga cacccatcaa cagtattatt ttctcccatg 2160

aagacggtac gcgactgggc gtggagcatc tggtcgcatt gggtcaccag caaatcgcgc 2220

tgttagcggg cccattaagt tctgtctcgg cgcgtctgcg tctggctggc tggcataaat 2280

atctcactcg caatcaaatt cagccgatag cggaacggga aggcgactgg agtgccatgt 2340

ccggttttca acaaaccatg caaatgctga atgagggcat cgttcccact gcgatgctgg 2400

ttgccaacga tcagatggcg ctgggcgcaa tgcgcgccat taccgagtcc gggctgcgcg 2460

ttggtgcgga tatctcggta gtgggatacg acgataccga agacagctca tgttatatcc 2520

cgccgttaac caccatcaaa caggattttc gcctgctggg gcaaaccagc gtggaccgct 2580

tgctgcaact ctctcagggc caggcggtga agggcaatca gctgttgccc gtctcactgg 2640

tgaaaagaaa aaccaccctg gcgcccaata cgcaaaccgc ctctccccgc gcgttggccg 2700

attcattaat gcagctggca cgacaggttt cccgactgga aagcgggcag tgagcgcaac 2760

gcaattaatg taagttagct cactcattag gcaccgggat ctcgaccgat gcccttgaga 2820

gccttcaacc cagtcagctc cttccggtgg gcgcggggca tgactatcgt cgccgcactt 2880

atgactgtct tctttatcat gcaactcgta ggacaggtgc cggcagcgct ctgggtcatt 2940

ttcggcgagg accgctttcg ctggagcgcg acgatgatcg gcctgtcgct tgcggtattc 3000

ggaatcttgc acgccctcgc tcaagccttc gtcactggtc ccgccaccaa acgtttcggc 3060

gagaagcagg ccattatcgc cggcatggcg gccccacggg tgcgcatgat cgtgctcctg 3120

tcgttgagga cccggctagg ctggcggggt tgccttactg gttagcagaa tgaatcaccg 3180

atacgcgagc gaacgtgaag cgactgctgc tgcaaaacgt ctgcgacctg agcaacaaca 3240

tgaatggtct tcggtttccg tgtttcgtaa agtctggaaa cgcggaagtc agcgccctgc 3300

accattatgt tccggatctg catcgcagga tgctgctggc taccctgtgg aacacctaca 3360

tctgtattaa cgaagcgctg gcattgaccc tgagtgattt ttctctggtc ccgccgcatc 3420

cataccgcca gttgtttacc ctcacaacgt tccagtaacc gggcatgttc atcatcagta 3480

acccgtatcg tgagcatcct ctctcgtttc atcggtatca ttacccccat gaacagaaat 3540

cccccttaca cggaggcatc agtgaccaaa caggaaaaaa ccgcccttaa catggcccgc 3600

tttatcagaa gccagacatt aacgcttctg gagaaactca acgagctgga cgcggatgaa 3660

caggcagaca tctgtgaatc gcttcacgac cacgctgatg agctttaccg cagctgcctc 3720

gcgcgtttcg gtgatgacgg tgaaaacctc tgacacatgc agctcccgga gacggtcaca 3780

gcttgtctgt aagcggatgc cgggagcaga caagcccgtc agggcgcgtc agcgggtgtt 3840

ggcgggtgtc ggggcgcagc catgacccag tcacgtagcg atagcggagt gtatactggc 3900

ttaactatgc ggcatcagag cagattgtac tgagagtgca ccatatatgc ggtgtgaaat 3960

accgcacaga tgcgtaagga gaaaataccg catcaggcgc tcttccgctt cctcgctcac 4020

tgactcgctg cgctcggtcg ttcggctgcg gcgagcggta tcagctcact caaaggcggt 4080

aatacggtta tccacagaat caggggataa cgcaggaaag aacatgtgag caaaaggcca 4140

gcaaaaggcc aggaaccgta aaaaggccgc gttgctggcg tttttccata ggctccgccc 4200

ccctgacgag catcacaaaa atcgacgctc aagtcagagg tggcgaaacc cgacaggact 4260

ataaagatac caggcgtttc cccctggaag ctccctcgtg cgctctcctg ttccgaccct 4320

gccgcttacc ggatacctgt ccgcctttct cccttcggga agcgtggcgc tttctcatag 4380

ctcacgctgt aggtatctca gttcggtgta ggtcgttcgc tccaagctgg gctgtgtgca 4440

cgaacccccc gttcagcccg accgctgcgc cttatccggt aactatcgtc ttgagtccaa 4500

cccggtaaga cacgacttat cgccactggc agcagccact ggtaacagga ttagcagagc 4560

gaggtatgta ggcggtgcta cagagttctt gaagtggtgg cctaactacg gctacactag 4620

aaggacagta tttggtatct gcgctctgct gaagccagtt accttcggaa aaagagttgg 4680

tagctcttga tccggcaaac aaaccaccgc tggtagcggt ggtttttttg tttgcaagca 4740

gcagattacg cgcagaaaaa aaggatctca agaagatcct ttgatctttt ctacggggtc 4800

tgacgctcag tggaacgaaa actcacgtta agggattttg gtcatgaaca ataaaactgt 4860

ctgcttacat aaacagtaat acaaggggtg ttatgagcca tattcaacgg gaaacgtctt 4920

gctctaggcc gcgattaaat tccaacatgg atgctgattt atatgggtat aaatgggctc 4980

gcgataatgt cgggcaatca ggtgcgacaa tctatcgatt gtatgggaag cccgatgcgc 5040

cagagttgtt tctgaaacat ggcaaaggta gcgttgccaa tgatgttaca gatgagatgg 5100

tcagactaaa ctggctgacg gaatttatgc ctcttccgac catcaagcat tttatccgta 5160

ctcctgatga tgcatggtta ctcaccactg cgatccccgg gaaaacagca ttccaggtat 5220

tagaagaata tcctgattca ggtgaaaata ttgttgatgc gctggcagtg ttcctgcgcc 5280

ggttgcattc gattcctgtt tgtaattgtc cttttaacag cgatcgcgta tttcgtctcg 5340

ctcaggcgca atcacgaatg aataacggtt tggttgatgc gagtgatttt gatgacgagc 5400

gtaatggctg gcctgttgaa caagtctgga aagaaatgca taaacttttg ccattctcac 5460

cggattcagt cgtcactcat ggtgatttct cacttgataa ccttattttt gacgagggga 5520

aattaatagg ttgtattgat gttggacgag tcggaatcgc agaccgatac caggatcttg 5580

ccatcctatg gaactgcctc ggtgagtttt ctccttcatt acagaaacgg ctttttcaaa 5640

aatatggtat tgataatcct gatatgaata aattgcagtt tcatttgatg ctcgatgagt 5700

ttttctaaga attaattcat gagcggatac atatttgaat gtatttagaa aaataaacaa 5760

ataggggttc cgcgcacatt tccccgaaaa gtgccacctg aaattgtaaa cgttaatatt 5820

ttgttaaaat tcgcgttaaa tttttgttaa atcagctcat tttttaacca ataggccgaa 5880

atcggcaaaa tcccttataa atcaaaagaa tagaccgaga tagggttgag tgttgttcca 5940

gtttggaaca agagtccact attaaagaac gtggactcca acgtcaaagg gcgaaaaacc 6000

gtctatcagg gcgatggccc actacgtgaa ccatcaccct aatcaagttt tttggggtcg 6060

aggtgccgta aagcactaaa tcggaaccct aaagggagcc cccgatttag agcttgacgg 6120

ggaaagccgg cgaacgtggc gagaaaggaa gggaagaaag cgaaaggagc gggcgctagg 6180

gcgctggcaa gtgtagcggt cacgctgcgc gtaaccacca cacccgccgc gcttaatgcg 6240

ccgctacagg gcgcgtccca ttcgccatcc ggatatagtt cctcctttca gcaaaaaacc 6300

cctcaagacc cgtttagagg ccccaagggg ttatgctagt tattgctcag cggtggcagc 6360

agccaactca gcttcctttc gggctttgtt agcagccgga tctcagtggt ggtggtggtg 6420

gtgctcgagt gc 6432

<210> 16
<211> 6418
<212> DNA
<213> Artificial Sequence

<220>

<223>  pET-28b{Ptac}[hGLYAT3(co_Ec)]

<220>
<221>  misc_feature
<222>  (1)..(6418)
<223>  pET-28b{Ptac}[hGLYAT3(co_Ec)]

<400>  16
ggccgccctt cattttggat ccaattgtga gcggataaca attacgagct tcatgcacag      60

tgatcgacgc tgttgacaat taatcatcgg ctcgtataat gtgtggatgt ggaattgtga      120

gcgctcacaa ttccacaacg gtttccctct agaaataatt ttgtttaaca ggaggtaaaa      180

catatgctgg ttctgaattg ctctactaaa ctgctgatcc tggagaaaat gctgaaatcc      240

tgtttcccgg aatctctgaa agtttatggt gcggtaatga atatcaaccg tggtaatccg      300

tttcaaaaag aagttgttct ggactcttgg ccagatttca aggcggttat cacccgtcgt      360

cagcgtgaag cggaaaccga caacctggac cactatacca acgcgtatgc ggttttctat      420

aaagatgttc gcgcgtaccg tcagctgctg gaagaatgcg atgttttcaa ctgggaccag      480

gtttttcaga tccaaggtct gcaatccgag ctgtacgacg tttctaaggc cgtggcgaac      540

tctaaacaac tgaacatcaa gctgacctct ttcaaagcgg ttcatttctc tccggtatcc      600

agcctgccgg acacctcctt cctgaaaggt ccgtctccgc gtctgaccta cctgtctgtt      660

gcgaatgcgg atctgctgaa ccgtacttgg tcccgtggtg gcaacgaaca gtgcctgcgt      720

tacattgcta acctgatctc ttgcttcccg agcgtctgcg ttcgtgatga aaaaggtaac      780

ccagtatctt ggtctatcac cgaccagttc gcgaccatgt gtcatggcta caccctgcct      840

gaacaccgtc gtaagggtta ctctcgtctg gttgcgctga ccctggcgcg taagctgcag      900

tctcgtggtt ttccgtccca gggtaacgtc ctggacgaca acactgcgtc catctccctg      960

ctgaagtccc tgcatgccga attcctgccg tgccgtttcc accgtctgat tctgacccca      1020

gcgaccttct ctggtctgcc gcacctgtaa gcgttaagtc tgagaggtga gagctcgaat      1080

tcggatcccg acccatttgc tgtccaccag tcatgctagc catatggctg ccgcgcggca      1140

ccaggccgct gctgtgatga tgatgatgat ggctgctgcc catggtatat ctccttctta      1200

aagttaaaca aaattatttc tagaggggaa ttgttatccg ctcacaattc ccctatagtg      1260

agtcgtatta atttcgcggg atcgagatct cgatcctcta cgccggacgc atcgtggccg      1320

gcatcaccgg cgccacaggt gcggttgctg gcgcctatat cgccgacatc accgatgggg      1380

aagatcgggc tcgccacttc gggctcatga gcgcttgttt cggcgtgggt atggtggcag      1440

gccccgtggc cgggggactg ttgggcgcca tctccttgca tgcaccattc cttgcggcgg      1500

cggtgctcaa cggcctcaac ctactactgg gctgcttcct aatgcaggag tcgcataagg      1560

gagagcgtcg agatcccgga caccatcgaa tggcgcaaaa cctttcgcgg tatggcatga      1620

tagcgcccgg aagagagtca attcagggtg gtgaatgtga aaccagtaac gttatacgat 1680

gtcgcagagt atgccggtgt ctcttatcag accgtttccc gcgtggtgaa ccaggccagc 1740

cacgtttctg cgaaaacgcg ggaaaaagtg gaagcggcga tggcggagct gaattacatt 1800

cccaaccgcg tggcacaaca actggcgggc aaacagtcgt tgctgattgg cgttgccacc 1860

tccagtctgg ccctgcacgc gccgtcgcaa attgtcgcgg cgattaaatc tcgcgccgat 1920

caactgggtg ccagcgtggt ggtgtcgatg gtagaacgaa gcggcgtcga agcctgtaaa 1980

gcggcggtgc acaatcttct cgcgcaacgc gtcagtgggc tgatcattaa ctatccgctg 2040

gatgaccagg atgccattgc tgtggaagct gcctgcacta atgttccggc gttatttctt 2100

gatgtctctg accagacacc catcaacagt attattttct cccatgaaga cggtacgcga 2160

ctgggcgtgg agcatctggt cgcattgggt caccagcaaa tcgcgctgtt agcgggccca 2220

ttaagttctg tctcggcgcg tctgcgtctg gctggctggc ataaatatct cactcgcaat 2280

caaattcagc cgatagcgga acgggaaggc gactggagtg ccatgtccgg ttttcaacaa 2340

accatgcaaa tgctgaatga gggcatcgtt cccactgcga tgctggttgc caacgatcag 2400

atggcgctgg gcgcaatgcg cgccattacc gagtccgggc tgcgcgttgg tgcggatatc 2460

tcggtagtgg gatacgacga taccgaagac agctcatgtt atatcccgcc gttaaccacc 2520

atcaaacagg attttcgcct gctggggcaa accagcgtgg accgcttgct gcaactctct 2580

cagggccagg cggtgaaggg caatcagctg ttgcccgtct cactggtgaa aagaaaaacc 2640

accctggcgc ccaatacgca aaccgcctct ccccgcgcgt tggccgattc attaatgcag 2700

ctggcacgac aggtttcccg actggaaagc gggcagtgag cgcaacgcaa ttaatgtaag 2760

ttagctcact cattaggcac cgggatctcg accgatgccc ttgagagcct tcaacccagt 2820

cagctccttc cggtgggcgc ggggcatgac tatcgtcgcc gcacttatga ctgtcttctt 2880

tatcatgcaa ctcgtaggac aggtgccggc agcgctctgg gtcattttcg gcgaggaccg 2940

ctttcgctgg agcgcgacga tgatcggcct gtcgcttgcg gtattcggaa tcttgcacgc 3000

cctcgctcaa gccttcgtca ctggtcccgc caccaaacgt ttcggcgaga agcaggccat 3060

tatcgccggc atggcggccc cacgggtgcg catgatcgtg ctcctgtcgt tgaggacccg 3120

gctaggctgg cggggttgcc ttactggtta gcagaatgaa tcaccgatac gcgagcgaac 3180

gtgaagcgac tgctgctgca aaacgtctgc gacctgagca acaacatgaa tggtcttcgg 3240

tttccgtgtt tcgtaaagtc tggaaacgcg gaagtcagcg ccctgcacca ttatgttccg 3300

gatctgcatc gcaggatgct gctggctacc ctgtggaaca cctacatctg tattaacgaa 3360

gcgctggcat tgaccctgag tgatttttct ctggtcccgc cgcatccata ccgccagttg 3420

tttaccctca caacgttcca gtaaccgggc atgttcatca tcagtaaccc gtatcgtgag 3480

catcctctct cgtttcatcg gtatcattac ccccatgaac agaaatcccc cttacacgga 3540

ggcatcagtg accaaacagg aaaaaaccgc ccttaacatg gcccgcttta tcagaagcca 3600

gacattaacg cttctggaga aactcaacga gctggacgcg gatgaacagg cagacatctg 3660

tgaatcgctt cacgaccacg ctgatgagct ttaccgcagc tgcctcgcgc gtttcggtga 3720

tgacggtgaa aacctctgac acatgcagct cccggagacg gtcacagctt gtctgtaagc 3780

ggatgccggg agcagacaag cccgtcaggg cgcgtcagcg ggtgttggcg ggtgtcgggg 3840

cgcagccatg acccagtcac gtagcgatag cggagtgtat actggcttaa ctatgcggca 3900

tcagagcaga ttgtactgag agtgcaccat atatgcggtg tgaaataccg cacagatgcg 3960

taaggagaaa ataccgcatc aggcgctctt ccgcttcctc gctcactgac tcgctgcgct 4020

cggtcgttcg gctgcggcga gcggtatcag ctcactcaaa ggcggtaata cggttatcca 4080

cagaatcagg ggataacgca ggaaagaaca tgtgagcaaa aggccagcaa aaggccagga 4140

accgtaaaaa ggccgcgttg ctggcgtttt tccataggct ccgcccccct gacgagcatc 4200

acaaaaatcg acgctcaagt cagaggtggc gaaacccgac aggactataa agataccagg 4260

cgtttccccc tggaagctcc ctcgtgcgct ctcctgttcc gaccctgccg cttaccggat 4320

acctgtccgc ctttctccct tcgggaagcg tggcgctttc tcatagctca cgctgtaggt 4380

atctcagttc ggtgtaggtc gttcgctcca agctgggctg tgtgcacgaa ccccccgttc 4440

agcccgaccg ctgcgcctta tccggtaact atcgtcttga gtccaacccg gtaagacacg 4500

acttatcgcc actggcagca gccactggta acaggattag cagagcgagg tatgtaggcg 4560

gtgctacaga gttcttgaag tggtggccta actacggcta cactagaagg acagtatttg 4620

gtatctgcgc tctgctgaag ccagttacct tcggaaaaag agttggtagc tcttgatccg 4680

gcaaacaaac caccgctggt agcggtggtt tttttgtttg caagcagcag attacgcgca 4740

gaaaaaaagg atctcaagaa gatcctttga tcttttctac ggggtctgac gctcagtgga 4800

acgaaaactc acgttaaggg attttggtca tgaacaataa aactgtctgc ttacataaac 4860

agtaatacaa ggggtgttat gagccatatt caacgggaaa cgtcttgctc taggccgcga 4920

ttaaattcca acatggatgc tgatttatat gggtataaat gggctcgcga taatgtcggg 4980

caatcaggtg cgacaatcta tcgattgtat gggaagcccg atgcgccaga gttgtttctg 5040

aaacatggca aaggtagcgt tgccaatgat gttacagatg agatggtcag actaaactgg 5100

ctgacggaat ttatgcctct tccgaccatc aagcatttta tccgtactcc tgatgatgca 5160

tggttactca ccactgcgat ccccgggaaa acagcattcc aggtattaga agaatatcct 5220

gattcaggtg aaaatattgt tgatgcgctg gcagtgttcc tgcgccggtt gcattcgatt 5280

cctgtttgta attgtccttt taacagcgat cgcgtatttc gtctcgctca ggcgcaatca 5340

cgaatgaata acggtttggt tgatgcgagt gattttgatg acgagcgtaa tggctggcct 5400

gttgaacaag tctggaaaga aatgcataaa cttttgccat tctcaccgga ttcagtcgtc 5460

actcatggtg atttctcact tgataacctt atttttgacg aggggaaatt aataggttgt 5520

attgatgttg gacgagtcgg aatcgcagac cgataccagg atcttgccat cctatggaac 5580

tgcctcggtg agttttctcc ttcattacag aaacggcttt ttcaaaaata tggtattgat 5640

aatcctgata tgaataaatt gcagtttcat ttgatgctcg atgagttttt ctaagaatta 5700

attcatgagc ggatacatat ttgaatgtat ttagaaaaat aaacaaatag gggttccgcg 5760

cacatttccc cgaaaagtgc cacctgaaat tgtaaacgtt aatattttgt taaaattcgc 5820

gttaaatttt tgttaaatca gctcattttt taaccaatag gccgaaatcg gcaaaatccc 5880

ttataaatca aaagaataga ccgagatagg gttgagtgtt gttccagttt ggaacaagag 5940

tccactatta aagaacgtgg actccaacgt caaagggcga aaaaccgtct atcagggcga 6000

tggcccacta cgtgaaccat caccctaatc aagttttttg gggtcgaggt gccgtaaagc 6060

actaaatcgg aaccctaaag ggagcccccg atttagagct tgacggggaa agccggcgaa 6120

cgtggcgaga aaggaaggga agaaagcgaa aggagcgggc gctagggcgc tggcaagtgt 6180

agcggtcacg ctgcgcgtaa ccaccacacc cgccgcgctt aatgcgccgc tacagggcgc 6240

gtcccattcg ccatccggat atagttcctc ctttcagcaa aaaacccctc aagacccgtt 6300

tagaggcccc aaggggttat gctagttatt gctcagcggt ggcagcagcc aactcagctt 6360

cctttcgggc tttgttagca gccggatctc agtggtggtg gtggtggtgc tcgagtgc 6418

<210> 17
<211> 4334
<212> DNA
<213> Escherichia coli

<220>
<221> misc_feature
<222> (1)..(4334)
<223> pBMT-3_ccdAB

<400> 17
   ctgatggaca ggctgcgcct gcccacgagc ttgaccacag ggattgccca ccggctaccc 60
   agccttcgac cacataccca ccggctccaa ctgcgcggcc tgcggccttg ccccatcaat 120
   ttttttaatt ttctctgggg aaaagcctcc ggcctgcggc ctgcgcgctt cgcttgccgg 180
   ttggacacca agtggaaggc gggtcaaggc tcgcgcagcg accgcgcagc ggcttggcct 240
   tgacgcgcct ggaacgaccc aagcctatgc gagtgggggc agtcgaaggc gaagcccgcc 300
   cgcctgcccc ccgagcctca cggcggcgag tgcgggggtt ccaagggggc agcgccacct 360
   tgggcaaggc cgaaggccgc gcagtcgatc aacaagcccc ggaggggcca ctttttgccg 420
   gagggggagc cgcgccgaag gcgtggggga accccgcagg ggtgcccttc tttgggcacc 480
   aaagaactag atatagggcg aaatgcgaaa gacttaaaaa tcaacaactt aaaaaagggg 540
   ggtacgcaac agctcattgc ggcacccccc gcaatagctc attgcgtagg ttaaagaaaa 600
   tctgtaattg actgccactt ttacgcaacg cataattgtt gtcgcgctgc cgaaaagttg 660
   cagctgattg cgcatggtgc cgcaaccgtg cggcacccta ccgcatggag ataagcatgg 720
   ccacgcagtc cagagaaatc ggcattcaag ccaagaacaa gcccggtcac tgggtgcaaa 780
   cggaacgcaa agcgcatgag gcgtgggccg ggcttattgc gaggaaaccc acggcggcaa 840
   tgctgctgca tcacctcgtg gcgcagatgg gccaccagaa cgccgtggtg gtcagccaga 900
   agacactttc caagctcatc ggacgttctt tgcggacggt ccaatacgca gtcaaggact 960
   tggtggccga gcgctggatc tccgtcgtga agctcaacgg ccccggcacc gtgtcggcct 1020
   acgtggtcaa tgaccgcgtg gcgtggggcc agccccgcga ccagttgcgc ctgtcggtgt 1080
   tcagtgccgc cgtggtggtt gatcacgacg accaggacga atcgctgttg gggcatggcg 1140
   acctgcgccg catcccgacc ctgtatccgg gcgagcagca actaccgacc ggccccggcg 1200
   aggagccgcc cagccagccc ggcattccgg gcatggaacc agacctgcca gccttgaccg 1260
   aaacggagga atgggaacgg cgcgggcagc agcgcctgcc gatgcccgat gagccgtgtt 1320
   ttctggacga tggcgagccg ttggagccgc cgacacgggt cacgctgccg cgccggtagt 1380
   acgtacccgg aattgccagc tggggcgccc tctggtaagg ttgggaagcc ctgcaaagta 1440
   aactggatgg ctttcttgcc gccaaggatc tgatggcgca ggggatcaag ctctgatcaa 1500
   gagacaggat gaggatcgtt tcgatgaagc agcgtattac agtgacagtt gacagcgaca 1560
   gctatcagtt gctcaaggca tatgatgtca atatctccgg tctggtaagc acaaccatgc 1620
   agaatgaagc ccgtcgtctg cgtgccgaac gctggaaagc ggaaaatcag gaagggatgg 1680
   ctgaggtcgc ccggtttatt gaaatgaacg gctcttttgc tgacgagaac agggactggt 1740
   gaaatgcagt ttaaggttta cacctataaa agagagagcc gttatcgtct gtttgtggat 1800
   gtacagagcg atattattga cacgcccggg cgacggatgg tgatccccct ggccagtgca 1860
   cgtctgctgt cagataaagt ctcccgtgaa ctttacccgg tggtgcatat cggggatgaa 1920
   agctggcgca tgatgaccac cgatatggcc agtgtgccgg tctccgttat cggggaagaa 1980
   gtggctgatc tcagccaccg cgaaaatgac atcaaaaacg ccattaacct gatgttctgg 2040
   ggaatataaa tcctagacga attctctagt agaggttcca actttcacca taatgaaata 2100
   agatcactac cgggcgtatt ttttgagtta tcgagatttt caggagctaa ggaagctaaa 2160
   atggagaaaa aaatcactgg atataccacc gttgatatat cccaatggca tcgtaaagaa 2220
   cattttgagg catttcagtc agttgctcaa tgtacctata accagaccgt tcagctggat 2280
   attacggcct ttttaaagac cgtaaagaaa aataagcaca agttttatcc ggcctttatt 2340
   cacattcttg cccgcctgat gaatgctcat ccggaattcc gtatggcaat gaaagacggt 2400
   gagctggtga tatgggatag tgttcaccct tgttacaccg ttttccatga gcaaactgaa 2460
   acgttttcat cgctctggag tgaataccac gacgatttcc ggcagtttct acacatatat 2520
   tcgcaagatg tggcgtgtta cggtgaaaac ctggcctatt tccctaaagg gtttattgag 2580
   aatatgtttt tcgtctcagc caatccctgg gtgagtttca ccagttttga tttaaacgtg 2640
   gccaatatgg acaacttctt cgcccccgtt ttcaccatgg gcaaatatta tacgcaaggc 2700
   gacaaggtgc tgatgccgct ggcgattcag gttcatcatg ccgtttgtga tggcttccat 2760
   gtcggcagaa tgcttaatga attacaacag tactgcgatg agtggcaggg cggggcgtaa 2820
   agatctggat ccccctcaag tcaaaagcct ccggtcggag gcttttgact ttctgctatg 2880
   gaggtcaggt atgatttaaa tggtcagtat tgagcgatat ctagagaatt cgtcaacgaa 2940
   ttcaagcttg atatcattca ggacgagcct cagactccag cgtaactgga ctgaaaacaa 3000
   actaaagcgc ccttgtggcg ctttagtttt gttccgctca tgataataat ggtttcttag 3060
   acgtcaggtg gcacttttcg gggaaatgtg cgcgcccgcg ttcctgctgg cgctgggcct 3120
   gtttctggcg ctggacttcc cgctgttccg tcagcagctt ttcgcccacg gccttgatga 3180
   tcgcggcggc cttggcctgc atatcccgat tcaacggccc cagggcgtcc agaacgggct 3240
   tcaggcgctc ccgaaggtct cgggccgtct cttgggcttg atcggccttc ttgcgcatct 3300
   cacgcgctcc tgcggcggcc tgtagggcag gctcataccc ctgccgaacc gcttttgtca 3360
   gccggtcggc cacggcttcc ggcgtctcaa cgcgctttga gattcccagc ttttcggcca 3420
   atccctgcgg tgcataggcg cgtggctcga ccgcttgcgg gctgatggtg acgtggccca 3480
   ctggtggccg ctccagggcc tcgtagaacg cctgaatgcg cgtgtgacgt gccttgctgc 3540
   cctcgatgcc ccgttgcagc cctagatcgg ccacagcggc cgcaaacgtg gtctggtcgc 3600
   gggtcatctg cgctttgttg ccgatgaact ccttggccga cagcctgccg tcctgcgtca 3660
   gcggcaccac gaacgcggtc atgtgcgggc tggtttcgtc acggtggatg ctggccgtca 3720
   cgatgcgatc cgccccgtac ttgtccgcca gccacttgtg cgccttctcg aagaacgccg 3780
   cctgctgttc ttggctggcc gacttccacc attccgggct ggccgtcatg acgtactcga 3840
   ccgccaacac agcgtccttg cgccgcttct ctggcagcaa ctcgcgcagt cggcccatcg 3900
   cttcatcggt gctgctggcc gcccagtgct cgttctctgg cgtcctgctg gcgtcagcgt 3960
   tgggcgtctc gcgctcgcgg taggcgtgct tgagactggc cgccacgttg cccattttcg 4020
   ccagcttctt gcatcgcatg atcgcgtatg ccgccatgcc tgcccctccc ttttggtgtc 4080
   caaccggctc gacgggggca gcgcaaggcg gtgcctccgg cgggccactc aatgcttgag 4140
   tatactcact agactttgct tcgcaaagtc gtgaccgcct acggcggctg cggcgcccta 4200
   cgggcttgct ctccgggctt cgccctgcgc ggtcgctgcg ctcccttgcc agcccgtgga 4260
   tatgtggacg atggccgcga gcggccaccg gctggctcgc ttcgctcggc ccgtggacaa 4320
   ccctgctgga caag 4334

<210> 18
<211> 5024
<212> DNA
<213> Escherichia coli

<220>
<221> misc_feature
<222> (1)..(5024)
<223> pBMT-3_ccdAB_PT7-'tesA

<400> 18
caaaaaaccc ctcaagaccc gtttagaggc cccaaggggt tatgctagtt attgctcagc 60

ggtggcagca gcctaggtta attaagctgc gctagtagac gagtccatgt gctggcgttc 120

aaatttcgca gcagcggttt ctttaccaga ctcgaaaatt caggagtcgt ggttgaccag 180

cggctgcagt tgcttggcca tccagtcggc gatgaacggc tgcgcgtcgc ggttcgggtg 240

gatgccgtcg tcctgcatcc actgcggctt caggtagact tcctccatga agaacggcag 300

cagcggcacg tcgaattcct tggccagctt cgggtagatc gcggagaagg cctcgttgta 360

gcgacggccg tagttcgccg gcaggcggat ctgcatcagc agcggctcgg cgttcgcggc 420

cttcacgtcc tgcaggatct ggcgcagcgt ctgttcggtc tgctgcggct ggaagccgcg 480

caggccgtcg ttgccgccca gctcgaccag cacccagcgc ggctggtgct gcttcagcag 540

cgccggcagg cgggccaggc cctgctggct cgtgtcgccg ctgatcgagg cgttgaccac 600

cgaggtcttg gactgccact tgtcgttcag cagcgccggc cacgccgccg acgccgacat 660

gcggtagccc gccgacaggg agtcgcccag gatcagcagg gtgtccgccg ccatggtata 720

tctccttatt aaagttaaac aaaattattt ctacagggga attgttatcc gctcacaatt 780

cccctatagt gagtcgtatt aatttcctaa tgcaggagtc gatcattcag gacgagcctc 840

agactccagc gtaactggac tgaaaacaaa ctaaagcgcc cttgtggcgc tttagttttg 900

ttccgctcat gataataatg gtttcttaga cgtcaggtgg cacttttcgg ggaaatgtgc 960

gcgcccgcgt tcctgctggc gctgggcctg tttctggcgc tggacttccc gctgttccgt 1020

cagcagcttt tcgcccacgg ccttgatgat cgcggcggcc ttggcctgca tatcccgatt 1080

caacggcccc agggcgtcca gaacgggctt caggcgctcc cgaaggtctc gggccgtctc 1140

ttgggcttga tcggccttct tgcgcatctc acgcgctcct gcggcggcct gtagggcagg 1200

ctcatacccc tgccgaaccg cttttgtcag ccggtcggcc acggcttccg gcgtctcaac 1260

gcgctttgag attcccagct tttcggccaa tccctgcggt gcataggcgc gtggctcgac 1320

cgcttgcggg ctgatggtga cgtggcccac tggtggccgc tccagggcct cgtagaacgc 1380

ctgaatgcgc gtgtgacgtg ccttgctgcc ctcgatgccc cgttgcagcc ctagatcggc 1440

cacagcggcc gcaaacgtgg tctggtcgcg ggtcatctgc gctttgttgc cgatgaactc 1500

cttggccgac agcctgccgt cctgcgtcag cggcaccacg aacgcggtca tgtgcgggct 1560

ggtttcgtca cggtggatgc tggccgtcac gatgcgatcc gccccgtact tgtccgccag 1620

ccacttgtgc gccttctcga agaacgccgc ctgctgttct tggctggccg acttccacca 1680

ttccgggctg gccgtcatga cgtactcgac cgccaacaca gcgtccttgc gccgcttctc 1740

tggcagcaac tcgcgcagtc ggcccatcgc ttcatcggtg ctgctggccg cccagtgctc 1800

gttctctggc gtcctgctgg cgtcagcgtt gggcgtctcg cgctcgcggt aggcgtgctt 1860

gagactggcc gccacgttgc ccattttcgc cagcttcttg catcgcatga tcgcgtatgc 1920

cgccatgcct gcccctccct tttggtgtcc aaccggctcg acgggggcag cgcaaggcgg 1980

tgcctccggc gggccactca atgcttgagt atactcacta gactttgctt cgcaaagtcg 2040

tgaccgccta cggcggctgc ggcgccctac gggcttgctc tccgggcttc gccctgcgcg 2100

gtcgctgcgc tcccttgcca gcccgtggat atgtggacga tggccgcgag cggccaccgg 2160

ctggctcgct tcgctcggcc cgtggacaac cctgctggac aagctgatgg acaggctgcg 2220

cctgcccacg agcttgacca cagggattgc ccaccggcta cccagccttc gaccacatac 2280

ccaccggctc caactgcgcg gcctgcggcc ttgccccatc aattttttta attttctctg 2340

gggaaaagcc tccggcctgc ggcctgcgcg cttcgcttgc cggttggaca ccaagtggaa 2400

ggcgggtcaa ggctcgcgca gcgaccgcgc agcggcttgg ccttgacgcg cctggaacga 2460

cccaagccta tgcgagtggg ggcagtcgaa ggcgaagccc gcccgcctgc cccccgagcc 2520

tcacggcggc gagtgcgggg gttccaaggg ggcagcgcca ccttgggcaa ggccgaaggc 2580

cgcgcagtcg atcaacaagc cccggagggg ccactttttg ccggaggggg agccgcgccg 2640

aaggcgtggg ggaaccccgc aggggtgccc ttctttgggc accaaagaac tagatatagg 2700

gcgaaatgcg aaagacttaa aaatcaacaa cttaaaaaag gggggtacgc aacagctcat 2760

tgcggcaccc cccgcaatag ctcattgcgt aggttaaaga aaatctgtaa ttgactgcca 2820

cttttacgca acgcataatt gttgtcgcgc tgccgaaaag ttgcagctga ttgcgcatgg 2880

tgccgcaacc gtgcggcacc ctaccgcatg gagataagca tggccacgca gtccagagaa 2940

atcggcattc aagccaagaa caagcccggt cactgggtgc aaacggaacg caaagcgcat 3000

gaggcgtggg ccgggcttat tgcgaggaaa cccacggcgg caatgctgct gcatcacctc 3060

gtggcgcaga tgggccacca gaacgccgtg gtggtcagcc agaagacact ttccaagctc 3120

atcggacgtt ctttgcggac ggtccaatac gcagtcaagg acttggtggc cgagcgctgg 3180

atctccgtcg tgaagctcaa cggccccggc accgtgtcgg cctacgtggt caatgaccgc 3240

gtggcgtggg gccagccccg cgaccagttg cgcctgtcgg tgttcagtgc cgccgtggtg 3300

gttgatcacg acgaccagga cgaatcgctg ttggggcatg gcgacctgcg ccgcatcccg 3360

accctgtatc cgggcgagca gcaactaccg accggccccg gcgaggagcc gcccagccag 3420

cccggcattc cgggcatgga accagacctg ccagccttga ccgaaacgga ggaatgggaa 3480

cggcgcgggc agcagcgcct gccgatgccc gatgagccgt gttttctgga cgatggcgag 3540

ccgttggagc cgccgacacg ggtcacgctg ccgcgccggt agtacgtacc cggaattgcc 3600

agctggggcg ccctctggta aggttgggaa gccctgcaaa gtaaactgga tggctttctt 3660

gccgccaagg atctgatggc gcaggggatc aagctctgat caagagacag gatgaggatc 3720

gtttcgatga agcagcgtat tacagtgaca gttgacagcg acagctatca gttgctcaag 3780

gcatatgatg tcaatatctc cggtctggta agcacaacca tgcagaatga agcccgtcgt 3840

ctgcgtgccg aacgctggaa agcggaaaat caggaaggga tggctgaggt cgcccggttt 3900

attgaaatga acggctcttt tgctgacgag aacagggact ggtgaaatgc agtttaaggt 3960

ttacacctat aaaagagaga gccgttatcg tctgtttgtg gatgtacaga gcgatattat 4020

tgacacgccc gggcgacgga tggtgatccc cctggccagt gcacgtctgc tgtcagataa 4080

agtctcccgt gaactttacc cggtggtgca tatcggggat gaaagctggc gcatgatgac 4140

caccgatatg gccagtgtgc cggtctccgt tatcggggaa gaagtggctg atctcagcca 4200

ccgcgaaaat gacatcaaaa acgccattaa cctgatgttc tggggaatat aaatcctaga 4260

cgaattctct agtagaggtt ccaactttca ccataatgaa ataagatcac taccgggcgt 4320

attttttgag ttatcgagat tttcaggagc taaggaagct aaaatggaga aaaaaatcac 4380

tggatatacc accgttgata tatcccaatg gcatcgtaaa gaacattttg aggcatttca 4440

gtcagttgct caatgtacct ataaccagac cgttcagctg gatattacgg cctttttaaa 4500

gaccgtaaag aaaaataagc acaagtttta tccggccttt attcacattc ttgcccgcct 4560

gatgaatgct catccggaat tccgtatggc aatgaaagac ggtgagctgg tgatatggga 4620

tagtgttcac ccttgttaca ccgttttcca tgagcaaact gaaacgtttt catcgctctg 4680

gagtgaatac cacgacgatt tccggcagtt tctacacata tattcgcaag atgtggcgtg 4740

ttacggtgaa aacctggcct atttccctaa agggtttatt gagaatatgt ttttcgtctc 4800

agccaatccc tgggtgagtt tcaccagttt tgatttaaac gtggccaata tggacaactt 4860

cttcgccccc gttttcacca tgggcaaata ttatacgcaa ggcgacaagg tgctgatgcc 4920

gctggcgatt caggttcatc atgccgtttg tgatggcttc catgtcggca gaatgcttaa 4980

tgaattacaa cagtactgcg atgagtggca gggcggggcg taaa 5024

<210> 19
<211> 8268
<212> DNA

<213>  Escherichia coli

<220>
<221>  misc_feature
<222>  (1)..(8268)
<223>  pBMT-3_ccdAB_PT7-nphT7-hbd-crt-ter

<400>  19
ccttgccagc ccgtggatat gtggacgatg gccgcgagcg gccaccggct ggctcgcttc      60

gctcggcccg tggacaaccc tgctggacaa gctgatggac aggctgcgcc tgcccacgag     120

cttgaccaca gggattgccc accggctacc cagccttcga ccacataccc accggctcca     180

actgcgcggc ctgcggcctt gccccatcaa tttttttaat tttctctggg gaaaagcctc     240

cggcctgcgg cctgcgcgct tcgcttgccg gttggacacc aagtggaagg cgggtcaagg     300

ctcgcgcagc gaccgcgcag cggcttggcc ttgacgcgcc tggaacgacc caagcctatg     360

cgagtggggg cagtcgaagg cgaagcccgc ccgcctgccc cccgagcctc acggcggcga     420

gtgcgggggt tccaaggggg cagcgccacc ttgggcaagg ccgaaggccg cgcagtcgat     480

caacaagccc cggaggggcc actttttgcc ggagggggag ccgcgccgaa ggcgtggggg     540

aaccccgcag gggtgccctt ctttgggcac caaagaacta gatatagggc gaaatgcgaa     600

agacttaaaa atcaacaact taaaaaaggg gggtacgcaa cagctcattg cggcaccccc     660

cgcaatagct cattgcgtag gttaaagaaa atctgtaatt gactgccact tttacgcaac     720

gcataattgt tgtcgcgctg ccgaaaagtt gcagctgatt gcgcatggtg ccgcaaccgt     780

gcggcaccct accgcatgga gataagcatg gccacgcagt ccagagaaat cggcattcaa     840

gccaagaaca agcccggtca ctgggtgcaa acggaacgca aagcgcatga ggcgtgggcc     900

gggcttattg cgaggaaacc cacggcggca atgctgctgc atcacctcgt ggcgcagatg     960

ggccaccaga acgccgtggt ggtcagccag aagacacttt ccaagctcat cggacgttct    1020

ttgcggacgg tccaatacgc agtcaaggac ttggtggccg agcgctggat ctccgtcgtg    1080

aagctcaacg gccccggcac cgtgtcggcc tacgtggtca atgaccgcgt ggcgtggggc    1140

cagccccgcg accagttgcg cctgtcggtg ttcagtgccg ccgtggtggt tgatcacgac    1200

gaccaggacg aatcgctgtt ggggcatggc gacctgcgcc gcatcccgac cctgtatccg    1260

ggcgagcagc aactaccgac cggccccggc gaggagccgc ccagccagcc cggcattccg    1320

ggcatggaac cagacctgcc agccttgacc gaaacggagg aatgggaacg gcgcgggcag    1380

cagcgcctgc cgatgcccga tgagccgtgt tttctggacg atggcgagcc gttggagccg    1440

ccgacacggg tcacgctgcc gcgccggtag tacgtacccg gaattgccag ctggggcgcc    1500

ctctggtaag gttgggaagc cctgcaaagt aaactggatg gctttcttgc cgccaaggat    1560

ctgatggcgc aggggatcaa gctctgatca agagacagga tgaggatcgt ttcgatgaag    1620

cagcgtatta cagtgacagt tgacagcgac agctatcagt tgctcaaggc atatgatgtc 1680

aatatctccg gtctggtaag cacaaccatg cagaatgaag cccgtcgtct gcgtgccgaa 1740

cgctggaaag cggaaaatca ggaagggatg gctgaggtcg cccggtttat tgaaatgaac 1800

ggctcttttg ctgacgagaa cagggactgg tgaaatgcag tttaaggttt acacctataa 1860

aagagagagc cgttatcgtc tgtttgtgga tgtacagagc gatattattg acacgcccgg 1920

gcgacggatg gtgatccccc tggccagtgc acgtctgctg tcagataaag tctcccgtga 1980

actttacccg gtggtgcata tcggggatga aagctggcgc atgatgacca ccgatatggc 2040

cagtgtgccg gtctccgtta tcggggaaga agtggctgat ctcagccacc gcgaaaatga 2100

catcaaaaac gccattaacc tgatgttctg gggaatataa atcctagacg aattctctag 2160

tagaggttcc aactttcacc ataatgaaat aagatcacta ccgggcgtat tttttgagtt 2220

atcgagattt tcaggagcta aggaagctaa aatggagaaa aaaatcactg gatataccac 2280

cgttgatata tcccaatggc atcgtaaaga acattttgag gcatttcagt cagttgctca 2340

atgtacctat aaccagaccg ttcagctgga tattacggcc tttttaaaga ccgtaaagaa 2400

aaataagcac aagttttatc cggcctttat tcacattctt gcccgcctga tgaatgctca 2460

tccggaattc cgtatggcaa tgaaagacgg tgagctggtg atatgggata gtgttcaccc 2520

ttgttacacc gttttccatg agcaaactga aacgttttca tcgctctgga gtgaatacca 2580

cgacgatttc cggcagtttc tacacatata ttcgcaagat gtggcgtgtt acggtgaaaa 2640

cctggcctat ttccctaaag ggtttattga gaatatgttt ttcgtctcag ccaatccctg 2700

ggtgagtttc accagttttg atttaaacgt ggccaatatg gacaacttct tcgcccccgt 2760

tttcaccatg ggcaaatatt atacgcaagg cgacaaggtg ctgatgccgc tggcgattca 2820

ggttcatcat gccgtttgtg atggcttcca tgtcggcaga atgcttaatg aattacaaca 2880

gtactgcgat gagtggcagg gcggggcgta aaaaatccct tatgcgactc ctgcattagg 2940

aaattaatac gactcactat aggggaattg tgagcggata acaattcccc tgtagaaata 3000

attttgttta actttaataa ggagatatac catgacggat gtccgctttc gtatcattgg 3060

cacgggcgct tacgtcccgg aacgcattgt cagcaacgac gaagtcggcg caccggctgg 3120

tgtggatgac gattggatta cgcgtaaaac cggtatccgc cagcgtcgct gggcggccga 3180

cgatcaagca acgagcgatc tggctaccgc agctggtcgt gcagcactga aagcagctgg 3240

cattacgccg gaacagctga ccgtcatcgc agtggctacc tctacgccgg accgtccgca 3300

gccgccgacc gcggcctatg ttcaacatca cctgggtgca accggcacgg cagcttttga 3360

tgttaacgct gtgtgcagcg gtacggtttt cgcgctgagc tctgttgccg gcaccctggt 3420

ctatcgtggc ggttacgcac tggtcattgg tgctgatctg tactcacgta tcctgaatcc 3480

ggcggaccgc aaaaccgtgg ttctgttcgg cgatggtgcg ggcgcgatgg tgctgggccc 3540

gaccagcacc ggcaccggtc cgattgtgcg tcgcgttgca ctgcatacgt ttggcggtct 3600

gaccgatctg atccgtgttc cggccggcgg ttcccgccag ccgctggaca ccgatggtct 3660

ggacgcaggc ctgcaatatt ttgcgatgga tggccgcgaa gtccgtcgct tcgtgaccga 3720

acatctgccg cagctgatta aaggttttct gcacgaagcg ggcgtggatg cggcggatat 3780

tagccatttc gtgccgcacc aagccaacgg tgtgatgctg gacgaagttt ttggcgaact 3840

gcatctgccg cgtgcaacca tgcaccgtac ggtggaaacc tacggtaata cgggcgcagc 3900

tagtattccg atcacgatgg atgcggccgt tcgtgcaggt tccttccgtc cgggcgaact 3960

ggttctgctg gcgggctttg gcggcggtat ggcggcttcg tttgctctga ttgaatggtg 4020

acctaatgca ggctgcaggc ggatacgagg aggaataaac catgaaaaag gtatgtgtta 4080

taggtgcagg tactatgggt tcaggaattg ctcaggcatt tgcagctaaa ggatttgaag 4140

tagtattaag agatattaaa gatgaatttg ttgatagagg attagatttt atcaataaaa 4200

atctttctaa attagttaaa aaaggaaaga tagaagaagc tactaaagtt gaaatcttaa 4260

ctagaatttc cggaacagtt gaccttaata tggcagctga ttgcgattta gttatagaag 4320

cagctgttga aagaatggat attaaaaagc agatttttgc tgacttagac aatatatgca 4380

agccagaaac aattcttgca tcaaatacat catcactttc aataacagaa gtggcatcag 4440

caactaaaag acctgataag gttataggta tgcatttctt taatccagct cctgttatga 4500

agcttgtaga ggtaataaga ggaatagcta catcacaaga aacttttgat gcagttaaag 4560

agacatctat agcaatagga aaagatcctg tagaagtagc agaagcacca ggatttgttg 4620

taaatagaat attaatacca atgattaatg aagcagttgg tatattagca gaaggaatag 4680

cttcagtaga agacatagat aaagctatga aacttggagc taatcaccca atgggaccat 4740

tagaattagg tgattttata ggtcttgata tatgtcttgc tataatggat gttttatact 4800

cagaaactgg agattctaag tatagaccac atacattact taagaagtat gtaagagcag 4860

gatggcttgg aagaaaatca ggaaaaggtt tctacgatta ttcaaaataa gtttacagga 4920

tctgcaggga ggaggaaatc atggagttga acaacgttat tctggagaaa gaaggcaagg 4980

tggcggttgt caccattaac cgtccaaagg ccctgaacgc tctgaactcg gataccctga 5040

aagagatgga ttacgttatt ggcgagattg agaatgacag cgaagtgctg gctgtgattc 5100

tgaccggtgc gggtgagaag agctttgtcg cgggtgcgga catcagcgag atgaaagaaa 5160

tgaacaccat cgaaggccgt aagttcggta ttctgggcaa caaggtgttt cgtcgtctgg 5220

aactgctgga gaaacctgtc attgctgccg tgaacggttt cgcgctgggc ggtggttgcg 5280

agatcgctat gagctgcgat attcgtatcg catcgtccaa cgcacgcttt ggtcaaccgg 5340

aggtcggtct gggtatcact ccgggtttcg gcggtacgca acgtctgagc cgcctggttg 5400

gcatgggcat ggcgaaacag ttgattttca cggcacagaa cattaaggcg gatgaggcgc 5460

tgcgtattgg tctggtgaat aaggtcgttg agccaagcga actgatgaat accgcgaaag 5520

aaattgcgaa caagatcgtt agcaatgccc cggtggccgt taagctgtcg aaacaggcaa 5580

tcaaccgtgg catgcagtgt gacatcgaca ccgccctggc gtttgagagc gaggcgtttg 5640

gtgagtgctt ctccaccgag gaccaaaagg atgcgatgac cgcgttcatt gagaaacgca 5700

agatcgaggg tttcaagaat cgttaataga ggaggatagg aggttttcat atgattgtga 5760

aaccgatggt ccgtaataat atctgtctga atgctcaccc gcagggctgt aaaaaaggcg 5820

tggaagatca aattgaatat accaaaaaac gtattacggc agaagtgaaa gccggcgcaa 5880

aagctccgaa aaacgtgctg gttctgggtt gcagcaatgg ctatggtctg gcttctcgca 5940

ttaccgcggc ctttggctac ggtgcagcta cgatcggcgt tagtttcgaa aaagcaggtt 6000

ccgaaaccaa atatggcacg ccgggttggt acaacaatct ggcttttgat gaagcggcca 6060

aacgtgaagg cctgtatagt gtcaccattg atggtgacgc gttctccgat gaaattaaag 6120

cacaggtgat cgaagaagcg aagaaaaaag gcattaaatt tgacctgatc gtttacagcc 6180

tggcatctcc ggtccgtacc gatccggaca cgggtatcat gcataaatct gtgctgaaac 6240

cgtttggcaa aaccttcacg ggtaaaaccg ttgatccgtt cacgggcgaa ctgaaagaaa 6300

ttagcgcgga accggccaac gatgaagaag cagctgcgac cgtcaaagtg atgggcggtg 6360

aagactggga acgttggatc aaacagctga gtaaagaagg cctgctggaa gaaggttgca 6420

ttaccctggc gtattcctac atcggcccgg aagcaaccca agctctgtat cgcaaaggca 6480

cgattggtaa agcgaaagaa catctggaag cgaccgccca ccgtctgaac aaagaaaatc 6540

cgtcaatccg cgccttcgtt tcggtcaata aaggtctggt tacccgtgca tcagctgtga 6600

ttccggttat cccgctgtac ctggcatcgc tgtttaaagt catgaaagaa aaaggcaacc 6660

atgaaggttg tattgaacag atcacccgcc tgtatgccga acgtctgtac cgcaaagatg 6720

gtacgattcc ggtggacgaa gaaaatcgta ttcgcatcga tgactgggaa ctggaagaag 6780

atgtccaaaa agccgtgagc gccctgatgg aaaaagttac cggcgaaaac gcggaatctc 6840

tgacggatct ggccggttat cgtcacgact ttctggcgag taatggtttt gatgttgaag 6900

gcattaacta cgaagctgaa gtggaacgct ttgatcgcat ttgatctaga gaattcgtca 6960

acgaattcaa gcttgatatc attcaggacg agcctcagac tccagcgtaa ctggactgaa 7020

aacaaactaa agcgcccttg tggcgcttta gttttgttcc gctcatgata ataatggttt 7080

cttagacgtc aggtggcact tttcggggaa atgtgcgcgc ccgcgttcct gctggcgctg 7140

ggcctgtttc tggcgctgga cttcccgctg ttccgtcagc agcttttcgc ccacggcctt 7200

gatgatcgcg gcggccttgg cctgcatatc ccgattcaac ggccccaggg cgtccagaac 7260

gggcttcagg cgctcccgaa ggtctcgggc cgtctcttgg gcttgatcgg ccttcttgcg 7320

catctcacgc gctcctgcgg cggcctgtag ggcaggctca tacccctgcc gaaccgcttt 7380

tgtcagccgg tcggccacgg cttccggcgt ctcaacgcgc tttgagattc ccagcttttc 7440

ggccaatccc tgcggtgcat aggcgcgtgg ctcgaccgct tgcgggctga tggtgacgtg 7500

gcccactggt ggccgctcca gggcctcgta gaacgcctga atgcgcgtgt gacgtgcctt 7560

gctgccctcg atgccccgtt gcagccctag atcggccaca gcggccgcaa acgtggtctg 7620

gtcgcgggtc atctgcgctt tgttgccgat gaactccttg gccgacagcc tgccgtcctg 7680

cgtcagcggc accacgaacg cggtcatgtg cgggctggtt tcgtcacggt ggatgctggc 7740

cgtcacgatg cgatccgccc cgtacttgtc cgccagccac ttgtgcgcct tctcgaagaa 7800

cgccgcctgc tgttcttggc tggccgactt ccaccattcc gggctggccg tcatgacgta 7860

ctcgaccgcc aacacagcgt ccttgcgccg cttctctggc agcaactcgc gcagtcggcc 7920

catcgcttca tcggtgctgc tggccgccca gtgctcgttc tctggcgtcc tgctggcgtc 7980

agcgttgggc gtctcgcgct cgcggtaggc gtgcttgaga ctggccgcca cgttgcccat 8040

tttcgccagc ttcttgcatc gcatgatcgc gtatgccgcc atgcctgccc ctcccttttg 8100

gtgtccaacc ggctcgacgg gggcagcgca aggcggtgcc tccggcgggc cactcaatgc 8160

ttgagtatac tcactagact ttgcttcgca aagtcgtgac cgcctacggc ggctgcggcg 8220

ccctacgggc ttgctctccg ggcttcgccc tgcgcggtcg ctgcgctc 8268

<210> 20
<211> 8959
<212> DNA
<213> Escherichia coli


<220>
<221> misc_feature
<222> (1)..(8959)
<223> pBMT-3_ccdAB_PT7-'tesA_PT7-nphT7-hbd-crt-ter

<400> 20
ccttgccagc ccgtggatat gtggacgatg gccgcgagcg gccaccggct ggctcgcttc 60

gctcggcccg tggacaaccc tgctggacaa gctgatggac aggctgcgcc tgcccacgag 120

cttgaccaca gggattgccc accggctacc cagccttcga ccacataccc accggctcca 180

actgcgcggc ctgcggcctt gccccatcaa tttttttaat tttctctggg gaaaagcctc 240

cggcctgcgg cctgcgcgct tcgcttgccg gttggacacc aagtggaagg cgggtcaagg 300

ctcgcgcagc gaccgcgcag cggcttggcc ttgacgcgcc tggaacgacc caagcctatg 360

cgagtggggg cagtcgaagg cgaagcccgc ccgcctgccc cccgagcctc acggcggcga 420

gtgcgggggt tccaaggggg cagcgccacc ttgggcaagg ccgaaggccg cgcagtcgat 480

caacaagccc cggaggggcc actttttgcc ggagggggag ccgcgccgaa ggcgtggggg 540

aaccccgcag gggtgccctt ctttgggcac caaagaacta gatatagggc gaaatgcgaa 600

agacttaaaa atcaacaact taaaaaaggg gggtacgcaa cagctcattg cggcaccccc 660

cgcaatagct cattgcgtag gttaaagaaa atctgtaatt gactgccact tttacgcaac 720

gcataattgt tgtcgcgctg ccgaaaagtt gcagctgatt gcgcatggtg ccgcaaccgt 780

gcggcaccct accgcatgga gataagcatg gccacgcagt ccagagaaat cggcattcaa 840

gccaagaaca agcccggtca ctgggtgcaa acggaacgca aagcgcatga ggcgtgggcc 900

gggcttattg cgaggaaacc cacggcggca atgctgctgc atcacctcgt ggcgcagatg 960

ggccaccaga acgccgtggt ggtcagccag aagacacttt ccaagctcat cggacgttct 1020

ttgcggacgg tccaatacgc agtcaaggac ttggtggccg agcgctggat ctccgtcgtg 1080

aagctcaacg gccccggcac cgtgtcggcc tacgtggtca atgaccgcgt ggcgtggggc 1140

cagccccgcg accagttgcg cctgtcggtg ttcagtgccg ccgtggtggt tgatcacgac 1200

gaccaggacg aatcgctgtt ggggcatggc gacctgcgcc gcatcccgac cctgtatccg 1260

ggcgagcagc aactaccgac cggccccggc gaggagccgc ccagccagcc cggcattccg 1320

ggcatggaac cagacctgcc agccttgacc gaaacggagg aatgggaacg gcgcgggcag 1380

cagcgcctgc cgatgcccga tgagccgtgt tttctggacg atggcgagcc gttggagccg 1440

ccgacacggg tcacgctgcc gcgccggtag tacgtacccg gaattgccag ctggggcgcc 1500

ctctggtaag gttgggaagc cctgcaaagt aaactggatg gctttcttgc cgccaaggat 1560

ctgatggcgc aggggatcaa gctctgatca agagacagga tgaggatcgt ttcgatgaag 1620

cagcgtatta cagtgacagt tgacagcgac agctatcagt tgctcaaggc atatgatgtc 1680

aatatctccg gtctggtaag cacaaccatg cagaatgaag cccgtcgtct gcgtgccgaa 1740

cgctggaaag cggaaaatca ggaagggatg gctgaggtcg cccggtttat tgaaatgaac 1800

ggctcttttg ctgacgagaa cagggactgg tgaaatgcag tttaaggttt acacctataa 1860

aagagagagc cgttatcgtc tgtttgtgga tgtacagagc gatattattg acacgcccgg 1920

gcgacggatg gtgatccccc tggccagtgc acgtctgctg tcagataaag tctcccgtga 1980

actttacccg gtggtgcata tcggggatga aagctggcgc atgatgacca ccgatatggc 2040

cagtgtgccg gtctccgtta tcggggaaga agtggctgat ctcagccacc gcgaaaatga 2100

catcaaaaac gccattaacc tgatgttctg gggaatataa atcctagacg aattctctag 2160

tagaggttcc aactttcacc ataatgaaat aagatcacta ccgggcgtat tttttgagtt 2220

atcgagattt tcaggagcta aggaagctaa aatggagaaa aaaatcactg gatataccac 2280

cgttgatata tcccaatggc atcgtaaaga acattttgag gcatttcagt cagttgctca 2340

atgtacctat aaccagaccg ttcagctgga tattacggcc tttttaaaga ccgtaaagaa 2400

aaataagcac aagttttatc cggcctttat tcacattctt gcccgcctga tgaatgctca 2460

tccggaattc cgtatggcaa tgaaagacgg tgagctggtg atatgggata gtgttcaccc 2520

ttgttacacc gttttccatg agcaaactga aacgttttca tcgctctgga gtgaatacca 2580

cgacgatttc cggcagtttc tacacatata ttcgcaagat gtggcgtgtt acggtgaaaa 2640

cctggcctat ttccctaaag ggtttattga gaatatgttt ttcgtctcag ccaatccctg 2700

ggtgagtttc accagttttg atttaaacgt ggccaatatg gacaacttct tcgcccccgt 2760

tttcaccatg ggcaaatatt atacgcaagg cgacaaggtg ctgatgccgc tggcgattca 2820

ggttcatcat gccgtttgtg atggcttcca tgtcggcaga atgcttaatg aattacaaca 2880

gtactgcgat gagtggcagg gcggggcgta aagatcttct cgacgctctc ccttatgcga 2940

ctcctgcatt aggaaattaa tacgactcac tataggggaa ttgtgagcgg ataacaattc 3000

ccctgtagaa ataattttgt ttaactttaa taaggagata taccatggcg gcggacaccc 3060

tgctgatcct gggcgactcc ctgtcggcgg gctaccgcat gtcggcgtcg gcggcgtggc 3120

cggcgctgct gaacgacaag tggcagtcca agacctcggt ggtcaacgcc tcgatcagcg 3180

gcgacacgag ccagcagggc ctggcccgcc tgccggcgct gctgaagcag caccagccgc 3240

gctgggtgct ggtcgagctg ggcggcaacg acggcctgcg cggcttccag ccgcagcaga 3300

ccgaacagac gctgcgccag atcctgcagg acgtgaaggc cgcgaacgcc gagccgctgc 3360

tgatgcagat ccgcctgccg gcgaactacg gccgtcgcta caacgaggcc ttctccgcga 3420

tctacccgaa gctggccaag gaattcgacg tgccgctgct gccgttcttc atggaggaag 3480

tctacctgaa gccgcagtgg atgcaggacg acggcatcca cccgaaccgc gacgcgcagc 3540

cgttcatcgc cgactggatg gccaagcaac tgcagccgct ggtcaaccac gactcctgaa 3600

tttaaatccc ttatgcgact cctgcattag gaaattaata cgactcacta taggggaatt 3660

gtgagcggat aacaattccc ctgtagaaat aattttgttt aactttaata aggagatata 3720

ccatgacgga tgtccgcttt cgtatcattg gcacgggcgc ttacgtcccg gaacgcattg 3780

tcagcaacga cgaagtcggc gcaccggctg gtgtggatga cgattggatt acgcgtaaaa 3840

ccggtatccg ccagcgtcgc tgggcggccg acgatcaagc aacgagcgat ctggctaccg 3900

cagctggtcg tgcagcactg aaagcagctg gcattacgcc ggaacagctg accgtcatcg 3960

cagtggctac ctctacgccg gaccgtccgc agccgccgac cgcggcctat gttcaacatc 4020

acctgggtgc aaccggcacg gcagcttttg atgttaacgc tgtgtgcagc ggtacggttt 4080

tcgcgctgag ctctgttgcc ggcaccctgg tctatcgtgg cggttacgca ctggtcattg 4140

gtgctgatct gtactcacgt atcctgaatc cggcggaccg caaaaccgtg gttctgttcg 4200

gcgatggtgc gggcgcgatg gtgctgggcc cgaccagcac cggcaccggt ccgattgtgc 4260

gtcgcgttgc actgcatacg tttggcggtc tgaccgatct gatccgtgtt ccggccggcg 4320

gttcccgcca gccgctggac accgatggtc tggacgcagg cctgcaatat tttgcgatgg 4380

atggccgcga agtccgtcgc ttcgtgaccg aacatctgcc gcagctgatt aaaggttttc 4440

tgcacgaagc gggcgtggat gcggcggata ttagccattt cgtgccgcac caagccaacg 4500

gtgtgatgct ggacgaagtt tttggcgaac tgcatctgcc gcgtgcaacc atgcaccgta 4560

cggtggaaac ctacggtaat acgggcgcag ctagtattcc gatcacgatg gatgcggccg 4620

ttcgtgcagg ttccttccgt ccgggcgaac tggttctgct ggcgggcttt ggcggcggta 4680

tggcggcttc gtttgctctg attgaatggt gacctaatgc aggctgcagg cggatacgag 4740

gaggaataaa ccatgaaaaa ggtatgtgtt ataggtgcag gtactatggg ttcaggaatt 4800

gctcaggcat ttgcagctaa aggatttgaa gtagtattaa gagatattaa agatgaattt 4860

gttgatagag gattagattt tatcaataaa aatctttcta aattagttaa aaaaggaaag 4920

atagaagaag ctactaaagt tgaaatctta actagaattt ccggaacagt tgaccttaat 4980

atggcagctg attgcgattt agttatagaa gcagctgttg aaagaatgga tattaaaaag 5040

cagatttttg ctgacttaga caatatatgc aagccagaaa caattcttgc atcaaataca 5100

tcatcacttt caataacaga agtggcatca gcaactaaaa gacctgataa ggttataggt 5160

atgcatttct ttaatccagc tcctgttatg aagcttgtag aggtaataag aggaatagct 5220

acatcacaag aaacttttga tgcagttaaa gagacatcta tagcaatagg aaaagatcct 5280

gtagaagtag cagaagcacc aggatttgtt gtaaatagaa tattaatacc aatgattaat 5340

gaagcagttg gtatattagc agaaggaata gcttcagtag aagacataga taaagctatg 5400

aaacttggag ctaatcaccc aatgggacca ttagaattag gtgattttat aggtcttgat 5460

atatgtcttg ctataatgga tgttttatac tcagaaactg gagattctaa gtatagacca 5520

catacattac ttaagaagta tgtaagagca ggatggcttg gaagaaaatc aggaaaaggt 5580

ttctacgatt attcaaaata agtttacagg atctgcaggg aggaggaaat catggagttg 5640

aacaacgtta ttctggagaa agaaggcaag gtggcggttg tcaccattaa ccgtccaaag 5700

gccctgaacg ctctgaactc ggataccctg aaagagatgg attacgttat tggcgagatt 5760

gagaatgaca gcgaagtgct ggctgtgatt ctgaccggtg cgggtgagaa gagctttgtc 5820

gcgggtgcgg acatcagcga gatgaaagaa atgaacacca tcgaaggccg taagttcggt 5880

attctgggca acaaggtgtt tcgtcgtctg gaactgctgg agaaacctgt cattgctgcc 5940

gtgaacggtt tcgcgctggg cggtggttgc gagatcgcta tgagctgcga tattcgtatc 6000

gcatcgtcca acgcacgctt tggtcaaccg gaggtcggtc tgggtatcac tccgggtttc 6060

ggcggtacgc aacgtctgag ccgcctggtt ggcatgggca tggcgaaaca gttgattttc 6120

acggcacaga acattaaggc ggatgaggcg ctgcgtattg gtctggtgaa taaggtcgtt 6180

gagccaagcg aactgatgaa taccgcgaaa gaaattgcga acaagatcgt tagcaatgcc 6240

ccggtggccg ttaagctgtc gaaacaggca atcaaccgtg gcatgcagtg tgacatcgac 6300

accgccctgg cgtttgagag cgaggcgttt ggtgagtgct tctccaccga ggaccaaaag 6360

gatgcgatga ccgcgttcat tgagaaacgc aagatcgagg gtttcaagaa tcgttaatag 6420

aggaggatag gaggttttca tatgattgtg aaaccgatgg tccgtaataa tatctgtctg 6480

aatgctcacc cgcagggctg taaaaaaggc gtggaagatc aaattgaata taccaaaaaa 6540

cgtattacgg cagaagtgaa agccggcgca aaagctccga aaaacgtgct ggttctgggt 6600

tgcagcaatg gctatggtct ggcttctcgc attaccgcgg cctttggcta cggtgcagct 6660

acgatcggcg ttagtttcga aaaagcaggt tccgaaacca aatatggcac gccgggttgg 6720

tacaacaatc tggcttttga tgaagcggcc aaacgtgaag gcctgtatag tgtcaccatt 6780

gatggtgacg cgttctccga tgaaattaaa gcacaggtga tcgaagaagc gaagaaaaaa 6840

ggcattaaat ttgacctgat cgtttacagc ctggcatctc cggtccgtac cgatccggac 6900

acgggtatca tgcataaatc tgtgctgaaa ccgtttggca aaaccttcac gggtaaaacc 6960

gttgatccgt tcacgggcga actgaaagaa attagcgcgg aaccggccaa cgatgaagaa 7020

gcagctgcga ccgtcaaagt gatgggcggt gaagactggg aacgttggat caaacagctg 7080

agtaaagaag gcctgctgga agaaggttgc attaccctgg cgtattccta catcggcccg 7140

gaagcaaccc aagctctgta tcgcaaaggc acgattggta aagcgaaaga acatctggaa 7200

gcgaccgccc accgtctgaa caaagaaaat ccgtcaatcc gcgccttcgt ttcggtcaat 7260

aaaggtctgg ttacccgtgc atcagctgtg attccggtta tcccgctgta cctggcatcg 7320

ctgtttaaag tcatgaaaga aaaaggcaac catgaaggtt gtattgaaca gatcacccgc 7380

ctgtatgccg aacgtctgta ccgcaaagat ggtacgattc cggtggacga agaaaatcgt 7440

attcgcatcg atgactggga actggaagaa gatgtccaaa aagccgtgag cgccctgatg 7500

gaaaaagtta ccggcgaaaa cgcggaatct ctgacggatc tggccggtta tcgtcacgac 7560

tttctggcga gtaatggttt tgatgttgaa ggcattaact acgaagctga agtggaacgc 7620

tttgatcgca tttgatctag agaattcgtc aacgaattca agcttgatat cattcaggac 7680

gagcctcaga ctccagcgta actggactga aaacaaacta aagcgccctt gtggcgcttt 7740

agttttgttc cgctcatgat aataatggtt tcttagacgt caggtggcac ttttcgggga 7800

aatgtgcgcg cccgcgttcc tgctggcgct gggcctgttt ctggcgctgg acttcccgct 7860

gttccgtcag cagcttttcg cccacggcct tgatgatcgc ggcggccttg gcctgcatat 7920

cccgattcaa cggccccagg gcgtccagaa cgggcttcag gcgctcccga aggtctcggg 7980

ccgtctcttg ggcttgatcg gccttcttgc gcatctcacg cgctcctgcg gcggcctgta 8040

gggcaggctc atacccctgc cgaaccgctt ttgtcagccg gtcggccacg gcttccggcg 8100

tctcaacgcg ctttgagatt cccagctttt cggccaatcc ctgcggtgca taggcgcgtg 8160

gctcgaccgc ttgcgggctg atggtgacgt ggcccactgg tggccgctcc agggcctcgt 8220

agaacgcctg aatgcgcgtg tgacgtgcct tgctgccctc gatgccccgt tgcagcccta 8280

gatcggccac agcggccgca aacgtggtct ggtcgcgggt catctgcgct ttgttgccga 8340

tgaactcctt ggccgacagc ctgccgtcct gcgtcagcgg caccacgaac gcggtcatgt 8400

gcgggctggt ttcgtcacgg tggatgctgg ccgtcacgat gcgatccgcc ccgtacttgt 8460

ccgccagcca cttgtgcgcc ttctcgaaga acgccgcctg ctgttcttgg ctggccgact 8520

tccaccattc cgggctggcc gtcatgacgt actcgaccgc caacacagcg tccttgcgcc 8580

gcttctctgg cagcaactcg cgcagtcggc ccatcgcttc atcggtgctg ctggccgccc 8640

agtgctcgtt ctctggcgtc ctgctggcgt cagcgttggg cgtctcgcgc tcgcggtagg 8700

cgtgcttgag actggccgcc acgttgccca ttttcgccag cttcttgcat cgcatgatcg 8760

cgtatgccgc catgcctgcc cctccctttt ggtgtccaac cggctcgacg ggggcagcgc 8820

aaggcggtgc ctccggcggg ccactcaatg cttgagtata ctcactagac tttgcttcgc 8880

aaagtcgtga ccgcctacgg cggctgcggc gccctacggg cttgctctcc gggcttcgcc 8940

ctgcgcggtc gctgcgctc 8959

<210> 21
<211> 5367
<212> DNA
<213> Escherichia coli

<220>
<221> misc_feature
<222> (1)..(5367)
<223> pET-28b(empty vector)

<400> 21
ggcgaatggg acgcgccctg tagcggcgca ttaagcgcgg cgggtgtggt ggttacgcgc 60

agcgtgaccg ctacacttgc cagcgcccta gcgcccgctc ctttcgcttt cttcccttcc 120

tttctcgcca cgttcgccgg ctttccccgt caagctctaa atcgggggct ccctttaggg 180

ttccgattta gtgctttacg gcacctcgac cccaaaaaac ttgattaggg tgatggttca 240

cgtagtgggc catcgccctg atagacggtt tttcgccctt tgacgttgga gtccacgttc 300

tttaatagtg gactcttgtt ccaaactgga acaacactca accctatctc ggtctattct 360

tttgatttat aagggatttt gccgatttcg gcctattggt taaaaaatga gctgatttaa 420

caaaaattta acgcgaattt taacaaaata ttaacgttta caatttcagg tggcactttt 480

cggggaaatg tgcgcggaac ccctatttgt ttatttttct aaatacattc aaatatgtat 540

ccgctcatga attaattctt agaaaaactc atcgagcatc aaatgaaact gcaatttatt 600

catatcagga ttatcaatac catatttttg aaaaagccgt ttctgtaatg aaggagaaaa 660

ctcaccgagg cagttccata ggatggcaag atcctggtat cggtctgcga ttccgactcg 720

tccaacatca atacaaccta ttaatttccc ctcgtcaaaa ataaggttat caagtgagaa 780

atcaccatga gtgacgactg aatccggtga gaatggcaaa agtttatgca tttctttcca 840

gacttgttca acaggccagc cattacgctc gtcatcaaaa tcactcgcat caaccaaacc 900

gttattcatt cgtgattgcg cctgagcgag acgaaatacg cgatcgctgt taaaaggaca 960

attacaaaca ggaatcgaat gcaaccggcg caggaacact gccagcgcat caacaatatt 1020

ttcacctgaa tcaggatatt cttctaatac ctggaatgct gttttcccgg ggatcgcagt 1080

ggtgagtaac catgcatcat caggagtacg gataaaatgc ttgatggtcg gaagaggcat 1140

aaattccgtc agccagttta gtctgaccat ctcatctgta acatcattgg caacgctacc 1200

tttgccatgt ttcagaaaca actctggcgc atcgggcttc ccatacaatc gatagattgt 1260

cgcacctgat tgcccgacat tatcgcgagc ccatttatac ccatataaat cagcatccat 1320

gttggaattt aatcgcggcc tagagcaaga cgtttcccgt tgaatatggc tcataacacc 1380

ccttgtatta ctgtttatgt aagcagacag ttttattgtt catgaccaaa atcccttaac 1440

gtgagttttc gttccactga gcgtcagacc ccgtagaaaa gatcaaagga tcttcttgag 1500

atcctttttt tctgcgcgta atctgctgct tgcaaacaaa aaaaccaccg ctaccagcgg 1560

tggtttgttt gccggatcaa gagctaccaa ctctttttcc gaaggtaact ggcttcagca 1620

gagcgcagat accaaatact gtccttctag tgtagccgta gttaggccac cacttcaaga 1680

actctgtagc accgcctaca tacctcgctc tgctaatcct gttaccagtg gctgctgcca 1740

gtggcgataa gtcgtgtctt accgggttgg actcaagacg atagttaccg gataaggcgc 1800

agcggtcggg ctgaacgggg ggttcgtgca cacagcccag cttggagcga acgacctaca 1860

ccgaactgag atacctacag cgtgagctat gagaaagcgc cacgcttccc gaagggagaa 1920

aggcggacag gtatccggta agcggcaggg tcggaacagg agagcgcacg agggagcttc 1980

cagggggaaa cgcctggtat ctttatagtc ctgtcgggtt tcgccacctc tgacttgagc 2040

gtcgattttt gtgatgctcg tcaggggggc ggagcctatg gaaaaacgcc agcaacgcgg 2100

cctttttacg gttcctggcc ttttgctggc cttttgctca catgttcttt cctgcgttat 2160

cccctgattc tgtggataac cgtattaccg cctttgagtg agctgatacc gctcgccgca 2220

gccgaacgac cgagcgcagc gagtcagtga gcgaggaagc ggaagagcgc ctgatgcggt 2280

attttctcct tacgcatctg tgcggtattt cacaccgcat atatggtgca ctctcagtac 2340

aatctgctct gatgccgcat agttaagcca gtatacactc cgctatcgct acgtgactgg 2400

gtcatggctg cgccccgaca cccgccaaca cccgctgacg cgccctgacg ggcttgtctg 2460

ctcccggcat ccgcttacag acaagctgtg accgtctccg ggagctgcat gtgtcagagg 2520

ttttcaccgt catcaccgaa acgcgcgagg cagctgcggt aaagctcatc agcgtggtcg 2580

tgaagcgatt cacagatgtc tgcctgttca tccgcgtcca gctcgttgag tttctccaga 2640

agcgttaatg tctggcttct gataaagcgg gccatgttaa gggcggtttt ttcctgtttg 2700

gtcactgatg cctccgtgta agggggattt ctgttcatgg gggtaatgat accgatgaaa 2760

cgagagagga tgctcacgat acgggttact gatgatgaac atgcccggtt actggaacgt 2820

tgtgagggta aacaactggc ggtatggatg cggcgggacc agagaaaaat cactcagggt 2880

caatgccagc gcttcgttaa tacagatgta ggtgttccac agggtagcca gcagcatcct 2940

gcgatgcaga tccggaacat aatggtgcag ggcgctgact tccgcgtttc cagactttac 3000

gaaacacgga aaccgaagac cattcatgtt gttgctcagg tcgcagacgt tttgcagcag 3060

cagtcgcttc acgttcgctc gcgtatcggt gattcattct gctaaccagt aaggcaaccc 3120

cgccagccta gccgggtcct caacgacagg agcacgatca tgcgcacccg tggggccgcc 3180

atgccggcga taatggcctg cttctcgccg aaacgtttgg tggcgggacc agtgacgaag 3240

gcttgagcga gggcgtgcaa gattccgaat accgcaagcg acaggccgat catcgtcgcg 3300

ctccagcgaa agcggtcctc gccgaaaatg acccagagcg ctgccggcac ctgtcctacg 3360

agttgcatga taaagaagac agtcataagt gcggcgacga tagtcatgcc ccgcgcccac 3420

cggaaggagc tgactgggtt gaaggctctc aagggcatcg gtcgagatcc cggtgcctaa 3480

tgagtgagct aacttacatt aattgcgttg cgctcactgc ccgctttcca gtcgggaaac 3540

ctgtcgtgcc agctgcatta atgaatcggc caacgcgcgg ggagaggcgg tttgcgtatt 3600

gggcgccagg gtggtttttc ttttcaccag tgagacgggc aacagctgat tgcccttcac 3660

cgcctggccc tgagagagtt gcagcaagcg gtccacgctg gtttgcccca gcaggcgaaa 3720

atcctgtttg atggtggtta acggcgggat ataacatgag ctgtcttcgg tatcgtcgta 3780

tcccactacc gagatatccg caccaacgcg cagcccggac tcggtaatgg cgcgcattgc 3840

gcccagcgcc atctgatcgt tggcaaccag catcgcagtg ggaacgatgc cctcattcag 3900

catttgcatg gtttgttgaa aaccggacat ggcactccag tcgccttccc gttccgctat 3960

cggctgaatt tgattgcgag tgagatattt atgccagcca gccagacgca gacgcgccga 4020

gacagaactt aatgggcccg ctaacagcgc gatttgctgg tgacccaatg cgaccagatg 4080

ctccacgccc agtcgcgtac cgtcttcatg ggagaaaata atactgttga tgggtgtctg 4140

gtcagagaca tcaagaaata acgccggaac attagtgcag gcagcttcca cagcaatggc 4200

atcctggtca tccagcggat agttaatgat cagcccactg acgcgttgcg cgagaagatt 4260

gtgcaccgcc gctttacagg cttcgacgcc gcttcgttct accatcgaca ccaccacgct 4320

ggcacccagt tgatcggcgc gagatttaat cgccgcgaca atttgcgacg gcgcgtgcag 4380

ggccagactg gaggtggcaa cgccaatcag caacgactgt ttgcccgcca gttgttgtgc 4440

cacgcggttg ggaatgtaat tcagctccgc catcgccgct tccacttttt cccgcgtttt 4500

cgcagaaacg tggctggcct ggttcaccac gcgggaaacg gtctgataag agacaccggc 4560

atactctgcg acatcgtata acgttactgg tttcacattc accaccctga attgactctc 4620

ttccgggcgc tatcatgcca taccgcgaaa ggttttgcgc cattcgatgg tgtccgggat 4680

ctcgacgctc tcccttatgc gactcctgca ttaggaagca gcccagtagt aggttgaggc 4740

cgttgagcac cgccgccgca aggaatggtg catgcaagga gatggcgccc aacagtcccc 4800

cggccacggg gcctgccacc atacccacgc cgaaacaagc gctcatgagc ccgaagtggc 4860

gagcccgatc ttccccatcg gtgatgtcgg cgatataggc gccagcaacc gcacctgtgg 4920

cgccggtgat gccggccacg atgcgtccgg cgtagaggat cgagatctcg atcccgcgaa 4980

attaatacga ctcactatag gggaattgtg agcggataac aattcccctc tagaaataat 5040

tttgtttaac tttaagaagg agatatacca tgggcagcag ccatcatcat catcatcaca 5100

gcagcggcct ggtgccgcgc ggcagccata tggctagcat gactggtgga cagcaaatgg 5160

gtcgggatcc gaattcgagc tccgtcgaca agcttgcggc cgcactcgag caccaccacc 5220

accaccactg agatccggct gctaacaaag cccgaaagga agctgagttg gctgctgcca 5280

ccgctgagca ataactagca taaccccttg gggcctctaa acgggtcttg aggggttttt 5340

tgctgaaagg aggaactata tccggat 5367

<210> 22
<211> 43
<212> DNA
<213> Artificial Sequence

<220>
<223> oligonucleotide o-MO-40 for gcvTHP amplification

<400> 22
ccggggatcg cggccgcgga ccgaacttat ccgccaggca atg 43

<210> 23
<211> 51
<212> DNA
<213> Artificial Sequence

<220>
<223> oligonucleotide o-MO-41 for gcvTHP amplification

<400> 23
ttactggtat tcgctaatcg gctcgagcat cttgtcctca ttgaataagc g 51

<210> 24
<211> 30
<212> DNA

<213>  Artificial Sequence

<220>
<223>  oligonucleotide o-MO-42 for gcvTHP amplification

<400>  24
ctcgagccga ttagcgaata ccagtaattc                                        30


<210>  25
<211>  41
<212>  DNA
<213>  Artificial Sequence

<220>
<223>  oligonucleotide o-MO-43 for gcvTHP amplification

<400>  25
ggatcgcggc cgctctagag ttcatcgtgc tgatcgattg c                           41


<210>  26
<211>  553
<212>  DNA
<213>  Artificial Sequence

<220>
<223>  PCR product 1 from example 12, 1553bp

<400>  26
ccggggatcg cggccgcgga ccgaacttat ccgccaggca atgggattaa acgatttgcc        60

tgaatggctg cgttaaaaat ttctcctctg ttgtttattt gatacccatc acactttcat       120

ctcccggttt tttcgccggg agattttcct catttgaaat aaactaattt cacctccgtt       180

ttcgcattat attttctaat gccattattt tttgatttag tgttttttga cattttttta       240

gctcttaata ttgtcttatt caaattgact ttctcatcac atcatctttg tatagaaact       300

ggtgtatttt ttggtttttt attctgtcgc gatttttgca ttttttaacc ataagctaat       360

gtgatgatca attttacctt atggttaaca gtctgtttcg gtggtaagtt caggcaaaag       420

agaacgattg cgttggggac cgggagtggc tccgatgctg ggtttcgtgg tgataatttc       480

accatgaaaa agttgtcagc cccgcttatt caatgaggac aagatgctcg agccgattag       540

cgaataccag taa                                                          553


<210>  27
<211>  547
<212>  DNA
<213>  Artificial Sequence

<220>
<223>  PCR product 2 from example 12, 1553bp

<400>  27
ctcgagccga ttagcgaata ccagtaattc actgattcga ctattttcta aaggcgcttc        60

ggcgcctttt tagtcagatg acaaagtaca aaagtgctca gacagtcccc tcgccccttt       120

ggggagaggg ttagggtgag gggaacaggc cggcactggc gcgaatattt accctcaccc 180

cggccctctc cctgaaaggg cgagggggaa aagcgtgcca acattgaaga ttgagccagt 240

ttgttagcaa tctcaaagat acgtcaacga attaattttt ctcggaaaaa caaatggcta 300

tagcacttgt gactggtggc agtcgcggca tcgggcgggc aactgcatta ctgttggcgc 360

aagaagggta tacggtggcg gttaattatc agcaaaacct ccacgcggcg caggaagtga 420

tgaacttaat aacgcaagcc ggtggcaaag cattcgtgct ccaggcggat atcagcgacg 480

aaaaccaggt cgttgcgatg tttacagcaa tcgatcagca cgatgaactc tagagcggcc 540

gcgatcc 547

<210> 28
<211> 5667
<212> DNA
<213> Artificial Sequence

<220>
<223> vector pKO3

<400> 28
cctttcgtct tcgaataaat acctgtgacg gaagatcact tcgcagaata aataaatcct 60

ggtgtccctg ttgataccgg gaagccctgg gccaactttt ggcgaaaatg agacgttgat 120

cggcacgtaa gaggttccaa ctttcaccat aatgaaataa gatcactacc gggcgtattt 180

tttgagttat cgagattttc aggagctaag gaagctaaaa tggagaaaaa aatcactgga 240

tataccaccg ttgatatatc ccaatggcat cgtaaagaac attttgaggc atttcagtca 300

gttgctcaat gtacctataa ccagaccgtt cagctggata ttacggcctt tttaaagacc 360

gtaaagaaaa ataagcacaa gttttatccg gcctttattc acattcttgc ccgcctgatg 420

aatgctcatc cggaattccg tatggcaatg aaagacggtg agctggtgat atgggatagt 480

gttcaccctt gttacaccgt tttccatgag caaactgaaa cgttttcatc gctctggagt 540

gaataccacg acgatttccg gcagtttcta cacatatatt cgcaagatgt ggcgtgttac 600

ggtgaaaacc tggcctattt ccctaaaggg tttattgaga atatgttttt cgtctcagcc 660

aatccctggg tgagtttcac cagttttgat ttaaacgtgg ccaatatgga caacttcttc 720

gcccccgttt tcaccatggg caaatattat acgcaaggcg acaaggtgct gatgccgctg 780

gcgattcagg ttcatcatgc cgtttgtgat ggcttccatg tcggcagaat gcttaatgaa 840

ttacaacagt actgcgatga gtggcagggc ggggcgtaat ttttttaagg cagttattgg 900

tgcccttaaa cgcctggttg ctacgcctga ataagtgata ataagcggat gaatggcaga 960

aattcgaaag caaattcgac ccggtcgtcg gttcagggca gggtcgttaa atagccgctt 1020

atgtctattg ctggtctcgg tacccgggga tcgcggccgc ggaccggatc ctctagagcg 1080

gccgcgatcc tctagagtcg accggtggcg aatgggacgc gccctgtagc ggcgcattaa 1140

gcgcggcggg tgtggtggtt acgcgcagcg tgaccgctac acttgccagc gccctagcgc 1200

ccgctccttt cgctttcttc ccttcctttc tcgccacgtt cgccggcttt ccccgtcaag 1260

ctctaaatcg ggggctccct ttagggttcc gatttagtgc tttacggcac ctcgacccca 1320

aaaaacttga ttagggtgat ggttcacgta gtgggccatc gccctgatag acggtttttc 1380

gccctttgac gttggagtcc acgttcttta atagtggact cttgttccaa actggaacaa 1440

cactcaaccc tatctcggtc tattcttttg atttataagg gattttgccg atttcggcct 1500

attggttaaa aaatgagctg atttaacaaa aatttaacgc gaattttaac aaaatattaa 1560

cgcttacaat ttaggtggca cttttcgggg aaatgtgcgc ggaaccccta tttgtttatt 1620

tttctaaata cattcaaata tgtatccgct caccgcgatc ctttttaacc catcacatat 1680

acctgccgtt cactattatt tagtgaaatg agatattatg atattttctg aattgtgatt 1740

aaaaaggcaa ctttatgccc atgcaacaga aactataaaa aatacagaga atgaaaagaa 1800

acagatagat tttttagttc tttaggcccg tagtctgcaa atccttttat gattttctat 1860

caaacaaaag aggaaaatag accagttgca atccaaacga gagtctaata gaatgaggtc 1920

gaaaagtaaa tcgcgcgggt ttgttactga taaagcaggc aagacctaaa atgtgtaaag 1980

ggcaaagtgt atactttggc gtcacccctt acatatttta ggtctttttt tattgtgcgt 2040

aactaacttg ccatcttcaa acaggagggc tggaagaagc agaccgctaa cacagtacat 2100

aaaaaaggag acatgaacga tgaacatcaa aaagtttgca aaacaagcaa cagtattaac 2160

ctttactacc gcactgctgg caggaggcgc aactcaagcg tttgcgaaag aaacgaacca 2220

aaagccatat aaggaaacat acggcatttc ccatattaca cgccatgata tgctgcaaat 2280

ccctgaacag caaaaaaatg aaaaatatca agttcctgag ttcgattcgt ccacaattaa 2340

aaatatctct tctgcaaaag gcctggacgt ttgggacagc tggccattac aaaacgctga 2400

cggcactgtc gcaaactatc acggctacca catcgtcttt gcattagccg gagatcctaa 2460

aaatgcggat gacacatcga tttacatgtt ctatcaaaaa gtcggcgaaa cttctattga 2520

cagctggaaa aacgctggcc gcgtctttaa agacagcgac aaattcgatg caaatgattc 2580

tatcctaaaa gaccaaacac aagaatggtc aggttcagcc acatttacat ctgacggaaa 2640

aatccgttta ttctacactg atttctccgg taaacattac ggcaaacaaa cactgacaac 2700

tgcacaagtt aacgtatcag catcagacag ctctttgaac atcaacggtg tagaggatta 2760

taaatcaatc tttgacggtg acggaaaaac gtatcaaaat gtacagcagt tcatcgatga 2820

aggcaactac agctcaggcg acaaccatac gctgagagat cctcactacg tagaagataa 2880

aggccacaaa tacttagtat ttgaagcaaa cactggaact gaagatggct accaaggcga 2940

agaatcttta tttaacaaag catactatgg caaaagcaca tcattcttcc gtcaagaaag 3000

tcaaaaactt ctgcaaagcg ataaaaaacg cacggctgag ttagcaaacg gcgctctcgg 3060

tatgattgag ctaaacgatg attacacact gaaaaaagtg atgaaaccgc tgattgcatc 3120

taacacagta acagatgaaa ttgaacgcgc gaacgtcttt aaaatgaacg gcaaatggta 3180

cctgttcact gactcccgcg gatcaaaaat gacgattgac ggcattacgt ctaacgatat 3240

ttacatgctt ggttatgttt ctaattcttt aactggccca tacaagccgc tgaacaaaac 3300

tggccttgtg ttaaaaatgg atcttgatcc taacgatgta acctttactt actcacactt 3360

cgctgtacct caagcgaaag gaaacaatgt cgtgattaca agctatatga caaacagagg 3420

attctacgca gacaaacaat caacgtttgc gccaagcttc ctgctgaaca tcaaaggcaa 3480

gaaaacatct gttgtcaaag acagcatcct tgaacaagga caattaacag ttaacaaata 3540

aaaacgcaaa agaaaatgcc gatattgact accggaagca gtgtgaccgt gtgcttctca 3600

aatgcctgat tcaggctgtc tatgtgtgac tgttgagctg taacaagttg tctcaggtgt 3660

tcaatttcat gttctagttg ctttgtttta ctggtttcac ctgttctatt aggtgttaca 3720

tgctgttcat ctgttacatt gtcgatctgt tcatggtgaa cagctttaaa tgcaccaaaa 3780

actcgtaaaa gctctgatgt atctatcttt tttacaccgt tttcatctgt gcatatggac 3840

agttttccct ttgatatgta acggtgaaca gttgttctac ttttgtttgt tagtcttgat 3900

gcttcactga tagatacaag agccataaga acctcagatc cttccgtatt tagccagtat 3960

gttctctagt gtggttcgtt gtttttgcgt gagccatgag aacgaaccat tgagatcata 4020

cttactttgc atgtcactca aaaattttgc ctcaaaactg gtgagctgaa tttttgcagt 4080

taaagcatcg tgtagtgttt ttcttagtcc gttatgtagg taggaatctg atgtaatggt 4140

tgttggtatt ttgtcaccat tcatttttat ctggttgttc tcaagttcgg ttacgagatc 4200

catttgtcta tctagttcaa cttggaaaat caacgtatca gtcgggcggc ctcgcttatc 4260

aaccaccaat ttcatattgc tgtaagtgtt taaatcttta cttattggtt tcaaaaccca 4320

ttggttaagc cttttaaact catggtagtt attttcaagc attaacatga acttaaattc 4380

atcaaggcta atctctatat ttgccttgtg agttttcttt tgtgttagtt cttttaataa 4440

ccactcataa atcctcatag agtatttgtt ttcaaaagac ttaacatgtt ccagattata 4500

ttttatgaat ttttttaact ggaaaagata aggcaatatc tcttcactaa aaactaattc 4560

taatttttcg cttgagaact tggcatagtt tgtccactgg aaaatctcaa agcctttaac 4620

caaaggattc ctgatttcca cagttctcgt catcagctct ctggttgctt tagctaatac 4680

accataagca ttttccctac tgatgttcat catctgaacg tattggttat aagtgaacga 4740

taccgtccgt tctttccttg tagggttttc aatcgtgggg ttgagtagtg ccacacagca 4800

taaaattagc ttggtttcat gctccgttaa gtcatagcga ctaatcgcta gttcatttgc 4860

tttgaaaaca actaattcag acatacatct caattggtct aggtgatttt aatcactata 4920

ccaattgaga tgggctagtc aatgataatt actagtcctt ttcctttgag ttgtgggtat 4980

ctgtaaattc tgctagacct ttgctggaaa acttgtaaat tctgctagac cctctgtaaa 5040

ttccgctaga cctttgtgtg ttttttttgt ttatattcaa gtggttataa tttatagaat 5100

aaagaaagaa taaaaaaaga taaaaagaat agatcccagc cctgtgtata actcactact 5160

ttagtcagtt ccgcagtatt acaaaaggat gtcgcaaacg ctgtttgctc ctctacaaaa 5220

cagaccttaa aaccctaaag gcttaagtag caccctcgca agctcgggca aatcgctgaa 5280

tattcctttt gtctccgacc atcaggcacc tgagtcgctg tctttttcgt gacattcagt 5340

tcgctgcgct cacggctctg gcagtgaatg ggggtaaatg gcactacagg cgccttttat 5400

ggattcatgc aaggaaacta cccataatac aagaaaagcc cgtcacgggc ttctcagggc 5460

gttttatggc gggtctgcta tgtggtgcta tctgactttt tgctgttcag cagttcctgc 5520

cctctgattt tccagtctga ccacttcgga ttatcccgtg acaggtcatt cagactggct 5580

aatgcaccca gtaaggcagc ggtatcatca acaggcttac ccgtcttact gtcggggatc 5640

gacgctctcc cttatgcgac tcctgca 5667

<210> 29
<211> 6693
<212> DNA
<213> Artificial Sequence

<220>
<223> knock-out vector pKO3 delta gcvTHP

<400> 29
cctttcgtct tcgaataaat acctgtgacg gaagatcact tcgcagaata aataaatcct 60

ggtgtccctg ttgataccgg gaagccctgg gccaactttt ggcgaaaatg agacgttgat 120

cggcacgtaa gaggttccaa ctttcaccat aatgaaataa gatcactacc gggcgtattt 180

tttgagttat cgagattttc aggagctaag gaagctaaaa tggagaaaaa aatcactgga 240

tataccaccg ttgatatatc ccaatggcat cgtaaagaac attttgaggc atttcagtca 300

gttgctcaat gtacctataa ccagaccgtt cagctggata ttacggcctt tttaaagacc 360

gtaaagaaaa ataagcacaa gttttatccg gcctttattc acattcttgc ccgcctgatg 420

aatgctcatc cggaattccg tatggcaatg aaagacggtg agctggtgat atgggatagt 480

gttcaccctt gttacaccgt tttccatgag caaactgaaa cgttttcatc gctctggagt 540

gaataccacg acgatttccg gcagtttcta cacatatatt cgcaagatgt ggcgtgttac 600

ggtgaaaacc tggcctattt ccctaaaggg tttattgaga atatgttttt cgtctcagcc 660

aatccctggg tgagtttcac cagttttgat ttaaacgtgg ccaatatgga caacttcttc 720

gcccccgttt tcaccatggg caaatattat acgcaaggcg acaaggtgct gatgccgctg 780

gcgattcagg ttcatcatgc cgtttgtgat ggcttccatg tcggcagaat gcttaatgaa 840

ttacaacagt actgcgatga gtggcagggc ggggcgtaat ttttttaagg cagttattgg 900

tgcccttaaa cgcctggttg ctacgcctga ataagtgata ataagcggat gaatggcaga 960

aattcgaaag caaattcgac ccggtcgtcg gttcagggca gggtcgttaa atagccgctt 1020

atgtctattg ctggtctcgg tacccgggga tcgcggccgc ggaccgaact tatccgccag 1080

gcaatgggat taaacgattt gcctgaatgg ctgcgttaaa aatttctcct ctgttgttta 1140

tttgataccc atcacacttt catctcccgg ttttttcgcc gggagatttt cctcatttga 1200

aataaactaa tttcacctcc gttttcgcat tatattttct aatgccatta ttttttgatt 1260

tagtgttttt tgacattttt ttagctctta atattgtctt attcaaattg actttctcat 1320

cacatcatct ttgtatagaa actggtgtat tttttggttt tttattctgt cgcgattttt 1380

gcatttttta accataagct aatgtgatga tcaattttac cttatggtta acagtctgtt 1440

tcggtggtaa gttcaggcaa aagagaacga ttgcgttggg gaccgggagt ggctccgatg 1500

ctgggtttcg tggtgataat ttcaccatga aaaagttgtc agccccgctt attcaatgag 1560

gacaagatgc tcgagccgat tagcgaatac cagtaattca ctgattcgac tattttctaa 1620

aggcgcttcg gcgccttttt agtcagatga caaagtacaa aagtgctcag acagtcccct 1680

cgcccctttg gggagagggt tagggtgagg ggaacaggcc ggcactggcg cgaatattta 1740

ccctcacccc ggccctctcc ctgaaagggc gagggggaaa agcgtgccaa cattgaagat 1800

tgagccagtt tgttagcaat ctcaaagata cgtcaacgaa ttaatttttc tcggaaaaac 1860

aaatggctat agcacttgtg actggtggca gtcgcggcat cgggcgggca actgcattac 1920

tgttggcgca agaagggtat acggtggcgg ttaattatca gcaaaacctc cacgcggcgc 1980

aggaagtgat gaacttaata acgcaagccg gtggcaaagc attcgtgctc caggcggata 2040

tcagcgacga aaaccaggtc gttgcgatgt ttacagcaat cgatcagcac gatgaactct 2100

agagcggccg cgatcctcta gagtcgaccg gtggcgaatg ggacgcgccc tgtagcggcg 2160

cattaagcgc ggcgggtgtg gtggttacgc gcagcgtgac cgctacactt gccagcgccc 2220

tagcgcccgc tcctttcgct ttcttccctt cctttctcgc cacgttcgcc ggctttcccc 2280

gtcaagctct aaatcggggg ctccctttag ggttccgatt tagtgcttta cggcacctcg 2340

accccaaaaa acttgattag ggtgatggtt cacgtagtgg gccatcgccc tgatagacgg 2400

tttttcgccc tttgacgttg gagtccacgt tctttaatag tggactcttg ttccaaactg 2460

gaacaacact caaccctatc tcggtctatt cttttgattt ataagggatt ttgccgattt 2520

cggcctattg gttaaaaaat gagctgattt aacaaaaatt taacgcgaat tttaacaaaa 2580

tattaacgct tacaatttag gtggcacttt tcggggaaat gtgcgcggaa cccctatttg 2640

tttatttttc taaatacatt caaatatgta tccgctcacc gcgatccttt ttaacccatc 2700

acatatacct gccgttcact attatttagt gaaatgagat attatgatat tttctgaatt 2760

gtgattaaaa aggcaacttt atgcccatgc aacagaaact ataaaaaata cagagaatga 2820

aaagaaacag atagattttt tagttcttta ggcccgtagt ctgcaaatcc ttttatgatt 2880

ttctatcaaa caaaagagga aaatagacca gttgcaatcc aaacgagagt ctaatagaat 2940

gaggtcgaaa agtaaatcgc gcgggtttgt tactgataaa gcaggcaaga cctaaaatgt 3000

gtaaagggca aagtgtatac tttggcgtca ccccttacat attttaggtc tttttttatt 3060

gtgcgtaact aacttgccat cttcaaacag gagggctgga agaagcagac cgctaacaca 3120

gtacataaaa aaggagacat gaacgatgaa catcaaaaag tttgcaaaac aagcaacagt 3180

attaaccttt actaccgcac tgctggcagg aggcgcaact caagcgtttg cgaaagaaac 3240

gaaccaaaag ccatataagg aaacatacgg catttcccat attacacgcc atgatatgct 3300

gcaaatccct gaacagcaaa aaaatgaaaa atatcaagtt cctgagttcg attcgtccac 3360

aattaaaaat atctcttctg caaaaggcct ggacgtttgg gacagctggc cattacaaaa 3420

cgctgacggc actgtcgcaa actatcacgg ctaccacatc gtctttgcat tagccggaga 3480

tcctaaaaat gcggatgaca catcgattta catgttctat caaaaagtcg gcgaaacttc 3540

tattgacagc tggaaaaacg ctggccgcgt ctttaaagac agcgacaaat tcgatgcaaa 3600

tgattctatc ctaaaagacc aaacacaaga atggtcaggt tcagccacat ttacatctga 3660

cggaaaaatc cgtttattct acactgattt ctccggtaaa cattacggca aacaaacact 3720

gacaactgca caagttaacg tatcagcatc agacagctct ttgaacatca acggtgtaga 3780

ggattataaa tcaatctttg acggtgacgg aaaaacgtat caaaatgtac agcagttcat 3840

cgatgaaggc aactacagct caggcgacaa ccatacgctg agagatcctc actacgtaga 3900

agataaaggc cacaaatact tagtatttga agcaaacact ggaactgaag atggctacca 3960

aggcgaagaa tctttattta acaaagcata ctatggcaaa agcacatcat tcttccgtca 4020

agaaagtcaa aaacttctgc aaagcgataa aaaacgcacg gctgagttag caaacggcgc 4080

tctcggtatg attgagctaa acgatgatta cacactgaaa aaagtgatga aaccgctgat 4140

tgcatctaac acagtaacag atgaaattga acgcgcgaac gtctttaaaa tgaacggcaa 4200

atggtacctg ttcactgact cccgcggatc aaaaatgacg attgacggca ttacgtctaa 4260

cgatatttac atgcttggtt atgtttctaa ttctttaact ggcccataca agccgctgaa 4320

caaaactggc cttgtgttaa aaatggatct tgatcctaac gatgtaacct ttacttactc 4380

acacttcgct gtacctcaag cgaaaggaaa caatgtcgtg attacaagct atatgacaaa 4440

cagaggattc tacgcagaca aacaatcaac gtttgcgcca agcttcctgc tgaacatcaa 4500

aggcaagaaa acatctgttg tcaaagacag catccttgaa caaggacaat taacagttaa 4560

caaataaaaa cgcaaaagaa aatgccgata ttgactaccg gaagcagtgt gaccgtgtgc 4620

ttctcaaatg cctgattcag gctgtctatg tgtgactgtt gagctgtaac aagttgtctc 4680

aggtgttcaa tttcatgttc tagttgcttt gttttactgg tttcacctgt tctattaggt 4740

gttacatgct gttcatctgt tacattgtcg atctgttcat ggtgaacagc tttaaatgca 4800

ccaaaaactc gtaaaagctc tgatgtatct atctttttta caccgttttc atctgtgcat 4860

atggacagtt ttccctttga tatgtaacgg tgaacagttg ttctactttt gtttgttagt 4920

cttgatgctt cactgataga tacaagagcc ataagaacct cagatccttc cgtatttagc 4980

cagtatgttc tctagtgtgg ttcgttgttt ttgcgtgagc catgagaacg aaccattgag 5040

atcatactta ctttgcatgt cactcaaaaa ttttgcctca aaactggtga gctgaatttt 5100

tgcagttaaa gcatcgtgta gtgtttttct tagtccgtta tgtaggtagg aatctgatgt 5160

aatggttgtt ggtattttgt caccattcat ttttatctgg ttgttctcaa gttcggttac 5220

gagatccatt tgtctatcta gttcaacttg gaaaatcaac gtatcagtcg ggcggcctcg 5280

cttatcaacc accaatttca tattgctgta agtgtttaaa tctttactta ttggtttcaa 5340

aacccattgg ttaagccttt taaactcatg gtagttattt tcaagcatta acatgaactt 5400

aaattcatca aggctaatct ctatatttgc cttgtgagtt ttcttttgtg ttagttcttt 5460

taataaccac tcataaatcc tcatagagta tttgttttca aaagacttaa catgttccag 5520

attatatttt atgaattttt ttaactggaa aagataaggc aatatctctt cactaaaaac 5580

taattctaat ttttcgcttg agaacttggc atagtttgtc cactggaaaa tctcaaagcc 5640

tttaaccaaa ggattcctga tttccacagt tctcgtcatc agctctctgg ttgctttagc 5700

taatacacca taagcatttt ccctactgat gttcatcatc tgaacgtatt ggttataagt 5760

gaacgatacc gtccgttctt tccttgtagg gttttcaatc gtggggttga gtagtgccac 5820

acagcataaa attagcttgg tttcatgctc cgttaagtca tagcgactaa tcgctagttc 5880

atttgctttg aaaacaacta attcagacat acatctcaat tggtctaggt gattttaatc 5940

actataccaa ttgagatggg ctagtcaatg ataattacta gtccttttcc tttgagttgt 6000

gggtatctgt aaattctgct agacctttgc tggaaaactt gtaaattctg ctagaccctc 6060

tgtaaattcc gctagacctt tgtgtgtttt ttttgtttat attcaagtgg ttataattta 6120

tagaataaag aaagaataaa aaaagataaa aagaatagat cccagccctg tgtataactc 6180

actactttag tcagttccgc agtattacaa aaggatgtcg caaacgctgt ttgctcctct 6240

acaaaacaga ccttaaaacc ctaaaggctt aagtagcacc ctcgcaagct cgggcaaatc 6300

gctgaatatt ccttttgtct ccgaccatca ggcacctgag tcgctgtctt tttcgtgaca 6360

ttcagttcgc tgcgctcacg gctctggcag tgaatggggg taaatggcac tacaggcgcc 6420

ttttatggat tcatgcaagg aaactaccca taatacaaga aaagcccgtc acgggcttct 6480

cagggcgttt tatggcgggt ctgctatgtg gtgctatctg actttttgct gttcagcagt 6540

tcctgccctc tgattttcca gtctgaccac ttcggattat cccgtgacag gtcattcaga 6600

ctggctaatg cacccagtaa ggcagcggta tcatcaacag gcttacccgt cttactgtcg 6660

gggatcgacg ctctccctta tgcgactcct gca 6693

<210> 30
<211> 30
<212> DNA
<213> Artificial Sequence

<220>
<223> DNA sequence after deletion of gcvTHP

<400> 30
atgctcgagc cgattagcga ataccagtaa 30

<210> 31
<211> 38
<212> DNA
<213> Artificial Sequence

<220>
<223> oligonucleotide o-MO-44 for amplifying the upstream region of
 glyA

<400> 31
gggatcgcgg ccgcggaccg agggcttcac gttgatcg 38

<210> 32
<211> 45
<212> DNA
<213> Artificial Sequence

<220>
<223> oligonucleotide o-MO-45 for amplifying the upstream region of
 glyA

<400> 32
tatgcgtaaa ccgggtaacg ctcgagcatc cgcatctcct gactc 45

<210> 33
<211> 22
<212> DNA
<213> Artificial Sequence

<220>
<223> oligonucleotide o-MO-46 for amplifying the downstream region of
 glyA

<400> 33
cgttacccgg tttacgcata ag 22

<210> 34

<211>  39
<212>  DNA
<213>  Artificial Sequence

<220>
<223>  oligonucleotide o-MO-47 for amplifying the downstream region of
       glyA

<400>  34
ggatcgcggc cgctctagag atcaccagct gggttgatc                              39


<210>  35
<211>  546
<212>  DNA
<213>  Artificial Sequence

<220>
<223>  PCR product 1 from Example 14

<400>  35
gggatcgcgg ccgcggaccg agggcttcac gttgatcgcc attacgctgg ttactcatgt       60

taaaaatttc tttgagttct gggttatgag taaacatacg gtcgtagaaa tgggcggtta      120

actttggccc cgtttccacc agtaaaggga tggtggcttt tactgtagcg atggtttgag      180

cgtcaagcat atggtcttcc tttttttgca tcttaattga tgtatctcaa atgcatctta      240

taaaaaatag ccctgcaatg taaatggttc tttggtgttt ttcagaaaga atgtgatgaa      300

gtgaaaaatt tgcatcacaa acctgaaaag aaatccgttt ccggttgcaa gctctttatt      360

ctccaaagcc ttgcgtagcc tgaaggtaat cgtttgcgta aattcctttg tcaagacctg      420

ttatcgcaca atgattcggt tatactgttc gccgttgtcc aacaggaccg cctataaagg      480

ccaaaaattt tattgttagc tgagtcagga gatgcggatg ctcgagcgtt acccggttta      540

cgcata                                                                 546


<210>  36
<211>  520
<212>  DNA
<213>  Artificial Sequence

<220>
<223>  PCR product 2 from Example 14

<400>  36
cgttacccgg tttacgcata agcgaaacgg tgatttgctg tcaatgtgct cgttgttcat       60

gccggatgcg gcgtgaacgc cttatccggc ctacaaaact ttgcaaattc aatatattgc      120

aatctccgtg taggcctgat aagcgtagcg catcaggcaa tttttcgttt atgatcatca      180

aggcttcctt cgggaagcct ttctacgtta tcgcgccatc aaatctgtcg taactgcgcc      240

tcaacataca aatagccaat tcccagcacc tgttgtgcgc ggcttaattg cccaaagcca      300

atttgcgtcg ctttcacgct cggtccttcg tcgcggtcga acgggttaaa tcctgtctgg      360

cgaataatag tattaagcca gttctcacca aaggcacaac tgcgaccgta caaccaaatc 420

tgattaatat tgaggatgtt gaggaagtta tacagactca aaccaatggc attggcagag 480

cgatcaaccc agctggtgat ctctagagcg gccgcgatcc 520

<210> 37
<211> 26
<212> DNA
<213> Artificial Sequence

<220>
<223> oligonucleotide o-MO-52

<400> 37
actgaggatc cgggatcgcg gccgcg 26

<210> 38
<211> 31
<212> DNA
<213> Artificial Sequence

<220>
<223> oligonucleotide o-MO-53

<400> 38
actgaggatc cggatcgcgg ccgctctaga g 31

<210> 39
<211> 1052
<212> DNA
<213> Artificial Sequence

<220>
<223> PCR product 3 from Example 14

<400> 39
gatccgggat cgcggccgcg gaccgagggc ttcacgttga tcgccattac gctggttact 60

catgttaaaa atttctttga gttctgggtt atgagtaaac atacggtcgt agaaatgggc 120

ggttaacttt ggccccgttt ccaccagtaa agggatggtg gcttttactg tagcgatggt 180

ttgagcgtca agcatatggt cttccttttt ttgcatctta attgatgtat ctcaaatgca 240

tcttataaaa aatagccctg caatgtaaat ggttctttgg tgtttttcag aaagaatgtg 300

atgaagtgaa aaatttgcat cacaaacctg aaaagaaatc cgtttccggt tgcaagctct 360

ttattctcca aagccttgcg tagcctgaag gtaatcgttt gcgtaaattc ctttgtcaag 420

acctgttatc gcacaatgat tcggttatac tgttcgccgt tgtccaacag gaccgcctat 480

aaaggccaaa aattttattg ttagctgagt caggagatgc ggatgctcga gcgttacccg 540

gtttacgcat aagcgaaacg gtgatttgct gtcaatgtgc tcgttgttca tgccggatgc 600

ggcgtgaacg ccttatccgg cctacaaaac tttgcaaatt caatatattg caatctccgt 660

gtaggcctga taagcgtagc gcatcaggca atttttcgtt tatgatcatc aaggcttcct 720

tcgggaagcc tttctacgtt atcgcgccat caaatctgtc gtaactgcgc ctcaacatac 780

aaatagccaa ttcccagcac ctgttgtgcg cggcttaatt gcccaaagcc aatttgcgtc 840

gctttcacgc tcggtccttc gtcgcggtcg aacgggttaa atcctgtctg gcgaataata 900

gtattaagcc agttctcacc aaaggcacaa ctgcgaccgt acaaccaaat ctgattaata 960

ttgaggatgt tgaggaagtt atacagactc aaaccaatgg cattggcaga gcgatcaacc 1020

cagctggtga tctctagagc ggccgcgatc cg 1052

<210> 40
<211> 6719
<212> DNA
<213> Artificial Sequence

<220>
<223> pKO3 delta glyA

<400> 40
cctttcgtct tcgaataaat acctgtgacg gaagatcact tcgcagaata aataaatcct 60

ggtgtccctg ttgataccgg gaagccctgg gccaactttt ggcgaaaatg agacgttgat 120

cggcacgtaa gaggttccaa ctttcaccat aatgaaataa gatcactacc gggcgtattt 180

tttgagttat cgagattttc aggagctaag gaagctaaaa tggagaaaaa aatcactgga 240

tataccaccg ttgatatatc ccaatggcat cgtaaagaac attttgaggc atttcagtca 300

gttgctcaat gtacctataa ccagaccgtt cagctggata ttacggcctt tttaaagacc 360

gtaaagaaaa ataagcacaa gttttatccg gcctttattc acattcttgc ccgcctgatg 420

aatgctcatc cggaattccg tatggcaatg aaagacggtg agctggtgat atgggatagt 480

gttcaccctt gttacaccgt tttccatgag caaactgaaa cgttttcatc gctctggagt 540

gaataccacg acgatttccg gcagtttcta cacatatatt cgcaagatgt ggcgtgttac 600

ggtgaaaacc tggcctattt ccctaaaggg tttattgaga atatgttttt cgtctcagcc 660

aatccctggg tgagtttcac cagttttgat ttaaacgtgg ccaatatgga caacttcttc 720

gcccccgttt tcaccatggg caaatattat acgcaaggcg acaaggtgct gatgccgctg 780

gcgattcagg ttcatcatgc cgtttgtgat ggcttccatg tcggcagaat gcttaatgaa 840

ttacaacagt actgcgatga gtggcagggc ggggcgtaat ttttttaagg cagttattgg 900

tgcccttaaa cgcctggttg ctacgcctga ataagtgata ataagcggat gaatggcaga 960

aattcgaaag caaattcgac ccggtcgtcg gttcagggca gggtcgttaa atagccgctt 1020

atgtctattg ctggtctcgg tacccgggga tcgcggccgc ggaccggatc cgggatcgcg 1080

gccgcggacc gagggcttca cgttgatcgc cattacgctg gttactcatg ttaaaaattt 1140

ctttgagttc tgggttatga gtaaacatac ggtcgtagaa atgggcggtt aactttggcc 1200

ccgtttccac cagtaaaggg atggtggctt ttactgtagc gatggtttga gcgtcaagca 1260

tatggtcttc ctttttttgc atcttaattg atgtatctca aatgcatctt ataaaaaata 1320

gccctgcaat gtaaatggtt ctttggtgtt tttcagaaag aatgtgatga agtgaaaaat 1380

ttgcatcaca aacctgaaaa gaaatccgtt tccggttgca agctctttat tctccaaagc 1440

cttgcgtagc ctgaaggtaa tcgtttgcgt aaattccttt gtcaagacct gttatcgcac 1500

aatgattcgg ttatactgtt cgccgttgtc caacaggacc gcctataaag gccaaaaatt 1560

ttattgttag ctgagtcagg agatgcggat gctcgagcgt tacccggttt acgcataagc 1620

gaaacggtga tttgctgtca atgtgctcgt tgttcatgcc ggatgcggcg tgaacgcctt 1680

atccggccta caaaactttg caaattcaat atattgcaat ctccgtgtag gcctgataag 1740

cgtagcgcat caggcaattt ttcgtttatg atcatcaagg cttccttcgg gaagcctttc 1800

tacgttatcg cgccatcaaa tctgtcgtaa ctgcgcctca acatacaaat agccaattcc 1860

cagcacctgt tgtgcgcggc ttaattgccc aaagccaatt tgcgtcgctt tcacgctcgg 1920

tccttcgtcg cggtcgaacg ggttaaatcc tgtctggcga ataatagtat taagccagtt 1980

ctcaccaaag gcacaactgc gaccgtacaa ccaaatctga ttaatattga ggatgttgag 2040

gaagttatac agactcaaac caatggcatt ggcagagcga tcaacccagc tggtgatctc 2100

tagagcggcc gcgatccgga tcctctagag cggccgcgat cctctagagt cgaccggtgg 2160

cgaatgggac gcgccctgta gcggcgcatt aagcgcggcg ggtgtggtgg ttacgcgcag 2220

cgtgaccgct acacttgcca gcgccctagc gcccgctcct ttcgctttct tcccttcctt 2280

tctcgccacg ttcgccggct ttccccgtca agctctaaat cgggggctcc ctttagggtt 2340

ccgatttagt gctttacggc acctcgaccc caaaaaactt gattagggtg atggttcacg 2400

tagtgggcca tcgccctgat agacggtttt tcgccctttg acgttggagt ccacgttctt 2460

taatagtgga ctcttgttcc aaactggaac aacactcaac cctatctcgg tctattcttt 2520

tgatttataa gggattttgc cgatttcggc ctattggtta aaaaatgagc tgatttaaca 2580

aaaatttaac gcgaatttta acaaaatatt aacgcttaca atttaggtgg cacttttcgg 2640

ggaaatgtgc gcggaacccc tatttgttta tttttctaaa tacattcaaa tatgtatccg 2700

ctcaccgcga tcctttttaa cccatcacat atacctgccg ttcactatta tttagtgaaa 2760

tgagatatta tgatattttc tgaattgtga ttaaaaaggc aactttatgc ccatgcaaca 2820

gaaactataa aaaatacaga gaatgaaaag aaacagatag attttttagt tctttaggcc 2880

cgtagtctgc aaatcctttt atgattttct atcaaacaaa agaggaaaat agaccagttg 2940

caatccaaac gagagtctaa tagaatgagg tcgaaaagta aatcgcgcgg gtttgttact 3000

gataaagcag gcaagaccta aaatgtgtaa agggcaaagt gtatactttg gcgtcacccc 3060

ttacatattt taggtctttt tttattgtgc gtaactaact tgccatcttc aaacaggagg 3120

gctggaagaa gcagaccgct aacacagtac ataaaaaagg agacatgaac gatgaacatc 3180

aaaaagtttg caaaacaagc aacagtatta acctttacta ccgcactgct ggcaggaggc 3240

gcaactcaag cgtttgcgaa agaaacgaac caaaagccat ataaggaaac atacggcatt 3300

tcccatatta cacgccatga tatgctgcaa atccctgaac agcaaaaaaa tgaaaaatat 3360

caagttcctg agttcgattc gtccacaatt aaaaatatct cttctgcaaa aggcctggac 3420

gtttgggaca gctggccatt acaaaacgct gacggcactg tcgcaaacta tcacggctac 3480

cacatcgtct ttgcattagc cggagatcct aaaaatgcgg atgacacatc gatttacatg 3540

ttctatcaaa aagtcggcga aacttctatt gacagctgga aaaacgctgg ccgcgtcttt 3600

aaagacagcg acaaattcga tgcaaatgat tctatcctaa aagaccaaac acaagaatgg 3660

tcaggttcag ccacatttac atctgacgga aaaatccgtt tattctacac tgatttctcc 3720

ggtaaacatt acggcaaaca aacactgaca actgcacaag ttaacgtatc agcatcagac 3780

agctctttga acatcaacgg tgtagaggat tataaatcaa tctttgacgg tgacggaaaa 3840

acgtatcaaa atgtacagca gttcatcgat gaaggcaact acagctcagg cgacaaccat 3900

acgctgagag atcctcacta cgtagaagat aaaggccaca aatacttagt atttgaagca 3960

aacactggaa ctgaagatgg ctaccaaggc gaagaatctt tatttaacaa agcatactat 4020

ggcaaaagca catcattctt ccgtcaagaa agtcaaaaac ttctgcaaag cgataaaaaa 4080

cgcacggctg agttagcaaa cggcgctctc ggtatgattg agctaaacga tgattacaca 4140

ctgaaaaaag tgatgaaacc gctgattgca tctaacacag taacagatga aattgaacgc 4200

gcgaacgtct ttaaaatgaa cggcaaatgg tacctgttca ctgactcccg cggatcaaaa 4260

atgacgattg acggcattac gtctaacgat atttacatgc ttggttatgt ttctaattct 4320

ttaactggcc catacaagcc gctgaacaaa actggccttg tgttaaaaat ggatcttgat 4380

cctaacgatg taacctttac ttactcacac ttcgctgtac ctcaagcgaa aggaaacaat 4440

gtcgtgatta caagctatat gacaaacaga ggattctacg cagacaaaca atcaacgttt 4500

gcgccaagct tcctgctgaa catcaaaggc aagaaaacat ctgttgtcaa agacagcatc 4560

cttgaacaag gacaattaac agttaacaaa taaaaacgca aaagaaaatg ccgatattga 4620

ctaccggaag cagtgtgacc gtgtgcttct caaatgcctg attcaggctg tctatgtgtg 4680

actgttgagc tgtaacaagt tgtctcaggt gttcaatttc atgttctagt tgctttgttt 4740

tactggtttc acctgttcta ttaggtgtta catgctgttc atctgttaca ttgtcgatct 4800

gttcatggtg aacagcttta aatgcaccaa aaactcgtaa aagctctgat gtatctatct 4860

tttttacacc gttttcatct gtgcatatgg acagttttcc ctttgatatg taacggtgaa 4920

cagttgttct acttttgttt gttagtcttg atgcttcact gatagataca agagccataa 4980

gaacctcaga tccttccgta tttagccagt atgttctcta gtgtggttcg ttgtttttgc 5040

gtgagccatg agaacgaacc attgagatca tacttacttt gcatgtcact caaaaatttt 5100

gcctcaaaac tggtgagctg aatttttgca gttaaagcat cgtgtagtgt ttttcttagt 5160

ccgttatgta ggtaggaatc tgatgtaatg gttgttggta ttttgtcacc attcattttt 5220

atctggttgt tctcaagttc ggttacgaga tccatttgtc tatctagttc aacttggaaa 5280

atcaacgtat cagtcgggcg gcctcgctta tcaaccacca atttcatatt gctgtaagtg 5340

tttaaatctt tacttattgg tttcaaaacc cattggttaa gccttttaaa ctcatggtag 5400

ttattttcaa gcattaacat gaacttaaat tcatcaaggc taatctctat atttgccttg 5460

tgagttttct tttgtgttag ttcttttaat aaccactcat aaatcctcat agagtatttg 5520

ttttcaaaag acttaacatg ttccagatta tattttatga atttttttaa ctggaaaaga 5580

taaggcaata tctcttcact aaaaactaat tctaattttt cgcttgagaa cttggcatag 5640

tttgtccact ggaaaatctc aaagccttta accaaaggat tcctgatttc cacagttctc 5700

gtcatcagct ctctggttgc tttagctaat acaccataag cattttccct actgatgttc 5760

atcatctgaa cgtattggtt ataagtgaac gataccgtcc gttctttcct tgtagggttt 5820

tcaatcgtgg ggttgagtag tgccacacag cataaaatta gcttggtttc atgctccgtt 5880

aagtcatagc gactaatcgc tagttcattt gctttgaaaa caactaattc agacatacat 5940

ctcaattggt ctaggtgatt ttaatcacta taccaattga gatgggctag tcaatgataa 6000

ttactagtcc ttttcctttg agttgtgggt atctgtaaat tctgctagac ctttgctgga 6060

aaacttgtaa attctgctag accctctgta aattccgcta gacctttgtg tgtttttttt 6120

gtttatattc aagtggttat aatttataga ataaagaaag aataaaaaaa gataaaaaga 6180

atagatccca gccctgtgta taactcacta ctttagtcag ttccgcagta ttacaaaagg 6240

atgtcgcaaa cgctgtttgc tcctctacaa aacagacctt aaaaccctaa aggcttaagt 6300

agcaccctcg caagctcggg caaatcgctg aatattcctt ttgtctccga ccatcaggca 6360

cctgagtcgc tgtctttttc gtgacattca gttcgctgcg ctcacggctc tggcagtgaa 6420

tgggggtaaa tggcactaca ggcgcctttt atggattcat gcaaggaaac tacccataat 6480

acaagaaaag cccgtcacgg gcttctcagg gcgttttatg gcgggtctgc tatgtggtgc 6540

tatctgactt tttgctgttc agcagttcct gccctctgat tttccagtct gaccacttcg 6600

gattatcccg tgacaggtca ttcagactgg ctaatgcacc cagtaaggca gcggtatcat 6660

caacaggctt acccgtctta ctgtcgggga tcgacgctct cccttatgcg actcctgca 6719

<210> 41
<211> 30
<212> DNA
<213> Artificial Sequence

<220>
<223>  DNA sequence after deletion of glyA

<400>  41
atgctcgagc gttacccggt ttacgcataa                                        30


<210>  42
<211>  39
<212>  DNA
<213>  Artificial Sequence

<220>
<223>  ltaE-UP_fw

<400>  42
ggatcgcggc cgcggaccgc aggcgacaga gccagaacg                              39


<210>  43
<211>  47
<212>  DNA
<213>  Artificial Sequence

<220>
<223>  ltAE-UP-XhoI_rev

<400>  43
ctctccttaa cgcgccagga actcgagcat ggcatgtcct tattatg                     47


<210>  44
<211>  21
<212>  DNA
<213>  Artificial Sequence

<220>
<223>  ltaE-DOWN_fw

<400>  44
ttcctggcgc gttaaggaga g                                                 21


<210>  45
<211>  40
<212>  DNA
<213>  Artificial Sequence

<220>
<223>  ltaE-DOWN_rev

<400>  45
ggatcgcggc cgctctagag tagaccatat cgcgcatgac                             40


<210>  46
<211>  540
<212>  DNA
<213>  Artificial Sequence

<220>
<223>  PCR product 4 from Example 15

<400>  46
ggatcgcggc cgcggaccgc aggcgacaga gccagaacgt caggtggtcg ccatgtgcgg      60

cgatggcggt tttagcatgt tgatgggcga tttcctctca gtagtgcaga tgaaactgcc     120

agtgaaaatt gtcgtcttta acaacagcgt gctgggcttt gtggcgatgg agatgaaagc     180

tggtggctat ttgactgacg gcaccgaact acacgacaca aactttgccc gcattgccga     240

agcgtgcggc attacgggta tccgtgtaga aaaagcgtct gaagttgatg aagccctgca     300

acgcgccttc tccatcgacg gtccggtgtt ggtggatgtg gtggtcgcca aagaagagtt     360

agccattcca ccgcagatca aactcgaaca ggccaaaggt ttcagcctgt atatgctgcg     420

cgcaatcatc agcggacgcg gtgatgaagt gatcgaactg gcgaaaacaa actggctaag     480

gtaaaaaggg tggcatttcc cgtcataata aggacatgcc atgctcgagt tcctggcgcg     540


<210>  47
<211>  536
<212>  DNA
<213>  Artificial Sequence

<220>
<223>  PCR product 5 from Example 15

<400>  47
ttcctggcgc gttaaggaga gagacgtgcc gcaacgcatt ttagttctcg gtgccagtgg      60

ctacattggt cagcatctgg tgcgcacact cagccagcaa gggcatcaga tcctggcggc     120

ggcacgtcat gtcgacaggc ttgcaaagct gcaactggca aacgtcagtt gccataaagt     180

cgatctcagc tggccggata acctaccggc cctgttgcag gatatcgata cggtctattt     240

tctggtgcac agcatgggcg aaggcggcga ttttatcgct caggagcgcc aggtggctct     300

caacgtccgc gatgcgctac gtgaagtacc agttaagcaa ttaatctttc ttagttcgtt     360

gcaggccccg ccacatgagc agtcggatca tctgcgtgct cgtcaggcta cggcggacat     420

tctgcgtgaa gcgaatgtac ccgtgaccga actgcgggcc ggaattatcg ttggcgcagg     480

ttccgcggcg ttcgaagtca tgcgcgatat ggtctactct agagcggccg cgatcc         536


<210>  48
<211>  26
<212>  DNA
<213>  Artificial Sequence

<220>
<223>  oligonucleotide o-MO-54 from Example 15

<400>  48
actgaggatc cggatcgcgg ccgcgg                                           26


<210>  49
<211>  29

<212>  DNA
<213>  Artificial Sequence

<220>
<223>  oligoneucleotide o-MO-55 from Example 15

<400>  49
actgaggatc cggatcgcgg ccgctctag                                          29


<210>  50
<211>  1071
<212>  DNA
<213>  Artificial Sequence

<220>
<223>  PCR product 6 from Example 15

<400>  50
gatccggatc gcggccgcgg accgcaggcg acagagccag aacgtcaggt ggtcgccatg        60

tgcggcgatg gcggttttag catgttgatg ggcgatttcc tctcagtagt gcagatgaaa       120

ctgccagtga aaattgtcgt ctttaacaac agcgtgctgg gctttgtggc gatggagatg       180

aaagctggtg gctatttgac tgacggcacc gaactacacg acacaaactt tgcccgcatt       240

gccgaagcgt gcggcattac gggtatccgt gtagaaaaag cgtctgaagt tgatgaagcc       300

ctgcaacgcg ccttctccat cgacggtccg gtgttggtgg atgtggtggt cgccaaagaa       360

gagttagcca ttccaccgca gatcaaactc gaacaggcca aaggtttcag cctgtatatg       420

ctgcgcgcaa tcatcagcgg acgcggtgat gaagtgatcg aactggcgaa aacaaactgg       480

ctaaggtaaa aagggtggca tttcccgtca taataaggac atgccatgct cgagttcctg       540

gcgcgttaag gagagagacg tgccgcaacg cattttagtt ctcggtgcca gtggctacat       600

tggtcagcat ctggtgcgca cactcagcca gcaagggcat cagatcctgg cggcggcacg       660

tcatgtcgac aggcttgcaa agctgcaact ggcaaacgtc agttgccata aagtcgatct       720

cagctggccg gataacctac cggccctgtt gcaggatatc gatacggtct attttctggt        780

gcacagcatg ggcgaaggcg gcgattttat cgctcaggag cgccaggtgg ctctcaacgt       840

ccgcgatgcg ctacgtgaag taccagttaa gcaattaatc tttcttagtt cgttgcaggc       900

cccgccacat gagcagtcgg atcatctgcg tgctcgtcag gctacggcgg acattctgcg       960

tgaagcgaat gtacccgtga ccgaactgcg ggccggaatt atcgttggcg caggttccgc      1020

ggcgttcgaa gtcatgcgcg atatggtcta ctctagagcg gccgcgatcc g               1071


<210>  51
<211>  6738
<212>  DNA
<213>  Artificial Sequence

<220>
<223>  pKO3 delta ltaE

<400>  51
cctttcgtct tcgaataaat acctgtgacg gaagatcact tcgcagaata aataaatcct      60

ggtgtccctg ttgataccgg gaagccctgg gccaactttt ggcgaaaatg agacgttgat     120

cggcacgtaa gaggttccaa ctttcaccat aatgaaataa gatcactacc gggcgtattt     180

tttgagttat cgagattttc aggagctaag gaagctaaaa tggagaaaaa aatcactgga     240

tataccaccg ttgatatatc ccaatggcat cgtaaagaac attttgaggc atttcagtca     300

gttgctcaat gtacctataa ccagaccgtt cagctggata ttacggcctt tttaaagacc     360

gtaaagaaaa ataagcacaa gttttatccg gcctttattc acattcttgc ccgcctgatg     420

aatgctcatc cggaattccg tatggcaatg aaagacggtg agctggtgat atgggatagt     480

gttcaccctt gttacaccgt tttccatgag caaactgaaa cgttttcatc gctctggagt     540

gaataccacg acgatttccg gcagtttcta cacatatatt cgcaagatgt ggcgtgttac     600

ggtgaaaacc tggcctattt ccctaaaggg tttattgaga atatgttttt cgtctcagcc     660

aatccctggg tgagtttcac cagttttgat ttaaacgtgg ccaatatgga caacttcttc     720

gcccccgttt tcaccatggg caaatattat acgcaaggcg acaaggtgct gatgccgctg     780

gcgattcagg ttcatcatgc cgtttgtgat ggcttccatg tcggcagaat gcttaatgaa     840

ttacaacagt actgcgatga gtggcagggc ggggcgtaat ttttttaagg cagttattgg     900

tgcccttaaa cgcctggttg ctacgcctga ataagtgata ataagcggat gaatggcaga     960

aattcgaaag caaattcgac ccggtcgtcg gttcagggca gggtcgttaa atagccgctt    1020

atgtctattg ctggtctcgg tacccgggga tcgcggccgc ggaccggatc cggatcgcgg    1080

ccgcggaccg caggcgacag agccagaacg tcaggtggtc gccatgtgcg gcgatggcgg    1140

ttttagcatg ttgatgggcg atttcctctc agtagtgcag atgaaactgc cagtgaaaat    1200

tgtcgtcttt aacaacagcg tgctgggctt tgtggcgatg gagatgaaag ctggtggcta    1260

tttgactgac ggcaccgaac tacacgacac aaactttgcc cgcattgccg aagcgtgcgg    1320

cattacgggt atccgtgtag aaaaagcgtc tgaagttgat gaagccctgc aacgcgcctt    1380

ctccatcgac ggtccggtgt tggtggatgt ggtggtcgcc aaagaagagt tagccattcc    1440

accgcagatc aaactcgaac aggccaaagg tttcagcctg tatatgctgc gcgcaatcat    1500

cagcggacgc ggtgatgaag tgatcgaact ggcgaaaaca aactggctaa ggtaaaaagg    1560

gtggcatttc ccgtcataat aaggacatgc catgctcgag ttcctggcgc gttaaggaga    1620

gagacgtgcc gcaacgcatt ttagttctcg gtgccagtgg ctacattggt cagcatctgg    1680

tgcgcacact cagccagcaa gggcatcaga tcctggcggc ggcacgtcat gtcgacaggc    1740

ttgcaaagct gcaactggca aacgtcagtt gccataaagt cgatctcagc tggccggata    1800

acctaccggc cctgttgcag gatatcgata cggtctattt tctggtgcac agcatgggcg    1860

aaggcggcga ttttatcgct caggagcgcc aggtggctct caacgtccgc gatgcgctac 1920

gtgaagtacc agttaagcaa ttaatctttc ttagttcgtt gcaggccccg ccacatgagc 1980

agtcggatca tctgcgtgct cgtcaggcta cggcggacat tctgcgtgaa gcgaatgtac 2040

ccgtgaccga actgcgggcc ggaattatcg ttggcgcagg ttccgcggcg ttcgaagtca 2100

tgcgcgatat ggtctactct agagcggccg cgatccggat cctctagagc ggccgcgatc 2160

ctctagagtc gaccggtggc gaatgggacg cgccctgtag cggcgcatta agcgcggcgg 2220

gtgtggtggt tacgcgcagc gtgaccgcta cacttgccag cgccctagcg cccgctcctt 2280

tcgctttctt cccttccttt ctcgccacgt tcgccggctt tccccgtcaa gctctaaatc 2340

gggggctccc tttagggttc cgatttagtg ctttacggca cctcgacccc aaaaaacttg 2400

attagggtga tggttcacgt agtgggccat cgccctgata gacggttttt cgccctttga 2460

cgttggagtc cacgttcttt aatagtggac tcttgttcca aactggaaca acactcaacc 2520

ctatctcggt ctattctttt gatttataag ggattttgcc gatttcggcc tattggttaa 2580

aaaatgagct gatttaacaa aaatttaacg cgaattttaa caaaatatta acgcttacaa 2640

tttaggtggc acttttcggg gaaatgtgcg cggaacccct atttgtttat ttttctaaat 2700

acattcaaat atgtatccgc tcaccgcgat cctttttaac ccatcacata tacctgccgt 2760

tcactattat ttagtgaaat gagatattat gatattttct gaattgtgat taaaaaggca 2820

actttatgcc catgcaacag aaactataaa aaatacagag aatgaaaaga aacagataga 2880

ttttttagtt ctttaggccc gtagtctgca aatcctttta tgattttcta tcaaacaaaa 2940

gaggaaaata gaccagttgc aatccaaacg agagtctaat agaatgaggt cgaaaagtaa 3000

atcgcgcggg tttgttactg ataaagcagg caagacctaa aatgtgtaaa gggcaaagtg 3060

tatactttgg cgtcacccct tacatatttt aggtcttttt ttattgtgcg taactaactt 3120

gccatcttca aacaggaggg ctggaagaag cagaccgcta acacagtaca taaaaaagga 3180

gacatgaacg atgaacatca aaaagtttgc aaaacaagca acagtattaa cctttactac 3240

cgcactgctg gcaggaggcg caactcaagc gtttgcgaaa gaaacgaacc aaaagccata 3300

taaggaaaca tacggcattt cccatattac acgccatgat atgctgcaaa tccctgaaca 3360

gcaaaaaaat gaaaaatatc aagttcctga gttcgattcg tccacaatta aaaatatctc 3420

ttctgcaaaa ggcctggacg tttgggacag ctggccatta caaaacgctg acggcactgt 3480

cgcaaactat cacggctacc acatcgtctt tgcattagcc ggagatccta aaaatgcgga 3540

tgacacatcg atttacatgt tctatcaaaa agtcggcgaa acttctattg acagctggaa 3600

aaacgctggc cgcgtcttta aagacagcga caaattcgat gcaaatgatt ctatcctaaa 3660

agaccaaaca caagaatggt caggttcagc cacatttaca tctgacggaa aaatccgttt 3720

attctacact gatttctccg gtaaacatta cggcaaacaa acactgacaa ctgcacaagt 3780
taacgtatca gcatcagaca gctctttgaa catcaacggt gtagaggatt ataaatcaat 3840
ctttgacggt gacggaaaaa cgtatcaaaa tgtacagcag ttcatcgatg aaggcaacta 3900
cagctcaggc gacaaccata cgctgagaga tcctcactac gtagaagata aaggccacaa 3960
atacttagta tttgaagcaa acactggaac tgaagatggc taccaaggcg aagaatcttt 4020
atttaacaaa gcatactatg gcaaaagcac atcattcttc cgtcaagaaa gtcaaaaact 4080
tctgcaaagc gataaaaaac gcacggctga gttagcaaac ggcgctctcg gtatgattga 4140
gctaaacgat gattacacac tgaaaaaagt gatgaaaccg ctgattgcat ctaacacagt 4200
aacagatgaa attgaacgcg cgaacgtctt taaaatgaac ggcaaatggt acctgttcac 4260
tgactcccgc ggatcaaaaa tgacgattga cggcattacg tctaacgata tttacatgct 4320
tggttatgtt tctaattctt taactggccc atacaagccg ctgaacaaaa ctggccttgt 4380
gttaaaaatg gatcttgatc ctaacgatgt aacctttact tactcacact tcgctgtacc 4440
tcaagcgaaa ggaaacaatg tcgtgattac aagctatatg acaaacagag gattctacgc 4500
agacaaacaa tcaacgtttg cgccaagctt cctgctgaac atcaaaggca agaaaacatc 4560
tgttgtcaaa gacagcatcc ttgaacaagg acaattaaca gttaacaaat aaaaacgcaa 4620
aagaaaatgc cgatattgac taccggaagc agtgtgaccg tgtgcttctc aaatgcctga 4680
ttcaggctgt ctatgtgtga ctgttgagct gtaacaagtt gtctcaggtg ttcaatttca 4740
tgttctagtt gctttgtttt actggtttca cctgttctat taggtgttac atgctgttca 4800
tctgttacat tgtcgatctg ttcatggtga acagctttaa atgcaccaaa aactcgtaaa 4860
agctctgatg tatctatctt ttttacaccg ttttcatctg tgcatatgga cagttttccc 4920
tttgatatgt aacggtgaac agttgttcta cttttgtttg ttagtcttga tgcttcactg 4980
atagatacaa gagccataag aacctcagat ccttccgtat ttagccagta tgttctctag 5040
tgtggttcgt tgtttttgcg tgagccatga gaacgaacca ttgagatcat acttactttg 5100
catgtcactc aaaaattttg cctcaaaact ggtgagctga atttttgcag ttaaagcatc 5160
gtgtagtgtt tttcttagtc cgttatgtag gtaggaatct gatgtaatgg ttgttggtat 5220
tttgtcacca ttcattttta tctggttgtt ctcaagttcg gttacgagat ccatttgtct 5280
atctagttca acttggaaaa tcaacgtatc agtcgggcgg cctcgcttat caaccaccaa 5340
tttcatattg ctgtaagtgt ttaaatcttt acttattggt ttcaaaaccc attggttaag 5400
ccttttaaac tcatggtagt tattttcaag cattaacatg aacttaaatt catcaaggct 5460
aatctctata tttgccttgt gagttttctt ttgtgttagt tcttttaata accactcata 5520
aatcctcata gagtatttgt tttcaaaaga cttaacatgt tccagattat attttatgaa 5580
tttttttaac tggaaaagat aaggcaatat ctcttcacta aaaactaatt ctaatttttc 5640

gcttgagaac ttggcatagt ttgtccactg gaaaatctca aagcctttaa ccaaaggatt 5700

cctgatttcc acagttctcg tcatcagctc tctggttgct ttagctaata caccataagc 5760

attttcccta ctgatgttca tcatctgaac gtattggtta taagtgaacg ataccgtccg 5820

ttctttcctt gtagggtttt caatcgtggg gttgagtagt gccacacagc ataaaattag 5880

cttggtttca tgctccgtta agtcatagcg actaatcgct agttcatttg ctttgaaaac 5940

aactaattca gacatacatc tcaattggtc taggtgattt taatcactat accaattgag 6000

atgggctagt caatgataat tactagtcct tttcctttga gttgtgggta tctgtaaatt 6060

ctgctagacc tttgctggaa aacttgtaaa ttctgctaga ccctctgtaa attccgctag 6120

acctttgtgt gttttttttg tttatattca agtggttata atttatagaa taaagaaaga 6180

ataaaaaaag ataaaaagaa tagatcccag ccctgtgtat aactcactac tttagtcagt 6240

tccgcagtat tacaaaagga tgtcgcaaac gctgtttgct cctctacaaa acagacctta 6300

aaaccctaaa ggcttaagta gcaccctcgc aagctcgggc aaatcgctga atattccttt 6360

tgtctccgac catcaggcac ctgagtcgct gtctttttcg tgacattcag ttcgctgcgc 6420

tcacggctct ggcagtgaat gggggtaaat ggcactacag gcgcctttta tggattcatg 6480

caaggaaact acccataata caagaaaagc ccgtcacggg cttctcaggg cgttttatgg 6540

cgggtctgct atgtggtgct atctgacttt ttgctgttca gcagttcctg ccctctgatt 6600

ttccagtctg accacttcgg attatcccgt gacaggtcat tcagactggc taatgcaccc 6660

agtaaggcag cggtatcatc aacaggctta cccgtcttac tgtcggggat cgacgctctc 6720

ccttatgcga ctcctgca 6738

<210> 52
<211> 24
<212> DNA
<213> Artificial Sequence

<220>
<223> DNA sequence after deletion of ltaE

<400> 52
atgctcgagt tcctggcgcg ttaa 24

<210> 53
<211> 294
<212> PRT
<213> Nomocharis pardanthina

<400> 53

Met Leu Val Leu His Asn Ala Gln Lys Leu Gln Ile Leu Tyr Lys Ser
1               5                   10                  15

Leu Glu Lys Ser Ile Pro Glu Ser Ile Lys Val Tyr Gly Ala Ile Phe
                20                  25                  30

Asn Ile Lys Asp Lys Asn Pro Phe Asn Met Glu Val Leu Val Asp Ala
            35                  40                  45

Trp Pro Asp Tyr Gln Ile Val Ile Thr Arg Pro Gln Lys Gln Glu Met
    50                  55                  60

Lys Asp Asp Gln Asp His Tyr Thr Asn Thr Tyr His Ile Phe Thr Lys
65                  70                  75                  80

Ala Pro Asp Lys Leu Glu Glu Val Leu Ser Tyr Ser Ser Val Ile Asn
                85                  90                  95

Trp Glu Gln Thr Leu Gln Ile Gln Gly Cys Gln Glu Gly Leu Asp Glu
            100                 105                 110

Ala Ile Arg Lys Ala Ala Thr Ser Lys Ser Val Gln Val Asp Tyr Met
        115                 120                 125

Lys Thr Met Leu Phe Ile Pro Glu Leu Pro Lys Lys His Lys Thr Ser
    130                 135                 140

Ser Asn Glu Lys Met Glu Leu Phe Lys Val Asp Asp Asp Asn Lys Glu
145                 150                 155                 160

Gly Asn Phe Ser Asn Met Phe Leu Asp Ala Ser His Ala Gly Leu Val
                165                 170                 175

Asn Glu His Trp Ala Phe Gly Lys Asn Glu Arg Ser Leu Lys Tyr Ile
            180                 185                 190

Glu Arg Cys Leu Gln Asp Phe Leu Gly Phe Gly Val Leu Gly Pro Glu
        195                 200                 205

Gly Gln Leu Val Ser Trp Ile Val Met Glu Gln Ser Cys Glu Leu Arg
    210                 215                 220

Met Gly Tyr Thr Val Pro Lys Tyr Arg His Gln Gly Asn Met Leu Gln
225                 230                 235                 240

Ile Gly Tyr His Phe Glu Lys Tyr Leu Ser Gln Lys Glu Ile Pro Phe
                245                 250                 255

Tyr Phe His Val Ala Asp Asn Asn Glu Lys Ser Leu Gln Ala Leu Lys
            260                 265                 270

Asn Leu Gly Phe Lys Ile Cys Pro Cys Gly Trp His Gln Trp Lys Cys
        275                 280                 285

Thr Pro Lys Lys Tyr Cys
    290

<210>  54
<211>  297
<212>  PRT
<213>  Saimiri boliviensis

<400>  54

Met Phe Val Leu His Asp Thr Gln Ile Leu Gln Thr Leu Tyr Lys Ser
1               5                   10                  15

Leu Glu Lys Arg Asn Pro Glu Ser Ile Lys Val Tyr Gly Ala Ile Phe
            20                  25                  30

Asn Ile Lys Asn Lys Asn Pro Phe Asn Met Glu Val Leu Val Asp Ala
        35                  40                  45

Trp Pro Asp Tyr Gln Thr Val Ile Ile Arg Pro Lys Lys Gln Glu Met
    50                  55                  60

Lys Asp Asp Gln Asp His Tyr Thr Asn Thr Tyr His Ile Phe Thr Lys
65                  70                  75                  80

Ala Pro Asp Lys Leu Glu Glu Ile Leu Ser Cys Ser Glu Val Ile Asn
                85                  90                  95

Trp Glu Gln Thr Phe Gln Ile Gln Gly Cys Gln Glu Gly Leu Asn Glu
            100                 105                 110

Ala Ile Arg Lys Val Ala Ala Ser Lys Thr Val Gln Val Asp Tyr Val
        115                 120                 125

Lys Thr Val Leu Phe Lys Pro Glu Leu Leu Lys Glu His Lys Thr Ser
    130                 135                 140

Ser Asn Asp Lys Met Glu Leu Phe Glu Met His Lys Val Asp Asp Asn
145                 150                 155                 160

Asn Lys Lys Arg Asn Phe Ser Ser Met Phe Leu Asp Ala Ser His Ala
                165                 170                 175

Gly Leu Ile Asn Glu His Trp Ala Phe Gly Lys Asn Glu Arg Ser Leu
            180                 185                 190

Lys Tyr Thr Glu Arg Cys Leu Gln Asp Phe Leu Gly Phe Gly Val Leu
        195                 200                 205

Gly Pro Glu Gly Glu Leu Ile Ser Trp Val Val Met Glu Gln Ser Cys
    210                 215                 220

Glu Leu Arg Met Gly Tyr Thr Ile Pro Lys Tyr Arg Asn Gln Arg His
225                 230                 235                 240

Met Ser Gln Ile Leu Cys Tyr Leu Glu Lys Tyr Leu Ser Gln Lys Glu
                245                 250                 255

Ile Pro Phe Tyr Ile His Val Ala Asp Tyr Asn Val Ala Gly Leu Gln
            260                 265                 270

Ile Met Lys Asn Met Gly Tyr Met Ile Cys Pro Cys Gly Trp His Gln
        275                 280                 285

Trp Lys Cys Thr Pro Lys Lys Tyr Cys
    290                 295

<210>  55
<211>  298
<212>  PRT
<213>  Felis catus

<400>  55

Met Phe Val Leu His Glu Ser Gln Lys Leu His Ile Leu Tyr Glu Ser
1               5                   10                  15

Leu Glu Lys Ser Ile Pro Glu Ser Leu Lys Val Tyr Gly Ala Val Phe
            20                  25                  30

Asn Ile Lys Asn Lys Asn Pro Phe Asn Met Glu Val Leu Val Asp Ala
        35                  40                  45

Trp Pro Asp Tyr Gln Ile Val Ile Thr Arg Pro Gln Arg Glu Glu Met
    50                  55                  60

Lys Asp Asp Leu Asp His Tyr Thr Asn Thr Tyr Gln Ile Phe Thr Lys
65                  70                  75                  80

Ala Pro Asp Lys Leu Glu Glu Val Leu Ala Gln Pro Gln Leu Ile Asn
                85                  90                  95

Trp Asp Gln Val Phe Gln Ile Gln Gly Cys Gln Glu Ser Leu Gln Glu
            100                 105                 110

Ala Ile Arg Lys Ile Ser Ala Ser Lys Ser Val Arg Val Asp Tyr Val
        115                 120                 125

Lys Thr Ile Arg Ile Met Thr Glu Met Pro Val Lys Pro Asn Asp Asp
    130                 135                 140

Gln Val Asp Val Met Glu Ser Phe Lys Met Pro Lys Val Asp Asp Asp
145                 150                 155                 160

Thr Lys Lys Gly Ser Phe Gln Asn Ile Ser Leu Asp Pro Ser His Ala
                165                 170                 175

Gly Leu Val Asn Glu Gln Trp Ala Phe Gly Lys Asn Asp Arg Ser Leu
            180                 185                 190

Lys Tyr Ile Glu Arg Cys Leu Gln Asn Phe Pro Gly Phe Gly Val Leu
        195                 200                 205

Gly Pro Glu Gly Arg Leu Ile Ser Trp Ile Val Met Glu Gln Ser Cys
    210                 215                 220

Glu Met Arg Met Gly Tyr Thr Val Pro Glu Tyr Arg Lys His Gly Tyr
225                 230                 235                 240

Met Lys Lys Thr Ser Val His Leu Met Asn Tyr Val Ile Gln Lys Lys
                245                 250                 255

Val Pro Cys Tyr Phe His Val Ser Glu His Lys Glu Glu Tyr Asn Gln
            260                 265                 270

Ala Leu Arg Asp Leu Lys Phe Lys Ala Ala Ser Cys Gly Trp His Gln
        275                 280                 285

Trp Lys Cys Ser Pro Glu Lys Tyr Val Asp
    290                 295

<210>  56
<211>  299
<212>  PRT
<213>  Bos taurus

<400>  56

Met Phe Val Leu Arg Glu Pro Gln Gln Leu Gln Ile Leu Tyr Glu Ser
1               5                   10                  15

Leu Glu Glu Ser Ile Pro Glu Ser Leu Lys Val Tyr Gly Thr Leu Phe
            20                  25                  30

His Ile Arg Asn Lys Asn Pro Phe Asn Leu Glu Val Leu Val Asp Ala
        35                  40                  45

Trp Pro Glu Tyr Gln Thr Val Val Ile Arg Pro Gln Lys Glu Glu Met
    50                  55                  60

Lys Asp Asp Leu Asp Tyr Tyr Thr Asn Thr Tyr His Ile Phe Thr Lys
65                  70                  75                  80

Ala Pro Asp Lys Leu Glu Glu Val Leu Ala Cys Pro Gln Val Ile Asn
                85                  90                  95

Trp Glu Gln Ala Phe Gln Ile Gln Gly Cys Gln Glu Ser Leu Gly Glu
            100                 105                 110

Ala Ile Gln Lys Val Ala Ala Ser Lys Ser Val Gln Val Asp Tyr Leu
        115                 120                 125

Arg Thr Val Leu Phe Val Phe Glu Lys Pro Thr Leu Glu Ala Ser Ser
    130                 135                 140

Gly Asp Lys Met Asp Leu Met Lys Leu Leu Lys Ile Pro Lys Val Leu
145                 150                 155                 160

Glu Asp Lys Arg Lys Glu Thr Phe Gln Asn Ile Phe Leu Asp Val Ser
                165                 170                 175

His Ala Arg Leu Val Asn Glu His Trp Glu Leu Gly Lys Asn Glu Lys
            180                 185                 190

Ser Leu Lys Tyr Val Glu Arg Cys Leu Gln Asn Phe Ala Gly Phe Gly
        195                 200                 205

Val Leu Ser Ser Glu Gly Lys Pro Ile Ser Trp Phe Leu Thr Glu Gln
    210                 215                 220

Ser Cys Glu Ile Arg Met Gly Tyr Thr Phe Pro Lys Tyr Arg Gly Gln
225                 230                 235                 240

Gly His Met Trp Gln Met Gly Cys His Ser Ile Ala Tyr Phe Phe Ser
                245                 250                 255

Lys Lys Ile Pro Phe Tyr Ile His Ala Ala Glu Asp Lys Glu Lys Thr
            260                 265                 270

Gln Gln Thr Leu Leu Ser Phe Gly Phe Lys Asn Val Leu Cys Gly Trp
        275                 280                 285

His Gln Trp Lys Cys Thr Pro Lys Lys Tyr Cys
    290                 295

<210>  57
<211>  296
<212>  PRT
<213>  Mus musculus

<400>  57

Met Ile Val Pro Leu Gln Gly Ala Gln Met Leu Gln Met Leu Glu Lys
1               5                   10                  15

Ser Leu Arg Lys Tyr Leu Pro Glu Ser Leu Lys Val Tyr Gly Thr Val
            20                  25                  30

Tyr His Met Ile His Gly Asn Pro Phe Asn Leu Lys Ala Leu Val Asp
        35                  40                  45

Lys Trp Pro Asp Phe Asn Thr Val Val Val Arg Pro Gln Glu Gln Glu
    50                  55                  60

Met Thr Asp Asp Leu Asp Phe Tyr Ile Asn Thr Tyr Gln Val Tyr Ser
65                  70                  75                  80

Lys Asp Pro Gln Asn Cys Gln Glu Phe Leu Glu Ser Ser Glu Val Ile
                85                  90                  95

Asn Trp Lys Gln His Leu Gln Ile Gln Ser Ser Gln Ser His Leu Asn
            100                 105                 110

Lys Thr Ile Gln Asn Leu Ala Ser Ile Gln Ser Phe Gln Ile Lys His
        115                 120                 125

Ser Glu Asn Ile Leu Tyr Val Ser Ser Glu Thr Ile Lys Lys Leu Phe
    130                 135                 140

Pro Ser Leu Leu Asp Thr Lys Asn Leu Ser Thr Gly Ser Gly Lys Pro
145                 150                 155                 160

Lys Ala Ile Asp Gln Asp Lys Phe Lys Leu Ser Ser Leu Asp Val Val
                165                 170                 175

His Ala Ala Leu Val Asn Lys Phe Trp Leu Phe Gly Gly Asn Glu Arg
            180                 185                 190

Ser Gln Arg Phe Ile Glu Arg Cys Ile Lys Asn Phe Pro Ser Ser Cys
        195                 200                 205

Val Leu Gly Pro Glu Gly Thr Pro Ala Ser Trp Thr Leu Met Asp Gln
    210                 215                 220

Thr Gly Glu Met Arg Met Gly Gly Thr Met Pro Glu Tyr Arg Leu Gln
225                 230                 235                 240

Gly Leu Val Ser Phe Val Val His Ser Gln Asp Gln Ile Met Thr Lys
                245                 250                 255

Arg Gly Tyr Pro Val Tyr Ser His Thr Glu Lys Ser Asn Ile Ala Met
            260                 265                 270

Gln Lys Met Ser Tyr Thr Leu Gln His Leu Pro Met Pro Cys Ala Trp
        275                 280                 285

Asn Gln Trp Lys Cys Met Pro Met
    290                 295

<210>  58
<211>  364
<212>  PRT
<213>  Artificial Sequence

<220>
<223>  gcvT

<400>  58

Met Ala Gln Gln Thr Pro Leu Tyr Glu Gln His Thr Leu Cys Gly Ala
1               5                   10                  15

Arg Met Val Asp Phe His Gly Trp Met Met Pro Leu His Tyr Gly Ser
            20                  25                  30

Gln Ile Asp Glu His His Ala Val Arg Thr Asp Ala Gly Met Phe Asp
        35                  40                  45

Val Ser His Met Thr Ile Val Asp Leu Arg Gly Ser Arg Thr Arg Glu
    50                  55                  60

Phe Leu Arg Tyr Leu Leu Ala Asn Asp Val Ala Lys Leu Thr Lys Ser
65                  70                  75                  80

Gly Lys Ala Leu Tyr Ser Gly Met Leu Asn Ala Ser Gly Gly Val Ile
                85                  90                  95

Asp Asp Leu Ile Val Tyr Tyr Phe Thr Glu Asp Phe Phe Arg Leu Val
            100                 105                 110

Val Asn Ser Ala Thr Arg Glu Lys Asp Leu Ser Trp Ile Thr Gln His
        115                 120                 125

Ala Glu Pro Phe Gly Ile Glu Ile Thr Val Arg Asp Asp Leu Ser Met
    130                 135                 140

Ile Ala Val Gln Gly Pro Asn Ala Gln Ala Lys Ala Ala Thr Leu Phe
145                 150                 155                 160

Asn Asp Ala Gln Arg Gln Ala Val Glu Gly Met Lys Pro Phe Phe Gly
                165                 170                 175

Val Gln Ala Gly Asp Leu Phe Ile Ala Thr Thr Gly Tyr Thr Gly Glu
            180                 185                 190

Ala Gly Tyr Glu Ile Ala Leu Pro Asn Glu Lys Ala Ala Asp Phe Trp
        195                 200                 205

Arg Ala Leu Val Glu Ala Gly Val Lys Pro Cys Gly Leu Gly Ala Arg
    210                 215                 220

Asp Thr Leu Arg Leu Glu Ala Gly Met Asn Leu Tyr Gly Gln Glu Met
225                 230                 235                 240

Asp Glu Thr Ile Ser Pro Leu Ala Ala Asn Met Gly Trp Thr Ile Ala
                245                 250                 255

Trp Glu Pro Ala Asp Arg Asp Phe Ile Gly Arg Glu Ala Leu Glu Val
            260                 265                 270

Gln Arg Glu His Gly Thr Glu Lys Leu Val Gly Leu Val Met Thr Glu
        275                 280                 285

Lys Gly Val Leu Arg Asn Glu Leu Pro Val Arg Phe Thr Asp Ala Gln
    290                 295                 300

Gly Asn Gln His Glu Gly Ile Ile Thr Ser Gly Thr Phe Ser Pro Thr
305                 310                 315                 320

Leu Gly Tyr Ser Ile Ala Leu Ala Arg Val Pro Glu Gly Ile Gly Glu
                325                 330                 335

Thr Ala Ile Val Gln Ile Arg Asn Arg Glu Met Pro Val Lys Val Thr
            340                 345                 350

Lys Pro Val Phe Val Arg Asn Gly Lys Ala Val Ala
        355                 360

<210>  59
<211>  129
<212>  PRT
<213>  Artificial Sequence

<220>
<223>  gcvH

<400>  59

Met Ser Asn Val Pro Ala Glu Leu Lys Tyr Ser Lys Glu His Glu Trp
1               5                   10                  15

Leu Arg Lys Glu Ala Asp Gly Thr Tyr Thr Val Gly Ile Thr Glu His
            20                  25                  30

Ala Gln Glu Leu Leu Gly Asp Met Val Phe Val Asp Leu Pro Glu Val
        35                  40                  45

Gly Ala Thr Val Ser Ala Gly Asp Asp Cys Ala Val Ala Glu Ser Val
    50                  55                  60

Lys Ala Ala Ser Asp Ile Tyr Ala Pro Val Ser Gly Glu Ile Val Ala
65                  70                  75                  80

Val Asn Asp Ala Leu Ser Asp Ser Pro Glu Leu Val Asn Ser Glu Pro
                85                  90                  95

Tyr Ala Gly Gly Trp Ile Phe Lys Ile Lys Ala Ser Asp Glu Ser Glu
            100                 105                 110

Leu Glu Ser Leu Leu Asp Ala Thr Ala Tyr Glu Ala Leu Leu Glu Asp
        115                 120                 125

Glu

<210>  60
<211>  957
<212>  PRT
<213>  Artificial Sequence

<220>
<223>  gcvP

<400>  60

Met Thr Gln Thr Leu Ser Gln Leu Glu Asn Ser Gly Ala Phe Ile Glu
1               5                   10                  15

Arg His Ile Gly Pro Asp Ala Ala Gln Gln Gln Glu Met Leu Asn Ala

20                  25                  30

Val Gly Ala Gln Ser Leu Asn Ala Leu Thr Gly Gln Ile Val Pro Lys
        35                  40                  45

Asp Ile Gln Leu Ala Thr Pro Pro Gln Val Gly Ala Pro Ala Thr Glu
    50                  55                  60

Tyr Ala Ala Leu Ala Glu Leu Lys Ala Ile Ala Ser Arg Asn Lys Arg
65                  70                  75                  80

Phe Thr Ser Tyr Ile Gly Met Gly Tyr Thr Ala Val Gln Leu Pro Pro
                85                  90                  95

Val Ile Leu Arg Asn Met Leu Glu Asn Pro Gly Trp Tyr Thr Ala Tyr
            100                 105                 110

Thr Pro Tyr Gln Pro Glu Val Ser Gln Gly Arg Leu Glu Ala Leu Leu
        115                 120                 125

Asn Phe Gln Gln Val Thr Leu Asp Leu Thr Gly Leu Asp Met Ala Ser
    130                 135                 140

Ala Ser Leu Leu Asp Glu Ala Thr Ala Ala Ala Glu Ala Met Ala Met
145                 150                 155                 160

Ala Lys Arg Val Ser Lys Leu Lys Asn Ala Asn Arg Phe Phe Val Ala
                165                 170                 175

Ser Asp Val His Pro Gln Thr Leu Asp Val Val Arg Thr Arg Ala Glu
            180                 185                 190

Thr Phe Gly Phe Glu Val Ile Val Asp Asp Ala Gln Lys Val Leu Asp
        195                 200                 205

His Gln Asp Val Phe Gly Val Leu Leu Gln Gln Val Gly Thr Thr Gly
    210                 215                 220

Glu Ile His Asp Tyr Thr Ala Leu Ile Ser Glu Leu Lys Ser Arg Lys
225                 230                 235                 240

Ile Val Val Ser Val Ala Ala Asp Ile Met Ala Leu Val Leu Leu Thr
                245                 250                 255

Ala Pro Gly Lys Gln Gly Ala Asp Ile Val Phe Gly Ser Ala Gln Arg
            260                 265                 270

Phe Gly Val Pro Met Gly Tyr Gly Gly Pro His Ala Ala Phe Phe Ala
        275                 280                 285

Ala Lys Asp Glu Tyr Lys Arg Ser Met Pro Gly Arg Ile Ile Gly Val
    290                 295                 300

Ser Lys Asp Ala Ala Gly Asn Thr Ala Leu Arg Met Ala Met Gln Thr
305                 310                 315                 320

Arg Glu Gln His Ile Arg Arg Glu Lys Ala Asn Ser Asn Ile Cys Thr
                325                 330                 335

Ser Gln Val Leu Leu Ala Asn Ile Ala Ser Leu Tyr Ala Val Tyr His
            340                 345                 350

Gly Pro Val Gly Leu Lys Arg Ile Ala Asn Arg Ile His Arg Leu Thr
        355                 360                 365

Asp Ile Leu Ala Ala Gly Leu Gln Gln Lys Gly Leu Lys Leu Arg His
    370                 375                 380

Ala His Tyr Phe Asp Thr Leu Cys Val Glu Val Ala Asp Lys Ala Gly
385                 390                 395                 400

Val Leu Thr Arg Ala Glu Ala Ala Glu Ile Asn Leu Arg Ser Asp Ile
                405                 410                 415

Leu Asn Ala Val Gly Ile Thr Leu Asp Glu Thr Thr Thr Arg Glu Asn
            420                 425                 430

Val Met Gln Leu Phe Asn Val Leu Leu Gly Asp Asn His Gly Leu Asp
        435                 440                 445

Ile Asp Thr Leu Asp Lys Asp Val Ala His Asp Ser Arg Ser Ile Gln
    450                 455                 460

Pro Ala Met Leu Arg Asp Asp Glu Ile Leu Thr His Pro Val Phe Asn
465                 470                 475                 480

Arg Tyr His Ser Glu Thr Glu Met Met Arg Tyr Met His Ser Leu Glu
                485                 490                 495

Arg Lys Asp Leu Ala Leu Asn Gln Ala Met Ile Pro Leu Gly Ser Cys
            500                 505                 510

Thr Met Lys Leu Asn Ala Ala Ala Glu Met Ile Pro Ile Thr Trp Pro
        515                 520                 525

Glu Phe Ala Glu Leu His Pro Phe Cys Pro Pro Glu Gln Ala Glu Gly
    530                 535                 540

Tyr Gln Gln Met Ile Ala Gln Leu Ala Asp Trp Leu Val Lys Leu Thr
545                 550                 555                 560

Gly Tyr Asp Ala Val Cys Met Gln Pro Asn Ser Gly Ala Gln Gly Glu
                565                 570                 575

Tyr Ala Gly Leu Leu Ala Ile Arg His Tyr His Glu Ser Arg Asn Glu
            580                 585                 590

Gly His Arg Asp Ile Cys Leu Ile Pro Ala Ser Ala His Gly Thr Asn
        595                 600                 605

Pro Ala Ser Ala His Met Ala Gly Met Gln Val Val Val Val Ala Cys
    610                 615                 620

Asp Lys Asn Gly Asn Ile Asp Leu Thr Asp Leu Arg Ala Lys Ala Glu
625                 630                 635                 640

Gln Ala Gly Asp Asn Leu Ser Cys Ile Met Val Thr Tyr Pro Ser Thr
                645                 650                 655

His Gly Val Tyr Glu Glu Thr Ile Arg Glu Val Cys Glu Val Val His
            660                 665                 670

Gln Phe Gly Gly Gln Val Tyr Leu Asp Gly Ala Asn Met Asn Ala Gln
        675                 680                 685

Val Gly Ile Thr Ser Pro Gly Phe Ile Gly Ala Asp Val Ser His Leu
    690                 695                 700

Asn Leu His Lys Thr Phe Cys Ile Pro His Gly Gly Gly Gly Pro Gly
705                 710                 715                 720

Met Gly Pro Ile Gly Val Lys Ala His Leu Ala Pro Phe Val Pro Gly
                725                 730                 735

His Ser Val Val Gln Ile Glu Gly Met Leu Thr Arg Gln Gly Ala Val
            740                 745                 750

Ser Ala Ala Pro Phe Gly Ser Ala Ser Ile Leu Pro Ile Ser Trp Met
        755                 760                 765

Tyr Ile Arg Met Met Gly Ala Glu Gly Leu Lys Lys Ala Ser Gln Val
    770                 775                 780

Ala Ile Leu Asn Ala Asn Tyr Ile Ala Ser Arg Leu Gln Asp Ala Phe
785                 790                 795                 800

Pro Val Leu Tyr Thr Gly Arg Asp Gly Arg Val Ala His Glu Cys Ile
                805                 810                 815

Leu Asp Ile Arg Pro Leu Lys Glu Glu Thr Gly Ile Ser Glu Leu Asp
            820                 825                 830

Ile Ala Lys Arg Leu Ile Asp Tyr Gly Phe His Ala Pro Thr Met Ser
        835                 840                 845

Phe Pro Val Ala Gly Thr Leu Met Val Glu Pro Thr Glu Ser Glu Ser
    850                 855                 860

Lys Val Glu Leu Asp Arg Phe Ile Asp Ala Met Leu Ala Ile Arg Ala
865                 870                 875                 880

Glu Ile Asp Gln Val Lys Ala Gly Val Trp Pro Leu Glu Asp Asn Pro
                885                 890                 895

Leu Val Asn Ala Pro His Ile Gln Ser Glu Leu Val Ala Glu Trp Ala
            900                 905                 910

His Pro Tyr Ser Arg Glu Val Ala Val Phe Pro Ala Gly Val Ala Asp
        915                 920                 925

Lys Tyr Trp Pro Thr Val Lys Arg Leu Asp Asp Val Tyr Gly Asp Arg
    930                 935                 940

Asn Leu Phe Cys Ser Cys Val Pro Ile Ser Glu Tyr Gln
945                 950                 955

<210>  61
<211>  417
<212>  PRT
<213>  Artificial Sequence

<220>
<223>  glyA

<400>  61

Met Leu Lys Arg Glu Met Asn Ile Ala Asp Tyr Asp Ala Glu Leu Trp
1               5                   10                  15

Gln Ala Met Glu Gln Glu Lys Val Arg Gln Glu Glu His Ile Glu Leu
            20                  25                  30

Ile Ala Ser Glu Asn Tyr Thr Ser Pro Arg Val Met Gln Ala Gln Gly
        35                  40                  45

Ser Gln Leu Thr Asn Lys Tyr Ala Glu Gly Tyr Pro Gly Lys Arg Tyr
    50                  55                  60

Tyr Gly Gly Cys Glu Tyr Val Asp Ile Val Glu Gln Leu Ala Ile Asp
65                  70                  75                  80

Arg Ala Lys Glu Leu Phe Gly Ala Asp Tyr Ala Asn Val Gln Pro His
                85                  90                  95

Ser Gly Ser Gln Ala Asn Phe Ala Val Tyr Thr Ala Leu Leu Glu Pro
            100                 105                 110

Gly Asp Thr Val Leu Gly Met Asn Leu Ala His Gly Gly His Leu Thr
        115                 120                 125

His Gly Ser Pro Val Asn Phe Ser Gly Lys Leu Tyr Asn Ile Val Pro
    130                 135                 140

Tyr Gly Ile Asp Ala Thr Gly His Ile Asp Tyr Ala Asp Leu Glu Lys
145                 150                 155                 160

Gln Ala Lys Glu His Lys Pro Lys Met Ile Ile Gly Gly Phe Ser Ala
                165                 170                 175

Tyr Ser Gly Val Val Asp Trp Ala Lys Met Arg Glu Ile Ala Asp Ser
            180                 185                 190

Ile Gly Ala Tyr Leu Phe Val Asp Met Ala His Val Ala Gly Leu Val
        195                 200                 205

Ala Ala Gly Val Tyr Pro Asn Pro Val Pro His Ala His Val Val Thr
    210                 215                 220

Thr Thr Thr His Lys Thr Leu Ala Gly Pro Arg Gly Gly Leu Ile Leu
225                 230                 235                 240

Ala Lys Gly Gly Ser Glu Glu Leu Tyr Lys Lys Leu Asn Ser Ala Val
                245                 250                 255

Phe Pro Gly Gly Gln Gly Gly Pro Leu Met His Val Ile Ala Gly Lys
            260                 265                 270

Ala Val Ala Leu Lys Glu Ala Met Glu Pro Glu Phe Lys Thr Tyr Gln
        275                 280                 285

Gln Gln Val Ala Lys Asn Ala Lys Ala Met Val Glu Val Phe Leu Glu
    290                 295                 300

Arg Gly Tyr Lys Val Val Ser Gly Gly Thr Asp Asn His Leu Phe Leu
305                 310                 315                 320

Val Asp Leu Val Asp Lys Asn Leu Thr Gly Lys Glu Ala Asp Ala Ala
                325                 330                 335

Leu Gly Arg Ala Asn Ile Thr Val Asn Lys Asn Ser Val Pro Asn Asp
            340                 345                 350

Pro Lys Ser Pro Phe Val Thr Ser Gly Ile Arg Val Gly Thr Pro Ala
        355                 360                 365

Ile Thr Arg Arg Gly Phe Lys Glu Ala Glu Ala Lys Glu Leu Ala Gly
    370                 375                 380

Trp Met Cys Asp Val Leu Asp Ser Ile Asn Asp Glu Ala Val Ile Glu
385                 390                 395                 400

Arg Ile Lys Gly Lys Val Leu Asp Ile Cys Ala Arg Tyr Pro Val Tyr
                405                 410                 415

Ala

<210>  62
<211>  333
<212>  PRT
<213>  Artificial Sequence

<220>
<223>  ltaE

<400>  62

Met Ile Asp Leu Arg Ser Asp Thr Val Thr Arg Pro Ser Arg Ala Met
1               5                   10                  15

Leu Glu Ala Met Met Ala Ala Pro Val Gly Asp Asp Val Tyr Gly Asp
            20                  25                  30

Asp Pro Thr Val Asn Ala Leu Gln Asp Tyr Ala Ala Glu Leu Ser Gly
        35                  40                  45

Lys Glu Ala Ala Ile Phe Leu Pro Thr Gly Thr Gln Ala Asn Leu Val
    50                  55                  60

Ala Leu Leu Ser His Cys Glu Arg Gly Glu Glu Tyr Ile Val Gly Gln
65                  70                  75                  80

Ala Ala His Asn Tyr Leu Phe Glu Ala Gly Gly Ala Ala Val Leu Gly
                85                  90                  95

Ser Ile Gln Pro Gln Pro Ile Asp Ala Ala Ala Asp Gly Thr Leu Pro
            100                 105                 110

Leu Asp Lys Val Ala Met Lys Ile Lys Pro Asp Asp Ile His Phe Ala
        115                 120                 125

Arg Thr Lys Leu Leu Ser Leu Glu Asn Thr His Asn Gly Lys Val Leu
    130                 135                 140

Pro Arg Glu Tyr Leu Lys Glu Ala Trp Glu Phe Thr Arg Glu Arg Asn
145                 150                 155                 160

Leu Ala Leu His Val Asp Gly Ala Arg Ile Phe Asn Ala Val Val Ala
                165                 170                 175

Tyr Gly Cys Glu Leu Lys Glu Ile Thr Gln Tyr Cys Asp Ser Phe Thr
            180                 185                 190

Ile Cys Leu Ser Lys Gly Leu Gly Thr Pro Val Gly Ser Leu Leu Val
        195                 200                 205

Gly Asn Arg Asp Tyr Ile Lys Arg Ala Ile Arg Trp Arg Lys Met Thr
    210                 215                 220

Gly Gly Gly Met Arg Gln Ser Gly Ile Leu Ala Ala Ala Gly Ile Tyr
225                 230                 235                 240

Ala Leu Lys Asn Asn Val Ala Arg Leu Gln Glu Asp His Asp Asn Ala
                245                 250                 255

Ala Trp Met Ala Glu Gln Leu Arg Glu Ala Gly Ala Asp Val Met Arg
            260                 265                 270

Gln Asp Thr Asn Met Leu Phe Val Arg Val Gly Glu Glu Asn Ala Ala
        275                 280                 285

Ala Leu Gly Glu Tyr Met Lys Ala Arg Asn Val Leu Ile Asn Ala Ser
    290                 295                 300

Pro Ile Val Arg Leu Val Thr His Leu Asp Val Ser Arg Glu Gln Leu
305                 310                 315                 320

Ala Glu Val Ala Ala His Trp Arg Ala Phe Leu Ala Arg
                325                 330

## Description

### FIELD OF THE INVENTION

The present invention relates to biotechnological methods and cells for producing at least one acyl amino acid. In particular, the acyl amino acid is from at least one fatty acid.

### BACKGROUND OF THE INVENTION

Acyl amino acids are a class of surface-active agents with a variety of uses, for example as detergents for washing purposes, emulsifiers in food products and as essential ingredients in various personal care products such as shampoos, soaps, moisturizing agents and the like. In addition to having both hydrophobic and hydrophilic regions, a prerequisite for use as a surfactant, the compounds are made of naturally occurring molecules, more specifically amino acids and fatty acids, which are not only non-hazardous and environmentally acceptable but may be readily produced at a large scale using inexpensive biological raw materials. In pharmacological research, acyl amino acids are used as neuromodulators and probes for new drug targets.

Acyl amino acids have been isolated from a multitude of biological sources and are believed to have a range of functions, including for example as signalling molecules in mammalian tissues. Also, identification of endogenous acyl amino acids based on a targeted lipidomics approach, allow them to be used as building blocks for antibiotics in bacterial cultures. For example, since cyclic AMP directly activates NasP, an N-acyl amino acid antibiotic biosynthetic enzyme cloned from an uncultured beta-proteobacterium, these N-acyl amino acids can be easily used in antibiotic production. They are also used as compounds involved in bacterial protein sorting.

Conventionally, acyl amino acids have been produced at an industrial scale starting with materials derived from petrochemicals. More specifically, activated fatty acids provided in the form of acid chlorides may be used to acylate amino acids in an aqueous alkaline medium as described in GB1483500. Shortcomings of such approaches include the need to add hazardous chemicals such as sulphuric acid or anhydrides thereof. Other synthetic approaches are associated with the accumulation of by-products such as chloride salts which have undesirable effect on surfactancy.

A range of biotechnological routes towards production of acyl amino acids has been described in the art. However, none of them is adequate for commercial large-scale production of acyl amino acids owing to low yields, insufficient purities and the need for multi-step purification procedures. In particular, only a small proportion of the carbon substrates fed to biotechnologically useful organisms is actually converted to the desired product, whilst much of it is consumed by reactions of the primary metabolism.

Another problem associated with the available biotechnological routes is the fact that a mixture of products is obtained and thus the composition is difficult to control. More specifically, a range of fatty acids may be converted to acyl amino acids, even though production of a single adduct may be desirable. Since the mixture comprises compounds highly related in terms of chemical structure, purifying or at least enriching a single component in an efficient and straightforward manner is usually beyond technical feasibility.

Accordingly, there is a need in the art for an efficient and effective biotechnological method of producing acyl amino acids.

### DESCRIPTION OF THE INVENTION

The present invention attempts to solve the problems above by providing at least one method of producing acyl amino acids using a biotechnological method that may be efficient and specific in producing the desired acyl amino acid. In particular, the acyl amino acids are produced from genetically engineered microorganisms. More in particular, the acyl amino acids are produced by culturing a microorganism that is genetically engineered to express at least one amino acid-N-acyl-transferase and at least one acyl-CoA synthetase and to not express/ decrease the expression of at least one enzyme involved in glycine metabolism. In particular, the enzyme involved in glycine metabolism may be selected from the group consisting of enzyme of the glycine cleavage system, glycine hydroxymethyltransferase (GlyA) and threonine aldolase (ItaE).

These problems may be solved by the subject matter of the attached claims.

According to one aspect of the present invention, there is provided a cell for producing acyl amino acids wherein the cell is genetically modified to comprise
- at least a first genetic mutation that increases the expression relative to the wild type cell of an amino acid-N-acyl-transferase,
- at least a second genetic mutation that increases the expression relative to the wild type cell of an acyl-CoA synthetase, and/or
- at least a third genetic mutation that decreases the expression relative to the wild type cell of at least one enzyme selected from the group consisting of an enzyme of the glycine cleavage system, glycine hydroxymethyltransferase (GlyA) and threonine aldolase (ItaE).

In particular, the cells and methods according to any aspect of the present invention may provide an efficient biotechnological route towards acyl amino acids. The yield and purity of the product from the cells and methods according to any aspect of the present invention, in terms of catalysts or unwanted by-products, may be improved compared to the processes in the state of the art.

The cells and methods according to any aspect of the present invention may also provide a method for making acyl amino acids, wherein the spectrum of fatty acids converted to acyl amino acids may be broader than the processes known in the state of the art. For example, the cells and methods according to any aspect of the present invention may be suitable for converting short and unsaturated fatty acids to acyl amino acids. The cells and methods according to any aspect of the present invention may also provide a biotechnological method for making acyl amino acids, wherein the length of the acyl residue in the acyl amino acid product may be controlled, for example lauryl may be enriched or prevalent.

The cells and methods according to any aspect of the present invention is based on the surprising finding that the combination of amino acid-N-acyl transferase, an acyl-CoA synthetase together with a decrease in expression of at least one enzyme involved in glycine metabolism may be used to convert a variety of fatty acids to acyl amino acids. In particular, amino acid-N-acyl transferases may exist that can be used to convert short unsaturated fatty acids such as lauroleic acid to an acyl amino acid. More in particular, these amino acid-N-acyltransferases may be capable of converting short unsaturated fatty acids such as lauroleic acid to a larger yield of an acyl amino acid compared to the methods known in the art. Even more in particular, the composition of acyl amino acids produced in the cell according to any aspect of the present invention may be adjusted by varying specifically the length of fatty acids incorporated into such acyl amino acids. For example, the resultant composition of acyl amino acids produced in the cell may be controlled by introducing into the cell one or more specific acyl-CoA thioesterases or altering the expression of one or more acyl-CoA thioesterases endogenously expressed by the cell. The combination of increasing the expression relative to the wild type cell of an amino acid-N-acyl-transferase and acyl-CoA synthetase, and decreasing the expression relative to the wild type cell of at least one enzyme involved in glycine metabolism may result in a synergistic effect enabling an increase in the yield of acyl amino acid compared to the methods known in the art. In one example, the acyl amino acid formed may be lauroylglycinate.

The term "amino acid-N-acyl transferase", as used herein, refers to an enzyme capable of catalysing the conversion of acyl-CoA, for example the CoA ester of lauroleic acid, and an amino acid, for example a proteinogenic amino acid, in particular glycine, to an acyl amino acid. Suitable amino acid-N-acyl transferases have been described in the prior art, for example in Waluk, D. P. et al, 2010. In one example, the amino acid-N-acyl transferase used according to any aspect of the present invention may comprises a nucleotide sequence of SEQ ID NO:4. In particular, the amino acid sequence of amino acid-N-acyl transferase may be selected from the group consisting of NP_001010904.1,NP_659453.3, XP_001147054.1, AAH16789.1, AA073139.1, XP_003275392.1, XP_002755356.1, XP_003920208.1, XP_004051278.1, XP_006147456.1, XP_006214970.1, XP_003801413.1, XP_006189704.1, XP_003993512.1, XP_005862181.1, XP_007092708.1, XP_006772167.1, XP_006091892.1, XP_005660936.1, XP_005911029.1, NP_001178259.1, XP_004016547.1, XP_005954684.1, ELR45061.1, XP_005690354.1, XP_004409352.1, XP_007519553.1, XP_004777729.1, XP_005660935.1, XP_004824058.1, XP_006068141.1, XP_006900486.1, XP_007497585.1, XP_002821801.2, XP_007497583.1, XP_003774260.1, XP_001377648.2, XP_003909843.1, XP_003801448.1, XP_001091958.1, XP_002821798.1, XP_005577840.1, XP_001092197.1, NP_001207423.1, NP_001207425.1, XP_003954287.1, NP_001271595.1, XP_003909848.1, XP_004087850.1, XP_004051279.1, XP_003920209.1, XP_005577835.1, XP_003774402.1, XP_003909846.1, XP_004389401.1, XP_002821802.1, XP_003774401.1, XP_007497581.1, EHH21814.1, XP_003909845.1, XP_005577839.1, XP_003774403.1, XP_001092427.1, XP_003275395.2, NP_542392.2, XP_001147271.1, XP_005577837.1, XP_003826420.1, XP_004051281.1, XP_001147649.2, XP_003826678.1, XP_003909847.1, XP_004682812.1, XP_004682811.1, XP_003734315.1, XP_004715052.1, BAG62195.1, XP_003777804.1, XP_003909849.1, XP_001092316.2, XP_006167891.1, XP_540580.2, XP_001512426.1, EAW73833.1, XP_003464217.1, XP_007519551.1, XP_003774037.1, XP_005954680.1, XP_003801411.1, NP_803479.1, XP_004437460.1, XP_006875830.1, XP_004328969.1, XP_004264206.1, XP_004683490.1, XP_004777683.1, XP_005954681.1, XP_003480745.1, XP_004777682.1, XP_004878093.1, XP_007519550.1, XP_003421399.1, EHH53167.1, XP_006172214.1 ,XP_003993453.1, AAI12537.1, XP_006189705.1 ,Q2KIR7.2, XP_003421465.1, NP_001009648.1, XP_003464328.1, XP_001504745.1, ELV11036.1, XP_005690351.1, XP_005216632.1, EPY77465.1, XP_005690352.1, XP_004016544.1, XP_001498276.2, XP_004264205.1, XP_005690353.1, XP_005954683.1, XP_004667759.1, XP_004479306.1, XP_004645843.1, XP_004016543.1, XP_002928268.1, XP_006091904.1, XP_005331614.1, XP_007196549.1, XP_007092705.1, XP_004620532.1, XP_004869789.1, EHA98800.1, XP_004016545.1, XP_004479307.1, XP_004093105.1, NP_001095518.1, XP_005408101.1, XP_004409350.1, XP_001498290.1, XP_006056693.1, XP_005216639.1, XP_007455745.1, XP_005352049.1, XP_004328970.1, XP_002709220.1, XP_004878092.1, XP_007196553.1, XP_006996816.1, XP_005331615.1, XP_006772157.1, XP_007196552.1, XP_004016546.1, XP_007628721.1, NP_803452.1, XP_004479304.1, DAA21601.1, XP_003920207.1, XP_006091906.1, XP_003464227.1, XP_006091903.1, XP_006189706.1, XP_007455744.1, XP_004585544.1, XP_003801410.1, XP_007124812.1, XP_006900488.1, XP_004777680.1, XP_005907436..1, XP_004389356.1, XP_007124811.1, XP_005660937.1, XP_007628724.1, XP_003513512.1, XP_004437813.1, XP_007628723.1, ERE78858.1, EPQ15380.1, XP_005862178.1, XP_005878672.1, XP_540581.1, XP_002928267.1, XP_004645845.1, EPQ05184.1, XP_003513511.1, XP_006214972.1, XP_007196545.1, XP_007196547.1, XP_006772160.1, XP_003801409.1, NP_001119750.1, XP_003801412.1, XP_006772159.1, EAW73832.1, XP_006091897.1, XP_006772163.1, XP_006091898.1, XP_005408105.1, XP_006900487.1, XP_003993454.1, XP_003122754.3, XP_007455746.1, XP_005331618.1, XP_004585337.1, XP_005063305.1, XP_006091895.1, XP_006772156.1, XP_004051276.1, XP_004683488.1, NP_666047.1, NP_001013784.2, XP_006996815.1, XP_006996821.1, XP_006091893.1, XP_006173036.1, XP_006214971.1, EPY89845.1, XP_003826423.1, NP_964011.2, XP_007092707.1, XP_005063858.1, BAL43174.1, XP_001161154.2, XP_007124813.1, NP_083826.1XP_003464239.1, XP_003275394.1, ELK23978.1, XP_004878097.1, XP_004878098.1, XP_004437459.1, XP_004264204.1, XP_004409351.1, XP_005352047.1, Q5RFP0.1, XP_005408107.1, XP_007659164.1, XP_003909852.1, XP_002755355.1, NP_001126806.1, AAP92593.1, NP_001244199.1, BAA34427.1, XP_005063859.1, NP_599157.2, XP_004667761.1, XP_006900489.1, XP_006215013.1, XP_005408100.1, XP_007628718.1, XP_003514769.1, XP_006160935.1, XP_004683489.1, XP_003464329.1, XP_004921258.1, XP_003801447.1, XP_006167892.1, XP_004921305.1, AAH89619.1, XP_004706162.1, XP_003583243.1, EFB16804.1, XP_006728603.1, EPQ05185.1, XP_002709040.1, XP_006875861.1, XP_005408103.1, XP_004391425.1, EDL41477.1, XP_006772158.1, EGW06527.1, AAH 15294.1, XP_006772162.1, XP_005660939.1, XP_005352050.1, XP_006091901.1, XP_005878675.1, XP_004051323.1, EHA98803.1, XP_003779925.1, EDM12924.1, XP_003421400.1, XP_006160939.1, XP_006160938.1, XP_006160937.1, XP_006160936.1, XP_005702185.1, XP_005313023.1, XP_003769190.1, XP_002714424.1, XP_004715051.1, XP_007661593.1, XP_004590594.1, ELK23975.1, XP_004674085.1, XP_004780477.1, XP_006231186.1, XP_003803573.1, XP_004803176.1, EFB16803.1, XP_006056694.1, XP_005441626.1, XP_005318647.1XP_004605904.1, XP_005862182.1, XP_003430682.1, XP_004780478.1, XP_005239278.1, XP_003897760.1, XP_007484121.1, XP_004892683.1, XP_004414286.1, XP_006927013.1, XP_003923145.1, XP_852587.2, AAP97178.1, EHH53105.1, XP_005408113.1, XP_002915474.1, XP_005377590.1, XP_527404.2, XP_005552830.1, XP_004044211.1, NP_001180996.1, XP_003513513.2, XP_001498599.2, XP_002746654.1, XP_005072349.1, XP_006149181.1, EAX04334.1, XP_003833230.1, XP_005216635.1, XP_003404197.1, XP_007523363.1, XP_007433902.1, XP_003254235.1, XP_004471242.1, XP_005216634.1, XP_006860675.1, XP_004771956.1, XP_006038833.1, NP_001138534.1, XP_007068532.1, XP_003510714.1, ERE87950.1, XP_003986313.1, XP_006728644.1, XP_004878099.1, XP_003468014.1, XP_007095614.1, XP_004648849.1, XP_004869795.1, XP_004018927.1, XP_005696454.1, XP_006201985.1, XP_005960697.1, XP_004813725.1, XP_005496926.1, ELR45088.1, XP_004696625.1, XP_005860982.1, XP_005911003.1, XP_006260162.1, EPQ04414.1, XP_006099775.1, NP_001138532.1, XP_006190795.1, XP_004649775.1, XP_004424497.1, XP_004390885.1, XP_005911004.1, XP_003777803.1, XP_004312259.1, XP_005529140.1, XP_005314582.1, XP_006926523.1, XP_006926522.1, XP_004683491.1, XP_003826680.1, XP_003215018.1, XP _003215087.1, EGW12611.1, XP_006113023.1, XP_006882182.1, XP_007425200.1, XP_006041342.1, NP_001138533.1, EMP27694.1, XP_007497753.1, XP_006034252.1 and a variant thereof. The term 'variant' as used herein in conjunction with amino acid-N-acyl transferase refers to polypeptides having essentially the amino acid sequence of at least one amino acid-N-acyl transferase mentioned above, but wherein one or more insertions, deletions, additions and/or substitutions intentionally have been made by conventional methods. The variants may vary in the sequence compared to any one of the amino acid-N-acyl transferases mentioned above but they are capable of maintaining theirfunction. In one example, the variant comprises 50, 60, 65, 70, 80, 85, 90, 92, 94, 95, 97, 98, 99 % sequence identity in relation to any one of the amino acid-N-acyl transferases mentioned above. In particular, every variant of amino acid-N-acyl transferase is capable of maintaining the function of amino acid-N-acyl transferases.

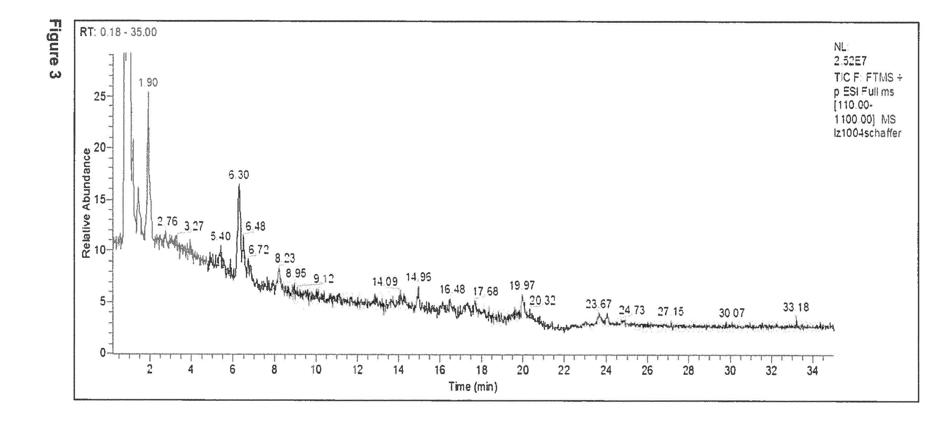

According to any aspect of the present invention, the term "variant" refers to polynucleotides and/or polypeptides that comprise differences in amino acids other than those essential for the function. For example, a variant of a specific enzyme comprises the sequences/parts that result in the catalytic activity of the enzyme, or the fold or structure of the enzyme. The only variations are in the not essential parts of the enzyme. These not so essential amino acids may be deleted, substituted or replaced by insertions or essential amino acids may be replaced in a conservative manner to the effect that the biological activity of the reference sequence or a molecule derived therefrom is preserved. The state of the art comprises algorithms that may be used to align two given nucleic acid or amino acid sequences and to calculate the degree of identity, for example in see Lesk, 2008, Thompson *et al.,* 1994, and Katoh *et al.,* 2005. The term "variant" is used synonymously and interchangeably with the term "homologue". Such variants may be prepared by introducing deletions, insertions or substitutions in amino acid or nucleic acid sequences as well as fusions comprising such macromolecules or variants thereof. In one example, the term "variant", with regard to amino acid sequence, comprises, in addition to the sequence identity, amino acid sequences that comprise one or more conservative amino acid changes with respect to the respective reference or wild type sequence or comprises nucleic acid sequences encoding amino acid sequences that comprise one or more conservative amino acid changes. In another example, the term "variant" of an amino acid sequence or nucleic acid sequence comprises, in addition to the degree of sequence identity, any active portion and/or fragment of the amino acid sequence or nucleic acid sequence, respectively, or any nucleic acid sequence encoding an active portion and/or fragment of an amino acid sequence. In a preferred embodiment, the term "active portion", as used herein, refers to an amino acid sequence or a nucleic acid sequence, which is less than the full length amino acid sequence or codes for less than the full length amino acid sequence, respectively, wherein the amino acid sequence or the amino acid sequence encoded, respectively retains at least some of its essential biological activity. For example an active portion and/or fragment of a protease are capable of hydrolysing peptide bonds in polypeptides. In one example, the term "retains at least some of its essential biological activity", as used herein, means that the amino acid sequence in question has a biological activity exceeding and distinct from the background activity and the kinetic parameters characterising said activity, more specifically k_{cat} and K_{M}, are within 3, 2, or 1 order of magnitude of the values displayed by the reference molecule with respect to a specific substrate. In one example, the term "variant" of a nucleic acid comprises nucleic acids the complementary strand of which hybridises, under stringent conditions, to the reference or wild type nucleic acid. Examples of variants of amino acid-N-acyl-transferases may at least be provided in Figure 3. Stringency of hybridisation reactions may be readily determinable by one of ordinary skilled in the art, and in general is an empirical calculation dependent on probe length, washing temperature and salt concentration. In general, longer probes require higher temperatures for proper annealing, while shorter probes need lower temperatures. Hybridisation generally depends on the ability of denatured DNA to reanneal to complementary strands are present in an environment below their melting temperature. The higher the degree of desired homology between the probe and hybridisable sequence, the higher the relative temperature which may be used. As a result it follows that higher relative temperatures would tend to make the reaction conditions more stringent, while lower temperature less so. For additional details and explanation of stringency of hybridisation reactions, see F. M. Ausubel (1995). The person skilled in the art may follow the instructions provided in the manual "The DIG System Users Guide for Filter Hybridization", 1993 and in Liebl *et al.* (1991) on how to identify DNA sequences by means of hybridisation.

Stringent conditions may be applied for any hybridisation, i.e. hybridisation occurs only if the probe is 70% or more identical to the target sequence. Probes having a lower degree of identity with respect to the target sequence may hybridise, but such hybrids are unstable and will be removed in a washing step under stringent conditions, for example lowering the concentration of salt to 2 x SSC or, optionally and subsequently, to 0,5 x SSC, while the temperature is, in order of increasing preference, approximately 50 °C - 68 °C, approximately 52 °C - 68 °C, approximately 54 °C - 68 °C, approximately 56 °C - 68 °C, approximately 58 °C - 68 °C, approximately 60 °C - 68 °C, approximately 62 °C - 68 °C, approximately 64 °C - 68 °C, approximately 66 °C - 68 °C. In one example, the temperature may be approximately 64°C-68 °C or approximately 66 °C - 68 °C. It may be possible to adjust the concentration of salt to 0.2 x SSC or even 0.1 x SSC. Polynucleotide fragments having a degree of identity with respect to the reference or wild type sequence of at least 70, 80, 90, 91, 92, 93, 94, 95, 96, 97, 98, 99 % may be isolated. In one example, the term "homologue" of a nucleic acid sequence, as used herein, refers to any nucleic acid sequence that encodes the same amino acid sequence as the reference nucleic acid sequence, in line with the degeneracy of the genetic code.

A skilled person would be able to easily determine the amino acid-N-acyl-transferases that will be capable of making proteinogenic amino acids and/or fatty acids. In particular, the variants may include but are not limited to an amino acid-N-acyl-transferase from any organism selected from the group of organisms consisting of *Nomascus leucogenys* (NI, XP_003275392.1), *Saimiri boliviensis* (Sb, XP_003920208.1), *Felis catus* (Fc, XP_003993512.1), *Bos taurus* (Bt, NP_001178259.1), and *Mus musculus* (Mm, NP_666047.1).

The term "acyl amino acid", as used herein, refers to the product of the reaction catalysed by an amino acid-N-acyl transferase, in particular, a compound represented by the formula acyl-CO-NH-CHR-COOH, wherein R is the side chain of a proteinogenic amino acid, and wherein the term "acyl" refers to the acyl residue of a fatty acid. According to any aspect of the present invention, the term "fatty acid", as used herein, means a carboxylic acid, In one example, the fatty acid comprises 6, 7, 8, 9, 10, 11, 12, 13, 14, 15, 16, 17, 18, 19, 20, 21, 22, 23, 24, 25, 26, 27, 28, 29, 30 carbon atoms. In particular, the fatty acid may be an alkanoic acid, with at least 6, 8, 10, or 12 carbon atoms. More in particular, the fatty acid has 12 carbon atoms. In one example, a linear fatty acid may be used. In another example, the fatty acid may be branched. In one example, the fatty acid used according to any aspect of the present invention may be a saturated fatty acid. In another example, the fatty acid may be unsaturated. In particular, the fatty acid used according to any aspect of the present invention may be a linear fatty acid with at least 12 carbon atoms comprising a double bond, for example at position 9. In a further example, the fatty acid may be a simple unsaturated fatty acid having one double bond, where the double bond may be located at position 9 or 11. More in particular, the fatty acid used according to any aspect of the present invention may be lauroleic acid (9-dodecenoic acid).

According to any aspect of the present invention, a formula referring to a chemical group that represents the dissociated or undissociated state of a compound capable of dissociating in an aqueous solution, comprises both the dissociated and the undissociated state and the various salt forms of the group. For example, the residue -COOH comprises both the protonated (-COOH) as well as the unprotonated (-COO⁻) carboxylic acid.

The acyl-CoA substrate used according to any aspect of the present invention may be purified from at least one cell, chemically synthesised and/or produced using an acyl-CoA synthetase. In particular, the acyl-CoA substrate used according to any aspect of the present invention may be produced using an acyl-CoA synthetase.

The term "acyl-CoA synthetase", as used herein, refers to an enzyme capable of catalysing the ATP-dependent conversion of a fatty acid and CoA to acyl CoA. In particular, acyl-CoA synthetase may refer to an acyl-CoA/ACP synthetase that may be capable of producing acyl glycinates and/or catalysing the following reaction:

fatty acid + CoA/ACP + ATP → acyl-CoA/ACP + ADP + Pi

Examples of acyl-CoA/ACP synthetases may include but are not limited to EC 6.2.1.3, EC 6.2.1.10, EC 6.2.1.15, EC 6.2.1.20 and the like. Acyl-CoA synthetases used according to any aspect of the present invention may be selected from the group consisting of YP_001724804.1, WP_001563489.1, NP_707317.1 and the like. In one example, the acyl-CoA synthetase may comprise SEQ ID NO:6 or YP_001724804.1 or a variant thereof. The term 'variant' as used herein in conjunction with Acyl-CoA synthetase refers to polypeptides having essentially the amino acid sequence of at least one Acyl-CoA synthetase mentioned above, but wherein one or more insertions, deletions, additions and/or substitutions intentionally have been made by conventional methods. The variants may vary in the sequence compared to any one of the Acyl-CoA synthetase mentioned above but they are capable of maintaining their function. In one example, the variant comprises 50, 60, 65, 70, 80, 85, 90, 92, 94, 95, 97, 98, 99 % sequence identity in relation to any one of the Acyl-CoA synthetases mentioned above.

Various methods to detect acyl-CoA synthetase activity are known in the art. For example, the activity of an acyl-CoA synthetase may be assayed by incubating the sample of interest in 100 mM Tris-HCl at pH 8 in the presence of 250 µM lauroyl-CoA, 500 µM glycine and DTNB (5,5'-dithiobis-2-nitrobenzoic acid, also referred to as Ellman's reagent) and spectrophotometrically monitoring the absorbance at 410 nm following release of free thiol groups in the form of CoASH as the reaction progresses and reaction with Ellman's reagent. The activity of an acyl-CoA synthetase may be assayed as described in the state of the art, for example Kang, Y., 2010. Briefly, the amount of free thiol in the form of unreacted CoASH is determined by adding Ellmann's reagent and spectrophotometrically monitoring the absorbance at 410 nm, preferably in a reaction buffer comprising 150 mM Tris-HCl (pH 7.2), 10 mM MgCl₂, 2 mM EDTA, 0.1 % Triton X-100, 5 mM ATP, 0.5 mM coenzyme A (CoASH) and a fatty acid (30 to 300 mM).

In one example, the cell according to any aspect of the present invention may be genetically modified to increase the expression of at least the enzymes amino acid-N-acyl transferase and acyl-CoA synthetase. In particular, the cell may overexpress enzymes glycine N-acyl transferase and acyl-CoA/ACP synthetase. The phrase "increased activity of an enzyme" and "overexpress an enzyme", as used herein is to be understood as increased intracellular activity. Basically, an increase in enzymatic activity can be achieved by increasing the copy number of the gene sequence or gene sequences that code for the enzyme, using a strong promoter or employing a gene or allele that code for a corresponding enzyme with increased activity and optionally by combining these measures. Genetically modified cells used according to any aspect of the present invention are for example produced by transformation, transduction, conjugation or a combination of these methods with a vector that contains the desired gene, an allele of this gene or parts thereof and a vector that makes expression of the gene possible. Heterologous expression is in particular achieved by integration of the gene or of the alleles in the chromosome of the cell or an extra-chromosomally replicating vector.

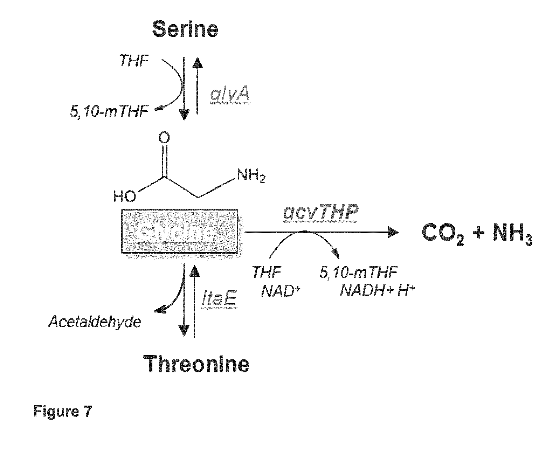

The term 'an enzyme involved in glycine metabolism' as used herein refers to at least one enzyme that may be capable of reducing the total amount of glycine in a cell by catalysing the breakdown of glycine and/or catalysing the conversion of glycine to other amino acids. In one example, the glycine may be broken down to carbon dioxide and/or ammonia. In another example, the glycine may be converted to serine and/ or threonine. In a further example, the glycine may be metabolised in a cell by being broken down to carbon dioxide and/or ammonia and converted to serine and/ or threonine simultaneously. Figure 7 shows a summary of the possible glycine metabolism pathways. In particular, an enzyme involved in glycine metabolism may be selected from the group consisting of at least one enzyme of the glycine cleavage system, glycine hydroxymethyltransferase (GlyA) and threonine aldolase (ItaE).

The term 'glycine hydroxymethyltransferase' according to any aspect of the present invention may be directed to an enzyme that catalyses the chemical reaction

5,10-methylenetetrahydrofolate + glycine + H₂O ⇄ tetrahydrofolate + L-serine

The glycine hydroxymethyltransferase (EC 2.1.2.1) belongs to the family oftransferases that transfer one-carbon groups, specifically the hydroxymethyl-, formyl- and related transferases. In particular, GlyA used according to any aspect of the present invention may comprise a polypeptide sequence of SEQ ID NO:61 or variants thereof. The term 'variant' as used herein in conjunction with GlyA refers to polypeptides having essentially the same sequence as SEQ ID NO:61 but wherein one or more insertions, deletions, additions and/or substitutions intentionally have been made by conventional methods. The variants may vary in the sequence compared to SEQ ID NO:61 but they are capable of maintaining their function. In one example, the variant comprises 50, 60, 65, 70, 80, 85, 90, 92, 94, 95, 97, 98, 99 % sequence identity in relation to SEQ ID NO:61. Similarly, ItaE used according to any aspect of the present invention may comprise a polypeptide sequence of SEQ ID NO:62 or variants thereof. The term 'variant' as used herein in conjunction with ItaE refers to polypeptides having essentially the same sequence as SEQ ID NO:62 but wherein one or more insertions, deletions, additions and/or substitutions intentionally have been made by conventional methods. The variants may vary in the sequence compared to SEQ ID NO:62 but they are capable of maintaining their function. In particular, the variant of ItaE may comprise 50, 60, 65, 70, 80, 85, 90, 92, 94, 95, 97, 98, 99 % sequence identity in relation to SEQ ID NO:62.

The phrase "at least one enzyme from the glycine cleavage system" refers to a series of enzymes that are triggered in response to high concentrations of the amino acid glycine. The glycine cleavage system is composed of four proteins: the T-protein (GcvT), P-protein (GcvP), L-protein (GcvL), and H-protein (GcvH). The H-protein is responsible for interacting with the three other proteins and acts as a shuttle for some of the intermediate products in glycine decarboxylation. In both animals and plants the glycine cleavage system is loosely attached to the inner membrane of the mitochondria. The expression of any one of these enzymes may be decreased according to any aspect of the present invention. More in particular, the glycine cleavage system H protein, carries lipoic acid and interacts with the glycine cleavage system proteins P, L and T; the glycine cleavage system P protein (EC 1.4.4.2), catalyses the reaction glycine + [glycine-cleavage complex H protein]-N(6)-lipoyl-L-lysine → [glycine-cleavage complex H protein]-S-aminomethyl-N(6)-dihydrolipoyl-L-lysine + CO2; glycine cleavage system L protein (EC 1.8.1.4), catalyses the reaction protein N6-(dihydrolipoyl)lysine + NAD+ → protein N6-(lipoyl)lysine + NADH + H+; glycine cleavage system T protein (EC 2.1.2.10), catalyses the reaction [protein]-S8-aminomethyldihydrolipoyllysine + tetrahydrofolate → [protein]-dihydrolipoyllysine + 5,10-methylenetetrahydrofolate + NH3; threonine aldolase (EC 4.2.1.48), catalyses the reaction L-threonine → glycine + acetaldehyde; serine hydroxylmethyltransferase (EC 2.1.2.1), catalyses the reaction 5,10-methylenetetrahydrofolate + glycine + H₂O + tetrahydrofolate + L-serine.

Enzymes from the glycine cleavage system used according to any aspect of the present invention may be selected from the sequences mentioned in Table 1 below and variants thereof. The term 'variant' as used herein in conjunction with an enzyme from the glycine cleavage system refers to polypeptides having essentially the amino acid sequence of at least one enzyme from the glycine cleavage system mentioned in Table 1, but wherein one or more insertions, deletions, additions and/or substitutions intentionally have been made by conventional methods. The variants may vary in the sequence compared to any one of the enzyme from the glycine cleavage system mentioned below but they are capable of maintaining their function. In one example, the variant comprises 50, 60, 65, 70, 80, 85, 90, 92, 94, 95, 96, 97, 98 or 99 % sequence identity in relation to any one of the enzyme from the glycine cleavage system mentioned below. More in particular, the sequence of GcvT may be SEQ ID NO:58. The sequence of GcvP may be SEQ ID NO:60. The sequence of GcvH may be SEQ ID NO:59. Even more in particular the sequences of GcvT, GcvP and GcvH may comprise SEQ ID NO:58, SEQ ID NO:60 and SEQ ID NO:59 respectively. Throughout this application, any data base code, unless specified to the contrary, refers to a sequence available from the NCBI data bases, more specifically the version online on 6^{th} August 2014, and comprises, if such sequence is a nucleotide sequence, the polypeptide sequence obtained by translating the former.

**Table 1. Sequences of enzymes that are part of the Gcv system.**

| **Enzyme** | **Accession Number** |
|---|---|
| GcvT | WP_000068701; CAA52144.1, YP_079782.1; NP_980596.1; YP_021091.1; AA11139.1;YP_148276.1;YP_175989.1; BAB06533.1; NP_464875.1; NP_470723.1; YP_013965.1; ZP_00231385.1; ZP_00538577.1; NP_692823.1; NP_764775.1; YP_188676.1; NP_372059.1; CAG43268.1; YP_253296.1; YP_186433.1; YP_041008.1; NP_621985.1; ZP_00560257.1; AAU84894.1; ZP_00574838.1; YP_075748.1; NP_143816.1; CAB50682.1; NP_662997.1; NP_972230.1; AAL82124.1; ZP_00590815.1; ZP_00528533.1; AD85568.1; NP_228029.1; ZP_00511104.1; ZP_00591209.1; ZP_00532593.1; ZP_00525162.1; ZP_00355911.1; YP_112880.1; ZP_00399764.1; YP_143792.1; CAD75448.1; YP_004126.1; ZP_00535960.1; ZP_00334892.1; CAG35027.1; CAF23008.1; YP_010643.1; NP_951437.1; NP_840694.1; EAN28088.1; YP_125490.1; YP_094168.1; AAO91208.1; YP_122478.1; NP_110817.1; NP_214308.1; CAC12478.1; ZP_00602311.1; YP_256007.1; |
| | EAM94548.1; BAB66249.1; NP_342409.1; YP_023949.1; ZP_00054699.1; AAK25314.1; ZP_00270640.1; YP_169455.1; NP_148400.1; ZP_00577149.1; ZP_00375766.1; ZP_00303628.1; NP_280387.1; AAV46419.1; EAO04791.1; CAC32302.1; CAI36361.1; AAZ12256.1; NP_013914.1; YP_034020.1; AC74698.1; Q827D7; ZP_00396527.1; EAK92665.1; EAK92694.1; ZP_00292858.1; ZP_00625542.1; NP_298674.1; EAO14274.1; NP_883104.1; NP_887405.1; ZP_00038971.2; NP_778843.1; ZP_00327636.1; EAA72140.1; NP_879086.1; NP_102591.1; CAJ04455.1; CAG61762.1; NP_716412.1; CAG88846.1; ZP_00582521.1; CAD17083.1; 36086.1; YP_191522.1; EAM75690.1; CAH00726.1; CAF26479.1; AAF11360.1; YP_202186.1; ZP_00308932.1; EAA47849.1; ZP_00278041.1; CAA81076.1; CAA91000.1; CAA85353.1; EAM85167.1; YP_071681.1; ZP_00638529.1; CAB16911.1 EAN06353.1; BAD35509.1; EAL84525.1; YP_118701.1; AAQ24377.1; YP_223286.1; NP_541539.1; ZP_00634544.1; CAB16916.1; BAD82264.1; AAN47559.1; NP_772393.1; YP_000299.1; CAB16918.1; CAD52982.1; XP_314216.2; NP_930808.1;AAD56281.1;AAQ66378.1; AAO76254.1; AAN33907.1; CAG73659.1; XP_219785.3; ZP_00111607.1; ZP_00585062.1; AAL21928.1; AAH42245.1; AAX66900.1; AAS46734.1; NP_949187.1; NP_989653.1; AAC31228.1;; AAA63798.1; ZP_00004510.1; CAA42443.1; ZP_00555139.1; YP_132995.1; NP_251135.1; NP_636487.1; ZP_00213263.1; ZP_00140178.2; ZP_00516950.1; AAB82711.1; AAZ27773.1; EAA61388.1; ZP_00167208.2; EAN04068.1; AA007159.1; NP_936747.1; ZP_00499479.1; NP_806663.1; AAW42121.1; CAG83849.1; NP_923192.1; NP_441838.1; ZP_00593912.1; CAA52146.1; BAA14286.1; Q8XD33; AAA69071.1; EAM28058.1; ZP_00494650.1; ZP_00489316.1; ZP_00470629.1; YP_152074.1; AAM14125.1; AAM91322.1; ZP_00569907.1; ZP_00459442.1; NP_755358.1; ZP_00436271.1; NP_613061.1; CAE59244.1; BAC38022.1; CAA91099.1; ZP_00264533.1; AAC46780.1; AAG58030.1; YP_261725.1; AAL24244.1; NP_708666.1; CAH07776.1; Q8YNF9; YP_099306.1; ZP_00474391.1; AAL57651.1; YP_055456.1; NP_682393.1; NP_893785.1; YP_172756.1; CAH74116.1; ZP_00622638.1; YP_234188.1; NP_791106.1; NP_967658.1; EAN85656.1; ZP_00379711.1; |
| | XP_520482.1; AAN66613.1; ZP_00162707.1; ZP_00417692.1; YP_264083.1; AAQ61092.1; NP_216348.1; NP_855515.1; CAE76410.1; AA039460.1; CAE22343.1; NP_800311.1; YP_206661.1; ZP_00506636.1; ZP_00417032.1; YP_164890.1; XP_760322.1; YP_266091.1; YP_156473.1; CAC46126.1; ZP_00244924.1; EAN91112.1; XP_637330.1; NP_960479.1; YP_160522.1; ZP_00317484.1; ZP_00412767.1; NP_253900.1; AAQ00873.1; NP_532152.1; ZP_00151464.1; EA009970.1; ZP_00631036.1; ZP_00141690.2; NP_302381.1; CAG08109.1; CAE08889.1; YP_263017.1; AAN70757.1; ZP_00264788.1; XP_538655.1; AAZ27305.1; AAN17423.1; AAO38610.1; CAB85154.1; AAW89976.1; AAO44232; NP_789087.1; ZP_00496567.1; AAH57478.1; AAS16361.1; AAL33595.1; ZP_00048418.1; CAA72255.1; XP_598207.1; BAD82265.1; AAK26613.1; AAM93931.1; XP_517277.1; CAB50683.1; NP_143817.1; AAL82123.1; BAD82266.1; EAN80863.1; BAD85569.1; ZP_00574837.1; BAB26854.1; ZP_00560256.1; AAU84893.1; AAD33990.1; YP_175990.1; ZP_00641578.1; NP_390336.1; YP_023948.1; AAK25315.1; CAA38252.1; EAM94547.1; ZP_00530977.1; YP_013964.1; NP_470722.1; NP_464874.1; ZP_00525161.1; NP_148401.1; ZP_00589915.1; YP_079783.1; ZP_00233535.1; ZP_00577150.1; NP_228028.1; ZP_00238490.1; NP_980597.1; YP_021092.1; AAP11140.1 YP_038289.1; BAB06534.1; ZP_00399763.1; YP_148277.1; XP_606427.1; AAL04442.1; YP_075749.1; ZP_00527784.1; NP_692824.1; ZP_00334895.1; CAD75449.1; NP_840693.1; ZP_00591425.1; CAF23007.1; ZP_00054162.1; NP_621986.1; CAC12479.1; NP_662508.1; ZP_00512041.1; AAV46420.1; ZP_00375765.1; NP_110816.1; NP_280388.1; BAB66248.1; ZP_00538578.1; NP_951436.1; ZP_00367500.1; YP_169454.1; AAX77943.1; YP_094170.1; YP_122480.1; YP_125492.1;; ZP_00270641.1; YP_178313.1; XP_473181.1; ZP_00535961.1; NP_372060.1; ZP_00602310.1; YP_255986.1; CAE05443.2; CAB72709.1; CAG43269.1; NP_342410.1; BAB95353.1; AAA92036.1; YP_186434.1; YP_041009.1; AAM63785.1; XP_584346.1; AAM51302.1; AAF99780.1; ZP_00563258.1; EAN09988.1; NP_148290.1; YP_253295.1; ZP_00627278.1; YP_256008.1; AAO91209.1; XP_655257.1; ZP_00303629.1; CAC11669.1; NP_394004.1; EAM93962.1; CAB57769.1; |
| | CAB50124.1; ZP_00520055.1; ZP_00369709.1; EAN28089.1; CAC12627.1; AAB85605.1; Q9HI38; AAH26135.1; EAM24571.1; AAC03768.1; AAP98645.1; CAJ18422.1; NP_213757.1; CAA05909.1; NP_142859.1; BAD32415.1; BAC31437.1; NP_947805.1; YP_080623.1; CAH93356.1; ZP_00600159.1; NP_775139.1; ZP_00400734.1; CAA09590.2; XP_321361.2; AAL81283.1; BAB81491.1; ZP_00506445.1; ZP_00533640.1; YP_160688.1; NP_764776.1; NP_967201.1; AAH52991.1; AAO38289.1; NP_837365.1; XP_521504.1; NP_371248.1; NP_753970.1; CAG42465.1; AAC74750.1; AAU93791.1; Q7ADI4; 1KMK; 1JF9; BAA86566.1; YP_170184.1; YP_165179.1; NP_624289.1; AAX77932.1; CAD14721.1; BAD84717.1; ZP_00048472.2; AAF73526.1; XP_641773.1; ZP_00570672.1; ZP_00400089.1; NP_391148.1; NP_001007947.1; CAE08806.1; CAB50330.1; YP_192068.1; ZP_00152262.1; ZP_00502920.1; YP_096188.1; NP_796975.1; YP_254084.1; YP_124440.1; BAD86003.1; CAH04407.1; BAB07188.1; Q9K7A0; XP_313472.2; NP_103175.1; AAF63783.1; AAN58019.1; YP_127437.1; AAM62682.1; CAG90500.1; AAB85857.1; ZP_00387883.1; XP_562557.1; 027433; ZP_00170920.2; CAA63066.1; EAN08419.1; CAB73027.1; ZP_00335679.1; AAB85866.1; ZP_00129032.1; YP_178855.1; YP_145876.1; AAV94639.1; NP_623991.1; YP_040300.1; YP_040204.1; YP_080545.1; YP_253952.1; ZP_00638523.1; NP_371368.1; YP_255707.1; AAU38404.1; NP_085678.1; AAK68403.2; BAB66416.1; XP_624944.1; ZP_00135005.2; ZP_00595047.1; NP_950100.1; ZP_00458730.1; AAQ75173.1; AAP99129.1; YP_004062.1; XP_601722.1; CAC45089.1; CAA07007.1; EAL01528.1; XP_694140.1; NP_770978.1; AAC49935.1; EAA21518.1 |
| GcvH | WP_001295377; NP_391159; YP_152075.1; NP_806664.1; NP_755359.1; Q8FE66; AAX66901.1; ZP_00585063.1; NP_716411.1; ZP_00582520.1; NP_930809.1; YP_071682.1; ZP_00638530.1; CAG73658.1; YP_156474.1; ZP_00634545.1; YP_131233.1; YP_268018.1; ZP_00417033.1; AAN70758.1; ZP_00141691.2; NP_253901.1; AAZ18651.1; EAO18275.1; ZP_00264789.1; ZP_00474390.1; ZP_00499480.1; ZP_00459441.1; EAM28059.1; ZP_00318113.1; AAQ61093.1; NP_790167.1; AAO91210.1; CAH37374.1; YP_169453.1; AAW49868.1; NP_840692.1; AAO07160.1; |
| | YP_263019.1; Q9K0L7; YP_160523.1; NP_800312.1; ZP_00334896.1; ZP_00278042.1; YP_233358.1; AAW90049.1; ZP_00213264.1; AAF96187.1; CAB84042.1; AAM37905.1; ZP_00658478.1; YP_112591.1; YP_132994.1; YP_200434.1; ZP_00593911.1; YP_206660.1; ZP_00167207.1; XP_464281.1; NP_638224.1; EAM75684.1; ZP_00574836.1; ZP_00151463.1; YP_270507.1; CAD17082.1; BAD45416.1; YP_004124.1; YP_143790.1; YP_075750.1; AAF11361.1; NP_465948.1; CAG35029.1; NP_960473.1; ZP_00568923.1; EAN06352.1; ZP_00506635.1; NP_532153.1; YP_223285.1; NP_541538.1; YP_014985.1; AAO63775.1; CAA20174.1; ZP_00399762.1; YP_010645.1; NP_471849.1; AAO77626.1; YP_094171.1; NP_693309.1; BAC70485.1; NP_228027.1; YP_253966.1; 1ZKO; YP_148857.1; YP_046528.1; YP_040289.1; ZP_00131107.1; YP_176481.1; NP_297474.1; Q9PGW7; ZP_00355907.1; ZP_00651773.1; NP_778393.1; YP_021882.1; NP_855509.1; CAF26480.1; CAG42548.1; ZP_00389942.1; NP_981423.1; AAP11863.1; NP_302386.1; NP_764151.1; YP_266090.1; ZP_00237746.1; ZP_00244923.1; ZP_00396528.1; YP_188077.1; CAC46127.1; ZP_00375764.1; ZP_00560255.1; ZP_00270642.1; BAB07203.1; EAN28788.1; NP_216342.1; CAH09835.1; NP_251136.1; YP_185749.1; AAT49691.1; ZP_00264532.1; NP_102590.1; YP_034021.1; NP_879085.1; AAP54618.1; ZP_00625541.1; ZP_00525160.1; AAV46421.1; ZP_00412765.1; CAH02746.1; CAA81075.1; CAB16912.1; CAB16710.1; CAA85761.1; ZP_00622637.1; CAB16914.1; CAA85759.1; NP_143205.1; YP_101635.1; ZP_00380048.1; ZP_00004511.1; ZP_00414055.1; AAC61829.1; AAL81616.1; YP_261724.1; CAB49742.1; AAN66614.1; Q9V0G1; YP_080558.1; BAD84339.1; AAK25316.1; NP_949186.1; CAJ13836.1; AAQ67414.1; 1DXM; CAA85768.1; CAA85757.1; P93255; CAJ13723.1; CAA85755.1; ZP_00292299.1; ZP_00308328.1; NP_772392.1; CAI36363.1; NP_147622.1; NP_391159.1; ZP_00577152.1; CAA85756.1; AAQ66080.1; AAV94183.1; CAA88734.1; ZP_00547429.1; NP_621789.1; CAA85760.1; AAR37471.1; ZP_00555140.1; AAW47059.1; ZP_00303630.1; NP_621987.1; AAG48828.1; CAG86839.1; YP_191521.1; YP_055457.1; ZP_00631037.1; YP_118696.1; CAA85767.1; XP_637044.1; AAM64413.1; CAC19751.1; ZP_00379709.1; NP_280389.1; CAF23006.1; |
| | EAO25275.1; AAU84892.1; CAF92157.1; EAN04067.1; AAH91548.1; YP_164889.1; NP_213756.1; CAG62852.1; AAW49010.1; CAA94317.1; ZP_00527786.1; YP_234189.1; CAD52976.1; NP_967650.1; CAE66592.1; EAA66192.1; AAW27708.1; ZP_00565058.1; XP_536768.1; AB66246.1; CAA95820.1; AAL68248.1; AAH14745.1; NP_791107.1; EAL90537.1; XP_579628.1; XP_316586.2; AAP88829.1; NP_004474.2; NP_080848.1; NP_951435.1; XP_523434.1; CAA85754.1; Q9N121; AAS59848.1; YP_172757.1; ZP_00534758.1; ZP_00591423.1; ZP_00512042.1; ZP_00661686.1; NP_883103.1; ZP_00054163.1; NP_598282.1; AAW31875.1; XP_756407.1; AAH76212.1; CAF99616.1; CAA94316.1; NP_953067.1; EAA72139.1; NP_110807.1; AAS52315.1; ZP_00575020.1; P20821; NP_662509.1; CAG33353.1; CAE63163.1; ZP_00589916.1; XP_217678.1; YP_256010.1; XP_582835.1; CAC12487.1; NP_394822.1; ZP_00535962.1; NP_001004372.1; EAL29812.1; CAB05472.1; XP_615385.1; EAL03567.1; O22535; AAH82740.1; AAX07637.1; NP_926477.1; EAM93557.1; XP_584988.1; AAM92707.1 ZP_00515529.1; ZP_00530979.1; AAH81062.1; ZP_00111606.1; NP_682468.1; Q8DIB2; EAA77334.1; AAN47560.1; AAL33596.1; ZP_00162706.2; NP_342412.1; Q8G4Z7; ZP_00120558.2; ZP_00136571.1; NP_972232.1; CAE18120.1; Q8YNF8; XP_604979.1; CAE08890.1; CAE76092.1; YP_023402.1; NP_009355.2; P39726; CAD75450.1; NP_440920.1; CAE47935.1; XP_498178.1; NP_893786.1; ZP_00327635.1; EAN76953.1; ZP_00140179.2; EAN99694.1; AAZ14696.1; CAE22344.1; EAN83079.1; BAB26349.1; YP_169819.1; AAX78078.1; AAQ00874.1; AAC36844.1; CAG78944.1; XP_694123.1; ZP_00050263.1; AAO44734.1; XP_414165.1; ZP_00654389.1; ZP_00575538.1; XP_518701.1; BAB66989.1; NP_342534.1; XP_583383.1; NP_213643.1; YP_255059.1; NP_214139.1; NP_213280.1; ZP_00540302.1; YP_055788.1; AAP05107.1; AAF39393.1; ZP_00399135.1; NP_701199.1; CAD75020.1; XP_343995.2; AAP98380.1; P_300490.1; XP_635521.1; YP_219766.1; XP_637059.1; AAV71155.1; XP_739104.1; EAA18076.1; NP_219787.1; ZP_00400682.1; NP_967270.1; ZP_00384691.1; ZP_00401280.1; NP_816146.1; ZP_00526092.1; NP_213714.1; YP_255058.1; NP_785822.1; BAB66988.1; |
| | NP_253484.1; ZP_00141248.1; ZP_00399137.1; XP_676766.1; EAO22790.1; NP_342572.1; AAF07900.1; ZP_00152140.2; XP_518003.1; AAA23866.1; AAP06383.1; AAM99940.1; ZP_00523572.1; AAH09065.1; YP_039780.1; NP_735539.1; NP_326272.1; YP_252181.1; NP_802319.1; XP_637062.1; AAK70873.1; ZP_00523574.1; NP_784119.1; XP_356748.3; YP_115837.1; XP_520481.1; BAB94166.1; XP_527720.1; NP_975513.1; ZP_00511802.1; ZP_00660576.1; XP_598843.1; ZP_00335312.1; ZP_00501149.1; ZP_00496208.1; ZP_00488962.1; ZP_00481092.1; ZP_00470769.1; YP_112037.1; ZP_00315286.1; ZP_00591859.1; NP_622848.1; CAB59889.1; ZP_00566435.1; ZP_00633779.1; AAG13505.2; CAB16915.1; ZP_00585787.1; ZP_00531610.1; AAW51218.1; YP_263215.1; AAK22373.1; AAT58044.1; EAA20319.1; NP_842410.1; EAN32517.1; NP_532021.1; AAH75478.1; NP_533976.1; ZP_00385854.1; AAL04441.1; AAK89915.1; BAD16654.1; NP_216731.1; NP_770578.1; ZP_00547768.1; NP_522769.1; ZP_00383064.1; ZP_00322497.1; YP_022846.1; NP_961247.1;YP_238125.1; ZP_00412124.1; XP_475165.1; XP_470945.1; NP_910410.1; NP_635909.1; CAE01575.2; XP_419793.1; ZP_00170555.1; NP_217017.1;; ZP_00556997.1; AAP99073.1; NP_297341.1; AAX73221.1; NP_214109.1; NP_440434.1; CAG87711.1; ZP_00051435.1; BAB80778.1; XP_393389.2; ZP_00651791.1; ZP_00269419.1; ZP_00423019.1; YP_170419.1; NP_001006383.1; XP_482561.1; NP_778293.1; CAB03400.1; CAH04871.1; XP_655127.1; AAF61288.1; ZP_00356683.1; ZP_00565878.1; CAJ01708.1; XP_758384.1; AAU38115.1; AAK23858.1; XP_688912.1; BAB06344. |
| GcvP | WP_000195062; CAA52144, NP_390336; NP_708668.1; Q8XD32; NP_755360.1; 1VLO; YP_152076.1; AAX66902.1; YP_071683.1; NP_670593.1; NP_930810.1; GAG73657.1; ZP_00585064.1; ZP_00634546.1; NP_716410.1; ZP_00638531.1; ZP_00582519.1; YP_156475.1; YP_268017.1; ZP_00141692.2; ZP_00417034.1; NP_253902.1; AAT51348.1; ZP_00318114.1; YP_233357.1; AAO91211.1; AAM37906.1; ZP_00264790.1; NP_790166.1; YP_125494.1; YP_263020.1; YP_122482.1; YP_094172.1; AAN70759.1; Q5ZZ93; YP_200433.1; NP_638225.1; YP_112882.1; NP_778394.1; NP_840691.1; EAO18276.1; |
| | NP_297476.1; ZP_00651772.1; YP_160524.1; AAQ61094.1; ZP_00334897.1; YP_104498.1; ZP_00213265.1; EAM28060.1; NP_887403.1; NP_883102.1; ZP_00151462.1; ZP_00654390.1; NP_879084.1; ZP_00459440.1; ZP_00454903.1; ZP_00278043.1; ZP_00499481.1; EAN28090.1; CAD17081.1; Q9K0L8; AAZ18652.1; CAB84041.1; Q9JVP2; ZP_00167206.1; YP_169452.1; AAW90051.1; AAW49997.1; ZP_00593910.1; ZP_00419736.1; ZP_00560254.1; YP_041010.1; YP_186435.1; YP_253294.1; NP_764777.1; YP_079784.1; ZP_00325013.1; YP_172504.1; BAB06535.1; NP_621988.1; NP_390337.1; NP_464873.1; ZP_00233534.1; ZP_00165293.2; YP_013963.1; NP_470721.1; NP_681534.1; BAB76308.1; ZP_00399761.1; ZP_00517920.1; NP_692825.1; Q8CXD9; NP_926476.1; Q7NFJ5; YP_075751.1; NP_228026.1; ZP_00162705.1; YP_148278.1; AAQ00895.1; 1YX2; ZP_00355906.1; ZP_00111605.1; AAU84891.1; NP_441988.1; YP_085561.1; ZP_00238491.1; NP_980598.1; ZP_00530333.1; NP_662667.1; AAP11141.1; CAE22389.1; CAE08940.1; EAO25274.1; ZP_00574835.1; ZP_00511544.1; NP_967651.1; YP_004123.1; ZP_00590889.1; YP_175991.1; ZP_00538579.1; YP_143789.1; ZP_00533333.1; ZP_00307846.1; ZP_00396529.1; ZP_00660916.1; ZP_00588126.1; ZP_00525159.1; ZP_00531283.1; NP_893804.1; CAI36362.1; AAO79689; AAX16385.1; NP_393488.1; CAH07007.1; CAA20175.1; NP_110577.1; ZP_00292300.1; YP_055458.1; BAB59199.1; BAC70484.1; EAM94419.1; NP_960885.1; ZP_00412766.1; YP_117906.1; NP_301653.1; NP_972233.1; NP_295535.1; AAQ66593.1; NP_216727.1; P64220; ZP_00646130.1; YP_023281.1; CAC11159.1; O67441; EAM73669.1; CAF23005.1; ZP_00656447.1; AAN47561.1; YP_000301.1; CAD75451.1; Q8F935; AAV46422.1; Q5V230; ZP_00631038.1; AAO07163.1; ZP_00379710.1; AAK25317.1; ZP_00574284.1; ZP_00549460.1; AAL33597.1; NP_936752.1; XP_473945.1; CAA81081.1; ZP_00054164.1; ZP_00577153.1; CAB16917.1; NP_143049.1; O58888; CAB50008.1; BAD86224.1; AAB38502.1; YP_164888.1; EAL00308.1; NP_280390.1; YP_206658.1; Q9HPJ7; CAA52800.1; EAL00186.1; ZP_00130510.2; AAP21169.1; NP_800315.1; CAA81077.1; CAA94902.1; NP_789581.1; CAA10976.1; AAO44735.1; XP_756577.1; YP_266089.1; |
| | CAB11698.1; ZP_00535963.1; NP_949185.1; XP_629708.1; ZP_00270643.1; AAK87256.1; AAL81465.1; YP_261727.1; NP_010302.1; EAN06351.1; YP_010902.1; CAG77727.1; AAB05000.1; ZP_00303631.1; NP_001006021.1; ZP_00625540.1; CAG85941.1; NP_951434.1; NP_772391.1; CAF93361.1; CAH02226.1; YP_270503.1; CAF26481.1; ZP_00601921.1; XP_394029.2; ZP_00474388.1; CAC46128.1; AAN66611.1; ZP_00264535.1; NP_251132.1; ZP_00375763.1; ZP_00140175.2; ZP_00555141.1; YP_234187.1; AAT51611.1; ZP_00622636.1; ZP_00506634.1; NP_791105.1; AAR21108.1; ZP_00417689.1; EAN86200.1; AAW42395.1; EAL33114.1; BAB66247.1; NP_532154.1; YP_034022.1; NP_102589.1; XP_331926.1; EAN85387.1; CAG58515.1;; AAB37080.1; ZP_00244922.1; YP_191520.1; CAJ09347.1; CAJ09346.1; CAE64583.1; AAN33909.1; YP_223284.1; NP_214006.1; BAA02967.1; BAA03512.1; AAF52996.1; AAX33383.1; XP_322034.2; NP_541537.1; NP_001013836.1; EAA68431.1; NP_001014026.1; XP_620786.1; EAA51066.1; EAN04066.1; XP_541886.1; YP_256009.1; Q9TSZ7; YP_132990.1; NP_990119.1; AAH07546.2; EAA65791.1; Q9YBA2; EAL90405.1; CAC41491.1; NP_342411.1; EAN79919.1; XP_517018.1; NP_107651.1; ZP_00004512.2; BAA12709.1; NP_148104.1; AAV94849.1; YP_266710.1; XP_516459.1; NP_105581.1; ZP_00620069.1; ZP_00631895.1; AAL13520.1; YP_047137.1; AAF68432.3; CAC46853.1; XP_542460.1; ZP_00460296.1; ZP_00554633.1; ZP_00282393.1; NP_102909.1; YP_235303.1; AAQ87218.1; ZP_00213445.1; NP_106044.1; ZP_00169723.2; ZP_00500952.1; ZP_00489849.1; ZP_00480439.1; ZP_00450235.1; ZP_00436058.1; AAY59105.1; EAM32228.1; AAW21506.1; CAD14805.1; ZP_00410721.1; NP_792264.1; Q46337; NP_521609.1; BAD97818.1; 1X31; NP_534554.1; ZP_00602139.1; AAK16489.1; YP_266475.1; EAA22341.1; XP_318114.2; CAC47432.1; ZP_00004192.1; NP_534790.1; AAL52901.1; ZP_00565350.1; ZP_00657316.1; YP_134767.1; ZP_00379371.1; YP_134764.1; XP_742683.1; AAN29180.1; ZP_00602144.1; CAG35030.1; EAM31940.1; ZP_00556129.1; CAC49374.1; ZP_00050264.2; ZP_00379741.1; NP_705537.1; ZP_00471825.1; NP_104736.1; ZP_00600293.1; AAY87206.1; YP_266690.1; AAL76414.1; AAC31611.1; AAR38319.1; |
| | ZP_00658996.1; YP_266673.1; NP_105928.1; ZP_00630198.1; YP_266631.1; NP_254105.1; NP_107666.1; NP_104289.1; NP_102901.1; AAV96623.1; BAC74662.1; AAV95607.1; ZP_00620942.1; ZP_00645790.1; CAC41486.1; EAN05741.1; AAN65213.1; YP_269196.1; ZP_00620654.1; ZP_00660136.1; NP_885663.1; NP_436414.1; NP_881143.1; AAM75070.1; NP_572162.2; ZP_00379745.1; CAA39468.1; ZP_00264573.1; YP_262784.1; ZP_00380782.1; ZP_00278582.1; AAV94866.1; XP_414684.1; YP_237780.1; ZP_00602141.1; YP_165138.1; NP_790307.1; NP_620802.2; NP_534700.1; AAV95690.1; EAL32452.1; AAF21941.1; AAN65956.1; NP_083048.1; AAV94935.1; CAC46854.1; CAC41470.1; AAH24126.1; NP_037523.2; ZP_00565365.1; NP_102887.1; YP_134760.1; AAQ87217.1; AAH89599.1; ZP_00554816.1; AAK16482.1; NP_103085.1; ZP_00620147.1; NP_102906.1; ZP_00556188.1; XP_307967.2; ZP_00622120.1; CAC47100.1; NP_106776.1; CAH90377.1; ZP_00554918.1; CAD31640.1; XP_672544.1; AAT81177.1; AAK27867.2; AAD33412.1; NP_107653.1; CAI12276.1; BAD97122.1; ZP_00410737.1; AAD43585.1; NP_103793.1; XP_526883.1; XP_548398.1; CAB63337.2; YP_266661.1; ZP_00521798.1; EAA60688.1; AAL51865.1; AAV95026.1; EAL26357.1; ZP_00619907.1; CAE74368.1; NP_103190.1; AAV94915.1; AAG55663.1; XP_395831.2; AAK92969.1; NP_446116.1; AAF57796.1; AAN71380.1; ZP_00005411.1; CAE58942.1; EAA70894.1; AAV94873.1; ZP_00631887.1; ZP_00629673.1; BAB34711.1; ZP_00619923.1; ZP_00622863.1; EAL85410.1; AAL76413.1; AAR38318.1; CAD47921.1; AAH03456.1; NP_102854.1; AAH76859.1; AAL04443.1; AAH68953.1; YP_165137.1; XP_580581.1; ZP_00050273.2; ZP_00556400.1; ZP_00555517.1; AAV96627.1; AAV93943.1; AAH81271.1; AAK87410.1; ZP_00556086.1; AAV93879.1; AAH44792.1; XP_527208.1; ZP_00327983.1; ZP_00554213.1; NP_532318.1; CAI12274.1; AAH22388.1; XP_546052.1; XP_676622.1; AAV93533.1; YP_265671.1; AAV47350.1; AAV95190.1; CAD31286.1; ZP_00516133.1; ZP_00620892.1; AAH55193.1; AAY82706.1; ZP_00052785.2; AAR38102.1 |
| GcvL (LpdA) protein | P0A9P0; NP_706070.2; NP_752095.1; CAA24742.1; AAX64059.1; AAL19118.1; NP_804043.1; YP_149503.1; CAG76686.1; YP_069256.1; NP_930833.1; NP_935564.1; AAO10051.1; AAF95555.1; ZP_00585786.1; |
| | AAK02977.1; NP_798896.1; AAC46405.1; ZP_00122566.1; ZP_00132373.2; YP_131302.1; YP_205561.1; NP_716063.1; ZP_00637900.1; ZP_00633839.1; ZP_00582828.1; AAU37941.1; ZP_00134358.2; ZP_00157402.1; AAX88688.1; NP_439387.1; ZP_00154973.1; AAP96400.1; YP_154852.1; YP_271444.1; ZP_00464633.1; ZP_00451158.1; ZP_00212747.1; ZP_00500723.1; ZP_00486500.1; ZP_00463487.1; ZP_00467577.1; ZP_00423839.1; ZP_00423458.1; ZP_00283805.1; AAO90013.1; ZP_00595215.1; ZP_00170705.2; NP_879789.1; YP_123783.1; NP_889077.1; YP_126870.1; NP_240038.1; YP_095531.1; CAD15305.1; AAQ58205.1; NP_883762.1; NP_770362.1; YP_170418.1; CAA61894.1; AAV29309.1; CAB84783.1; AAF41719.1; ZP_00565931.1; CAA59171.1; CAA54878.1; AAW89295.1; CAA62435.1; ZP_00150164.2; 1 BHY; 1OJT; CAA61895.1; YP_157096.1; CAA57206.1; AAM38502.1; NP_635936.1; YP_199361.1; NP_660554.1; NP_842161.1; ZP_00507350.1 NP_779995.1; YP_115390.1; ZP_00651360.1; NP_298158.1; AAR38073.1; EAO17659.1; ZP_00245305.1; AAR38213.1; AAR38090.1; NP_777818.1; BAC24467.1; NP_891227.1; NP_879460.1; NP_878457.1; CAD71978.1; YP_078853.1; AAK50273.1; AAK50266.1; AAF11916.1; NP_389344.1; ZP_00396676.1; EAO21015.1; CAA37631.1; 1EBD; YP_146914.1; BAB06371.1; YP_085309.1; AAP10890.1; YP_020826.1; YP_175913.1; Q04829; AAN03817.1; NP_692336.1; AAG17888.1; ZP_00474314.1; NP_764349.1; AAA99234.1; 1 LPF; NP_250278.1; XP_475628.1; YP_253771.1; YP_143499.1; YP_257414.1; AAF34795.3; AAF79529.1; YP_040483.1; YP_005722.1; YP_074243.1; AAN23154.1; AAK50305.1; AAS20045.1; ZP_00540244.1; EAN07674.1; AAC26053.1; NP_815077.1; NP_908725.1; ZP_00307577.1; AAS47493.1; AAF34796.1; CAA11554.1; YP_013676.1; ZP_00317120.1; AAV48381.1; CAB84413.1; AAB30526.1; NP_969527.1; BAB44156.1; NP_464580.1; XP_635122.1; AAF41363.1; AAK50280.1; ZP_00397330.1; YP_265659.1; NP_470384.1; CAA44729.1; AAW89611.1; 1DXL; ZP_00401182.1 ZP_00418304.1; NP_792022.1; ZP_00625011.1; AAD53185.1; 3LAD; EAN08634.1; AAH18696.1; CAH93405.1; YP_235092.1; NP_967737.1; CAJ08862.1; NP_945538.1; NP_763632.1; BAE00452.1; BAD92940.1; |
| | NP_000099.1; IZMD; AAB01381.1; NP_999227.1; NP_767089.1; AAS47708.1; AAR21288.1; AAA35764.1; YP_034342.1; EAN90443.1; EAN96941.1; CAA61483.1; AAN69768.1; AAF12067.1; P31052; NP_105199.1; ZP_00263252.1; AAH62069.1; CAA72132.1; NP_031887.2; CAG58981.1; CAF26798.1; EAN80618.1; AAN15202.1; CAA72131.1; ZP_00269527.1; CAD72797.1; ZP_00554136.1; CAD61860.1; AAC53170.1; CAF05589.1; ZP_00622437.1; CAG81278.1; ZP_00284261.1; ZP_00497224.1; EAK93183.1; ZP_00492121.1; NP_533297.1; AAS53883.1; YP_160845.1; AAV93660.1; CAG31211.1; CAA49991.1; AAM93255.1; AAK11679.1; ZP_00427535.1; YP_258846.1; AAN30810.1;ZP_00449174.1; CAF92514.1;AAQ91233.1;AAH44432.1; XP_320877.2; AAH56016.1; YP_222565.1; CAC47627.1; AAA96487.1; ZP_00464142.1; NP_280867.1; YP_067405.1; CAB65609.1; AAL51327.1; AAK22329.1; YP_246823.1; NP_105334.1; XP_758608.1; CAH00655.1; NP_772974.1; AAB88282.1; ZP_00211386.1; EAN27796.1; AAN70931.1; EAL29693.1; ZP_00340462.1; ZP_00153792.2; ZP_00579524.1; AAZ17978.1; NP_266215.1; AAN33719.1; AAD30450.1; ZP_00383074.1; ZP_00597315.1; CAC47514.1; AAF49294.1; YP_223465.1; NP_220840.1; NP_360330.1; EAA26462.1; CAA39235.1; ZP_00578463.1; YP_047424.1; AAM36402.1; BAB03935.1; AAN69982.1; NP_116635.1; ZP_00654346.1; CAG85768.1; 1V59; NP_623271.1; AAA65618.1; ZP_00305550.1; XP_623438.1; ZP_00007570.1; ZP_00320049.1; AAN75183.1; ZP_00323583.1; YP_200681.1; 1LVL; ZP_00151187.2; AAP03132.1; CAD14973.1; ZP_00630163.1; ZP_00139957.1; NP_250940.1; NP_636857.1; CAB05249.2; ZP_00166998.2; AAN75720.1; CAA62982.1; ZP_00265019.1; ZP_00384289.1; AAV47687.1; ZP_00303079.1; NP_842316.1; XP_331183.1; AAV28779.1; AAN48422.1; ZP_00597992.1; AAN75618.1; AAV28746.1; ZP_00267415.1; ZP_00650982.1; AAQ58749.1; NP_298837.1; AAN75159.1; NP_778978.1; ZP_00575798.1; YP_002403.1; AAB97089.1; ZP_00511405.1; YP_005669.1; EAO21998.1; XP_613473.1; ZP_00245417.1; ZP_00210841.1; ZP_00561492.1; YP_259638.1; EAO16949.1; NP_785656.1; CAF23812.1; ZP_00055963.2; YP_143553.1; NP_953492.1; CAA63810.1; CAF22875.1; AAV89136.1; ZP_00536790.1; AAF39644.1; |
| | ZP_00621355.1; ZP_00486105.1; ZP_00020745.2; ZP_00589771.1; YP_180376.1; NP_220072.1; CAI27032.1; EAL87307.1; YP_112273.1; ZP_00376179.1; ZP_00498294.1; ZP_00492099.1; ZP_00463379.1; ZP_00217095.1; CAI27980.1; AAK23707.1; CAD60736.1; ZP_00268854.1; EAA77706.1; ZP_00629856.1; NP_879905.1; EAA51976.1; NP_885384.1; CA129613.1; AAA91879.1; ZP_00376555.1; ZP_00141283.2; NP_253516.1; NP_300890.1; AAB40885.1; AAN03814.1; ZP_00644737.1; AAO36548.1; AAP98791.1; YP_079735.1; NP_388690.1; AAP05672.1; NP_966507.1; P95596; EAN04065.1; NP_532124.1; ZP_00507305.1; NP_948204.1; ZP_00557093.1; YP_220287.1; ZP_00642506.1; ZP_00591535.1; NP_102193.1; NP_771418.1; AAA19188.1; AAK72471.1; AAK72470.1; NP_345630.1; NP_756887.1; ZP_00404212.1; AAK72472.1; AAN50085.1; CAC46029.1; YP_001129.1; AAL64341.1; ZP_00526430.1; ZP_00308867.1; YP_053282.1; YP_036862.1; ZP_00210426.1; ZP_00625423.1; ZP_00601791.1; YP_045732.1; YP_016277.1; P54533; AAV95488.1; AAW71149.1; YP_021029.1; YP_153983.1; ZP_00240355.1; XP_395801.2; EAN08156.1; AAP94898.1; NP_326592.1; AAP11076.1; ZP_00239726.1; NP_980528.1; ZP_00620223.1; ZP_00512893.1; YP_019413.1; NP_148088.1; XP_678378.1; YP_180009.1; NP_735347.1; CAI26632.1; AAN30046.1; BAB04498.1; AAN57909.1; ZP_00373647.1; NP_692788.1; ZP_00053288.1; EAM72947.1; YP_078075.1; ZP_00006401.1; EAA16706.1; YP_221832.1; XP_742153.1; AAL52038.1; NP_966125.1; YP_247286.1; AAA74473.1; ZP_00589476.1; CAI38117.1; AAO78292.1; EAA26057.1; BAD11090.1; ZP_00545191.1; CAE73952.1; YP_060098.1; BAB05544.1; NP_802451.1; NP_360876.1; AAL97648.1; ZP_00154188.2; ZP_00331725.1; AAV62625.1; CAG35032.1; YP_084091.1; ZP_00366080.1; YP_139515.1; YP_121481.1; ZP_00340821.1; ZP_00531539.1; ZP_00630106.1; CAB06298.1; NP_979105.1;; ZP_00162168.1; AAP09729.1; YP_175948.1; NP_938751.1; ZP_00277446.1; BAB76444.1; YP_148232.1; AAK33923.1; AAO75416.1; AAT58044.1; YP_116014.1; BAD11095.1; P_00571989.1; YP_033412.1; AAT47753.1; NP_221155.1; ZP_00111840.1 ; ZP_00293744.1; |
| | CAF34426.1; YP_055934.1; EAM93810.1; NP_214976.1; NP_975267.1; NP_701672.1; NP_662186.1; A AG12404.1; NP_390286.1; AAB96096.1; BAB64316.1; YP_075992.1; ZP_00656450.1; ZP_00413985.1 |

In one example, the cell according to any aspect of the present invention may comprise at least a first genetic mutation that increases the expression relative to the wild type cell of an amino acid-N-acyl-transferase and a second genetic mutation that increases the expression of an acyl-CoA synthetase, and at least a third genetic mutation that decreases the expression relative to the wild type cell of at least one enzyme selected from the group consisting of an enzyme of the glycine cleavage system, glycine hydroxymethyltransferase (GlyA) and threonine aldolase (ItaE). This enzyme from the glycine cleavage system may be GcvT, GcvP, GcvL or GcvH. In particular, the expression of GcvT and GcvP, GcvT and GcvH or GcvP and GcvH is decreased. In one example, the expression of GcvT, GcvP and GcvH may be decreased. More in particular, the sequence of GcvT, GcvP and GcvH may be SEQ ID NO:58, SEQ ID NO:60 and SEQ ID NO:59 respectively. The sequence of GlyA may be SEQ ID NO:61 and the sequence of ItaE may be SEQ ID NO:62.

In another example, the cell according to any aspect of the present invention may comprise at least a first genetic mutation that increases the expression relative to the wild type cell of an amino acid-N-acyl-transferase and a second genetic mutation that increases the expression of an acyl-CoA synthetase, and at least a third genetic mutation that decreases the expression relative to the wild type cell of GcvT, GcvP, GcvH, GlyA and ItaE; or GcvT, GcvP, GcvH, and GlyA; or GcvT, GcvP, GcvH, and ItaE; or GcvT and GcvH and GlyA and/or ItaE; or GcvP and GcvH and GlyA and/or ItaE; or GcvT and GcvP and GlyA and/or ItaE.

The expression of GcvL may be maintained or decreased according to any aspect of the present invention. The phrase "decreased activity of an enzyme" as used herein is to be understood as decreased intracellular activity. Basically, a decrease in enzymatic activity can be achieved by decreasing the copy number of the gene sequence or gene sequences that code for the enzyme, using a weak promoter or employing a gene or allele that silence the respective enzyme to decrease activity and optionally by combining these measures. Genetically modified cells used according to any aspect of the present invention are for example produced by transformation, transduction, conjugation or a combination of these methods with a vector that contains a silencer that binds to this gene or parts thereof and a vector that makes switches off the gene possible.

The cell according to any aspect of the present invention may comprise a further genetic mutation that results in the cell having a reduced fatty acid degradation capacity relative to the wild type cell. In particular, the reduced fatty acid degradation capacity may be a result of a decrease in expression relative to the wild type cell of at least one enzyme selected from the group consisting of acyl-CoA dehydrogenase, 2,4-dienoyl-CoA reductase, enoyl-CoA hydratase and 3-ketoacyl-CoA thiolase

In particular, the term "having a reduced fatty acid degradation capacity", as used herein, means that the respective cell degrades fatty acids, especially those taken up from the environment, at a lower rate than a comparable cell or wild type cell having normal fatty acid degradation capacity would under identical conditions. More in particular, the fatty acid degradation of such a cell is lower on account of deletion, inhibition or inactivation of at least one gene encoding an enzyme involved in the β-oxidation, pathway. In one example, at least one enzyme involved in the β-oxidation pathway has lost 5, 10, 20, 40, 50, 75, 90 or 99 % activity relative to the activity of the same enzyme under comparable conditions in the respective wild type microorganism. The person skilled in the art is familiar with various techniques that may be used to delete a gene encoding an enzyme or reduce the activity of such an enzyme in a cell, for example by exposition of cells to radioactivity followed by accumulation or screening of the resulting mutants, site-directed introduction of point mutations or knock out of a chromosomally integrated gene encoding for an active enzyme, as described in Sambrook/Fritsch/Maniatis (1989). In addition, the transcriptional repressor FadR may be over expressed to the effect that expression of enzymes involved in the β-oxidation, pathway is repressed (Fujita, Y., 2007).

The term "deletion of a gene", as used herein, means that the nucleic acid sequence encoding the gene may be modified such that the expression of active polypeptide encoded by the gene is reduced. For example, the gene may be deleted by removing in-frame a part of the sequence comprising the sequence encoding for the catalytic active centre of the polypeptide. Alternatively, the ribosome binding site may be altered such that the ribosomes no longer translate the corresponding RNA. The person skilled in the art is able to routinely measure the activity of enzymes expressed by living cells using standard assays as described in enzymology text books, for example Cornish-Bowden (1995).

Degradation of fatty acids may be accomplished by a sequence of enzymatically catalysed reactions. First of all, fatty acids are taken up and translocated across the cell membrane *via* a transport/acyl-activation mechanism involving at least one outer membrane protein and one inner membrane-associated protein which has fatty acid-CoA ligase activity, referred to in the case of *E. coli as* FadL and FadD / FadK, respectively. Inside the cell, the fatty acid to be degraded is subjected to enzymes catalysing other reactions of the β-oxidation, pathway. The first intracellular step involves the conversion of acyl-CoA to enoyl-CoA through acyl-CoA dehydrogenase, the latter referred to as FadE in the case of *E. coli.* The activity of an acyl-CoA dehydrogenase may be assayed using any method known in the art. For example by monitoring the concentration of NADH spectrophotometrically at 340 nm in 100 mM MOPS, pH 7.4, 0.2 mM Enoyl-CoA, 0.4 mM NAD⁺. The resulting enoyl-CoA is converted to 3-ketoacyl-CoA *via* 3-hydroxylacyl-CoA through hydration and oxidation, catalysed by enoyl-CoA hydratase/3-hydroxyacyl-CoA dehydrogenase, referred to as FadB and FadJ in *E*. *coli.* Enoyl-CoA hydratase/3-hydroxyacyl-CoA dehydrogenase activity, more specifically formation of the product NADH may be assayed spectrophotometrically as described in the state of the art, for example as outlined for FadE. Finally, 3-ketoacyl-CoA thiolase, FadA and FadI in *E. coli,* catalyses the cleavage of 3-ketoacyl-CoA, to give acetyl-CoA and the input acyl-CoA shortened by two carbon atoms. The activity of ketoacyl-CoA thiolase may be assayed as described in the state of the art, for example in Antonenkov, V., 1997.

In particular, the term "a cell having a reduced fatty acid degradation capacity", as used herein, refers to a cell having a reduced capability of taking up and/or degrading fatty acids, in particular those having at least eight carbon chains. The fatty acid degradation capacity of a cell may be reduced in various ways. In a one example, the cell has, compared to its wild type, a reduced activity of an enzyme involved in the β-oxidation, pathway. In one example, the term "enzyme involved in the β-oxidation, pathway", as used herein, refers to an enzyme that interacts directly with a fatty acid or a derivative thereof formed as part of the degradation of said fatty acid *via* the β-oxidation, pathway the sequence of reactions effecting the conversion of a fatty acid to acetyl-CoA and the CoA ester of the shortened fatty acid, preferably by recognizing the fatty acid or derivative thereof as a substrate, and converts it to a metabolite formed as a part of the β-oxidation, pathway. For example, the acyl-CoA dehydrogenase is an enzyme involved in the β-oxidation pathway as it interacts with fatty acid-CoA and converts fatty acid-CoA ester to enoyl-CoA, which is a metabolite formed as part of the β-oxidation. In one example, the term "enzyme involved in the β-oxidation pathway", as used herein, comprises any polypeptide from the group comprising acyl-CoA dehydrogenase, enoyl-CoA hydratase, 3-hydroxyacyl-CoA dehydrogenase and 3-keto-acyl-CoA thiolase. Subsequently, the acyl-CoA synthetase may catalyse the conversion a fatty acid to the CoA ester of a fatty acid, *i.e.* a molecule, wherein the functional group -OH of the carboxy group is replaced with -S-CoA, preferably for introducing said fatty acid into the β-oxidation pathway. For example, the polypeptides FadD and FadK in *E. coli* (Accession number: BAA15609.1) are acyl-CoA dehydrogenases. In one example, the term "acyl-CoA dehydrogenase", as used herein, is a polypeptide capable of catalysing the conversion of an acyl-CoA to enoyl-CoA, preferably as part of the β-oxidation pathway. For example, the polypeptide FadE in *E. coli* (Accession number: BAA77891.2) is an acyl-CoA dehydrogenase. In particular, the term "2,4-dienoyl-CoA reductase", as used herein, is a polypeptide capable of catalysing the conversion of the 2,4-dienoyl CoA from an unsaturated fatty acid into enoyl-CoA, more in particular as part of the β-oxidation pathway. For example, the polypeptide FadH in *E. coli* is a 2,4-dienoyl-CoA reductase. In one example, the term "enoyl-CoA hydratase", as used herein, also referred to as 3-hydroxyacyl-CoA dehydrogenase, refers to a polypeptide capable of catalysing the conversion of enoyl-CoA to 3-ketoacyl-CoA through hydration and oxidation, preferably as part of the β-oxidation pathway. For example, the polypeptides FadB and FadJ in *E. coli* (Accession number: BAE77457.1) are enoyl-CoA hydratases. In particular, the term "ketoacyl-CoA thiolase", as used herein, refers to a polypeptide capable of catalysing the conversion of cleaving 3-ketoacyl-CoA, resulting in an acyl-CoA shortened by two carbon atoms and acetyl-CoA, as the final step of the β-oxidation pathway. For example, the polypeptides FadA and Fadl in *E. coli* (Accession number: AP009048.1) are ketoacyl-CoA thiolases.

In one example, the cells according to any aspect of the present invention may be genetically modified to result in an increased activity of at least one amino acid N-acyl transferase in combination with increased activity of at least one acyl-CoA synthetase in combination with an increased activity of at least one transporter protein of the FadL and/or the AlkL. In particular, the cell according to any aspect of the present invention may be genetically modified to overexpress glycine N-acyl transferase, acyl-CoA/ACP synthetase, a transporter protein of the FadL and the AlkL compared to the wild type cell. These cells may be capable of producing acyl glycinates. In another example, the cell according to any aspect of the present invention may be genetically modified compared to the wild type cell to:
- increase the expression of amino acid N-acyl transferase, acyl-CoA synthetase; and
- reduce activity of at least one enzyme selected from the group consisting of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase and electron-transfer flavoprotein.

In yet another example, the cells according to any aspect of the present invention may be genetically modified compared to the wild type cell to result in:
- increase the expression of amino acid N-acyl transferase, acyl-CoA synthetase and at least one transporter protein of the FadL and/or the AlkL; and
- reduce activity of at least one enzyme selected from the group consisting of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase and electron-transfer flavoprotein.

In particular, the acyl-CoA dehydrogenase FadE (EC 1.3.8.7, EC 1.3.8.8 or EC 1.3.8.9) may catalyse the reaction acyl-CoA + electron-transfer flavoprotein = trans-2,3-dehydroacyl-CoA + reduced electron-transfer flavoprotein; the multifunctional 3-hydroxybutyryl-CoA epimerase (EC 5.1.2.3), Δ3-cis-Δ2-trans-enoyl-CoA isomerase (EC 5.3.3.8), enoyl-CoA hydratase (EC 4.2.1.17) and 3-hydroxyacyl-CoA dehydrogenase (EC 1.1.1.35) FadB, may catalyse the reactions (S)-3-hydroxybutanoyl-CoA → (R)-3-hydroxybutanoyl-CoA, (3Z)-3-enoyl-CoA → (2E)-2-enoyl-CoA, (3S)-3-hydroxyacyl-CoA → trans-2-enoyl-CoA + H2O, (S)-3-hydroxyacyl-CoA + NAD+ = 3-oxoacyl-CoA + NADH + H+; 3-ketoacyl-CoA thiolase (EC 2.3.1.16), may catalyse the reaction acyl-CoA + acetyl-CoA → CoA + 3-oxoacyl-CoA; and electron-transfer flavoprotein (EC 1.5.5.1), may catalyse the following reaction: reduced electron-transferring flavoprotein + ubiquinone → electron-transferring flavoprotein + ubiquinol.

More in particular, the cell according to any aspect of the present invention may be genetically modified compared to the wild type cell to increase the expression of glycine N-acyl transferase, acyl-CoA/ACP synthetase, a transporter protein of the FadL and the AlkL; and reduce activity of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase and electron-transfer flavoprotein.

In one example, the cell according to any aspect of the present invention may be genetically modified compared to the wild type cell to:
- increase the expression of amino acid N-acyl transferase, acyl-CoA synthetase; and
- reduce activity of at least one enzyme selected from the group consisting of glycine cleavage system H protein, glycine cleavage system P protein, glycine cleavage system L protein, glycine cleavage system T protein, threonine aldolase, and serine hydroxylmethyltransferase.

In another example, the cell according to any aspect of the present invention may be genetically modified compared to the wild type cell to:
- increase the expression of amino acid N-acyl transferase, acyl-CoA synthetase and at least one transporter protein of the FadL and/or the AlkL; and
- reduce activity of at least one enzyme selected from the group consisting of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase, and electron-transfer flavoprotein.

In yet another example, the cell according to any aspect of the present invention may be genetically modified compared to the wild type cell to:
- increase the expression of amino acid N-acyl transferase, acyl-CoA synthetase and at least one transporter protein of the FadL and/or the AlkL;
- reduce activity of at least one enzyme selected from the group consisting of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase, and electron-transfer flavoprotein; and
- reduce activity of at least one enzyme selected from the group consisting of glycine cleavage system H protein, glycine cleavage system P protein, glycine cleavage system L protein, glycine cleavage system T protein, threonine aldolase, and serine hydroxylmethyltransferase.

In particular, the cell according to any aspect of the present invention may be genetically modified compared to the wild type cell to increase the expression of glycine N-acyl transferase, acyl-CoA/ACP synthetase, a transporter protein of the FadL and the AlkL; reduce activity of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase, electron-transfer flavoprotein; and reduce activity of glycine cleavage system H protein, glycine cleavage system P protein, glycine cleavage system L protein, glycine cleavage system T protein, threonine aldolase, and serine hydroxylmethyltransferase.

More in particular, the glycine cleavage system H protein, carrying lipoic acid and interacts with the glycine cleavage system proteins P, L and T; the glycine cleavage system P protein (EC 1.4.4.2), catalyses the reaction glycine + [glycine-cleavage complex H protein]-N(6)-lipoyl-L-lysine → [glycine-cleavage complex H protein]-S-aminomethyl-N(6)-dihydrolipoyl-L-lysine + CO2; glycine cleavage system L protein (EC 1.8.1.4), catalyses the reaction protein N6-(dihydrolipoyl)lysine + NAD+ → protein N6-(lipoyl)lysine + NADH + H+; glycine cleavage system T protein (EC 2.1.2.10), catalyses the reaction [protein]-S8-aminomethyldihydrolipoyllysine + tetrahydrofolate → [protein]-dihydrolipoyllysine + 5,10-methylenetetrahydrofolate + NH3; threonine aldolase (EC 4.2.1.48), catalyses the reaction L-threonine → glycine + acetaldehyde; serine hydroxylmethyltransferase (EC 2.1.2.1), catalyses the reaction 5,10-methylenetetrahydrofolate + glycine + H₂O + tetrahydrofolate + L-serine.

The fatty acids that are to be converted to acyl amino acids may be produced by the cell according to any aspect of the present invention. In one example, the very cell that produces the acyl amino acids may be capable of producing the fatty acids from which the acyl amino acids are produced. In particular, the cells may be genetically modified to be able to produce fatty acids. In one example, the genetic modification may be to decrease a specific enzymatic activity and this may be done by a gene disruption or a genetic modification. The genetic modification may also increase a specific enzymatic activity. In particular, the genetic modification may increase microbial synthesis of a selected fatty acid or fatty acid derived chemical product above a rate of a control or wild type cell. This control or wild type cell may lack this genetic modification to produce a selected chemical product. Cells which may be able to produce fatty acids and/or proteinogenic amino acids are at least described in US20140051136. The fatty acid may be an unsaturated fatty acid and may be selected from the group consisting of myristoleic acid, lauroleic acid, palmitoleic acid and cis-vaccenic acid. In another example, the fatty acid may be a saturated fatty acid and may be selected from the group consisting of laurate, myristate and palmitate. The fatty acid according to any aspect of the present invention may be provided in the form of an organic phase comprising a liquid organic solvent and the fatty acid, wherein the organic solvent may be an ester of the fatty acid.

In one example, the cell according to any aspect of the present invention may be genetically modified to increase the expression of amino acid N-acyl transferase, acyl-CoA synthetase, and comprise a genetic modification in the cell capable of producing at least one fatty acid from at least one carbohydrate. A list of non-limiting genetic modification to enzymes or enzymatic activities is provided below in Table 2. The cells according to any aspect of the present invention may comprise a combination of genetic modification that produce fatty acids and convert the fatty acids to N-acyl amino acids. In particular, the cell according to any aspect of the present invention may be genetically modified to increase the expression of amino acid N-acyl transferase, acyl-CoA synthetase and comprise any of the genetic modifications listed in Table 2. More in particular, the cell may be genetically modified to increase the expression of N-acyl transferase, acyl-CoA synthetase, a transporter protein of the FadL and the AlkL and comprise any of the genetic modifications listed in Table 2. Even more in particular, the cell may be genetically modified to increase the expression of N-acyl transferase, acyl-CoA synthetase, a transporter protein of the FadL and the AlkL, reduce activity of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase, electron-transfer flavoprotein; reduce activity of glycine cleavage system H protein, glycine cleavage system P protein, glycine cleavage system L protein, glycine cleavage system T protein, threonine aldolase, serine hydroxylmethyltransferase, and comprise any of the genetic modifications listed in Table 2.

In one example, the cell according to any aspect of the present invention may be genetically modified to increase the expression of amino acid N-acyl transferase, acyl-CoA/ACP synthetase and decrease the expression of at least one enzyme selected from the group consisting of β-ketoacyl-ACP synthase I, 3-oxoacyl-ACP-synthase I, Malonyl-CoA-ACP transacylase, enoyl ACP reductase, and β-ketoacyl-ACP synthase III. In particular, the genetic modification in the cell according to any aspect of the present invention may comprise an increase in the expression compared to the wild type cell of amino acid N-acyl transferase, acyl-CoA/ACP synthetase and a decrease in the expression of β-ketoacyl-ACP synthase I, 3-oxoacyl-ACP-synthase I, Malonyl-CoA-ACP transacylase, enoyl ACP reductase, and β-ketoacyl-ACP synthase III.

Accordingly, the cells and methods of the present invention may comprise providing a genetically modified microorganism that comprises both a production pathway to a fatty acid or fatty acid derived product, and a modified polynucleotide that encodes an enzyme of the malonyl-ACP dependent fatty acid synthase system that exhibits reduced activity, so that utilisation of malonyl-CoA shifts toward the production pathway compared with a comparable (control) microorganism lacking such modifications. The methods involve producing the chemical product using a population of such genetically modified microorganism in a vessel, provided with a nutrient media. Other genetic modifications described herein, to other enzymes, such as acetyl-CoA carboxylase and/or NADPH-dependent transhydrogenase, may be present in some such examples. Providing additional copies of polynucleotides that encode polypeptides exhibiting these enzymatic activities is shown to increase a fatty acid or fatty acid derived product production. Other ways to increase these respective enzymatic activities is known in the art and may be applied to various examples of the present invention.

Also, without being limiting, a first step in some multi-phase methods of making a fatty acid may be exemplified by providing into a vessel, such as a culture or bioreactor vessel, a nutrient media, such as a minimal media as known to those skilled in the art, and an inoculum of a genetically modified microorganism so as to provide a population of such microorganism, such as a bacterium, and more particularly a member of the family *Enterobacteriaceae,* such as *E. coli,* where the genetically modified microorganism comprises a metabolic pathway that converts malonyl-CoA to a fatty acid. This inoculum is cultured in the vessel so that the cell density increases to a cell density suitable for reaching a production level of a fatty acid or fatty acid derived product that meets overall productivity metrics taking into consideration the next step of the method. In various alternative embodiments, a population of these genetically modified microorganisms may be cultured to a first cell density in a first, preparatory vessel, and then transferred to the noted vessel so as to provide the selected cell density. Numerous multi-vessel culturing strategies are known to those skilled in the art. Any such methods provide the selected cell density according to any aspect of the present invention.

Without being limiting, a subsequent step may be exemplified by two approaches, which also may be practiced in combination in various embodiments. A first approach provides a genetic modification to the genetically modified microorganism such that its enoyl-ACP reductase enzymatic activity may be controlled. As one example, a genetic modification may be made to substitute a temperature-sensitive mutant enoyl-ACP reductase (e.g., fabl^{TS} in *E. coli*) for the native enoyl-ACP reductase. The former may exhibit reduced enzymatic activity at temperatures above 30 °C but normal enzymatic activity at 30 °C, so that elevating the culture temperature to, for example to 34 °C, 35 °C, 36 °C, 37 °C or even 42 °C, reduces enzymatic activity of enoyl-ACP reductase. In such case, more malonyl-CoA is converted to a fatty acid or fatty acid derived product or another chemical product than at 30 °C, where conversion of malonyl-CoA to fatty acids is not impeded by a less effective enoyl-ACP reductase.

Other genetic modifications that may be useful in the production of fatty acids and/or amino acids may be included in the cell according to any aspect of the present invention. For example, the ability to utilize sucrose may be provided, and this would expand the range of feed stocks that can be utilized to produce a fatty acid or fatty acid derived product or other chemical products. Common laboratory and industrial strains of *E. coli,* such as the strains described herein, are not capable of utilizing sucrose as the sole carbon source. Since sucrose, and sucrose-containing feed stocks such as molasses, are abundant and often used as feed stocks for the production by microbial fermentation, adding appropriate genetic modifications to permit uptake and use of sucrose may be practiced in strains having other features as provided herein. Various sucrose uptake and metabolism systems are known in the art (for example, U.S. Pat. No. 6,960,455).

Also, genetic modifications may be provided to add functionality for breakdown of more complex carbon sources, such as cellulosic biomass or products thereof, for uptake, and/or for utilization of such carbon sources. For example, numerous cellulases and cellulase-based cellulose degradation systems have been studied and characterized (Beguin, P 1994 and Ohima, K. et al., 1997).

In some examples, genetic modifications increase the pool and availability of the cofactor NADPH, and/or, consequently, the NADPH/NADP⁺ ratio may also be provided. For example, in *E. coli,* this may be done by increasing activity, such as by genetic modification, of one or more of the following genes: *pgi* (in a mutated form), *pntAB,* overexpressed, *gapA:gapN* substitution/replacement, and disrupting or modifying a soluble transhydrogenase such as *sthA,* and/or genetic modifications of one or more of *zwf, gnd,* and *edd.*

Any such genetic modifications may be provided to species not having such functionality, or having a less than desired level of such functionality. More generally, and depending on the particular metabolic pathways of a microorganism selected for genetic modification, any subgroup of genetic modifications may be made to decrease cellular production of fermentation product(s) selected from the group consisting of acetate, acetoin, acetone, acrylic, malate, fatty acid ethyl esters, isoprenoids, glycerol, ethylene glycol, ethylene, propylene, butylene, isobutylene, ethyl acetate, vinyl acetate, other acetates, 1,4-butanediol, 2,3-butanediol, butanol, isobutanol, sec-butanol, butyrate, isobutyrate, 2-OH-isobutryate, 3-OH-butyrate, ethanol, isopropanol, D-lactate, L-lactate, pyruvate, itaconate, levulinate, glucarate, glutarate, caprolactam, adipic acid, propanol, isopropanol, fusel alcohols, and 1,2-propanediol, 1,3-propanediol, formate, fumaric acid, propionic acid, succinic acid, valeric acid, and maleic acid. Gene deletions may be made as disclosed generally herein, and other approaches may also be used to achieve a desired decreased cellular production of selected fermentation products.

In particular, the cell according to any aspect of the present invention may comprise at least:
- a first genetic mutation that increases the expression relative to the wild type cell of an amino acid-N-acyl-transferase that may be the human amino acid-N-acyl-transferase;
- at least a second genetic mutation that increases the expression relative to the wild type cell of an acyl-CoA synthetase, and
- at least a third genetic mutation that decreases the expression relative to the wild type cell of at least one enzyme selected from the group consisting of an enzyme of the glycine cleavage system, glycine hydroxymethyltransferase (GlyA) and threonine aldolase (ItaE).

In particular, the amino acid-N-acyl-transferase has 50, 60, 70, 75, 80, 85, 90, 92, 94, 95, 97, 98, 99% sequence identity to SEQ ID NO:4 or SEQ ID NO:5. More in particular, the amino acid-N-acyl-transferase has 85% sequence identity to SEQ ID NO:4 or SEQ ID NO:5.

**Table 2. Examples of genetic modifications in cells of microorganisms for production of fatty acids**

| Genetic Modifications | | | |
|---|---|---|---|
| ENZYME FUNCTION | E.C. CLASSIFICATION No. | GENE NAME IN *E. COLI* | COMMENTS |
| Glucose transporter | N/A | galP | Increase function |
| Pyruvate dehydrogenase E1p | 1.2.4.1 | accE | Increase function |
| lipoate acetyltransferase/ dihydrolipoamide acetyltransferase | 2.3.1.12 | aceF | Increase function |
| Pyruvate dehydrogenase E3 (lipoamide dehydrogenase) | 1.8.1.4 | lpd | Increase function or alter such as by mutation to increase resistance to NADH inhibition. |
| Lactate dehydrogenase | 1.1.1.28 | IdhA | Decrease function, including by mutation |
| Pyrivate formate lyase (B "inactive") | 2.3.1. | pflB | Decrease function, including by mutation |
| Pyruvate oxidase | 1.2.2.2 | poxB | Decrease function, including by mutation |
| Phosphate acetyltransferase | 2.3.1.8 | Pta | Decrease function, including by mutation |
| acetate kinase | 2.7.2.15 2.7.2.1 | ackA | Decrease function, including by mutation |
| methylglyoxal synthase | 4.2.3.3 | mgsA | Decrease function, including by mutation |
| Heat stable, histidyl phosphorylatable protein (of PTS) | N/A | ptsH (HPr) | Decrease function, including by mutation |
| Phosphoryl transfer protein (of PTS) | N/A | ptsI | Decrease function, including by mutation |
| Polypeptide chain (of PTS) | N/A | Crr | Decrease function, including by mutation |
| 3-oxoacyl-ACP synthase I | 2.3.1.179 | fabF | Decrease function, including by mutation |
| 3-oxoacyl-ACP synthase II monomer | 2.3.1.41 | | |
| β-ketoacyl-ACP synthase I. 3-oxoacyl-ACP-synthase I | 2.3.1.41 2.3.1. | fabB | Decrease function, including by mutation |
| Malonyl-CoA-ACP transacylase | 2.3.1.39 | fabD | Decrease function, including by mutation |
| enoyl acyl carrier protein reductase | 1.3.1.9. 1.3.1.10 | fabI | Decrease function, including by mutation |
| β-ketoacyl-acyl carrier protein synthase III | 2.3.1.180 | fabH | Decrease function, including by mutation |
| Carboxyl transferase subunit α subunit | 6.4.1.2 | accA | Increase function |
| Biotin carboxyl carrier protein | 6.4.1.2 | accB | Increase function |
| Biotin carboxylase subunit | 6.3.4.14 | accC | Increase function |
| Carboxyl transferase subunit β subunit | 6.4.1.2 | accD | Increase function |
| long chain fatty acyl thioesterase I | 3.1.2.2. 3.1.1.5 | tesA | Increase function as well as alter by mutation to express in cytoplasm or deletion |
| acyl-CoA synthase | 2.3.1.86 | fadD | Decrease via deletion or mutation |
| acetate CoA-transferase | 2.8.3.8 | atoD | Decrease via deletion or mutation |
| acetate CoA-transferase | 2.8.3.8 | atoA | Decrease via deletion or mutation |
| Transporter | N/A | atoE | Decrease via deletion or mutation |
| acetyl-CoA acetyltransferase | 2.3.1.9 | atoB | Decrease via deletion or mutation |
| pantothenate kinase | 2.7.1.33 | coaA | Increase via expression or feedback resistant mutation |
| lactose repressor | N/A | lacI | Decrease via deletion or mutation |
| γ-glutamyl-γ-aminobutyraldehyde dehydrogenase | 1.2.1. | puuC | Decrease via deletion or mutation |
| malate synthase A | 2.3.3.9 | aceB | Decrease via deletion or mutation |
| isocitratc lyase | 4.1.3.1 | aceA | Decrease via deletion or mutation |
| isocitrate dehydrogenase phosphatase/isocitrate dehydrogenase kinase | 3.1.3.- 2.7.11.5. | accK | Decrease via deletion or mutation |
| pyruvate formate-lyase deactivase | 1.2.1.10 1.1.1.1 | adhE | Decrease via deletion or mutation |
| aldehyde dehydrogenase A, NAD-linked | 1.2.1.21 1.2.1.22 | aldA | Decrease via deletion or mutation |
| acetaldehyde dehydrogenase | 1.2.1.4 | aldB | Decrease via deletion or mutation |
| Lambda phage DE3 lysogen | N/A | λDE3 | Increase |
| T7 mRNA polymerase | N/A | T7pol | Increase |
| trigger factor | 5.2.1.8 | tig | Decrease via deletion or mutation |
| 3-ketoacyl-CoA thiolase | 2.3.1.16 | fadA | Increase |
| dodecenoyl-CoA δ-isomerase, | 5.3.3.8 1.1.1.35 | fadB | Increase |
| enoyl-CoA hydratase, 3-hydroxybutyryl-CoA epimerase, 3-hydroxyacyl-CoA dehydrogenase | 5.1.2.3 4.2.1.17 | | |
| Sucrose permease | N/A | cscB | Increase |
| Invertase | 3.2.1.26 | cscA | Increase |
| fructokinase | 2.7.1.4 | cscK | Increase |
| carbonic anhydrase | 4.2.1.1 | cynT | Increase |
| carbonic anhydrase | 4.2.1.1 | can | Increase |
| pyridine nucleotide transhydrogenase | 1.6.1.2 | pntAB | Increase |
| pyridine nucleotide transhydrogenase | 1.6.1.1 | udhA | Increase |
| acyl-CoA thioesterase | 3.1.2.20 3.1.2.2 | yciA | Increase and or decrease |
| thioesterase II | 3.1.2.20 3.1.2.2 | tesB | Increase and or decrease |
| thioesterase III | 3.1.2. | fadM | Increase and or decrease |
| hydroxyphenylacetyl-CoA thioesterase | N/A | paaI | Increase and or decrease |
| esterase/thioesterase | 3.1.2.28 | ybgC | Increase and or decrease |
| proofreading thioesterase in enterobactin biosynthesis | | entH | Increase and or decrease |
| acetoacetyl-CoA synthase | 2.3.1.194 | npth07 | Increase |
| 3-ketoacyl-CoA synthase/elongase | 2.3.1 | Elol | Increase |
| 3-ketoacyl-CoA synthase/elongase | 2.3.1 | Elo2 | Increase |
| 3-Hydroxybutyryl-CoA dehydrogenase | 1.1.1.157 | hbd | Increase |
| 3-oxoacyl-CoA reductase | 1.1.1.100 | fabG | Increase |
| enoyl-CoA hydratase | 4.2.1.17 | crt | Increase |
| enoyl-CoA hydratase | 4.2.1.17 | ech2 | Increase |
| Trans-2-enoyl-reductase | 1.3.1.9 | ter | Increase |
| thioesterase | 3.1.2.20 | paaI | Decrease |

| | | | |
|---|---|---|---|
| E.C. No. = "Enzyme Commission number" | | | |

The cell according to any aspect of the present invention may be further genetically modified to comprise a fourth genetic mutation to increase the expression of at least one transporter protein compared to the wild type cell. The transporter protein may be FadL and AlkL. In one example, only FadL or AlkL may be expressed/ overexpressed, relative to the wild type cell, in the cell according to any aspect of the present invention. In another example, both FadL and AlkL may be expressed/ overexpressed relative to the wild type cell in the cell according to any aspect of the present invention.

The term "cell", as used herein, refers to any permanently unicellular organism comprising bacteria archaea, fungi, algae and the like. In particular, the cell used according to any aspect of the present invention may be may a bacterial cell. More in particular, the cell may be selected from the group consisting of *Pseudomonas, Corynebacterium, Bacillus* and *Escherichia.* Even more in particular, the cell may be *Escherichia coli.* In one example, the cell may be a lower eukaryote, in particular a fungus from the group consisting of *Saccharomyces, Candida, Pichia, Schizosaccharomyces* and *Yarrowia,* in particular, *Saccharomyces cerevisiae.* The microorganism may be an isolated cell, in other words a pure culture of a single strain, or may comprise a mixture of at least two strains. Biotechnologically relevant cells are commercially available, for example from the American Type Culture Collection (ATCC) or the German Collection of Microorganisms and Cell Cultures (DSMZ). Particles for keeping and modifying cells are available from the prior art, for example Sambrook/Fritsch/Maniatis (1989), and the like. The phrase "in comparison to its wild type has an increased or decreased activity" means that the microorganism has been genetically modified so as to have this increased or decreased activity respectively. A skilled person would understand that either an over-expression of an enzyme in the cell or an expression of an exogenous enzyme may be applicable.

Any aspect of the present invention may be practiced using wild type cells or recombinant cells. In particular, at least one of the enzymes mentioned according to any aspect of the present invention, in particular at least one or all from the group consisting of amino acid N-acyl-transferase, acyl-CoA synthetase acyl-CoA thioesterase an enzyme selected from the group consisting of an enzyme of the glycine cleavage system, glycine hydroxymethyltransferase (GlyA) and threonine aldolase (ItaE) may be recombinant. The term "recombinant" as used herein, refers to a molecule or is encoded by such a molecule, for example a polypeptide or nucleic acid that, as such, does not occur naturally but is the result of genetic engineering or refers to a cell that comprises a recombinant molecule. For example, a nucleic acid molecule is recombinant if it comprises a promoter functionally linked to a sequence encoding a catalytically active polypeptide and the promoter has been engineered such that the catalytically active polypeptide is overexpressed relative to the level of the polypeptide in the corresponding wild type cell that comprises the original unaltered nucleic acid molecule.

Whether or not a nucleic acid molecule, polypeptide, more specifically an enzyme required according to any aspect of the present invention is recombinant or not may not necessarily affect the level of its expression. However according to any aspect of the present invention, one or more recombinant nucleic acid molecules, polypeptides or enzymes may be required to allow the enzyme to result in the desired increase or decrease in the enzyme expression. In one example, the term "overexpressed", as used herein, means that the respective polypeptide encoded or expressed is expressed at a level higher or at higher activity than would normally be found in the cell under identical conditions in the absence of genetic modifications carried out to increase the expression, for example in the respective wild type cell. The person skilled in the art is familiar with numerous ways to bring about overexpression. For example, the nucleic acid molecule to be overexpressed or encoding the polypeptide or enzyme to be overexpressed may be placed under the control of a strong inducible promoter such as the lac promoter. The state of the art describes standard plasmids that may be used for this purpose, for example the pET system of vectors exemplified by pET-3a (commercially available from Novagen). Whether or not a nucleic acid or polypeptide is overexpressed may be determined by way of quantitative PCR reaction in the case of a nucleic acid molecule, SDS polyacrylamide electrophoreses, Western blotting or comparative activity assays in the case of a polypeptide. Genetic modifications may be directed to transcriptional, translational, and/or post-translational modifications that result in a change of enzyme activity and/or selectivity under selected and/or identified culture conditions. Thus, in various examples of the present invention, to function more efficiently, a microorganism may comprise one or more gene deletions. Gene deletions may be accomplished by mutational gene deletion approaches, and/or starting with a mutant strain having reduced or no expression of one or more of these enzymes, and/or other methods known to those skilled in the art.

In one example, at least one or all from the group consisting of N-acyl-transferase, acyl-CoA synthetase, acyl-CoA thioesterase, an enzyme of the glycine cleavage system, glycine hydroxymethyltransferase (GlyA) and threonine aldolase (ItaE) may be an isolated enzyme. In any event, any enzyme according to any aspect of the present invention may be used in an active state and in the presence of all cofactors, substrates, auxiliary and/or activating polypeptides or factors essential for its activity. The term "isolated", as used herein, means that the enzyme of interest is enriched compared to the cell in which it occurs naturally. Whether or not an enzyme is enriched may be determined by SDS polyacrylamide electrophoresis and/or activity assays. For example, the enzyme of interest may constitute more than 5, 10, 20, 50, 75, 80, 85, 90, 95 or 99 percent of all the polypeptides present in the preparation as judged by visual inspection of a polyacrylamide gel following staining with Coomassie blue dye.

The cell according to any aspect of the present invention with increased expression of amino acid-N-acyl-transferase and an acyl-CoA synthetase and a decreased expression of at least one enzyme selected from the group consisting of an enzyme of the glycine cleavage system, glycine hydroxymethyltransferase (GlyA) and threonine aldolase (ItaE), optionally having a reduced fatty acid degradation capacity may be capable of making proteinogenic amino acids and/or fatty acids. In one example, the term "proteinogenic amino acid", as used herein, refers to an amino acid selected from the group comprising alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine and valine. Proteinogenic amino acids and fatty acids are synthesized as part of the primary metabolism of many wild type cells in reactions and using enzymes that have been described in detail in biochemistry textbooks, for example Jeremy M Berg, 2002.

In one example, the cell according to any aspect of the present invention expresses an acyl-CoA thioesterase. In particular, the term "acyl-CoA thioesterase", as used herein, refers to an enzyme capable of hydrolyzing acyl-CoA. In one example, the acyl-CoA thioesterase comprises a sequence from the group consisting of SEQ ID NO:1, AEM72521.1 and AAC49180.1 or a variant thereof. In particular, the acyl-CoA thioesterase comprises SEQ ID NO:1. The activity of acyl-CoA thioesterase may be assayed using various assays described in the state of the art. Briefly, the reaction of Ellman's reagent, which reacts with free thiol groups associated with CoASH formed upon hydrolysis of acyl-CoA may be detected by spectophotometrically monitoring absorbance at 412 nm.

According to another aspect of the present invention, there is provided a method for producing acyl amino acids, comprising contacting an amino acid and a fatty acid and/or an acyl CoA thereof in the presence of at least one cell according to any aspect of the present invention. The amino acid-N-acyl-transferase, may be isolated and/or recombinant wherein the amino acid-N-acyl-transferase may be a human amino acid-N-acyl-transferase, in particular of SEQ ID NO:4, SEQ ID NO:5 or a variant thereof, wherein the acyl-CoA synthetase may be in particular SEQ ID NO:6 or a variant thereof, the GcvT, GcvP and GcvH may be SEQ ID NO:58, SEQ ID NO:60 and SEQ ID NO:59 respectively, the GlyA may be SEQ ID NO:61 and the ItaE may be SEQ ID NO:62 or culturing the cell according to any aspect of the present invention.

The method according to any aspect of the present invention my further comprise additional steps of
- converting a fatty acid to an acyl CoA using an acyl-CoA synthetase, which may be isolated and/or recombinant; and/or
- hydrogenating.

All the enzymes according to any aspect of the present invention, in particular selected from the group consisting of amino acid-N-acyl-transferase, acyl-CoA synthetase, GcvT, GcvP, GcvH, GlyA and ItaE may be provided in the form of a cell expressing said enzyme or enzymes.

The term "contacting", as used herein, means bringing about direct contact between the amino acid, the acyl CoA and the cell according to any aspect of the present invention. In one example, the amino acid, the acyl CoA and the cell according to any aspect of the present invention are brought together in an aqueous solution. For example, the cell, the amino acid and the acyl CoA may not be in different compartments separated by a barrier such as an inorganic membrane. If the amino acid or fatty acid is soluble and may be taken up by the cell or can diffuse across biological membranes, it may simply be added to the cell according to any aspect of the present invention in an aqueous solution. In case it is insufficiently soluble, it may be dissolved in a suitable organic solvent prior to addition to the aqueous solution. The person skilled in the art is able to prepare aqueous solutions of amino acids or fatty acids having insufficient solubility by adding suitable organic and/or polar solvents. Such solvents may be provided in the form of an organic phase comprising liquid organic solvent. In one example, the organic solvent or phase may be considered liquid when liquid at 25 °C and standard atmospheric pressure. In another example, the fatty acid may be provided in the form of a fatty acid ester such as the respective methyl or ethyl ester. For example, the fatty acid laurate may be dissolved in lauric acid methyl ester as described in EP11191520.3. According to any aspect of the present invention, the compounds and catalysts may be contacted *in vitro,* i.e. in a more or less enriched or even purified state, or may be contacted *in situ,* i.e. they are made as part of the metabolism of the cell and subsequently react inside the cell.

The term "an aqueous solution" comprises any solution comprising water, in particular mainly water as solvent that may be used to keep the cell according to any aspect of the present invention, at least temporarily, in a metabolically active and/or viable state and comprises, if such is necessary, any additional substrates. The person skilled in the art is familiar with the preparation of numerous aqueous solutions, usually referred to as media that may be used to keep the cells according to any aspect of the present invention, for example LB medium in the case of *E. coli.* It is advantageous to use as an aqueous solution a minimal medium, *i.e.* a medium of reasonably simple composition that comprises only the minimal set of salts and nutrients indispensable for keeping the cell in a metabolically active and/or viable state, by contrast to complex mediums, to avoid dispensable contamination of the products with unwanted side products. For example, M9 medium may be used as a minimal medium.

Advantageously, the cell and method according to any aspect of the present invention may use not only saturated fatty acids, but also unsaturated fatty acids may be converted to acyl amino acids. In case the end product sought-after is to comprise a higher yield of saturated acyl residues than is present at the beginning of the method according to any aspect of the present invention, it may be possible to complement the process by hydrogenating the acyl residues of the acyl amino acids. The hydrogenation may be carried out according to various state of the art processes, for example those described in US5734070. Briefly, the compound to be hydrogenated may be incubated at 100 °C in the presence of hydrogen and a suitable catalyst, for example a nickel catalyst on silicon oxide as a support.

According to another aspect of the present invention, there is provided a reaction mixture comprising
an amino acid-N-acyl-transferase, which may be isolated and/or recombinant,
an acyl-CoA synthetase, which may be isolated and/or recombinant,
an amino acid and/or
either an acyl CoA or a fatty acid and an acyl-CoA-synthetase,
wherein the amino acid-N-acyl-transferase may be a human amino acid-N-acyl-transferase, in particular of SEQ ID NO: 4, SEQ ID NO: 5 or a variant thereof,
wherein the acyl-CoA synthetase may be SEQ ID NO: 6 or a variant thereof,
wherein the GcvT, GcvP and GcvH may be SEQ ID NO:58, SEQ ID NO:60 and SEQ ID NO:59 or a variant thereof respectively,
wherein the GlyA may be SEQ ID NO:61; and
wherein the ItaE may be SEQ ID NO:62.

According to a further aspect of the present invention, there is provided a composition comprising
a first acyl amino acid consisting of a saturated acyl having 8 to 16 carbon atoms and glycine,
a second acyl amino acid consisting of an unsaturated acyl having 10 to 18 carbon atoms and glycine,
and optionally a third acyl amino acid consisting of a saturated or unsaturated acyl having 12 carbon atoms and an amino acid selected from the group consisting of glutamine, glutamic acid, alanine and asparagine.

The composition according to any aspect of the present invention may comprise a first acyl amino acid which comprises a saturated acyl having 12 carbon atoms, in particular lauryl, and glycine. The second acyl amino acid may comprise an unsaturated acyl having 12 or 14 carbon atoms and glycine. In particular, the acyl amino acids formed may be a mixture of amino acids. The mixture of amino acids may comprise at least two proteinogenic amino acids as defined below. In particular, the mixture of amino acids may have 3, 4, 5, 6, 7, 8, 9, 10, 11, 12, 13, 14, 15, 16, 17, 18, 19, 20, or 21 proteinogenic amino acids. In one example, the mixture of amino acids may be used to form cocoyl glycine and salts thereof.

Advantageously, the composition of acyl amino acids produced according to any aspect of the present invention, more specifically the length of fatty acids incorporated into such acyl amino acids, may be controlled by introducing into the cell one or more specific acyl-CoA thioesterases or altering the expression of one or more acyl-CoA thioesterases endogenously expressed by the cell.

### BRIEF DESCRIPTION OF FIGURES

The inventions are further illustrated by the following figures and non-limiting examples from which further embodiments, aspects and advantages of the present invention may be taken.
Figure 1 is a graph depicting a total ion chromatogram of the 48 h sample from the *E. coli* W3110 ΔfadE pJ294{Ptac}[synUcTE] / pCDF{Ptac}[hGLYAT2(co_Ec)_fadD_Ec] fermentation.
Figure 2 is a graph depicting a total ion chromatogram of the 48 h sample from the *E. coli* W3110 ΔfadE pJ294{Ptac}[synUcTE]/ pCDF{Ptac}[hGLYAT3(co_Ec)_fadD_Ec] fermentation.
Figure 3 is a graph depicting a total ion chromatogram of the 48 h sample from the *E. coli* W3110 ΔfadE pJ294{Ptac}[synUcTE] / pCDFDuet-1 fermentation (negative control).
Figure 4 is a graph showing the production of lauroylglycinate by *E.coli* strains W3110 Δ*fadE* pCDF{Ptac}[hGLYAT2(co_Ec)-fadD_Ec]{Placuv5}[alkLmod1].
Figure 5 is a graph showing the production of lauroylglycinate by *E. coli* strain W3110 Δ*fadE* Δ*GcvTHP* pCDF{Ptac}[hGLYAT2(co_Ec)-fadD_Ec]{Placuv5}[alkLmod1].

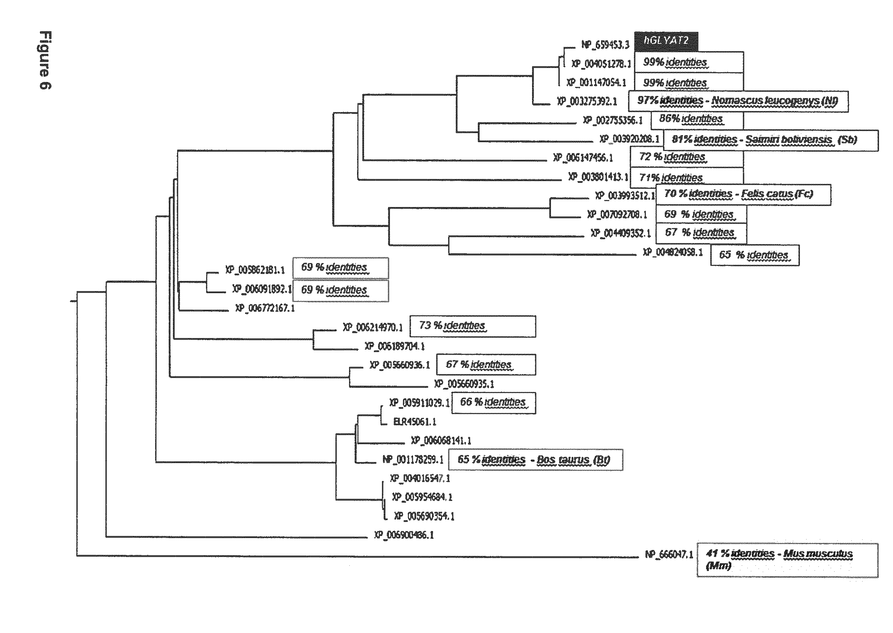

Figure 6 is a tree showing the percentage sequence identity of GLYAT2 in the various organisms tested in Example 18.
Figure 7 is an illustration showing the metabolic pathways of glycine.

### EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### Sequence ID NOs:

Throughout this application a range of SEQ ID NOs are used. These are shown in Table 3 below.

**Table 3. Sequences used in the Examples.**

| SEQ ID NO: | Comment |
|---|---|
| 1 | *Umbellularia californica synUcTE* (an acyl-CoA thioesterase) gene (codon-optimized) |
| 2 | tac promoter |
| 3 | Vector pJ294[Ptac-synUcTE], see Example 1 |
| 4 | *Homo sapiens* genes hGLYAT2 (an amino acid N-acyl transferase) |
| 5 | *Homo sapiens* genes hGLYAT3 (another amino acid N-acyl transferase) |
| 6 | *Escherichia coli fadD* (an acyl-CoA synthetase) |
| 7 | Vector pCDF[atfA1_Ab(co_Ec)-fadD_Ec], see Example 2 |
| 8 | Vector pCDF{Ptac}[hGLYAT2(co_Ec)-fadD_Ec], see Example 2 |
| 9 | Vector pCDF{Ptac}[hGLYAT3(co_Ec)-fadD_Ec], see Example 2 |
| 10 | alkL (an importer facilitating transport of hydrophobic acyl across cell membranes) gene, see Example 3 |
| 11 | lacuv5 promoter, see Example 3 |
| 12 | Vector pCDF[alkLmod1], see Example 3 |
| 13 | Vector pCDF{Ptac}[hGLYAT2(co_Ec)-fadD_Ec]{Placuv5}[alkLmod1], see Example 3 |
| 14 | Vector pET-28b, see Example 10 |
| 15 | Vector pET-28b{Ptac}[hGLYAT2(co_Ec)], see Example 10 |
| 16 | pET-28b{Ptac}[hGLYAT3(co_Ec)], see Example 10 |

### Example 1

### Generation of an expression vector for the Umbellularia californica gene synUcTE

To generate an expression vector for the *Umbellularia californica synUcTE* gene (SEQ ID NO:1), which encodes the *Umbellularia californica* acyl CoA-thioesterase, this gene was codon-optimized for expression in *Escherichia coli.* The gene was synthesized together with a *tac* promoter (SEQ ID NO:2), and, simultaneously, one cleavage site was introduced upstream of the promoter and one cleavage site downstream of the terminator. The synthesized DNA fragment P_{tac}-synUcTE was digested with the restriction endonucleases *BamH*I and *Not*I and ligated into the correspondingly cut vector pJ294 (DNA2.0 Inc., Menlo Park, CA, USA). The finished *E*. *coli* expression vector was referred to as pJ294[Ptac-synUcTE] (SEQ ID NO:3).

### Example 2

### Generation of vectors for coexpression of Escherichia coli fadD with either the Homo sapiens genes hGLYAT3 and hGLYAT2

To generate vectors for the coexpression of the *Homo sapiens* genes hGLYAT2 (SEQ ID NO:4) or hGYLAT3 (SEQ ID NO:5), which encodes human glycine-N-acyltransferase, with *Escherichia coli fadD* (SEQ ID NO:6), which encodes the *E*. *coli* acyl-CoA synthetase, the genes hGLYAT2 and hGLYAT3 were codon-optimized for expression in *Escherichia coli* and synthesized. The synthesized DNA fragments were digested with the restriction endonucleases *Sac*II and *Eco*47III and ligated into the correspondingly cut pCDF[atfA1_Ab(co_Ec)-fadD_Ec] (SEQ ID NO:7) with removal of the *aftA*1 gene. The sequence segments which were additionally removed in this process were cosynthesized during gene synthesis. The vector is a pCDF derivative which already comprises a synthetic tac promoter (SEQ ID NO:2) and the *Escherichia coli fadD* gene. The resulting expression vectors were named pCDF{Ptac}[hGLYAT2(co_Ec)-fadD_Ec] (SEQ ID NO:8) and pCDF{Ptac}[hGLYAT3(co_Ec)-fadD_Ec] (SEQ ID NO:9).

### Example 3

### Generation of vectors for the coexpression of the Homo sapiens hGLYAT2, Escherichia coli fadD and Pseudomonas putida alkL genes

To generate vectors for the coexpression of the hGLYAT2 genes with a modified *Pseudomonas putida* alkL gene, which encodes AlkL, an outer membrane protein that facilitates the import of hydrophobic substrates into a cell, the alkL gene (SEQ ID NO:10) was amplified together with the lacuv5 promoter (SEQ ID NO:11) from the plasmid pCDF[alkLmod1] (SEQ ID NO:12) by means of sequence-specific oligonucleotides. The PCR products were cleaved with the restriction endonucleases *Bam*HI and *Nsi*I and ligated into the correspondingly cleaved vector pCDF{Ptac}[hGLYAT2(co_Ec)-fadD_Ec] (SEQ ID NO:8). The correct insertion of the target genes was checked by restriction analysis and the authenticity of the introduced genes was verified by DNA sequencing. The resulting expression vector was named pCDF{Ptac}[hGLYAT2(co_Ec)-fadD_Ec]{Placuv5}[alkLmod1] (SEQ ID NO:13).

The following parameters were used for PCR: 1 x: initial denaturation, 98 °C, 3:00 min; 35 x denaturation, 98 °C, 0:10 min; annealing, 65 °C, 0:20 min; elongation, 72 °C, 0:17 min; 1 x: final elongation, 72 °C, 10 min. For amplification the Phusion™ High-Fidelity Master Mix from New England Biolabs (Frankfurt) was used according to manufacturer's manual. 50 µl of the PCR reaction were analyzed on a 1 % TAE agarose gel. Procedure of PCR, agarose gel electrophoresis, ethidium bromide staining of DNA and determination of PCR fragment size were carried out known to those skilled in the art.

### Example 4

### Generation of an E. coli strain with deletion in the fadE gene, which strain overexpresses the Umbellularia californica synUcTE, Escherichia coli fadD and Homo sapiens hGLYAT2 and hGLYAT3 genes

To generate *E. coli* strains which coexpress the *Umbellularia californica synUcTE* in combination with the *Escherichia coli fadD* and *Homo sapiens* hGLYAT2 or *Homo sapiens* hGLYAT3 genes, the strain *E.coli* W3110 ΔfadE was transformed with the plasmids pJ294{Ptac}[synUcTE] and pCDF{Ptac}[hGLYAT2(co_Ec)_fadD_Ec] or pCDF{Ptac}[hGLYAT3(co_Ec)_fadD_Ec] by means of electroporation and plated onto LB-agar plates supplemented with spectinomycin (100 µg/ml) and ampicillin (100 µg/ml). Transformants were checked for the presence of the correct plasmids by plasmid preparation and analytic restriction analysis. The strains *E.coli* W3110 ΔfadE pJ294{Ptac}[synUcTE] / pCDF{Ptac}[hGLYAT2(co_Ec)_fadD_Ec] and *E.coli* W3110 ΔfadE pJ294{Ptac}[synUcTE] / pCDF{Ptac}[hGLYAT3(co_Ec)_fadD_Ec] were generated thus.

### Example 5

### Generation of an E.coli strain with deletion in the fadE gene, which strain overexpresses the Escherichia coli fadD and either Homo sapiens hGLYAT2 or hGLYAT3 genes

To generate *E. coli* strains which overexpress the *Escherichia coli fadD* gene in combination with the *Homo sapiens* hGLYAT2 or hGLYAT3 genes, electrocompetent cells of *E. coli* strain W3110 ΔfadE were generated. *E.coli* W3110 ΔfadE was transformed with the plasmid pCDF{Ptac}[hGLYAT2(co_Ec)-fadD_Ec]{Placuv5}[alkLmod1] and plated onto LB-agar plates supplemented with spectinomycin (100 µg/ml). Transformants were checked for the presence of the correct plasmids by plasmid preparation and analytic restriction analysis. The strain generated thus was named *E. coli* W3110 ΔfadE pCDF{Ptac}[hGLYAT2(co_Ec)-fadD_Ec]{Placuv5}[alkLmod1].

### Example 6

### Production of fatty acid/amino acid adducts by E. coli strains with deletion in the fadE gene, which strains overexpress the synUcTE and fadD genes in combination with either hGLYAT2 or hGLYAT3

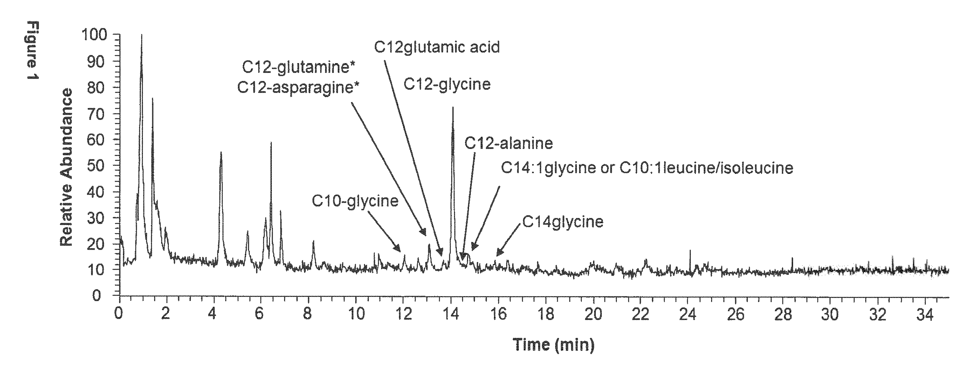

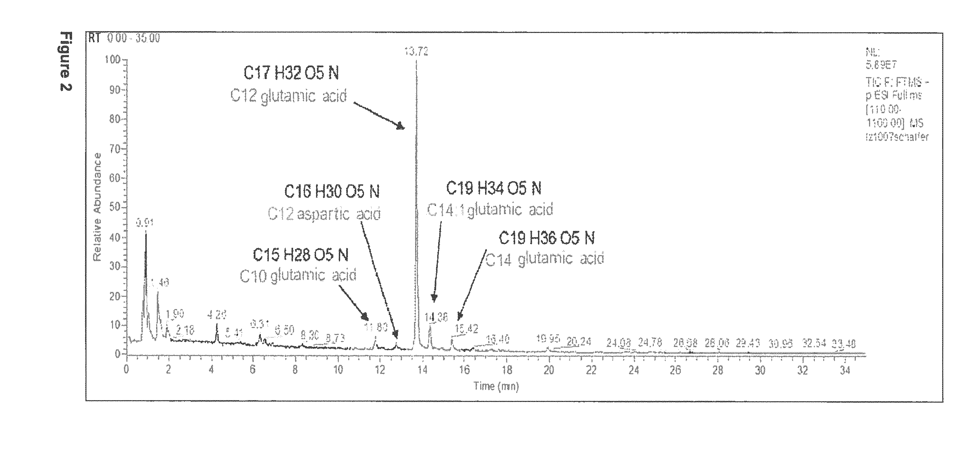

The strains generated in Example 4 were used to study their ability to produce fatty acid/amino acid adducts.Starting from a -80 °C glycerol culture, the strains to be studied were first plated onto an LB-agar plate supplemented with 100 µg/ml ampicillin and 100 µg/ml spectinomycin and incubated overnight at 37 °C. Starting from a single colony in each case, the strains were then grown as a 5-ml preculture in Luria-Bertani broth, Miller (Merck, Darmstadt) supplemented with 100 µg/ml ampicillin and 100 µg/ml spectinomycin. The further culture steps were performed in M9 medium. The medium, composed of 38 mM disodium hydrogenphosphate dihydrate, 22 mM potassium dihydrogenphosphate, 8.6 mM sodium chloride, 37 mM ammonium chloride, 2 % (w/v) glucose, 2 mM magnesium sulphate heptahydrate (all chemicals from Merck, Darmstadt) and 0.1 % (v/v) trace element solution, was brought to pH 7.4 with 25 % strength ammonium hydroxide solution. The trace element solution added, composed of 9.7 mM manganese(II) chloride tetrahydrate, 6.5 mM zinc sulphate heptahydrate, 2.5 mM sodium-EDTA (Titriplex III), 4.9 mM boric acid, 1 mM sodium molybdate dihydrate, 32 mM calcium chloride dihydrate, 64 mM iron(II) sulphate heptahydrate and 0.9 mM copper(II) chloride dihydrate, dissolved in 1 M hydrochloric acid (all chemicals from Merck, Darmstadt) was filter-sterilized before being added to the M9 medium. 20 ml of M9 medium supplemented with 100 µg/ml spectinomycin and 100 µg/ml ampicillin were introduced into baffled 100-ml Erlenmeyer flasks and inoculated with 0.5 ml preculture. The flasks were cultured at 37 °C and 200 rpm in a shaker-incubator. After a culture time of 8 hours, 50 ml of M9 medium supplemented with 100 µg/ml spectinomycin and 100 µg/ml ampicillin were introduced into a baffled 250-ml Erlenmeyer flask and inoculated with the 10-ml culture to achieve an optical density (600 nm) of 0.2. The flasks were cultured at 37 °C and 200 rpm in a shaker-incubator. When an optical density (600 nm) of 0.7 to 0.8 was reached, gene expression was induced by addition of 1 mM IPTG. The strains were cultured for a further 48 hours at 30 °C and 200 rpm. Simultaneously with the induction, 1 g/l glycine was added to some of the cultures. During culturing, samples were taken, and fatty acid/amino acid adducts present were analysed. The results are shown in Figures 1 and 2. It has been possible to demonstrate that both *E.coli* strain W3110 ΔfadE pJ294{Ptac}[synUcTE] / pCDF{Ptac}[hGLYAT2(co_Ec)_fadD_Ec] and *E.coli* strain W3110 *ΔfadE* pJ294{Ptac}[synUcTE] / pCDF{Ptac}[hGLYAT3(co_Ec)_fadD_Ec] are capable of forming various fatty acid/amino acid adducts, for example lauroyl-glutamic acid, from glucose. By contrast, no such adducts can be found in a cell that, as a negative control, lacks the plasmids (Figure 3). It appears that slight sequence variations, for example amino acid substitutions that distinguish hGLYAT2 from hGLYAT3, do not compromise the ability of the cell to make the fatty acid/acyl amino acid adducts as shown in Table 4.

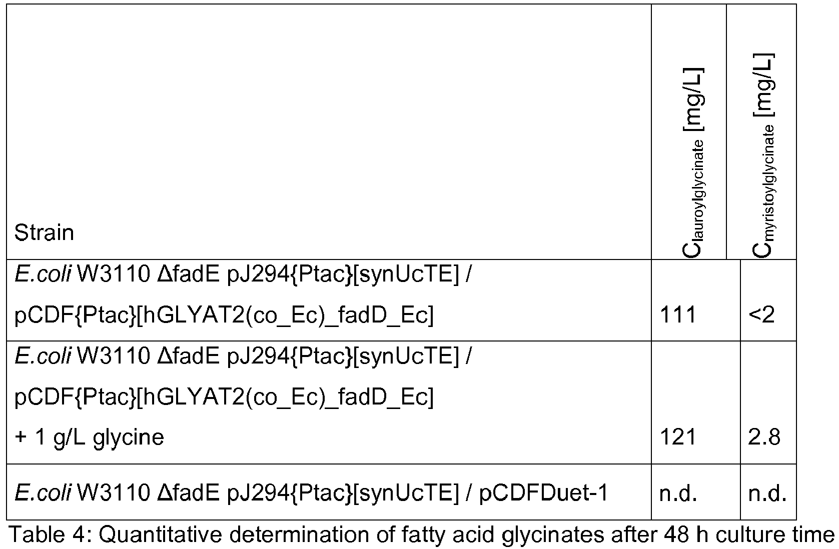

### Example 7

### Chromatographic quantification of products by HPLC/MS

The quantification of N-lauroylglycine, N-myristoylglycine and N-palmitoylglycine and the detection of other acylamino acids in fermentation samples was performed by HPLC-ESI/MS. The quantification was performed with the aid of an external calibration (approx. 0.1 - 50 mg/l) for the three target compounds in the "single ion monitoring" mode (SIM). In parallel, a scan was carried out over a mass range m/z = 100-1000 so as to identify further acylamino acids.

The samples for the determination of the fatty acid glycinates were prepared as follows: 800 µl of solvent (acetone) and 200 µl of sample were pipetted into a 2-ml reaction vessel. The mixture was shaken in a Retsch mill for 1 minute at 30 Hz and then centrifuged for 5 min at approximately 13 000 rpm. The clear supernatant was removed using a pipette and, after suitable dilution with diluent (80 % acetonitrile/20 % water + 0.1 % formic acid), analyzed. The calibration standards used were likewise dissolved and diluted in this diluent.

The following equipment was employed:
- Surveyor HPLC system (Thermo Scientific, Waltham, Massachusetts, USA) composed of MS pump, Autosampler Plus and PDA Detector Plus
- Mass spectrometer TSQ Vantage with HESI II source (Thermo Scientific, Waltham, Massachusetts, USA)
- HPLC column: 100 x 2 mm Pursuit XRS Ultra C8; 2.8 µm (Agilent, Santa Clara, California, USA)

Chemicals:
- Water from a Millipore system
- Acetonitrile for HPLC (Merck AG, Darmstadt, Germany)
- Formic acid, p.a. grade (Merck, Darmstadt, Germany)
- N-propanol Lichrosolv (Merck, Darmstadt, Germany)
- N-lauroylglycine 99 % (Chem-Impex International, Wood Dale, IL, USA)
- N-myristoylglycine > 98 % (Santa Cruz Biotechnology, Texas, USA)
- N-palmitoylglycine > 99 % (provenance unknown)

The HPLC separation was carried out using the abovementioned HPLC column. The injection volume amounted to 2 µl, the column temperature to 40 °C, the flow rate to 0.3 ml/min. The mobile phase consisted of Eluent A (0.1 % strength (v/v) aqueous formic acid) and Eluent B (75 % acetonitrile/25 % n-propanol (v/v) with 0.1 % (v/v) formic acid). The following gradient profile was used:

**Table 5. Gradient profile used in Example 7.**

| **Time** | **Eluent A** | **Eluent B** |
|---|---|---|
| **[min]** | **[%]** | **[%]** |
| 0 | 90 | 10 |
| 1 | 90 | 10 |
| 20 | 5 | 95 |
| 25 | 5 | 95 |

The HPLC/MS analysis was carried out under positive ionization mode with the following parameters of the ESI source:

| | |
|---|---|
| Spray Voltage: | 3500V |
| Vaporizer Temperature: | 50 °C |
| Sheath Gas Pressure: | 40 |
| Aux. Gas Pressure: | 10 |
| Capillary Temperature: | 250 °C |
| Sprayer Distance: | Ring C |

Detection and quantification of the three analytes were performed by "single ion monitoring" (SIM) with the following parameters shown in Table 6.

**Table 6. Parameters used in SIM of Example 7.**

| **Analyte** | **Ion [M+H] [m/z]** | **Scan range [m/z]** | **Scan time [ms]** | **Resolution Q3** |
|---|---|---|---|---|
| N-lauroylglycine | 258.2 | 0.002 | 50 | 0.7 |
| N-myristoylglycine | 286.2 | 0.002 | 50 | 0.7 |
| N-palmitoylglycine | 314.2 | 0.002 | 50 | 0.7 |

### Example 8

### Production of fatty acid amino acid adducts by E.coli strains with deletion in the fadE gene, which strains overexpress the synUcTE and fadD genes in combination with hGLYAT2 or hGLYAT3 in a parallel fermentation system

The strains generated in Example 4 were used for studying their ability to produce fatty acid amino acid adducts from glucose. For this purpose, the strain was cultured both in a shake flask and in a fed-batch fermentation. The fermentation was carried out in a parallel fermentation system from DASGIP with 8 bioreactors.

The production cells were prepared as described in Example 6.

The fermentation was performed using 1 I reactors equipped with overhead stirrers and impeller blades. pH and pO₂ were measured online for process monitoring. OTR/CTR measurements served for estimating the metabolic activity and cell fitness, inter alia.

The pH electrodes were calibrated by means of a two-point calibration using standard solutions of pH 4.0 and pH 7.0, as specified in DASGIP's technical instructions. The reactors were provided with the necessary sensors and connections as specified in the technical instructions, and the agitator shaft was fitted. The reactors were then charged with 300 ml of water and autoclaved for 20 min at 121 °C to ensure sterility. The pO₂ electrodes were connected to the measuring amplifiers and polarized overnight (for at least 6 h). Thereafter, the water was removed under a clean bench and replaced by M9 medium (pH 7.4) composed of KH₂PO₄ 3.0 g/l, Na₂HPO₄ 6.79 g/l, NaCl 0.5 g/l, NH₄Cl 2.0 g/l, 2 ml of a sterile 1 M MgSO₄*7H₂O solution and 1 ml/l of a filter-sterilized trace element stock solution (composed of HCl (37 %) 36.50 g/l, MnCl₂*4H₂O 1.91 g/l, ZnSO₄*7H₂O 1.87 g/l, ethylenediaminetetraacetic acid dihydrate 0.84 g/l, H₃BO₃0.30 g/l, Na₂MoO₄*2H₂O 0.25 g/l, CaCl₂*2H₂O 4.70 g/l, FeSO₄*7H₂O 17.80 g/l, CuCl₂*2H₂O 0.15 g/l) with 15 g/l glucose as the carbon source (added by metering in 30 ml/l of a sterile feed solution composed of 500 g/l glucose, 1.3 % (w/v) MgSO₄*7H₂O) supplemented with 100 mg/l spectinomycin and 3 ml/l DOW1500.

Thereafter, the pO₂ electrodes were calibrated to 100 % with a one-point calibration (stirrer: 400 rpm/aeration: 10 sl/h air), and the feed, correction agent and induction agent lines were cleaned by "cleaning in place" as specified in the technical instructions. To this end, the tubes were rinsed first with 70 % ethanol, then with 1 M NaOH, then with sterile fully-demineralized water and, finally, filled with the respective media.

Using the *E. coli* strain of Example 4, a dilution streak was first performed with a cryoculture on an LB agar plate supplemented with 100 mg/l spectinomycin, and the plate was incubated for approximately 16 h at 37 °C. LB medium (10 ml in a 100-ml baffle flask) supplemented with 100 mg/l spectinomycin was then inoculated with a single colony and the culture was grown overnight at 37 °C and 200 rpm for approximately 16 h. Thereafter, this culture was used for a second preculture stage with an initial OD of 0.2 in 50 ml of M9 medium, composed of KH₂PO₄ 3.0 g/l, Na₂HPO₄ 6.79 g/l, NaCl 0.5 g/l, NH₄Cl 2.0 g/l, 2 ml of a sterile 1 M MgSO₄*7H₂O solution and 1 ml/l of a filter-sterilized trace element stock solution (composed of HCl (37 %) 36.50 g/l, MnCl₂*4H₂O 1.91 g/l, ZnSO₄*7H₂O 1.87 g/l, ethylenediaminetetraacetic acid dihydrate 0.84 g/l, H₃BO₃0.30 g/l, Na₂MoO₄*2H₂O 0.25 g/l, CaCl₂*2H₂O 4.70 g/l, FeSO₄*7H₂O 17.80 g/l, CuCl₂*2H₂O 0.15 g/l) supplemented with 20 g/l glucose as carbon source (added by metering in 40 ml/l of a sterile feed solution composed of 500 g/l glucose) together with the above-described antibiotics was transferred into a 500-ml baffle flask and incubated for 8-12 h at 37 °C/200 rpm.

To inoculate the reactors with an optical density of 0.1, the OD₆₀₀ of the second preculture stage was measured and the amount of culture required for the inoculation was calculated. The required amount of culture was placed into the heated and aerated reactor with the aid of a 5-ml syringe through a septum.

The standard program shown in Table 7a-c was used:

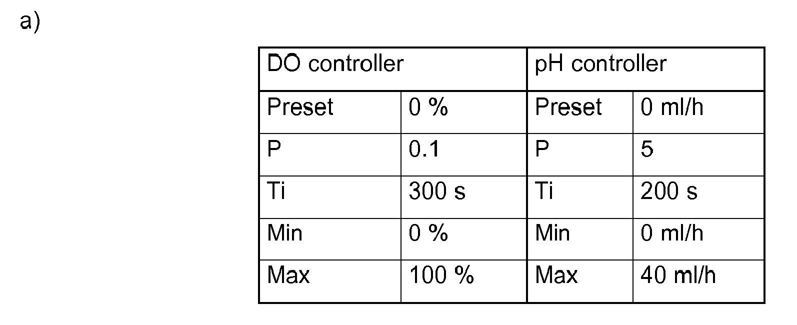

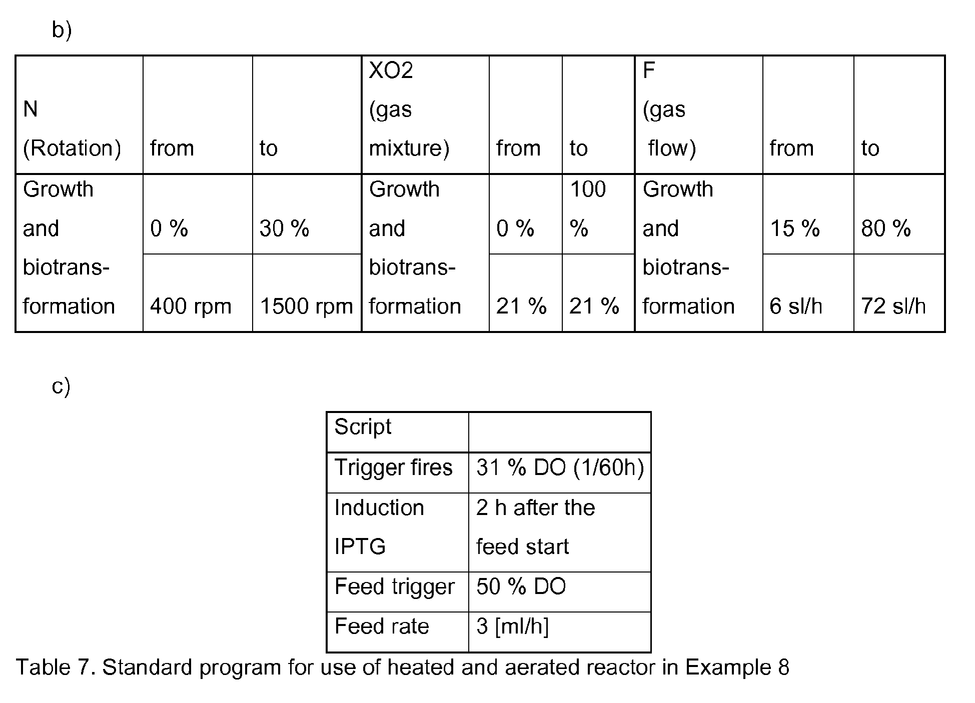

The pH was adjusted unilaterally to pH 7.0 with 12.5 % strength ammonia solution. During the growth phase and the biotransformation, the dissolved oxygen (pO₂ or DO) in the culture was adjusted to at least 30 % via the stirrer speed and the aeration rate. After the inoculation, the DO dropped from 100 % to these 30 %, where it was maintained stably for the remainder of the fermentation.

The fermentation was carried out as a fed batch, the feed start as the beginning of the feed phase with 5 g/l*h glucose feed, composed of 500 g/l glucose, 1.3 % (w/v) MgSO₄*7H₂O, being triggered via the DO peak which indicates the end of the batch phase. From the feed start onwards, the temperature was reduced from 37 °C to 30 °C. 2 h after the feed start, the expression was induced with 1 mM IPTG.

To quantify lauroyl, myristoyl and palmitoyl glycinate, samples were taken 47 h and 64 h after the start of the fermentation. These samples were prepared for analysis, and analyzed as described in Example 7.

It has been possible to demonstrate that strain *E. coli* W3110 ΔfadE pJ294{Ptac}[synUcTE] / pCDF{Ptac}[hGLYAT2(co_Ec)_fadD_Ec] is capable of forming lauroyl glycinate from glucose.

**Table 8: Quantification of fatty acid glycinates after 47 and 64 h fermentation time.**

| **Analyte** | **Ion [M+H] [m/z]** | **Scan range [m/z]** | **Scan time [ms]** | **Resolution Q3** |
|---|---|---|---|---|
| N-Lauroylglycine | 258.2 | 0.002 | 50 | 0.7 |
| N-Myristoylglycine | 286.2 | 0.002 | 50 | 0.7 |
| N-Palmitoylglycine | 314.2 | 0.002 | 50 | 0.7 |

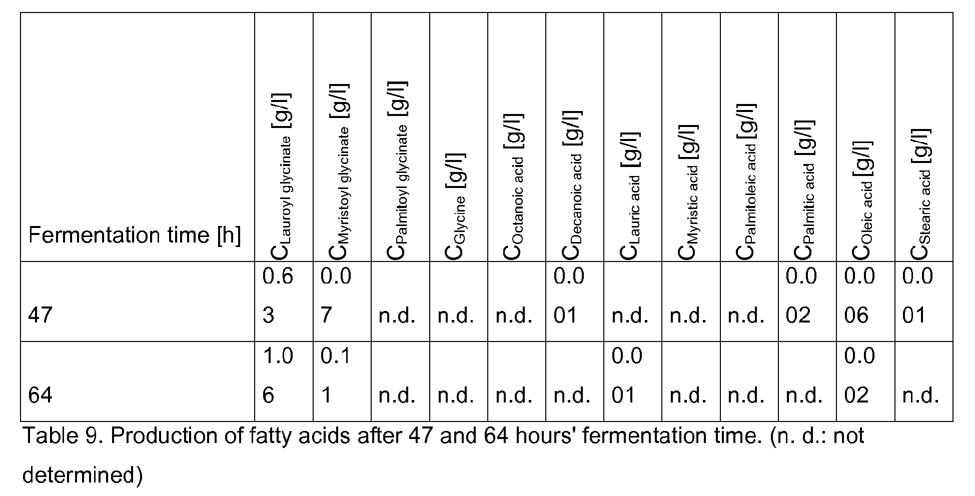

### Example 9

The strain of Example 5 was fermented in a fed-batch fermentation to study the ability of linking lauric acid and glycine to give lauroyl glycinate. This fermentation was carried out in a parallel fermentation system from DASGIP with 8 bioreactors.

The experimental setting was as described in Example 8 except that 100 g/l glycine in demineralized water and 100 g/l laurate in lauric acid methyl ester were fed rather than glucose.. To quantify lauroyl, myristoyl and palmitoyl glycinate in fermentation samples, samples were taken 23 h and 42 h after the start of the fermentation. These samples were prepared for analysis, and analyzed as described in Example 7. The results are shown in Tables 10 and 11.

It has been possible to demonstrate that the strain *E.coli* W3110 ΔfadE pCDF{Ptac}[hGLYAT2(co_Ec)_fadD_Ec] {Plavuv5} [alkLmod1] is capable of linking lauric acid and glycine and of producing lauroyl glycinate.

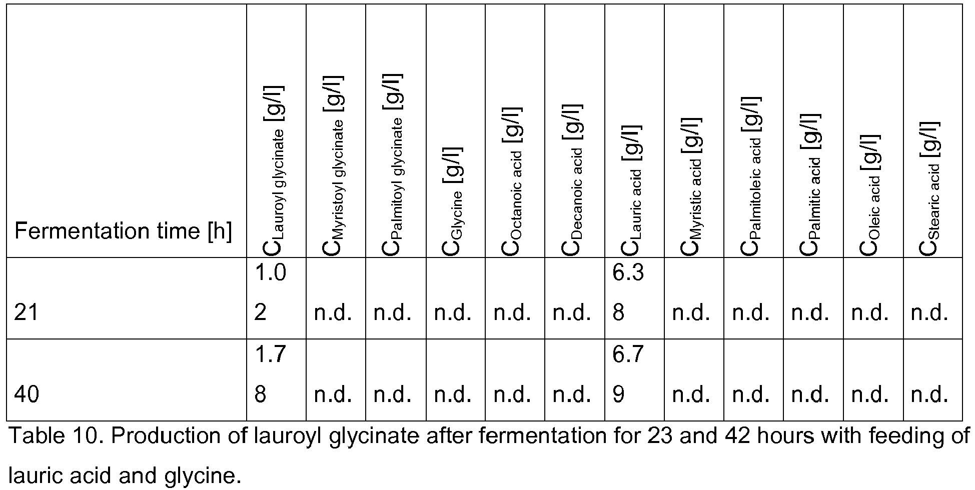

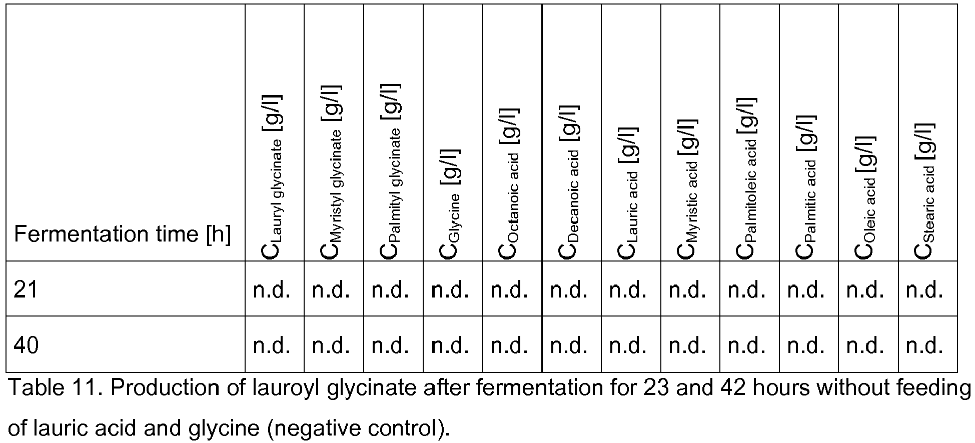

### Example 10

### Generation of vectors for expression of the Homo sapiens genes hGLYAT3 and hGLYAT2 in E.coli strains producing fatty acids via Malonyl-CoA and Acetyl-CoA

To generate vectors for the expression of the *Homo sapiens* genes hGLYAT2 (SEQ ID NO: 4) or hGYLAT3 (SEQ ID NO: 5) in the fatty acid producing strains listed in Table 3 below (from Table 3.2 of WO2014026162A1), the genes hGLYAT2 and hGYLAT3 were first amplified. The gene hGLYAT2 was amplified from the plasmid pCDF{Ptac}[hGLYAT2(co_Ec)-fadD_Ec]{Placuv5}[alkLmod1] (SEQ ID NO:13) and the hGYLAT3 gene was amplified from the plasmid pCDF{Ptac}[hGLYAT3(co_Ec)-fadD_Ec] (SEQ ID NO:9) by means of sequence-specific oligonucleotides. The PCR products were cleaved with the restriction endonucleases *NotI* and *SacI* and ligated in the correspondingly cleaved vector pET-28b (SEQ ID NO:14). The correct insertion of the target genes was checked by restriction analysis and the authenticity of the introduced genes was verified by DNA sequencing. The resulting expression vectors were named pET-28b{Ptac}[hGLYAT2(co_Ec)] (SEQ ID NO:15) and pET-28b{Ptac}[hGLYAT3(co_Ec)] (SEQ ID NO:16).

### Example 11

### Production of fatty acid amino acid adducts via Malonyl-CoA and Acetyl-CoA by strains overexpressing hGLYAT2 or hGLYAT3 in a shake flask experiment

The vectors produced according to Example 6 were then used to generate a microorganism strain from Table 12 below (OPX Biotechnologies Inc., USA) using any transformation method known in the art. In particular, the methods provided in section IV of WO2014026162A1 were used.

The strains generated were used for studying their ability to produce fatty acids, in particular amino acid adducts from glucose. For this purpose, the strains were transformed with the vectors pET-28b{Ptac}[hGLYAT2(co_Ec)] (SEQ ID NO:15) and pET-28b{Ptac}[hGLYAT3(co_Ec)] (SEQ ID NO:16) and cultured in shake flasks (Subsection C of section IV of WO2014026162A1). Strain BXF_031 (OPX Biotechnologies Inc., USA) harbouring the empty vector pET-28b was used as a control.

Triplicate evaluations were performed. Briefly, overnight starter cultures were made in 50 ml of Terrific Broth including the appropriate antibiotics and incubated 16-24 hours at 30 °C., while shaking at 225 rpm. These cultures were used to inoculate 150 ml cultures of each strain in SM11 minimal medium to an OD₆₀₀ of 0.8 and 5% TB culture carryover as starting inoculum, and antibiotics. 1 L SM11 medium consists of: 2 ml FM10 Trace Mineral Stock, 2.26 ml 1M MgSO₄, 30 g glucose, 200 mM MOPS (pH 7.4), 1 g/L yeast extract, 1.25 ml VM1 Vitamin Mix, 0.329 g K₂HPO₄, 0.173 g KH₂PO₄, 3 g (NH₄)₂SO₄, 0.15 g citric acid (anhydrous); FM10 Trace Mineral Stock consists of: 1 ml of concentrated HCl, 4.9 g CaCl₂*2H₂O,0.97 g FeCl₃*6H₂O, 0.04 g CoCl₂*6H₂O, 0.27 g CuCl₂*2H₂O, 0.02 g ZnCl₂, 0.024 g Na₂MoO₄*2H₂O, 0.007 g H₃BO₃, 0.036 g MnCl2*4H₂O, Q.S. with DI water to 100 ml; VM1 Vitamin Mix Solution consists of: 5 g Thiamine, 5.4 g Pantothenic acid, 6.0 g Niacin, 0.06 g, Q.S. with DI water to 1000 ml. All ingredients for the culture mediums used in this example are provided in (Subsection A of section IV of WO2014026162A1).

Cultures were incubated for 2 hours at 30 °C., while shaking at 225 rpm. After 2 hours, the cells were washed with SM11 (SM11 medium without phosphate). Cells were twice spun down (4,000 rpm, 15 min), the supernatant decanted, the pellet re-suspended in 150 ml of SM11 (SM11 medium without phosphate). The cultures were used to inoculate 3×50 ml of each strain in SM11 (no phosphate). The cultures were grown at 30° C. for approximately 2 h to an OD₆₀₀ of 1.0-1.5 after 2 h cells and shifted to 37° C. and samples removed periodically for product measurement over the course of 72 hrs.

The quantification of N-lauroylglycine, N-myristoylglycine and N-palmitoylglycine and the detection of other acylamino acids in fermentation samples was performed by HPLC-ESI/MS.

**Table 12. List of microorganism strains that were used to introduce the genes hGLYAT2 or hGLYAT3 in Examples 10 and 11. The method of production and the sequences of the strains are provided in Table 3.2 of WO2014026162A1 (OPX Biotechnologies Inc., USA).**

| **Strain designation** | **Host** | **Plasmid** | **SEQ ID NOs.** |
|---|---|---|---|
| BXF_0012 | BX_864 | 1)pBMT-3_ccdAB | 17 |
| BXF_0013 | BX_864 | 1)pBMT-3_ccdAB_P_{T7}-'tesA | 18 |
| BXF_0014 | BX_864 | 1)pBMT-3_ccdAB_P_{T7}-nphT7-hbd-crt-ter | 19 |
| BXF_0015 | BX_864 | 1)pBMT-3_ccdAB_P_{T7}-'tesA_PT7-nph_{T7}-hbd-crt-ter | 20 |
| BXF_0020 | BX_860 | 1)pBMT-3_ccdAB_PT7-'tesA_PT7-nphT7-hbd-crt-ter | 20 |
| BXF_0021 | BX_876 | 1)pBMT-3_ccdAB_P_{T7}-'tesA_PT7-nphT7-hbd-crt-ter | 20 |
| BXF_0022 | BX_874 | 1)pBMT-3_ccdAB | 17 |
| BXF_0023 | BX_874 | 1)pBMT-3_ccdAB_PT7-'tesA | 18 |
| BXF_0024 | BX_874 | 1)pBMT-3_ccdAB_PT7-'tesA_PT7-nphT7-hbd-crt-ter | 20 |
| BXF_0025 | BX_875 | 1)pBMT-3_ccdAB | 17 |
| BXF_0026 | BX_875 | 1)pBMT-3_ccdAB_PT7-'tesA | 18 |
| BXF_0027 | BX_875 | 1)pBMT-3_ccdAB_PT7-'tesA_PT7-nphT7-hbd-crt-ter | 20 |
| BXF_0028 | BX_878 | 1)pBMT-3ccdAB-T7-'tesA-PT7_nphT7_hbd_crt_ter | 20 |
| BXF_0028 | BX_878 | 1)pBMT-3_ccdAB_PT7-'tesA_PT7-nphT7-hbd-crt-ter | 20 |
| BXF_0029 | BX_879 | 1)pBMT-3_ccdAB_PT7-'tesA_PT7-nphT7-hbd-crt-ter | 20 |
| BXF_0030 | BX_881 | 1)pBMT-3_ccdAB_PT7-'tesA_PT7-nphT7-hbd-crt-ter | 20 |
| BXF_0031 | BX_864 | 1)pBMT-3_ccdAB_PT7-'tesA_FPT7-nphT7-hbd-crt-ter | 20 |
| | | 2)pET-28b(empty vector) | 21 |
| BXF_0033 | BX_878 | 1)pBMT-3_ccdAB_PT7-nphT7-hbd-crt-ter | 19 |
| BXF_0034 | BX_879 | 2)pBMT-3_ccdAB_PT7-nphT7-hbd-crt-ter | 19 |

### Example 12

### Generation of a vector for deletion of the GcvTHP operon in Escherichia coli W3110 ΔfadE

To generate a vector for the deletion of the *GcvTHP* operon of *E. coli* W3110, which encodes a glycine cleavage system (*GcvT:* aminomethyltransferase,tetrahydrofolate-dependent, subunit (T protein) of glycine cleavage complex; *GcvH:* glycine cleavage complex lipoylprotein; *GcvP:* glycine decarboxylase, PLP-dependent, subunit (protein P) of glycine cleavage complex), approx. 500 bp upstream and downstream of the *GcvTHP* operon were amplified via PCR. The upstream region of GcvTHP was amplified using the oligonucleotides o-MO-40 (SEQ ID NO:22) and o-MO-41 (SEQ ID NO:23) The downstream region of *GcvTHP* was amplified using the oligonucleotides o-MO-42 (SEQ ID NO:24) and o-MO-43 (SEQ ID NO:25). The PCR procedure is described above in Example 3.

In each case PCR fragments of the expected size could be amplified (PCR 1,553 bp, (SEQ ID NO:26); PCR 2,547 bp, SEQ ID NO:27). The PCR samples were separated via agarose gel electrophoresis and DNA fragments were isolated with *QiaQuick Gel extraction Kit* (Qiagen, Hilden). The purified PCR fragments were cloned into the vector pKO3 (SEQ ID NO:28), and cut with *Bam*HI using the Geneart® Seamless Cloning and Assembly Kit (Life Technologies, Carlsbad, CA, USA). The assembled product was transformed into chemically competent *E. coli* DH5α cells (New England Biolabs, Frankfurt). Procedure of PCR purification, in-vitro cloning and transformation were carried out according to manufacturer's manual. The correct insertion of the target genes was checked by restriction analysis and the authenticity of the introduced DNA fragments was verified by DNA sequencing. The resulting knock-out vector was named pKO3 delta GcvTHP (SEQ ID NO:29).

The construction of strain *E. coli* W3110 Δ*fadE* Δ*GcvTHP* was carried out with the help of pKO3 delta GcvTHP using the method described in Link et al., 1997. The DNA sequence after deletion of *GcvTHP* is SEQ ID NO:30. The *E*. *coli* strain W3110 Δ*fadE*Δ*GcvTHP* was transformed with the plasmid pCDF{Ptac}[hGLYAT2(co_Ec)-fadD_Ec]{Placuv5}[alkLmod1] (SEQ ID NO:13, Example 3) by means of electroporation and plated onto LB-agar plates supplemented with spectinomycin (100 µg/mL). Transformants were checked for the presence of the correct plasmids by plasmid preparation and analytic restriction analysis. The resulting strain was named *E. coli* W3110 Δ*fadE*Δ Δ*GcvTHP* pCOF{Ptac}[hGLYAT2(co_Ec)-fadD_Ec]{Placuv5}[alkLmod1].

### Example 13

### Production of lauroylglycinate by E. coli strains with deletion in the fadE or fadE / GcvTHP gene, overexpressing the hGLYAT2, fadD and alkL genes

The strain generated in Example 1 was used to study its ability to produce more lauroylglycinate, in comparison to the reference strain without *GcvTHP* deletion.

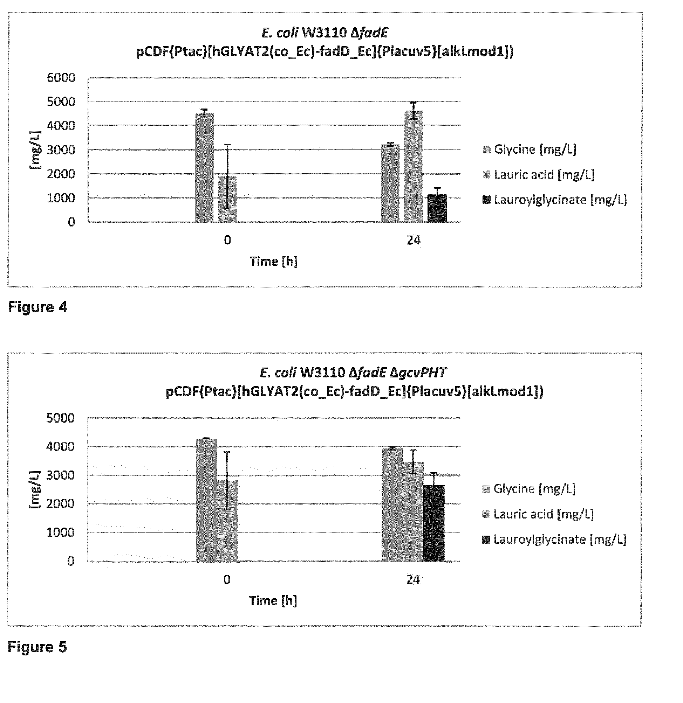

Starting from a -80 °C glycerol culture, the strains to be studied were first plated onto an LB-agar plate supplemented with 100 µg/mL spectinomycin and incubated overnight at 37 °C. Starting from a single colony in each case, the strains were then grown as a 5-mL preculture in LB-broth, Miller (Merck, Darmstadt) supplemented with 100 µg/mL spectinomycin. The further culture steps were performed in M9-FIT medium. The medium, composed of 38 mM disodium hydrogenphosphate dihydrate, 22 mM potassium dihydrogenphosphate, 8.6 mM sodium chloride, 37 mM ammonium chloride, 2 mM magnesium sulphate heptahydrate (all chemicals from Merck, Darmstadt), 5 % (w/v) maltodextrin solution (dextrose equivalent 13.0-17.0, Sigma Aldrich, Taufkirchen), 1 % (w/v) amyloglycosidase from *Aspergillus niger* (Sigma-Aldrich, Taufkirchen), 1 drop Delamex 180 (Bussetti & Co, Wien) and 0.1 % (v/v) trace element solution, was brought to pH 7.4 with 25 % strength ammonium hydroxide solution. The trace element solution added, composed of 9.7 mM manganese(II) chloride tetrahydrate, 6.5 mM zinc sulphate heptahydrate, 2.5 mM sodium-EDTA (Titriplex III), 4.9 mM boric acid, 1 mM sodium molybdate dihydrate, 32 mM calcium chloride dihydrate, 64 mM iron(II) sulphate heptahydrate and 0.9 mM copper(II) chloride dihydrate, dissolved in 1 M hydrochloric acid (all chemicals from Merck, Darmstadt) was filter-sterilized before being added to the M9 medium. 20 mL of M9 medium supplemented with 100 µg/mL spectinomycin were introduced into baffled 100-mL Erlenmeyer flasks and inoculated with 0.5 mL preculture. The flasks were cultured at 37 °C and 200 rpm in a shaker-incubator. After a culture time of 8 hours, 50 mL of M9 medium supplemented with 100 µg/mL spectinomycin and were introduced into a baffled 250-mL Erlenmeyer flask and inoculated with the 10-mL culture to achieve an optical density (600 nm) of 0.1. The flasks were cultured at 37 °C and 200 rpm in a shaker-incubator. When an optical density (600 nm) of 0.6 to 0.8 was reached, gene expression was induced by addition of 1 mM IPTG. The strains were cultured for a further 48 hours at 37 °C and 200 rpm. 1-3 h after the induction, 6 g/L glycine and 6 g/L lauric acid (dissolved in lauric acid methyl ester) were added to the cultures. After 0 h and 24 h cultivation samples were taken, and lauroylglycinate, lauric acid and glycine present were analysed. The results are shown in Figures 4 and 5. It has been possible to demonstrate that both *E. coli* strains W3110 Δ*fadE* pCDF{Ptac}[hGLYAT2(co_Ec)-fadD_Ec]{Placuv5}[alkLmod1] and *E*. *coli* strain W3110 Δ*fadE* Δ*GcvTHP* pCDF{Ptac}[hGLYAT2(co_Ec)-fadD_Ec]{Placuv5}[alkLmod1] are capable of forming lauroylglycinate. But in the new strain background Δ*fadE* Δ*GcvTHP* higher amounts of lauroylglycinate were synthesized and higher amounts of glycine are detected. It appears that less glycine is metabolized in the Δ*GcvTHP* background and can be used for lauroylglycinate synthesis.

### Example 14

### Generation of a vector for deletion of the GlyA gene in Escherichia coli W3110 ΔfadE

To generate a vector for the deletion of the *GlyA*-gene encoding a component of the Glycine hydroxymethyltransferase of *E*. *coli* W3110 approx. 500 bp upstream and downstream of the *GlyA*-gene were amplified via PCR. The upstream region of *GlyA* was amplified using the oligonucleotides o-MO-44 (SEQ ID NO:31) and o-MO-45 (SEQ ID NO:32). The downstream region of GlyA was amplified using the oligonucleotides o-MO-46 (SEQ ID NO:33) and o-MO-47 (SEQ ID NO:34).

In each case PCR fragments of the expected size could be amplified (PCR 1,546 bp, (SEQ ID NO:35); PCR 2,520 bp, SEQ ID NO:36). The PCR samples were separated via agarose gel electrophoresis and DNA fragments were isolated with *QiaQuick Gel extraction Kit* (Qiagen, Hilden). The purified PCR fragments were assembled via a crossover PCR. The generated fragment was purified and subcloned into the cloning vector pCR®-Blunt IITOPO (Life technologies) according to manufacturer's manual. To clone the fragment into the target plasmid pKO3 (SEQ ID NO:28) it was amplified with flanking *Bam*HI restriction sites, using the oligonucleotides o-MO-52 (SEQ ID NO:37) and o-MO-53 (SEQ ID NO:38). The purified, BamHI cleaved PCR 3 fragment (SEQ ID NO:39) was ligated into the correspondingly cleaved vector pKO3 (SEQ ID NO:28). The assembled product was transformed into chemically competent *E. coli* DH5α cells (New England Biolabs, Frankfurt). Procedure of PCR purification, in-vitro cloning and transformation were carried out according to manufacturer's manual. The correct insertion of the target genes was checked by restriction analysis and the authenticity of the introduced genes was verified by DNA sequencing. The resulting knock-out vector was named pKO3 delta GlyA (SEQ ID NO:40).

The construction of strain *E. coli* W3110 Δ*fadE* Δ*GlyA* was carried out with the help of pKO3 delta GlyA using the method described in Link et al., 1997. SEQ ID NO:41 is the DNA sequence after deletion of *GlyA.* The *E. coli* strain W3110 Δ*fadE* Δ*GcvTHP* was transformed with the plasmid pCDF{Ptac}[hGLYAT2(co_Ec)-fadD_Ec]{Placuv5}[alkLmod1] (SEQ ID NO:13 from Example 3), by means of electroporation and plated onto LB-agar plates supplemented with spectinomycin (100 µg/mL). Transformants were checked for the presence of the correct plasmids by plasmid preparation and analytic restriction analysis. The resulting strain was named *E. coli* W3110 Δ*fadE* ΔΔ*GlyA* pCDF{Ptac}[hGLYAT2(co_Ec)-fadD_Ec]{Placuv5}[alkLmod1].

### Example 15

### Generation of a vector for deletion of the ltaE gene in Escherichia coli W3110 ΔfadE

To generate a vector for the deletion of the *ltaE*-gene encoding the L-allo-threonine aldolase of *E. coli* W3110 approximately 500 bp upstream and downstream of the *ltaE* were amplified via PCR as described above. The upstream region of *ltaE* was amplified using the oligonucleotides ltaE-UP_fw (SEQ ID NO:42) and ltAE-UP-Xhol_rev (SEQ ID NO:43). The downstream region of *ltaE* was amplified using the oligonucleotides ltaE-DOWN_fw (SEQ ID NO:44) and ltaE-DOWN_rev (SEQ ID NO:45).

In each case PCR fragments of the expected size could be amplified (PCR 4,550 bp, (SEQ ID NO:46); PCR 5, 536 bp, SEQ ID NO:47). The PCR samples were separated via agarose gel electrophoresis and DNA fragments were isolated with *QiaQuick Gel extraction Kit* (Qiagen, Hilden). The purified PCR fragments were assembled via a crossover PCR. The generated fragment was purified and cloned into the cloning vector pCR®-Blunt IITOPO (Life technologies) according to manufacturer's manual. To clone the fragment into the target plasmid pKO3 (SEQ ID NO:28) it was amplified with flanking *Bam*HI restriction sites, using the oligonucleotides o-MO-54 (SEQ ID NO:48) and o-MO-55 (SEQ ID NO:49). The purified, *Bam*HI cleaved PCR 6 fragment (SEQ ID NO:50) was ligated into the correspondingly cleaved vector pKO3 (SEQ ID NO:28). The assembled product was transformed into chemically competent *E. coli* DH5α cells (New England Biolabs, Frankfurt). Procedure of PCR purification, in-vitro cloning and transformation were carried out according to manufacturer's manual. The correct insertion of the target genes was checked by restriction analysis and the authenticity of the introduced genes was verified by DNA sequencing. The resulting knock-out vector was named pKO3 delta ltaE (SEQ ID NO:51).

The construction of strain *E. coli* W3110 Δ*fadE* Δ*ltaE* was carried out with the help of pKO3 delta ltaE using the method described in Link et al., 1997. The DNA sequence after deletion of *ltaE is* described in SEQ ID NO:52). The *E*. *coli* strain W3110 Δ*fadE* Δ*ltaE* was transformed with the plasmid pCDF{Ptac}[hGLYAT2(co_Ec)-fadD_Ec]{Placuv5}[alkLmod1] (SEQ ID NO:13 from Example 3), by means of electroporation and plated onto LB-agar plates supplemented with spectinomycin (100 µg/mL). Transformants were checked for the presence of the correct plasmids by plasmid preparation and analytic restriction analysis. The resulting strain was named *E. coli* W3110 Δ*fadE*ΔΔ*ltaE* pCOF{Ptac}[hGLYAT2(co_Ec)-fadD_Ec]{Placuv5}[alkLmod1].

### Example 16

### LC-ESI/MS² -based quantification of lauric acid

Quantification of lauric acid in fermentation samples was carried out by means of LC-ESI/MS² on the basis of an external calibration for lauric acid (0.1 - 50 mg/L) and by using the internal standard d3-LS.

The following instruments were used:
- HPLC system 1260 (Agilent; Böblingen) with Autosampler (G1367E), binary pump (G1312B) and thermo-statted column (G1316A)
- Mass spectrometer TripelQuad 6410 (Agilent; Böblingen) with ESI source
- HPLC column: Kinetex C18, 100 x 2.1 mm, particle size: 2.6 µm, pore size 100 Å (Phenomenex; Aschaffenburg)
- Pre-column: KrudKatcher Ultra HPLC In-Line Filter; 0.5 µm filter depth and 0.004 mm inner diameter (Phenomenex; Aschaffenburg)
-

The samples were prepared by pipetting 1900 µL of solvent (80 % (v/v) ACN, 20% double-distilled H₂O (v/v), + 0.1 % formic acid) and 100 µL of sample into a 2 mL reaction vessel. The mixture was vortexed for approx. 10 seconds and then centrifuged at approx. 13 000 rpm for 5 min. The clear supernatant was removed using a pipette and analysed after appropriate dilution with a diluent (80 % (v/v) ACN, 20 % double-distilled H₂O (v/v), + 0.1 % formic acid). In each case, 100 µL of ISTD were added to 900 µl of sample (10 µl with a sample volume of 90 µl). HPLC separation was carried out using the above-mentioned column and pre-column. The injection volume is 1.0 µl, the column temperature 50 °C, the flow rate is 0.6 ml/min. the mobile phase consists of eluent A (0.1 % strength (v/v) aqueous formic acid) and eluent B (acetonitrile with 0.1 % (v/v) formic acid). The gradient shown it Table 4 was utilized:

**Table 13. Concentrations of Eluent A and B used in Example 12**

| **Time [min]** | **Eluent A [%]** | **Eluent B [%]** |
|---|---|---|
| 0 | 85 | 15 |
| 1 | 85 | 15 |
| 5 | 2 | 98 |
| 8 | 2 | 98 |
| 8.1 | 85 | 15 |
| 12 | 85 | 15 |

ESI-MS² analysis was carried out in positive mode with the following parameters of the ESI source:
- Gas temperature 320°C
- Gas flow 11 L/min
- Nebulizer pressure 50 psi
- Capillary voltage 4000 V

Detection and quantification of lauric acid was carried out with the following MRM parameters.

**Table 14. MRM parameters used in detection and quantification of lauric acid**

| **Analyte** | **Precursor ion [m/z]** | **Product ion [m/z]** | **Collision energy [eV]** |
|---|---|---|---|
| LS | 201.1 | 201.1 | 110 |
| d3-LS | 204.1 | 204.1 | 110 |

### Example 17

### Detection of glycine

Detection of glycine was performed via derivatization with ortho-phthaldialdehyde (OPA) and UV/VIS detection using an Agilent 1200 HPLC system.

200 µL of a homogeneous fermentation broth simple was mixed with 1800 µL of 30% (v/v) 1-propanol, vortexed for 10 s and subsequently centrifuged at 13,000 x g for 5 min. The supernatant was removed and used for HPLC analysis using the following parameters:

| | | | | |
|---|---|---|---|---|
| **Mobile phase:** | Eluent A | | | |
| | 2.5 mL acetic acid per 1 L distilled water, pH adjustment with NaOH @ 6.0 | | | |
| | Eluent B | | | |
| | Methanol | | | |
| **Column:** | Luna 5µ C8 100A (100 x 4.6 mm); Phenomenex | | | |
| **Column oven temperature:** | 40 °C | | | |
| **Flow:** | 1.0 mL/min | | | |

| **Gradient:** | **Time** | **%B** | **Flow** | **Max. Press.** |
|---|---|---|---|---|
| | 0.0 | 30.0 | 1.0 | 400 |
| | 1.0 | 30.0 | 1.0 | 400 |
| | 17.0 | 90.0 | 1.0 | 400 |
| | 19.5 | 90.0 | 1.0 | 400 |
| | 19.6 | 30.0 | 1.0 | 400 |
| | 20.5 | 30.0 | 1.0 | 400 |
| **Run time:** | 22 min | | | |
| **Detector:** | DAD | | | |
| | 334 nm | | | |
| | Spectrum | | | |
| | Store: all | | | |
| | Range: 200-400 nm | | | |
| | step 2 nm | | | |
| | FLD (excitation @ 330 nm; emission @ 450 nm, PMT gain 13) | | | |
| **Derivatization:** | automatically with injection program: | | | |

| | **Inject Programm** | | | |
|---|---|---|---|---|
| | # | Command | | |
| | 1 | DRAW 4.5 µL from Vial 1*, def. speed, def. offset | | |
| | 2 | DRAW 1.5 µL from sample, def. speed, def. offset | | |
| | 3 | DRAW 0.5 µL from air, def. speed | | |
| | 4 | NEEDLE wash in flush Port. 15.0 sec | | |
| | 5 | DRAW 4.5 µL from Vial 1, def. speed, def. offset | | |
| | 6 | MIX 11.0 µL in seat, def. speed, 1 times | | |
| | 7 | WAIT 1.00 min | | |
| | 8 | INJECT | | |
| | 9 | WAIT 0.50 min | | |
| | 10 | VALVE bypass | | |
| | 11 | Draw 100.0 µL from Vial 2*, def. speed, def. offset | | |
| | 12 | Eject 100.0 µL from Vial 2, def. speed, def. offset | | |
| | 13 | Valve mainpass | | |

| | | | | |
|---|---|---|---|---|
| *vial 1 contains OPA reagent (see below); vial 2 contains water | | | | |

### Preparation of OPA reagent

100 mg o-phthaldialdehyde was dissolved in 1 ml methanol and subsequently 0.4 mM borate buffer (pH 10.4) was added to give 10 mL. Subsequently, 50 µL mercaptoethanol was added and the reagent stored at 4 °C. Additional 10 µL mercaptoethanol was added before use.

### Preparation of borate buffer (0.4 mM H₃BO₄):

38.1 g Na₂B₄O₇ * 10 H₂O (0.1 mol) were dissolved in 1 L distilled water and the pH adjusted to 10.4 M NaOH auf 10,4 eingestellt. Subsequently, 1 mL 25% Brij35 (v/v) was added. Retention time: Glycine: 7.153 min

### Example 18

### Generation of vectors for the expression of hGLYAT2-homologs

To generate vectors for the expression of N-acyltransferases of different organisms, variants found in the NCBI databases with homology to HGLYAT2 were synthesized and codon-optimized for *E. coli.* These were glycine N-acyltransferase-like protein 2 isoform 1 of *Nomascus leucogenys* (NI, XP_003275392.1, SEQ ID NO:53), glycine N-acyltransferase-like protein 2 of *Saimiri boliviensis* (Sb, XP_003920208.1, SEQ ID NO:54), glycine-N-acyltransferase-like 2 of *Felis catus* (Fc, XP_003993512.1, SEQ ID NO:55), glycine N-acyltransferase-like protein 2 of *Bos taurus* (Bt, NP_001178259.1, SEQ ID NO:56), and glycine N-acyltransferase of *Mus musculus* (Mm, NP_666047.1, SEQ ID NO:57).

The hGLYAT2-gene of pCOF{Ptac}[hGLYAT2(co_Ec)-fadD_Ec]{Placuv5}[alkLmod1] (SEQ ID NO:13 of Example 3) was replaced by this variants as follows: The synthesized DNA fragments were digested with the restriction endonucleases *Bam*HI and *AsiS*I and ligated into the correspondingly cut pCDF{Ptac}[hGLYAT2(co_Ec)-fadD_Ec]{Placuv5}[alkLmod1].

The correct insertion of the target genes was checked by restriction analysis and the authenticity of the introduced genes was verified by DNA sequencing. The resulting expression vectors were named:
pCDF{Ptac}[GLYAT_NI(co_Ec)-fadD_Ec]{Placuv5}[alkLmod1]
pCDF{Ptac}[GLYAT_Sb(co_Ec)-fadD_Ec]{Placuv5}[alkLmod1]
pCDF{Ptac}[GLYAT_Fc(co_Ec)-fadD_Ec]{Placuv5}[alkLmod1]
pCDF{Ptac}[GLYAT_Bt(co_Ec)-fadD_Ec]{Placuv5}[alkLmod1]
pCDF{Ptac}[GLYAT_Mm(co_Ec)-fadD_Ec]{Placuv5}[alkLmod1]

### Example 19

### Production of lauroylglycinate by E. coli strains with deletion in the fadE gene, overexpressing the hGLYAT-variants, fadD and alkL genes

The strains generated in Example 18 were used to study their ability to produce lauroylglycinate, in comparison to the reference strain expressing hGLYAT2 harbouring the plasmid pCDF{Ptac}[hGLYAT2(co_Ec)-fadD_Ec]{Placuv5}[alkLmod1], applying the protocol described in Example 13.

1-3 h after the induction, 6 g/L glycine and 6 g/L lauric acid (dissolved in lauric acid methyl ester) were added to the cultures. After cultivation time of 48 h the entire broth of a shake flask was extracted with Acetone (ratio 1:2). Further sample treatment is described in Example 7. Samples were taken, and lauroylglycinate, lauric acid and glycine present were analysed. The results are shown in Table 15.

All strains except the none-plasmid control produced lauroylglycinate in amounts between 0.44 and 2109.8 mg/L.

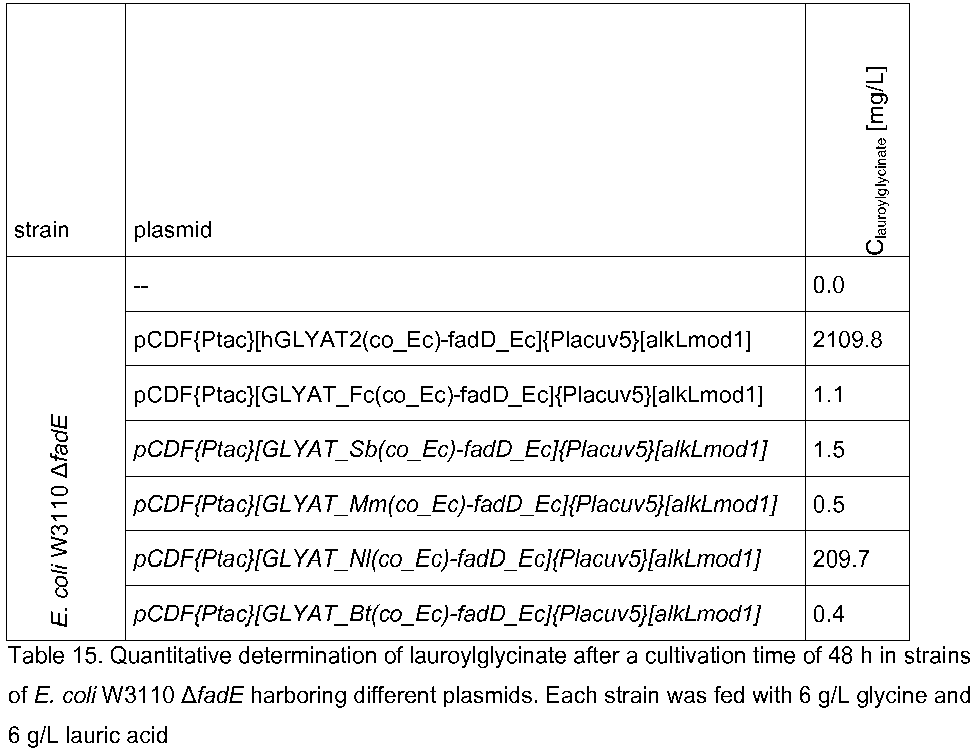

### Example 20

### Construction of mutants defect in different glycine-metabolizing pathways

With the Knock out-plasmid described in examples 12, 14 and 15, different mutants were constructed. The construction of each strain was performed with the help of the plasmids pKO3 delta ltaE (SEQ ID NO:51), pKO3 delta (SEQ ID NO: 40) and pKO3 delta GcvTHP (SEQ ID NO:29) using strain *E. coli* W3110 Δ*fadE* with the method described in Link et al., 1997. The *E. coli* strains W3110 Δ*fadE* Δ*GcvTHP* Δ*ltaE,* W3110 Δ*fadE* Δ*GcvTHP* Δ*GlyA,* W3110 Δ*fadE* Δ*GlyA* Δ*ltaE and* W3110 Δ*fadE* Δ*GcvTHP* Δ*ltaE* Δ*GlyA* were each transformed with the plasmid pCDF{Ptac}[hGLYAT2(co_Ec)-fadD_Ec]{Placuv5}[alkLmod1] (SEQ ID NO:13 from Example 3), by means of electroporation and plated onto LB-agar plates supplemented with spectinomycin (100 µg/mL). Transformants were checked for the presence of the correct plasmids by plasmid preparation and analytic restriction analysis. The resulting strains were named *E. coli* W3110 Δ*fadE* Δ*GcvTHP* Δ*ltaE* pCDF{Ptac}[hGLYAT2(co_Ec)-fadD_Ec]{Placuv5}[alkLmod1], *E*. *coli* W3110 Δ*fadE* Δ*GcvTHP* Δ*GlyA* pCDF{Ptac}[hGLYAT2(co_Ec)-fadD_Ec]{Placuv5}[alkLmod1], *E. coli W3110* Δ*fadE* Δ*GlyA* Δ*ltaE* pCDF{Ptac}[hGLYAT2(co_Ec)-fadD_Ec]{Placuv5}[alkLmod1] *and* W3110 Δ*fadE* Δ*GcvTHP* Δ*ltaE* Δ*GlyA* pCDF{Ptac}[hGLYAT2(co_Ec)-fad D_Ec]{Placuv5}[alkLmod1].

### REFERENCES

Waluk, D. P., Schultz, N., and Hunt, M. C. (2010), Identification of glycine N-acyltransferase-like 2 (GLYATL2) as a transferase that produces N-acyl glycines in humans, FASEB J. 24, 2795-2803.
Kang, Y., Zarzycki-Siek, J., Walton, C. B., Norris, M. H., and Hoang, T. T. (2010), Multiple FadD Acyl-CoA Synthetases Contribute to Differential Fatty Acid Degradation and Virulence in Pseudomonas aeruginosa, PLOS ONE 5 (10), e13557.
Arthur Lesk (2008), Introduction to bioinformatics, 3rd edition.
Thompson et al., (1994) Nucleic Acids Research 22, 4637-4680.
Katoh et al., Genome Information, 16(1), 22-33, 2005.
Fujita, Y., Matsuoka, H., and Hirooka, K. (2007) Mol. Microbiology 66(4), 829-839.
Cornish-Bowden (1995), Fundamentals of Enzyme Kinetics, Portland Press Limited, 1995.
Antonenkov, V., D.. Van Veldhoven, P., P., Waelkens, E., and Mannaerts, G.P. (1997) Sambrook/Fritsch/Maniatis (1989): Molecular cloning - A Laboratory Manual, Cold Spring Harbour Press, 2nd edition,
Allgemeine Mikrobiologie, 2008, Georg Thieme Verlag.
Jeremy M Berg, John L Tymoczko, and Lubert Stryer, Biochemistry, 5th edition, W. H. Freeman, 2002.
EP11191520.3, US5734070, US20140051136, GB1483500. U.S. Pat. No. 6,960,455
Link AJ, Phillips D, Church GM. J.Bacteriol. 1997. 179(20)
F. M. Ausubel (1995), Current Protocols in Molecular Biology. John Wiley & Sons, Inc.
"The DIG System Users Guide for Filter Hybridization", Boehringer Mannheim GmbH, Mannheim, Germany, 1993
Liebl et al. (1991) International Journal of Systematic Bacteriology 41: 255-260.Beguin, P and Aubert, J-P (1994) FEMS Microbial. Rev. 13: 25-58.
Ohima, K. et al. (1997) Biotechnol. Genet. Eng. Rev. 14: 365414.

## Claims

1. A cell for producing acyl amino acids wherein the cell is genetically modified to comprise
- at least a first genetic mutation that increases the expression relative to the wild type cell of an amino acid-N-acyl-transferase,
- at least a second genetic mutation that increases the expression relative to the wild type cell of an acyl-CoA synthetase, and
- at least a third genetic mutation that decreases the expression relative to the wild type cell of at least one enzyme selected from the group consisting of an enzyme of the glycine cleavage system, glycine hydroxymethyltransferase (GlyA) and threonine aldolase (ItaE).

2. The cell according to claim 1, wherein the enzyme from the glycine cleavage system is selected from the group consisting of glycine cleavage system T protein, the glycine cleavage system H protein, and the glycine cleavage system P protein.

3. The cell according to either claim 1 or 2, wherein the third genetic mutation decreases the expression relative to the wild type cell of the glycine hydroxymethyltransferase (GlyA), the threonine aldolase (ItaE), the glycine cleavage system T protein, the glycine cleavage system H protein and the glycine cleavage system P protein.

4. The cell according to claim 3, wherein the glycine cleavage system T protein has 85% sequence identity to SEQ ID NO:58, the glycine cleavage system H protein has 85% sequence identity to SEQ ID NO:59, and the glycine cleavage system P protein has 85% sequence identity to SEQ ID NO:60.

5. The cell according to any of the preceding claims, wherein the glycine hydroxymethyltransferase (GlyA) has 85% sequence identity to SEQ ID NO:61 and the threonine aldolase (ItaE) has 85% sequence identity to SEQ ID NO:62.

6. The cell according to any of the preceding claims, wherein the cell has a reduced fatty acid degradation capacity relative to the wild type cell.

7. The cell according to claim 6, wherein the reduced fatty acid degradation capacity is a result of a decrease in expression relative to the wild type cell of at least one enzyme selected from the group consisting of acyl-CoA dehydrogenase, 2,4-dienoyl-CoA reductase, enoyl-CoA hydratase and 3-ketoacyl-CoA thiolase.

8. The cell according to any of the preceding claims, wherein the amino acid-N-acyltransferase is a human amino acid-N-acyl-transferase.

9. The cell according to claim 8, wherein the amino acid-N-acyl-transferase has 85% sequence identity to SEQ ID NO:4 or SEQ ID NO:5.

10. The cell according to any one of the preceding claims, wherein the cell is a bacterial cell.

11. The cell according to claim 10, wherein the cell is *E. coli.*

12. The cell according to any of the preceding claims, wherein the cell is further genetically modified to comprise a fourth genetic mutation to increase the expression of at least one transporter protein compared to the wild type cell, wherein the transporter protein is selected from the group consisting of FadL and AlkL.

13. The cell according to any of the preceding claims, wherein the acyl amino acid is lauroylglycinate.

14. The cell according to any of the preceding claims, wherein the cell is capable of making proteinogenic amino acids and/or fatty acids.

15. A method for producing acyl amino acids, comprising contacting an amino acid and a fatty acid and/or an acyl CoA thereof in the presence of at least one cell according to any one of claims 1 to 14.

16. The method according to claim 15, comprising a further step of hydrogenating.
